# EUROPEAN PATENT APPLICATION

(11) **EP 4 610 356 A1**
(43) Date of publication of application: **03.09.2025**
(21) Application number: 23881768.8
(22) Date of filing: 23.10.2023
(51) Int. Cl.: C12N 15/113, A61K 31/713, A61P 1/16, A61P 3/06, A61P 9/10

(54) **RNA INHIBITOR FOR INHIBITING LPA GENE EXPRESSION AND USE THEREOF**

(30) Priority: 24.10.2022 CN 202211304230
(71) Applicant: Kylonova (Xiamen) Biopharma Co., Ltd., Xiamen, Fujian 361022 (CN)
(72) Inventor: CUI, Kunyuan, Xiamen, Fujian 361022 (CN); LU, Xueqin, Xiamen, Fujian 361022 (CN)
(74) Representative: Ter Meer Steinmeister & Partner
(86) International application number: PCT/CN2023/125861
(87) International publication number: WO 2024/088190

(57) **Abstract**

Provided is an RNA inhibitor for inhibiting LPA gene expression or a pharmaceutically acceptable salt thereof. The RNA inhibitor is formed by base pairing of a sense strand and an antisense strand having a chain length of 15-30, and the chain length is preferably 19-23; at least 85% of bases of the sense strand and the antisense strand are complementary to each other; -OH at position 2' of some or all of nucleotide sugar groups of the sense strand and/or the antisense strand is substituted with fluorine or methoxy; and phosphate bonds among three adjacent nucleotides of at least one of terminuses of the sense strand and/or antisense strand can be thioated. The structure of the RNA inhibitor may also contain ligand structures 5'MVIP and 3'MVIP. The RNA inhibitor provided in the present application interferes with the translation template function of the LPA mRNA, continuously and efficiently inhibits LPA gene expression, and can be used for treating and/or preventing diseases related to the horizontal increase of LP(a).

## Description

### Cross-reference of the relevant application

The present application claims the priority of the Chinese patent application 202211304230.8 with the name of "RNA inhibitor for inhibiting LPA gene expression and uses thereof" filed on October 24, 2022, the entire content of which are incorporated in the present application by reference.

### Technical field

The present application relates to the biomedical field, specifically involving an RNA inhibitor that inhibits LPA gene expression and its application.

### Background Art

### RNAi

RNAi (RNA interference) was discovered by Andrew Z. Fire et al. during antisense RNA inhibition experiments in Caenorhabditis elegans in 1998, and this process was called RNA interference. This discovery was rated as one of the top ten scientific advances in 2001 by Science magazine, and ranked first among the top ten scientific advances in 2002. Since then, siRNA with RNA interference as its mechanism of action has received widespread attention as a potential gene therapy drug. In 2006, Andrew Z. Fire and Craig C. Mello won the Nobel Prize in physiology or medicine for their contributions in the study of RNA interference mechanism. RNAi is triggered by double stranded RNA (dsRNA) in many organisms, including animals, plants and fungi. In the process of RNA inhibition, an endonuclease called "Dicer" cleaves or "dices" long strand of dsRNA into small segments of 21-25 nucleotides long. These small segments are known as small interfering RNAs (siRNAs), whose antisense strands are loaded onto Argonaute protein (AGO2). AGO2 loading occurs in the RISC-loading complex, a ternary complex consisting of Argonaute protein, Dicer, and dsRNA binding protein (TRBP for short). During loading, the sense strand is cleaved by AGO2 and discharged. Then, AGO2 uses the antisense strand to bind to mRNA containing fully complementary sequences, and then catalyzes the cleavage of these mRNA, resulting in mRNA cleavage to loss the role as a translation template, thereby preventing the synthesis of related proteins. After cleavage, the cleaved mRNA is released, and the RISC-loading complex loaded with the antisense strand is recycled for another round of cleavage.

According to statistics, among the disease-related proteins in the human body, about more than 80% of them cannot be targeted by current conventional small molecule drugs and biological macromolecule preparations, and are considered undruggable proteins. Gene therapy, which aims to treat diseases through gene expression, silencing and other functions, is considered by the industry to be the third generation of therapeutic drugs after chemical small molecule drugs and biological macromolecular drugs. This therapy treats diseases at the genetic level and is not restricted by undruggable proteins. As the most mainstream type of gene therapy, RNAi technology treats diseases at the mRNA level and has higher efficiency as compared with chemical small molecule drugs and biological macromolecular drugs at the protein level. RNAi technology can be used to design the sense strand and antisense strand sequences of siRNAs with high specificity and good inhibitory effect according to the specific gene sequence. These single strand sequences are synthesized through solid-phase synthesis, and then the sense strand and antisense strand are paired into siRNA in a specific annealing buffer according to the principle of base pairing. Finally, it is delivered to the corresponding target site in the body through the vector system, degrading the target mRNA, and destroying the function of the target mRNA as a translation template, thus preventing the synthesis of related proteins.

### Delivery system of siRNA

siRNA is unstable in blood and tissues and easily degraded by nucleases. In order to improve the stability of siRNA, the sense strand and/or antisense strand of siRNA can be modified, but these chemical modifications only provide limited protection from nuclease degradation and may ultimately affect the activity of siRNA. Therefore, a corresponding delivery system is needed to ensure that siRNA can safely and efficiently pass through cell membrane. Due to its large molecular weight, large amount of negative charge, and high water solubility, siRNA itself cannot smoothly pass through cell membrane to enter the cell.

Liposome is basically composed of a hydrophilic core and a phospholipid bilayer. It has a phospholipid bilayer similar to a biological membrane and has high biocompatibility, therefore liposome once became the most popular and widely used siRNA vector. Liposome-mediated siRNA delivery mainly encapsulates siRNA into liposomes to protect siRNA from degradation by nucleases, improves the efficiency of siRNA passing through cell membrane barriers, and thereby promotes cellular absorption. Examples of liposomes include, for example, anionic liposomes, pH sensitive liposomes, immunoliposomes, fusogenic liposomes, and cationic lipids. Despite some progress, liposomes themselves are prone to trigger inflammatory reactions, a variety of antihistamines and hormones such as cetirizine and dexamethasone must be used before administration to reduce possible acute inflammatory reactions. Therefore, they are not suitable for all treatment fields in actual clinical application, especially in the treatment of some chronic disease. Possible accumulated toxicity resulting from long-term use is a potential safety hazard. Therefore, a safer and more effective vector system is needed to deliver siRNA.

Asialoglycoprotein receptor (ASGPR) in the liver is a receptor specifically expressed on hepatocytes and is a highly efficient endocytic receptor. Due to the fact that under physiological conditions in the body, after acid and enzymatic hydrolysis of sialic acids, the exposed secondary ends of various glycoproteins are galactose residues, the sugar specifically bound to ASGPR is galactosyl, so ASGPR is also called galactose-specific receptor. Monosaccharide and polysaccharide molecules such as galactose, galactosamine, and N-acetylgalactosamine all have high affinity for ASGPR. ASGPR mainly has the physiological function of mediating the clearance of asialoglycoprotein, lipoprotein and other substances in the blood, and it is closely related to the occurrence and development of liver diseases such as viral hepatitis, liver cirrhosis, liver cancer and so on. The discovery of this characteristic of ASGPR plays an important role in the diagnosis and treatment of hepatogenic diseases (Ashwell G and Harford J, Carbohydrate specific Receptors of the Liver, Ann Rev Biochem 1982 51:531-554). The therapeutic drug for hepatogenic diseases containing galactose or galactosamine and their derivatives in the structure can have specific affinity with ASGPR, so it actively targets liver and does not need other vector systems to deliver.

### LPA, apo(a) and Lp(a)

LPA is the name of the gene encoding apolipoprotein (a) (apo (a)), which is mainly expressed in the liver, and its expression is restricted to humans and non-primates. Hydrophilic apolipoprotein fraction, apolipoprotein (a) is attached to apo(B)-100 via a disulfide bond and combined with the lipid core to form lipoprotein (a) (Lp (a)) particles. Lp(a) particles are special cholesterol-rich macromolecular lipoproteins with surface wrapped by cholesterol and phospholipids and embedded with the aforesaid apolipoprotein (a) and apo(B)-100. Lp (a) can enter and deposit on the blood vessel wall, promoting atherosclerosis. Lp (a) is structurally homologous to plasminogen (PLG) and can compete with plasminogens for binding to the fibrin site, thus inhibiting fibrinogen hydrolysis and promoting thrombosis. Therefore, LP (a) is closely associated with atherosclerosis and thrombosis. Studies have shown that blood Lp(a) level is an independent risk factor for cardiovascular disease, stroke, and atherosclerotic stenosis.

High Lp (a) levels are mainly related to genetics and will not change significantly with diet, exercise and other lifestyle changes. Lp (a) levels greater than 300 mg per liter in humans are considered high Lp (a). High Lp (a) levels often indicate a significantly increased risk of atherosclerosis and thrombosis. Detection of Lp (a) is relatively important for early identification of the risk of atherosclerosis. In China, there are about 330 million people suffering from cardiovascular diseases, but the awareness, treatment and control rates of dyslipidemia among the general public is generally at a low level, and the awareness of the risk of Lp(a) is even lower. Most hospitals do not include it in their routine lipid examination, and there is no targeted therapeutic drug in clinical practice at home and abroad. Therefore, there is an urgent need for a drug that effectively inhibits LPA gene expression.

### Content of the invention

In one aspect, the present application provides an RNA inhibitor or pharmaceutically acceptable salt thereof for inhibiting LPA gene expression.

In some embodiments, the RNA inhibitor of the present application is formed by base pairing of a sense strand and an antisense strand with a chain length of 15-30, preferably 19-23, and at least 85% of the bases between the sense strand and the antisense strand are complementary; the -OH at the 2' positions of part or all nucleotide sugar groups of the sense strand and/or the antisense strand can be substituted, the substituent group is fluorine or methoxy; and the phosphate bonds among 3 adjacent nucleotides at at least one of the ends of the sense and/or antisense strand can be thiolated.

In some embodiments, the RNA inhibitor or pharmaceutically acceptable salt thereof of the present application, wherein the antisense strand forms complementary regions with a target sequence, the target sequence is multiple regions at different positions of LPA mRNA, and the multiple regions have at least 15 consecutive nucleotides that are identical.

In some embodiments, the RNA inhibitor or pharmaceutically acceptable salt thereof of the present application, wherein the antisense strand forms complementary regions with a target sequence, the target sequence is multiple regions at different positions of LPA mRNA, the multiple regions have at least 15 consecutive nucleotides that are identical, and the target sequence is selected from any one of the nucleotide regions among 312-332, 654-674, 996-1016, 1338-1358, 1680-1700, 2022-2042 and 2364-2384 in LPA mRNA (NM_005577.4). The starting positions of these regions may vary depending on the version number of the LPA mRNA, for example, the nucleotide region among 654-674 in LPA mRNA NM_005577.4.

In some embodiments, the RNA inhibitor or pharmaceutically acceptable salt thereof of the present application, wherein the antisense strand forms complementary regions with a target sequence, the target sequence is multiple regions at different positions of LPA mRNA, the multiple regions have at least 15 consecutive nucleotides that are identical, and the target sequence is selected from any one of the nucleotide regions among 493-512, 1861-1880 and 2203-2222 in LPA mRNA (NM_005577.4). The starting positions of these regions may vary depending on the version number of the LPA mRNA, for example, the nucleotide region among 1861-1880 in LPA mRNA NM_005577.4.

In some embodiments, the antisense strand of the RNA inhibitor or pharmaceutically acceptable salt thereof of the present application is selected from the following sequences:
5' ucguauaacaauaaggagcug 3' SEQ ID NO. 25
5' auaacucuguccauuaccaug 3' SEQ ID NO. 21

or is a sequence having at least 15 consecutive nucleotides identical to the above antisense strand, or is a sequence differing from the above antisense strand by 1, 2 or 3 nucleotides,
wherein, g = guanylate, a = adenylate, u = uridylate, c = cytidylate.

In some embodiments, the sense strand of the RNA inhibitor or pharmaceutically acceptable salt thereof is selected from the following sequences:

| | |
|---|---|
| 5' cagcuccuuauuguuauacga 3' | SEQ ID NO. 11 |
| 5' ugguaauggacagaguuauca 3' | SEQ ID NO. 8 |

or is a sequence having at least 15 consecutive nucleotides identical to the above sense strand, or is a sequence differing from the above sense strand by 1, 2 or 3 nucleotides,
wherein, g = guanylate, a = adenylate, u = uridylate, c = cytidylate.

In some embodiments, the sense strand of the RNA inhibitor or pharmaceutically acceptable salt thereof of the present application is SEQ ID NO. 11, or a sequence having at least 15 consecutive nucleotides identical to it, or a sequence differing from it by 1, 2 or 3 nucleotides; and the antisense strand is SEQ ID NO. 25, or a sequence having at least 15 consecutive nucleotides identical to it, or a sequence differing from it by 1, 2 or 3 nucleotides,
Sense strand: 5' cagcuccuuauuguuauacga 3' SEQ ID NO. 11
Antisense strand: 5' ucguauaacaauaaggagcug 3' SEQ ID NO. 25;
alternatively, the sense strand is SEQ ID NO. 8, or a sequence having at least 15 consecutive nucleotides identical to it, or a sequence differing from it by 1, 2 or 3 nucleotides; and the antisense strand is SEQ ID NO. 21, or a sequence having at least 15 consecutive nucleotides identical to it, or a sequence differing from it by 1, 2 or 3 nucleotides,
Sense strand: 5' ugguaauggacagaguuauca 3' SEQ ID NO. 8
Antisense strand: 5' auaacucuguccauuaccaug 3' SEQ ID NO. 21;
wherein, g = guanylate, a = adenylate, u = uridylate, c = cytidylate.

In some embodiments, the RNA inhibitor or pharmaceutically acceptable salt thereof of the present application, wherein the sense strand is SEQ ID NO. 270, or a sequence differing from it by 1, 2 or 3 nucleotides; and the antisense strand is SEQ ID NO. 278, or a sequence differing from it by 1, 2 or 3 nucleotides,
Sense strand: 5' CsAsGCUCCUfUfAfUUGUUAUACsGsA 3' SEQ ID NO. 270
Antisense strand: 5' UsfCsGfUAfUAACAAfUAfAGfGAfGCsfUsG 3' SEQ ID NO. 278;
alternatively, the sense strand is SEQ ID NO. 239, or a sequence differing from it by 1, 2 or 3 nucleotides; and the antisense strand is SEQ ID NO. 344, or a sequence differing from it by 1, 2 or 3 nucleotides,
Sense strand: 5' UsGsGUfAAfUfGfGACAGAGUUAUsCsA 3' SEQ ID NO. 239
Antisense strand: 5' AsfUsAfACdTCfUGUCCAfUUfACCAsUsG 3' SEQ ID NO. 344;
wherein, G = 2'-O-methylguanylate, A = 2'-O-methyladenylate, U = 2'-O-methyluridylate, C = 2'-O-methylcytidylate; Gs = 2'-O-methyl-3'-thioguanylate, As = 2'-O-methyl-3'-thioadenylate, Us = 2'-O-methyl-3'-thiouridylate, Cs = 2'-O-methyl-3'-thiocytidylate, fG = 2'-fluoroguanylate, fA = 2'-fluoroadenylate, fU = 2'-fluorouridylate, fC = 2'-fluorocytidylate; fGs = 2'-fluoro-3'-thioguanylate, fAs = 2'-fluoro-3'-thioadenylate, fUs = 2'-fluoro-3'-thiouridylate, fCs = 2'-fluoro-3'-thio cytidylate, dT = 2'-deoxythymidylate.

In some embodiments, the sense strand or antisense strand of the RNA inhibitor of the present application can tolerate no more than 3 mismatched nucleotides, for example, within 5, 4, 3 or 2 nucleotides at the 5' end and/or 3' end.

In the above technical modes, preferably the RNA inhibitor or pharmaceutically acceptable salt thereof of the present application further contains ligand structures 5'MVIP and 3'MVIP, and the structure of the RNA inhibitor is shown in Formula Ia, Ib or Ic: 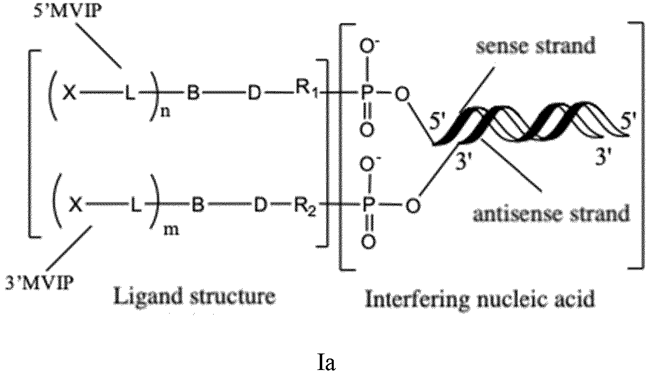 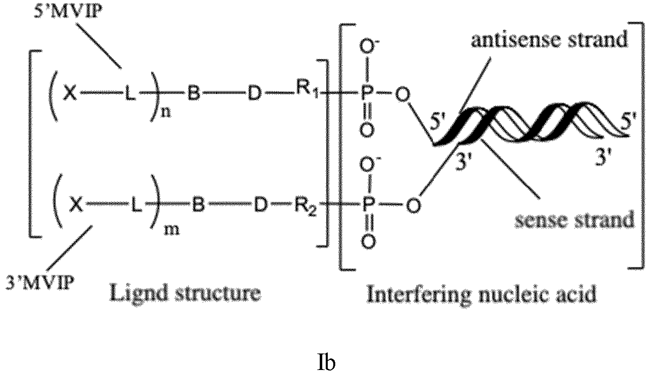 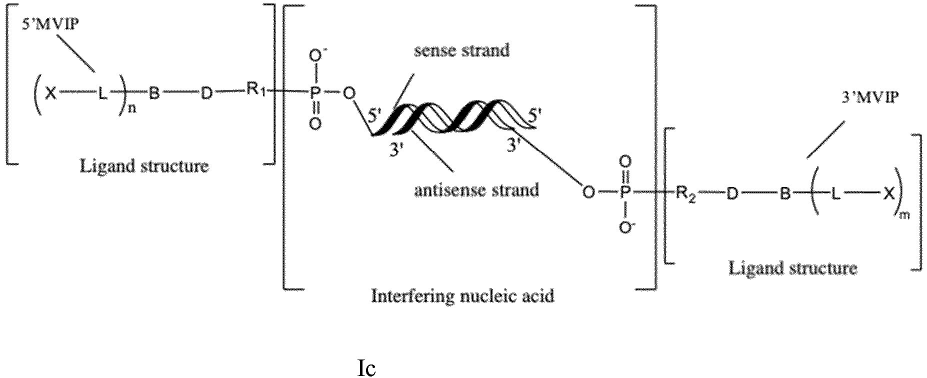 wherein,
the ligand structure includes 5'MVIP (5' MultiValent Import Platform) and 3'MVIP (3' MultiValent Import Platform);
the 5'MVIP is composed of a transition point R₁, a linking chain D, a linker B, a branched chain L and a liver targeting specific ligand X, which is connected to the 5' end of the sense strand or the 5' end of the antisense strand through the transition point R₁ and has the structure as shown in the general formula I:

   **(X-L)ₙ-B-D-R₁**- I
the 3'MVIP is composed of a transition point R₂, a linking chain D, a linker B, a branched chain L and a liver targeting specific ligand X, which is connected to the 3' end of the sense strand or the 3' end of the antisense strand through the transition point R₂ and has the structure as shown in the general formula II:

   **(X-L)ₘ-B**-**D-R₂**-, II
wherein,
n and m are each independently any integer from 0 to 4, preferably each independently an integer of 1-3, and n+m is an integer of 2-6, preferably n+m=2, 3 or 4, more preferably 4;
the transition point R₁ is a heterocyclic or carbocyclic structure containing N, S or O as shown below: 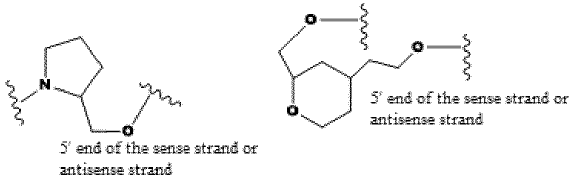 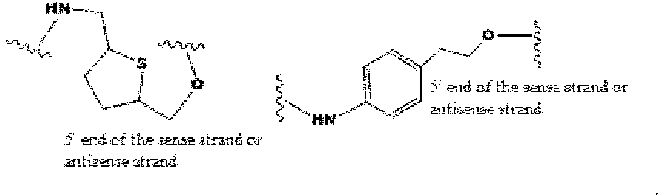
alternatively, the R₁ is -NH(CH₂)ₓCH₂O-, wherein x is any integer of 3-12, preferably any integer of 4-6,
the transition point R₂ is a heterocyclic or carbocyclic structure containing N, S or O as shown below: 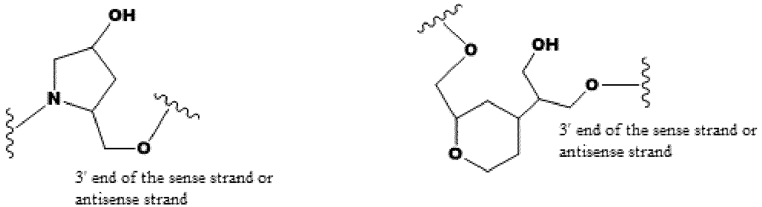 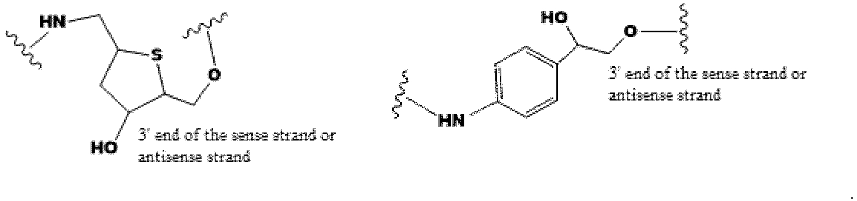
alternatively, the transition point R₂ is -NH(CH₂)ₓ₁CH(OH)(CH₂)ₓ₂CH₂O-, wherein x1 is any integer of 1-4, and x2 is any integer of 0-4,
the liver targeting specific ligand X is selected from structures used to enhance the uptake of RNA inhibitor by hepatocytes, and is the same or different within each of 5'MVIP and 3'MVIP or between 5'MVIP and 3'MVIP, and is selected from monosaccharides and their derivatives, preferably N-acetylgalactosamine and its derivatives, and more preferably selected from the following structures: 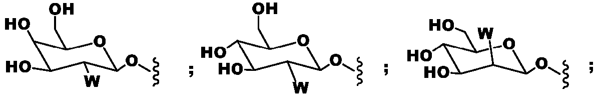 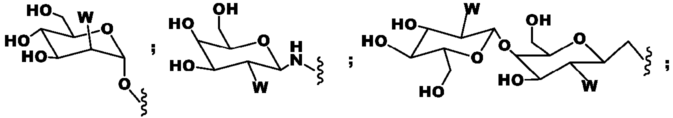 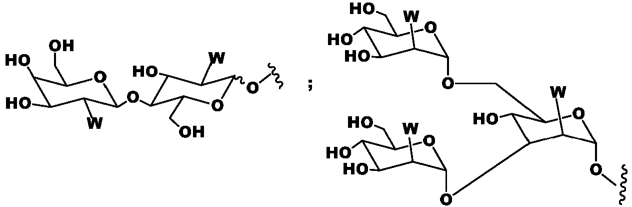 wherein W is selected from one or two of -OH, -NHCOOH and -NHCO(CH₂)_{q}CH₃, wherein q is an integer of 0-4,
the branched chain L is the same or different within each of 5'MVIP and 3'MVIP or between 5'MVIP and 3'MVIP, and is selected from one or more of the following structures: 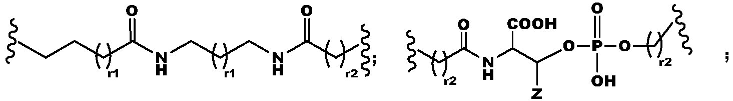 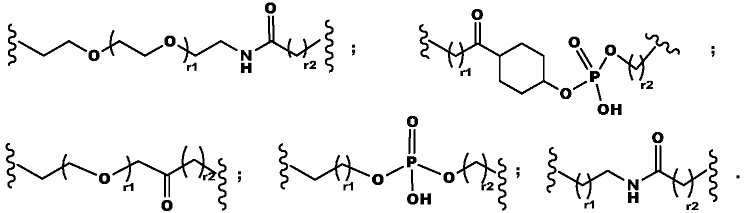 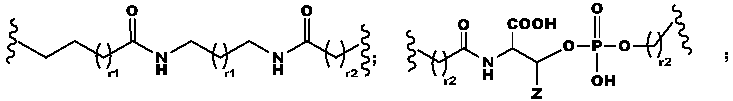 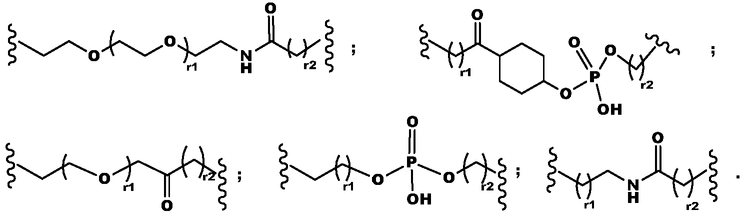 wherein r1 is any integer from 1 to 12, r2 is any integer from 0 to 20, and Z is H, alkyl or amido group, the alkyl group is such as C₁-C₅ alkyl,
the linker B is the same or different within each of 5'MVIP and 3'MVIP or between 5'MVIP and 3'MVIP, and is selected from the following structures: 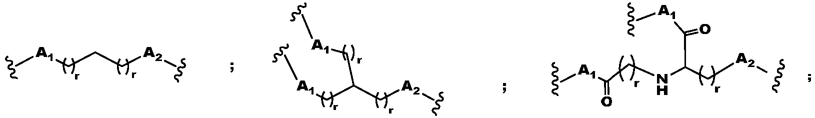 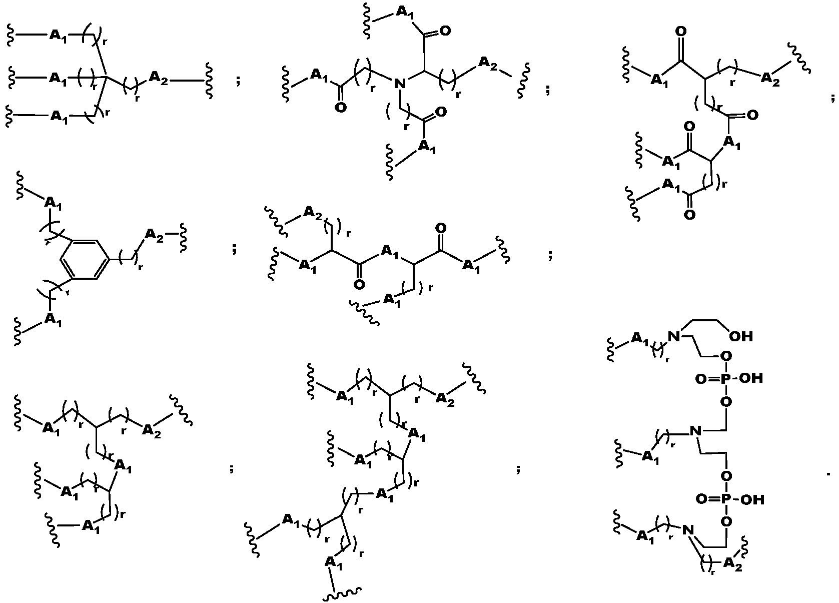 wherein A₁ and A₂ are each independently C, O, S, -NH-, carbonyl, amido, phosphoryl or thiophosphoryl, and r is any integer of 0-4.
the linking chain D is the same or different within each of 5'MVIP and 3'MVIP or between 5'MVIP and 3'MVIP, and is selected from the following structures: 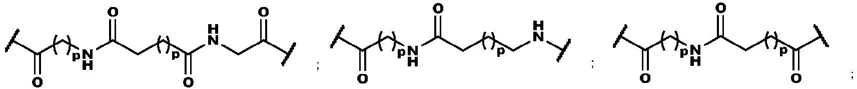 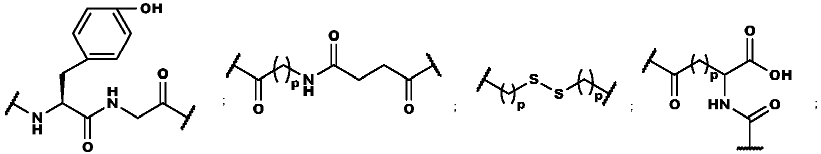 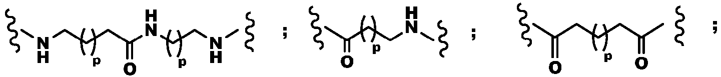 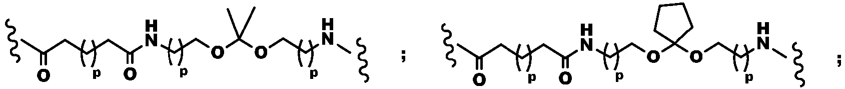 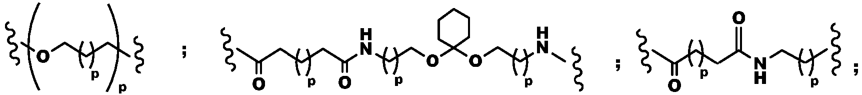 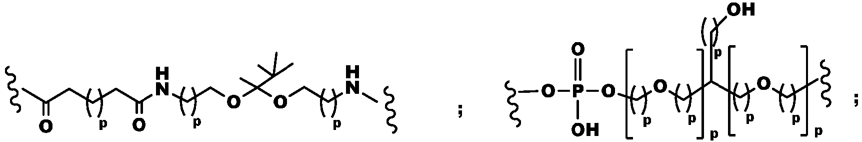 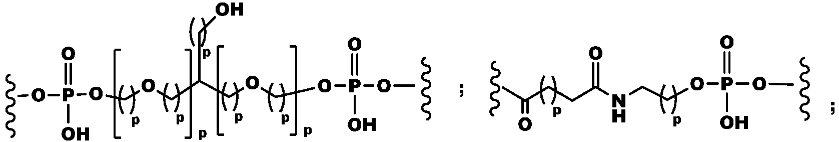 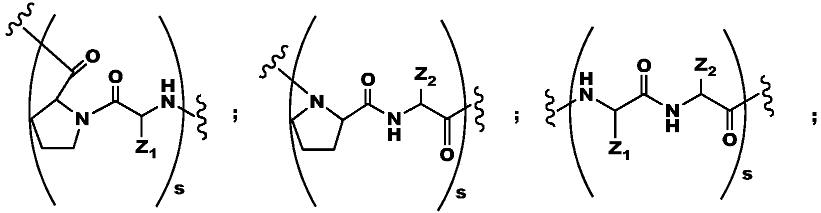 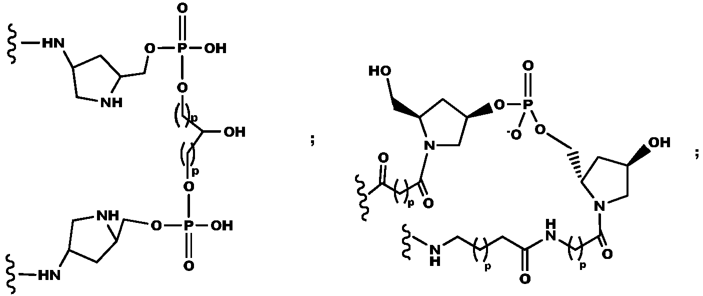 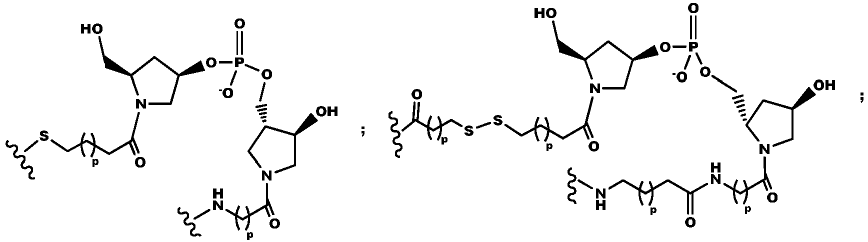 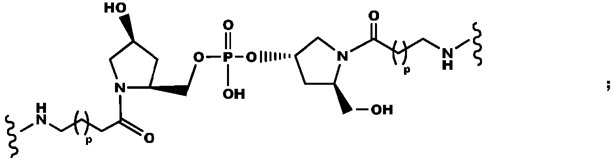 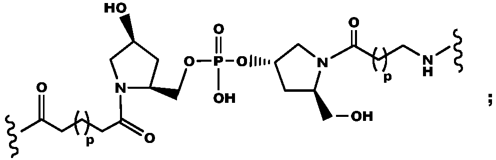 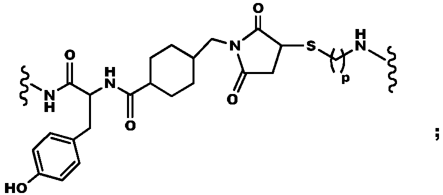 wherein, each p is independently any integer from 1 to 20; s is any integer of 2-13; Z₁ and Z₂ are the same or different substituents, such as C₃-C₁₀ alkyl.

In some embodiments, the 5'MVIP is selected from any one of 5'MVIP01 to 5'MVIP22 in Table 11.

In some embodiments, the 3'MVIP is selected from any one of 3'MVIP01 to 3'MVIP27 in Table 12.

In some embodiments, the RNA inhibitor or pharmaceutically acceptable salt thereof of the present application, wherein the 5'MVIP is 5'MVIP01 or 5'MVIP09 as shown below, and the 3'MVIP is 3'MVIP01, 3'MVIP09 or 3'MVIP17 as shown below:

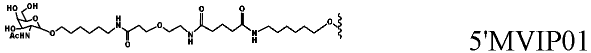

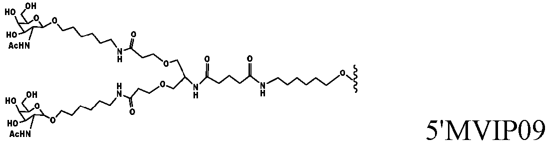

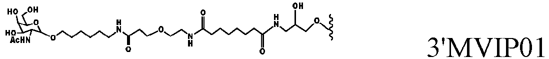

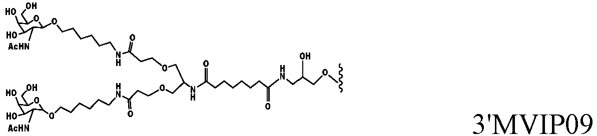

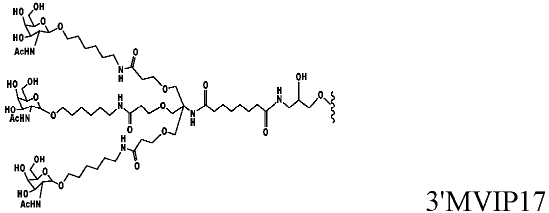

In some embodiments, the RNA inhibitor or pharmaceutically acceptable salt thereof of the present application, wherein the combination of the sense strand 5'MVIP and the antisense strand 3'MVIP is 5'MVIP01/3'MVIP01, 5'MVIP01/3'MVIP17 or 5'MVIP09/3'MVIP09, or the combination of the sense strand 5'MVIP and the antisense strand 3'MVIP is 5'MVIP01/3'MVIP09 or 5'MVIP09/3'MVIP01.

In another aspect, the present application also provides use of the aforementioned RNA inhibitor or pharmaceutically acceptable salt thereof in the preparation of a medicament for treating and/or preventing diseases associated with elevated LP(a) level, wherein the diseases associated with elevated LP(a) level include hepatogenic diseases, including inflammatory diseases, cardio-cerebrovascular diseases and metabolic diseases. Among them, the cardio-cerebrovascular diseases comprise hyperlipoprotein(a)emia, hyperlipidemia, stroke, atherosclerosis, thrombosis, coronary heart disease and aortic valve stenosis.

In yet another aspect, the present application provides a pharmaceutical composition containing the aforementioned RNA inhibitor or pharmaceutically acceptable salt thereof for inhibiting LPA gene expression, and optional pharmaceutically acceptable adjuvants, wherein the pharmaceutically acceptable adjuvants could be pharmaceutically acceptable excipients, carriers and/or diluents, and the dosage form of the pharmaceutical composition is oral administration, intravenous injection, or subcutaneous or intramuscular injection, preferably subcutaneous injection.

In yet another aspect, the present application provides a method for treating and/or preventing diseases, conditions or syndromes associated with elevated LP(a) levels, which includes administering to a subject/patient in need a therapeutically effective amount of the aforementioned RNA inhibitor or pharmaceutically acceptable salt thereof for inhibiting LPA gene expression, or the aforementioned pharmaceutical composition containing the pharmaceutically acceptable salt of the RNA inhibitor and optional pharmaceutically acceptable adjuvants, wherein the therapeutically effective amount is 1.0 mg/kg to 10 mg/kg of the aforementioned RNA inhibitor or pharmaceutically acceptable salt thereof, the administration methods (mode of administration) to the subject/ patient include oral, intravenous, subcutaneous or intramuscular injection, rectal or intraperitoneal administration. Those skilled in the art can easily perceive other aspects and advantages of the present application from the detailed description below. In the detailed description below, only exemplary embodiments of the present application are shown and described. As will be appreciated by those skilled in the art, the content of the present application enables those skilled in the art to modify the disclosed specific embodiments without departing from the spirit and scope of the invention involved in the present application. Accordingly, the description in the drawings and specification of the present application is merely exemplary and not restrictive.

### Brief Description of the Drawings

The specific features of the invention involved in the present application are shown in the appended claims. The features and advantages of the invention involved in the present application can be better understood by referring to the exemplary embodiments and drawings described in detail below. A brief description of the attached drawings is as follows:
Figure 1 shows the inhibitory effects of the RNA inhibitors in Table 3, which are obtained in Example 2 of the present application, on LPA mRNA level in Huh7 cells at different concentrations;
Figure 2 is a high-resolution mass spectrum of ERCd-01-c2 synthesized in 3.1.15 in Example 3 of the present application;
Figure 3 is a high-resolution mass spectrum of 3'MVIP17-c1 synthesized in 3.1.2.6 in Example 3 of the present application;
Figure 4 is a high-resolution mass spectrum of 5'MVIP09-ERCd-PFP-c2 synthesized in 3.2.1.2 in Example 3 of the present application;
Figure 5 shows the inhibitory effects of the RNA inhibitors in Table 4, which are obtained in Example 5 of the present application, on LPA mRNA level in Huh7 cells at different concentrations;
Figure 6 shows the inhibitory effects of the RNA inhibitors in Table 5, which are obtained in Example 6 of the present application, on LPA mRNA level in Huh7 cells at different concentrations;
Figure 7 shows the inhibitory effects of the RNA inhibitors in Table 6, which are obtained in Example 7 of the present application, on LPA mRNA level in Huh7 cells at different concentrations;
Figure 8 shows the inhibitory effects of the RNA inhibitors in Table 7, which are obtained in Example 8 of the present application, on LPA mRNA level in Huh7 cells at different concentrations;
Figure 9 shows the inhibitory effects of the RNA inhibitors in Table 8, which are obtained in Example 9 of the present application, on LPA mRNA level in Huh7 cells at different concentrations;
Figure 10 shows the effect of RNA inhibitors in Example 10 of the present application on reducing LDL-c levels in cynomolgus monkey plasma;
Figure 11 shows the effect of RNA inhibitors in Example 10 of the present application on reducing Lp(a) levels in cynomolgus monkey plasma.

### Embodiments

The implementation of the present invention is described by specific examples below. People familiar with this technology can easily understand other advantages and effects of the present invention from the content disclosed in the specification.

### Definition of terms

In the present application, the term "LPA" includes human LPA and cynomolgus monkey LPA, wherein the human LPA mRNA sequence can be found in, for example, GenBank NM_005577.4.

In the present application, the term "target sequence" refers to a continuous portion of the nucleotide sequence of an mRNA molecule formed during transcription of the LPA gene, including mRNA that is a product of RNA processing of the primary transcripts. In some embodiments, the target portion of the sequence will be at least long enough to serve as a substrate for RNA inhibitor-directed degradation at or near the portion of the nucleotide sequence of the mRNA molecule formed during transcription of the LPA gene. The length of the "target sequence" is generally about 15-30 nucleotides.

In the present application, the term "region" refers to the starting position to the ending position of the target sequence in the LPA mRNA included in GenBank. For example, the "region" 312-332 refers to the nucleotides from position 312 to position 332 in the LPA mRNA (NM_005577.4). The positions of these "regions" may vary due to updates of the LPA mRNA sequences included in GenBank, but the number and types of nucleotides covered by the regions will not change.

In the present application, the term "RNA inhibitor" generally refers to agents containing RNA as defined herein, which can mediate the targeted cleavage of RNA transcripts through RNA-induced silencing complex (RISC) pathway. It guides the sequence-specific degradation of mRNA via a process called RNA inhibition, regulates (e.g., inhibits) the expression of LPA genes in cells (e.g., cells in a subject such as a mammalian subject).

In some embodiments, the RNA inhibitor may be single stranded siRNA (ssRNA inhibitor) introduced into cell or organism to inhibit target mRNA (i.e., LPA gene). The single stranded RNA inhibitor binds to the RISC endonuclease Argonaute 2, which then cleaves the target mRNA. The single stranded siRNAs are generally 15 to 30 nucleotides in length and chemically modified.

In some embodiments, the "RNA inhibitor " used in the present application is a double stranded RNA, and is referred to as "double stranded RNA inhibitor ", "double stranded RNA (dsRNA, DS) molecule", "dsRNA agent" or "dsRNA" in the present application. The term "dsRNA" refers to a complex of ribonucleic acid molecules with a duplex structure containing two antiparallel and essentially complementary nucleic acid strands, known as having a "sense" and "antisense" orientations relative to the target mRNA. In some embodiments of the present application, double stranded RNA (dsRNA) triggers the degradation of target mRNA through a post transcriptional gene silencing mechanism (referred to as RNA inhibition or RNA interference in the present application).

The duplex structure can be any length that triggers specific degradation of LPA mRNA through RISC pathway, and can have a length in range of about 15 to 36 base pairs, for example, the length of about 15-30 base pairs, for example, the length of about 15, 16, 17, 18, 19, 20, 21, 22, 23, 24, 25, 26, 27, 28, 29, 30 or 36 base pairs. In some embodiments, the RNA inhibitor of the present application is a dsRNA containing 15-30 nucleotides, which interacts with the target sequence to guide the cleavage of LPA mRNA.

Generally, most nucleotides in the sense strand and antisense strand of dsRNA molecule are ribonucleotides, but as described in detail in the present application, may also include one or more non-ribonucleotides, such as deoxyribonucleotides or modified nucleotides. In addition, the RNA inhibitors involved in this specification may include ribonucleotides with chemical modifications, which may have modified nucleotides in multiple regions. The term "modified nucleotide" used in the present application refers to a nucleotide that independently has a modified sugar moiety, a modified internucleotide linkage or a modified nucleobase, or any combination thereof. Therefore, the term "modified nucleotide" covers the substitution, addition, or removal of functional groups or atoms of internucleotide linkages, sugar moieties, or nucleobases. The modifications applicable to the RNA inhibitors of the present invention include all types of modifications disclosed herein or known in the art.

In the present application, the term "nucleotide sequence" usually refers to a series or a certain sequence of nucleotides, whether modified or unmodified, described by a series of letters using standard nucleotide nomenclature and a list of symbols for modified nucleotides described in the present application. The nucleotide sequences described in the present application are polymers connected by phosphodiester bond (or related structural variant or synthetic analog thereof), including naturally occurring nucleotide polymers, but it should be understood that the scope of the term also includes various analogues, including but not limited to: peptide nucleic acid (PNA), aminophosphate, thiophosphate, methylphosphonate and 2'-O-methyl ribonucleic acid, etc. It is usually about 15-30 nucleotides, but the term can also refer to molecules of any length.

In some embodiments, the nucleotide sequence comprises one or more unmodified ribonucleosides (RNA) and/or unmodified deoxyribonucleosides (DNA) and/or one or more modified nucleotide. The term "modified nucleotide sequence" generally refers to a series or sequence of nucleotides comprising at least one modification and/or at least one modified internucleotide linkage.

In the present application, the term "modified nucleotide" generally means a nucleotide containing at least one chemical modification compared to a naturally occurring RNA or DNA nucleotide. For example, 2'-deoxy-thymidylate 2'-O-methyl modified nucleotides, 2'-fluoro modified nucleotides, 2'-deoxy-modified nucleotides, locked nucleotides, abasic nucleotides, 2'-amino-modified nucleotides, 2'-O-long chain alkyl-modified nucleotides (such as hexadecyl), morpholino nucleotides, phosphoramidate nucleotides, non-natural nucleobase nucleotides, 5'-phosphorothioate nucleotides and nucleotides with cholesteryl derivatives or dodecyl dodecylamide groups.

Modified nucleotide contains modified sugar groups and/or modified nucleobases.

In the present application, the term "nucleobase" or "base" generally refers to a heterocyclic pyrimidine or purine compound, which is a component of all nucleic acids and includes adenine, guanine, cytosine, thymine and uracil. Nucleotides may include modified nucleotides or nucleotide mimics, Abasics or the part substituted by the substituent. The term "unmodified nucleobase" or "naturally occurring nucleobase" generally means naturally occurring heterocyclic nucleobases of RNA or DNA: purine bases adenine and guanine; and the pyrimidine bases thymine, cytosine and uracil. "Modified nucleobase" usually means any nucleobase that is not a naturally occurring nucleobase.

In the present application, the term "sugar group" generally refers to the naturally occurring sugar group or modified sugar group of a nucleotide. The term "naturally occurring sugar group" generally refers to a ribofuranosyl as found in naturally occurring RNA or a deoxyribofuranosyl as found in naturally occurring DNA. "Modified sugar group" means a substituted sugar group or sugar substitute, for example, a fluoro or methoxy substitution at the 2' position of the sugar group.

In the present application, the term "internucleotide linkage" generally refers to the covalent linkage between adjacent nucleotides in a nucleotide sequence. "Naturally occurring internucleotide linkage" means 3' to 5' phosphodiester linkage. "Modified internucleotide linkage" means any internucleotide linkage other than the naturally occurring internucleotide linkage.

In the present application, the term "antisense strand" (AS) generally refers to the strand of RNA inhibitor (such as dsRNA) that includes a region that are substantially complementary to the target sequence. When used in the present application, the term "complementary region" generally refers to a region on the antisense strand that is substantially complementary to the sequence (e.g., target sequence) defined in the present application.

In the present application, the term "sense strand" (S) generally refers to such a strand of RNA inhibitor (such as dsRNA), which includes a region that is substantially complementary to a region of the "antisense strand" (AS). The "sense" strand is sometimes referred to as the "passenger" strand or "anti-guide" strand. With the sequence of the sense strand, the antisense strand targets the desired mRNA, while the sense strand may target a different target or be degraded. Therefore, if the antisense strand is incorporated into RISC, the correct target is targeted. The incorporation of sense strand can lead to off-target effects. These off-target effects can be limited by the use of modifications or 5' end caps on the sense strand.

In the present application, the term "complementary" refers to the ability of two nucleotide sequences to hybridize under certain conditions so as to form base pair hydrogen bonds and form duplex or double helix structures. For example, the antisense strand of RNA inhibitor hybridizes with the sense strand of RNA inhibitor or LPA mRNA to form Watson-Crick base pairs or non-Watson-Crick base pairs, and includes natural or modified nucleotides or nucleotide mimics. "Complementation" does not require nucleobase complementarity on each nucleoside. On the contrary, some mismatches can be tolerated.

In the present application, with regard to the term "mismatch", when the complementary region is not completely complementary to the target sequence, mismatch can be in the internal or terminal region of the molecule. Typically, the most tolerated mismatches are in the terminal region, for example, within 5, 4, 3 or 2 nucleotides at the 5' end and/or 3' end, and no more than 3.

In the present application, the term "ligand" generally refers to any compound or molecule that can be covalently or otherwise chemically bound to bioactive substances (such as dsRNA). In some embodiments, the ligand is able to interact directly or indirectly with another compound such as a receptor, the receptor interacting with the ligand may exist on the cell surface, or alternatively may be an intracellular and/or intercellular receptor, the interaction of the ligand with the receptor may lead to a biochemical reaction, or may simply be a physical interaction or binding.

In the present application, the term "pharmaceutically acceptable" generally refers to one or more non-toxic substances that do not inhibit the biological activity and effectiveness of the active ingredient. Such preparations may generally contain salts, excipients, buffers, preservatives, compatible carriers, and optionally other therapeutic agents. Such pharmaceutically acceptable preparations may also generally contain compatible solid or liquid fillers, diluents, or encapsulation materials suitable for administration to humans. When used in medicine, salts should be pharmaceutically acceptable salts, but non-pharmaceutically acceptable salts can be conveniently used to prepare pharmaceutically acceptable salts, which cannot be excluded from the scope of the present application. Such pharmacologically and pharmaceutically acceptable salts include but are not limited to those prepared from the following acids: hydrochloric acid, hydrobromic acid, sulfuric acid, nitric acid, phosphoric acid, maleic acid, acetic acid, salicylic acid, citric acid, boric acid, formic acid, malonic acid, succinic acid, etc. The pharmaceutically acceptable salts can also be prepared into alkali metal salts or alkaline earth metal salts, such as sodium salt, potassium salt or calcium salt.

In the present application, the term "lipid nanoparticles" or "LNP" generally refers to vesicles containing a lipid layer encapsulating pharmacologically active molecules (e.g., dsRNA). LNP is described in, for example, Chinese patent No. CN103189057B, the complete contents of which are incorporated herein by reference.

### Detail of the invention

In one aspect, the present application provides an RNA inhibitor or pharmaceutically acceptable salt thereof for inhibiting LPA gene expression.

In some embodiments, the target sequence acted upon by the RNA inhibitor or pharmaceutically acceptable salt thereof comprises multiple regions of LPA mRNA NM_005577.4 (SEQ ID NO. 1): 
SEQ ID NO. 1:
1 gtaagtcaac aatgtcctgg gattgggaca cactttctgg gcactgctgg ccagtcccaa
61 aatggaacat aaggaagtgg ttcttctact tcttttattt ctgaaatcag cagcacctga
121 gcaaagccat gtggtccagg attgctacca tggtgatgga cagagttatc gaggcacgta
181 ctccaccact gtcacaggaa ggacctgcca agcttggtca tctatgacac cacatcaaca
241 taataggacc acagaaaact acccaaatgc tggcttgatc atgaactact gcaggaatcc
301 agatgctgtg gcagctcctt attgttatac gagggatccc ggtgtcaggt gggagtactg
361 caacctgacg caatgctcag acgcagaagg gactgccgtc gcgcctccga ctgttacccc
421 ggttccaagc ctagaggctc cttccgaaca agcaccgact gagcaaaggc ctggggtgca
481 ggagtgctac catggtaatg gacagagtta tcgaggcaca tactccacca ctgtcacagg
541 aagaacctgc caagcttggt catctatgac accacactcg catagtcgga ccccagaata
601 ctacccaaat gctggcttga tcatgaacta ctgcaggaat ccagatgctg tggcagctcc
661 ttattgttat acgagggatc ccggtgtcag gtgggagtac tgcaacctga cgcaatgctc
721 agacgcagaa gggactgccg tcgcgcctcc gactgttacc ccggttccaa gcctagaggc
781 tccttccgaa caagcaccga ctgagcaaag gcctggggtg caggagtgct accatggtaa
841 tggacagagt tatcgaggca catactccac cactgtcaca ggaagaacct gccaagcttg
901 gtcatctatg acaccacact cgcatagtcg gaccccagaa tactacccaa atgctggctt
961 gatcatgaac tactgcagga atccagatgc tgtggcagct ccttattgtt atacgaggga
1021 tcccggtgtc aggtgggagt actgcaacct gacgcaatgc tcagacgcag aagggactgc
1081 cgtcgcgcct ccgactgtta ccccggttcc aagcctagag gctccttccg aacaagcacc
1141 gactgagcag aggcctgggg tgcaggagtg ctaccacggt aatggacaga gttatcgagg
1201 cacatactcc accactgtca ctggaagaac ctgccaagct tggtcatcta tgacaccaca
1261 ctcgcatagt cggaccccag aatactaccc aaatgctggc ttgatcatga actactgcag
1321 gaatccagat gctgtggcag ctccttattg ttatacgagg gatcccggtg tcaggtggga
1381 gtactgcaac ctgacgcaat gctcagacgc agaagggact gccgtcgcgc ctccgactgt
1441 taccccggtt ccaagcctag aggctccttc cgaacaagca ccgactgagc aaaggcctgg
1501 ggtgcaggag tgctaccatg gtaatggaca gagttatcga ggcacatact ccaccactgt
1561 cacaggaaga acctgccaag cttggtcatc tatgacacca cactcgcata gtcggacccc
1621 agaatactac ccaaatgctg gcttgatcat gaactactgc aggaatccag atgctgtggc
1681 agctccttat tgttatacga gggatcccgg tgtcaggtgg gagtactgca acctgacgca
1741 atgctcagac gcagaaggga ctgccgtcgc gcctccgact gttaccccgg ttccaagcct
1801 agaggctcct tccgaacaag caccgactga gcaaaggcct ggggtgcagg agtgctacca
1861 tggtaatgga cagagttatc gaggcacata ctccaccact gtcacaggaa gaacctgcca
1921 agcttggtca tctatgacac cacactcgca tagtcggacc ccagaatact acccaaatgc
1981 tggcttgatc atgaactact gcaggaatcc agatgctgtg gcagctcctt attgttatac
2041 gagggatccc ggtgtcaggt gggagtactg caacctgacg caatgctcag acgcagaagg
2101 gactgccgtc gcgcctccga ctgttacccc ggttccaagc ctagaggctc cttccgaaca
2161 agcaccgact gagcaaaggc ctggggtgca ggagtgctac catggtaatg gacagagtta
2221 tcgaggcaca tactccacca ctgtcacagg aagaacctgc caagcttggt catctatgac
2281 accacactcg catagtcgga ccccagaata ctacccaaat gctggcttga tcatgaacta
2341 ctgcaggaat ccagatgctg tggcagctcc ttattgttat acgagggatc ccggtgtcag
2401 gtgggagtac tgcaacctga cgcaatgctc agacgcagaa gggactgccg tcgcgcctcc
2461 gactgttacc ccggttccaa gcctagaggc tccttccgaa caagcaccga ctgagcagag
2521 gcctggggtg caggagtgct accacggtaa tggacagagt tatcgaggca catactccac
2581 cactgtcact ggaagaacct gccaagcttg gtcatctatg acaccacact cgcatagtcg
2641 gaccccagaa tactacccaa atgctggctt gatcatgaac tactgcagga atccagatcc
2701 tgtggcagcc ccttattgtt atacgaggga tcccagtgtc aggtgggagt actgcaacct
2761 gacacaatgc tcagacgcag aagggactgc cgtcgcgcct ccaactatta ccccgattcc
2821 aagcctagag gctccttctg aacaagcacc aactgagcaa aggcctgggg tgcaggagtg
2881 ctaccacgga aatggacaga gttatcaagg cacatacttc attactgtca caggaagaac
 
2941 ctgccaagct tggtcatcta tgacaccaca ctcgcatagt cggaccccag catactaccc
3001 aaatgctggc ttgatcaaga actactgccg aaatccagat cctgtggcag ccccttggtg
3061 ttatacaaca gatcccagtg tcaggtggga gtactgcaac ctgacacgat gctcagatgc
3121 agaatggact gccttcgtcc ctccgaatgt tattctggct ccaagcctag aggctttttt
3181 tgaacaagca ctgactgagg aaacccccgg ggtacaggac tgctactacc attatggaca
3241 gagttaccga ggcacatact ccaccactgt cacaggaaga acttgccaag cttggtcatc
3301 tatgacacca caccagcata gtcggacccc agaaaactac ccaaatgctg gcctgaccag
3361 gaactactgc aggaatccag atgctgagat tcgcccttgg tgttacacca tggatcccag
3421 tgtcaggtgg gagtactgca acctgacaca atgcctggtg acagaatcaa gtgtccttgc
3481 aactctcacg gtggtcccag atccaagcac agaggcttct tctgaagaag caccaacgga
3541 gcaaagcccc ggggtccagg attgctacca tggtgatgga cagagttatc gaggctcatt
3601 ctctaccact gtcacaggaa ggacatgtca gtcttggtcc tctatgacac cacactggca
3661 tcagaggaca acagaatatt atccaaatgg tggcctgacc aggaactact gcaggaatcc
3721 agatgctgag attagtcctt ggtgttatac catggatccc aatgtcagat gggagtactg
3781 caacctgaca caatgtccag tgacagaatc aagtgtcctt gcgacgtcca cggctgtttc
3841 tgaacaagca ccaacggagc aaagccccac agtccaggac tgctaccatg gtgatggaca
3901 gagttatcga ggctcattct ccaccactgt tacaggaagg acatgtcagt cttggtcctc
3961 tatgacacca cactggcatc agagaaccac agaatactac ccaaatggtg gcctgaccag
4021 gaactactgc aggaatccag atgctgagat tcgcccttgg tgttatacca tggatcccag
4081 tgtcagatgg gagtactgca acctgacgca atgtccagtg atggaatcaa ctctcctcac
4141 aactcccacg gtggtcccag ttccaagcac agagcttcct tctgaagaag caccaactga
4201 aaacagcact ggggtccagg actgctaccg aggtgatgga cagagttatc gaggcacact
4261 ctccaccact atcacaggaa gaacatgtca gtcttggtcg tctatgacac cacattggca
4321 tcggaggatc ccattatact atccaaatgc tggcctgacc aggaactact gcaggaatcc
4381 agatgctgag attcgccctt ggtgttacac catggatccc agtgtcaggt gggagtactg
4441 caacctgaca cgatgtccag tgacagaatc gagtgtcctc acaactccca cagtggcccc
4501 ggttccaagc acagaggctc cttctgaaca agcaccacct gagaaaagcc ctgtggtcca
4561 ggattgctac catggtgatg gacggagtta tcgaggcata tcctccacca ctgtcacagg
4621 aaggacctgt caatcttggt catctatgat accacactgg catcagagga ccccagaaaa
4681 ctacccaaat gctggcctga ccgagaacta ctgcaggaat ccagattctg ggaaacaacc
4741 ctggtgttac acaaccgatc cgtgtgtgag gtgggagtac tgcaatctga cacaatgctc
4801 agaaacagaa tcaggtgtcc tagagactcc cactgttgtt ccagttccaa gcatggaggc
4861 tcattctgaa gcagcaccaa ctgagcaaac ccctgtggtc cggcagtgct accatggtaa
4921 tggccagagt tatcgaggca cattctccac cactgtcaca ggaaggacat gtcaatcttg
4981 gtcatccatg acaccacacc ggcatcagag gaccccagaa aactacccaa atgatggcct
5041 gacaatgaac tactgcagga atccagatgc cgatacaggc ccttggtgtt ttaccatgga
5101 ccccagcatc aggtgggagt actgcaacct gacgcgatgc tcagacacag aagggactgt
5161 ggtcgctcct ccgactgtca tccaggttcc aagcctaggg cctccttctg aacaagactg
5221 tatgtttggg aatgggaaag gataccgggg caagaaggca accactgtta ctgggacgcc
5281 atgccaggaa tgggctgccc aggagcccca tagacacagc acgttcattc cagggacaaa
5341 taaatgggca ggtctggaaa aaaattactg ccgtaaccct gatggtgaca tcaatggtcc
5401 ctggtgctac acaatgaatc caagaaaact ttttgactac tgtgatatcc ctctctgtgc
5461 atcctcttca tttgattgtg ggaagcctca agtggagccg aagaaatgtc ctggaagcat
5521 tgtagggggg tgtgtggccc acccacattc ctggccctgg caagtcagtc tcagaacaag
5581 gtttggaaag cacttctgtg gaggcacctt aatatcccca gagtgggtgc tgactgctgc
5641 tcactgcttg aagaagtcct caaggccttc atcctacaag gtcatcctgg gtgcacacca
5701 agaagtgaac ctcgaatctc atgttcagga aatagaagtg tctaggctgt tcttggagcc
5761 cacacaagca gatattgcct tgctaaagct aagcaggcct gccgtcatca ctgacaaagt
5821 aatgccagct tgtctgccat ccccagacta catggtcacc gccaggactg aatgttacat
5881 cactggctgg ggagaaaccc aaggtacctt tgggactggc cttctcaagg aagcccagct
 
5941 ccttgttatt gagaatgaag tgtgcaatca ctataagtat atttgtgctg agcatttggc
6001 cagaggcact gacagttgcc agggtgacag tggagggcct ctggtttgct tcgagaagga
6061 caaatacatt ttacaaggag tcacttcttg gggtcttggc tgtgcacgcc ccaataagcc
6121 tggtgtctat gctcgtgttt caaggtttgt tacttggatt gagggaatga tgagaaataa
6181 ttaattggac gggagacaga gtgaagcatc aacctactta gaagctgaaa cgtgggtaag
6241 gatttagcat gctggaaata atagacagca atcaaacgaa gacactgttc ccagctacca
6301 gctatgccaa accttggcat ttttggtatt tttgtgtata agcttttaag gtctgactga
6361 caaattctgt attaaggtgt catagctatg acatttgtta aaaataaact ctgcacttat
6421 tttgatttga a
 wherein g = guanylate, a = adenylate, t = thymidylate, c = cytidylate.

In some embodiments, the target sequence acted upon by the RNA inhibitor or pharmaceutically acceptable salt thereof of the present application comprises the nucleotide region with double underline and single underline in SEQ ID NO. 1.

In some embodiments, the target sequence acted upon by the RNA inhibitor or pharmaceutically acceptable salt thereof of the present application is a sequence containing the nucleotide region between 301-2401 in SEQ ID NO. 1 and having at least 15 consecutive nucleotides identical thereto.

In some embodiments, the RNA inhibitor or pharmaceutically acceptable salt thereof of the present application, wherein the antisense strand forms a complementary region with the target sequence, and the target sequence is any one of the nucleotide regions between 312-332, 654-674, 996-1016, 1338-1358, 1680-1700, 2022-2042 and 2364-2384 in SEQ ID NO. 1.

In some embodiments, the RNA inhibitor or pharmaceutically acceptable salt thereof of the present application, wherein the antisense strand forms a complementary region with the target sequence, and the target sequence is any one of the nucleotide regions between 493-512, 1861-1880 and 2203-2222 in SEQ ID NO. 1.

In some embodiments, the target sequence of the RNA inhibitor of the present application is at least 15 consecutive nucleotides in these regions.

In some embodiments, the target sequence of the RNA inhibitor of the present application is at least 30 consecutive nucleotides extending before and after these regions.

In some embodiments, the target sequence of the RNA inhibitor of the present application is cagctcc ttattgttat acga (SEQ ID NO. 2), wherein g = guanylate, a = adenylate, t = thymidylate, c = cytidylate.

In some embodiments, the target sequence of the RNA inhibitor of the present application is tggtaatggacagagttat (SEQ ID NO. 3), wherein g = guanylate, a = adenylate, t = thymidylate, c = cytidylate.

In some embodiments, the target sequence of the RNA inhibitor of the present application is a target sequence that differs from SEQ ID NO. 2 by 1, 2 or 3 nucleotides.

In some embodiments, the target sequence of the RNA inhibitor of the present application is a target sequence having at least 15 consecutive nucleotides identical to SEQ ID NO. 2.

In some embodiments, the target sequence of the RNA inhibitor of the present application is a target sequence that differs from SEQ ID NO. 3 by 1, 2 or 3 nucleotides.

In some embodiments, the target sequence of the RNA inhibitor of the present application is a target sequence having at least 15 consecutive nucleotides identical to SEQ ID NO. 3.

In some embodiments, the target sequence of the RNA inhibitor of the present application may be a sequence of 15-30 nucleotides in any region other than those previously described in SEQ ID NO. 1.

In some embodiments, the RNA inhibitor of the present application includes double-stranded ribonucleic acid (dsRNA) molecules for inhibiting LPA gene expression in cells, such as cells of a subject (e.g., a mammal, such as a human who are susceptible to LPA-related disorders, such as high LP(a) levels). The antisense strand of the dsRNA includes a complementary region that is substantially complementary to the target sequence described above and is usually completely complementary. The sense strand includes a region complementary to the antisense strand so that when combined under suitable conditions, the two strands can hybridize and form a duplex structure. Typically, the duplex structure is 15 to 30 base pairs in length. Similarly, the region complementary to the target sequence is 15 to 30 nucleotides in length.

In some embodiments, the RNA inhibitor of the present application is formed by base pairing of a sense strand and an antisense strand with a chain length of 15-30, wherein the chain length is preferably 19-23.

In some embodiments, there is at least 85% base complementarity between the sense strand and the antisense strand in the RNA inhibitor of the present application.

In some embodiments, the sense strand of the RNA inhibitor of the present application is selected from the sequences in Table 1 as shown below:

**Table 1**

| sense strand code | SEQ ID NO. | sense strand sequence 5'-3' |
|---|---|---|
| Kylo-11-S01 | 4 | ugguaauggacagaguuauuu |
| Kylo-11-S02 | 5 | ugguaauggacagaguuau |
| Kylo-11-S03 | 6 | caugguaauggacagaguuau |
| Kylo-11-S04 | 7 | ugguaauggacagaguuaucg |
| Kylo-11-S05 | 8 | ugguaauggacagaguuauca |
| Kylo-11-S06 | 9 | cgguaauggacagaguuauca |
| Kylo-11-S07 | 10 | cagccccuuauuguuauacga |
| Kylo-11-S08 | 11 | cagcuccuuauuguuauacga |
| Kylo-11-S09 | 12 | gccccuuauuguuauacga |
| Kylo-11-S10 | 13 | cagccccuuauuguuauacgaga |
| Kylo-11-S11 | 14 | uccagccccuuauuguuauacga |
| Kylo-11-S12 | 15 | augguaauggacagaguuauc |
| Kylo-11-S13 | 16 | ugguaauggacagaguuacg |
| Kylo-11-S14 | 17 | ugguaauggacagaguuauug |
| Kylo-11-S15 | 33 | ggaaggaccugccaagcuu |
| Kylo-11-S16 | 34 | gacaccacaucaacauaau |
| Kylo-11-S17 | 35 | cauaauaggaccacagaaa |
| Kylo-11-S18 | 36 | gcagcuccuuauuguuaua |
| Kylo-11-S19 | 37 | uauuguuauacgagggauc |
| Kylo-11-S20 | 38 | aaugcucagacgcagaagg |
| Kylo-11-S21 | 39 | ccuuccgaacaagcaccga |
| Kylo-11-S22 | 40 | aagcaccgacugagcaaag |
| Kylo-11-S23 | 41 | cauacuccaccacugucac |
| Kylo-11-S24 | 42 | cacacucgcauagucggac |
| Kylo-11-S25 | 43 | cauagucggaccccagaau |
| Kylo-11-S26 | 44 | gaauacuacccaaaugcug |
| Kylo-11-S27 | 45 | aaugcuggcuugaucauga |
| Kylo-11-S28 | 46 | cuccuuauuguuauacgag |
| Kylo-11-S29 | 47 | caaugcucagacgcagaag |
| Kylo-11-S30 | 48 | acaagcaccgacugagcaa |
| Kylo-11-S31 | 49 | cagcuccuuauuguuauac |
| Kylo-11-S32 | 50 | cgcaaugcucagacgcaga |
| Kylo-11-S33 | 51 | aagcaccgacugagcagag |
| Kylo-11-S34 | 52 | agcuccuuauuguuauacg |
| Kylo-11-S35 | 53 | agacgcagaagggacugcc |
| Kylo-11-S36 | 54 | uuaccccgguuccaagccu |
| Kylo-11-S37 | 55 | aacaagcaccgacugagca |
| Kylo-11-S38 | 56 | uuauuguuauacgagggau |
| Kylo-11-S39 | 57 | gcucagacgcagaagggac |
| Kylo-11-S40 | 58 | gcaccgacugagcaaaggc |
| Kylo-11-S41 | 59 | guuauacgagggaucccgg |
| Kylo-11-S42 | 60 | augcucagacgcagaaggg |
| Kylo-11-S43 | 61 | acgcagaagggacugccgu |
| Kylo-11-S44 | 62 | acugccgucgcgccuccga |
| Kylo-11-S45 | 63 | caagcaccgacugagcaaa |
| Kylo-11-S46 | 64 | gcuccuuauuguuauacga |
| Kylo-11-S47 | 65 | gcaaugcucagacgcagaa |
| Kylo-11-S48 | 66 | caagcaccgacugagcaga |
| Kylo-11-S49 | 67 | aucgaggcacauacuccac |
| Kylo-11-S50 | 68 | accacugucacuggaagaa |
| Kylo-11-S51 | 69 | cuaugacaccacacucgca |
| Kylo-11-S52 | 70 | ccacacucgcauagucgga |
| Kylo-11-S53 | 71 | ccacggaaauggacagagu |
| Kylo-11-S54 | 72 | gcacauacuucauuacugu |
| Kylo-11-S55 | 73 | gccgaaauccagauccugu |
| Kylo-11-S56 | 74 | cuugguguuauacaacaga |
| Kylo-11-S57 | 75 | cucagaugcagaauggacu |
| Kylo-11-S58 | 76 | gaugcagaauggacugccu |
| Kylo-11-S59 | 77 | ggguacaggacugcuacua |
| Kylo-11-S60 | 78 | gcuacuaccauuauggaca |
| Kylo-11-S61 | 79 | cuaccauuauggacagagu |
| Kylo-11-S62 | 80 | cacaaugccuggugacaga |
| Kylo-11-S63 | 81 | cacagaggcuucuucugaa |
| Kylo-11-S64 | 82 | ggcuucuucugaagaagca |
| Kylo-11-S65 | 83 | cucuaccacugucacagga |
| Kylo-11-S66 | 84 | gcugagauuaguccuuggu |
| Kylo-11-S67 | 85 | gauuaguccuugguguuau |
| Kylo-11-S68 | 86 | cuugguguuauaccaugga |
| Kylo-11-S69 | 87 | caaccugacacaaugucca |
| Kylo-11-S70 | 88 | cugacacaauguccaguga |
| Kylo-11-S71 | 89 | cagugacagaaucaagugu |
| Kylo-11-S72 | 90 | guccacggcuguuucugaa |
| Kylo-11-S73 | 91 | cacggcuguuucugaacaa |
| Kylo-11-S74 | 92 | ggcuguuucugaacaagca |
| Kylo-11-S75 | 93 | caagcaccaacggagcaaa |
| Kylo-11-S76 | 94 | cagaguuaucgaggcucau |
| Kylo-11-S77 | 95 | cuccaccacuguuacagga |
| Kylo-11-S78 | 96 | cagagaaccacagaauacu |
| Kylo-11-S79 | 97 | agaauacuacccaaauggu |
| Kylo-11-S80 | 98 | cacauuggcaucggaggau |
| Kylo-11-S81 | 99 | gaggaucccauuauacuau |
| Kylo-11-S82 | 100 | gaucccauuauacuaucca |
| Kylo-11-S83 | 101 | caaccugacacgaugucca |
| Kylo-11-S84 | 102 | cugacacgauguccaguga |
| Kylo-11-S85 | 103 | gacacgauguccagugaca |
| Kylo-11-S86 | 104 | cagugacagaaucgagugu |
| Kylo-11-S87 | 105 | gacggaguuaucgaggcau |
| Kylo-11-S88 | 106 | gaccugucaaucuugguca |
| Kylo-11-S89 | 107 | cuuggucaucuaugauacc |
| Kylo-11-S90 | 108 | cugguguuacacaaccgau |
| Kylo-11-S91 | 109 | caaucugacacaaugcuca |
| Kylo-11-S92 | 110 | auagacacagcacguucau |
| Kylo-11-S93 | 111 | cacguucauuccagggaca |
| Kylo-11-S94 | 112 | ggugacaucaauggucccu |
| Kylo-11-S95 | 113 | gugcuacacaaugaaucca |
| Kylo-11-S96 | 114 | aagguuuggaaagcacuuc |
| Kylo-11-S97 | 115 | gcugcucacugcuugaaga |
| Kylo-11-S98 | 116 | gcuaaagcuaagcaggccu |
| Kylo-11-S99 | 117 | gucaccgccaggacugaau |
| Kylo-11-S100 | 118 | gcuccuuguuauugagaau |
| Kylo-11-S101 | 119 | gggagacagagugaagcau |
| Kylo-11-S102 | 120 | cagagugaagcaucaaccu |
| Kylo-11-S103 | 121 | uacuuagaagcugaaacgu |
| Kylo-11-S104 | 122 | cuggaaauaauagacagca |
| Kylo-11-S105 | 123 | cagcaaucaaacgaagaca |
| Kylo-11-S106 | 124 | ugccaaaccuuggcauuuu |

| | | |
|---|---|---|
| wherein g = guanylate, a = adenylate, t = thymidylate, c = cytidylate. | | |

In some embodiments, the antisense strand of the RNA inhibitor of the present application is selected from the sequences in Table 2 as shown below:

**Table 2**

| antisense strand code | SEQ ID NO. | antisense strand sequence 5'-3' |
|---|---|---|
| Kylo-11-AS01 | 18 | auaacucuguccauuaccauu |
| Kylo-11-AS02 | 19 | uuaacucuguccauuaccauu |
| Kylo-11-AS03 | 20 | uauaacucuguccauuaccauu |
| Kylo-11-AS04 | 21 | auaacucuguccauuaccaug |
| Kylo-11-AS05 | 22 | aacucuguccauuaccauggu |
| Kylo-11-AS06 | 23 | auaactcuguccauuaccauu |
| Kylo-11-AS07 | 24 | ucguauaacaauaaggggcug |
| Kylo-11-AS08 | 25 | ucguauaacaauaaggagcug |
| Kylo-11-AS09 | 26 | ucguauaacaauaaggggcugga |
| Kylo-11-AS10 | 27 | auaacucuguccauuaccaua |
| Kylo-11-AS11 | 28 | gauaacucuguccauuaccau |
| Kylo-11-AS12 | 29 | auaactcuguccauuaccaua |
| Kylo-11-AS13 | 30 | auaacucuguccauuaccaut |
| Kylo-11-AS14 | 31 | caauaacucuguccauuaccauu |
| Kylo-11-AS15 | 32 | auaacucuguccauuaccauggu |
| Kylo-11-AS16 | 125 | aagcuuggcagguccuucc |
| Kylo-11-AS17 | 126 | auuauguugaugugguguc |
| Kylo-11-AS18 | 127 | uuucugugguccuauuaug |
| Kylo-11-AS19 | 128 | uauaacaauaaggagcugc |
| Kylo-11-AS20 | 129 | gaucccucguauaacaaua |
| Kylo-11-AS21 | 130 | ccuucugcgucugagcauu |
| Kylo-11-AS22 | 131 | ucggugcuuguucggaagg |
| Kylo-11-AS23 | 132 | cuuugcucagucggugcuu |
| Kylo-11-AS24 | 133 | gugacagugguggaguaug |
| Kylo-11-AS25 | 134 | guccgacuaugcgagugug |
| Kylo-11-AS26 | 135 | auucugggguccgacuaug |
| Kylo-11-AS27 | 136 | cagcauuuggguaguauuc |
| Kylo-11-AS28 | 137 | ucaugaucaagccagcauu |
| Kylo-11-AS29 | 138 | cucguauaacaauaaggag |
| Kylo-11-AS30 | 139 | cuucugcgucugagcauug |
| Kylo-11-AS31 | 140 | uugcucagucggugcuugu |
| Kylo-11-AS32 | 141 | guauaacaauaaggagcug |
| Kylo-11-AS33 | 142 | ucugcgucugagcauugcg |
| Kylo-11-AS34 | 143 | cucugcucagucggugcuu |
| Kylo-11-AS35 | 144 | cguauaacaauaaggagcu |
| Kylo-11-AS36 | 145 | ggcagucccuucugcgucu |
| Kylo-11-AS37 | 146 | aggcuuggaaccgggguaa |
| Kylo-11-AS38 | 147 | ugcucagucggugcuuguu |
| Kylo-11-AS39 | 148 | aucccucguauaacaauaa |
| Kylo-11-AS40 | 149 | gucccuucugcgucugagc |
| Kylo-11-AS41 | 150 | gccuuugcucagucggugc |
| Kylo-11-AS42 | 151 | ccgggaucccucguauaac |
| Kylo-11-AS43 | 152 | cccuucugcgucugagcau |
| Kylo-11-AS44 | 153 | acggcagucccuucugcgu |
| Kylo-11-AS45 | 154 | ucggaggcgcgacggcagu |
| Kylo-11-AS46 | 155 | uuugcucagucggugcuug |
| Kylo-11-AS47 | 156 | ucguauaacaauaaggagc |
| Kylo-11-AS48 | 157 | uucugcgucugagcauugc |
| Kylo-11-AS49 | 158 | ucugcucagucggugcuug |
| Kylo-11-AS50 | 159 | guggaguaugugccucgau |
| Kylo-11-AS51 | 160 | uucuuccagugacaguggu |
| Kylo-11-AS52 | 161 | ugcgaguguggugucauag |
| Kylo-11-AS53 | 162 | uccgacuaugcgagugugg |
| Kylo-11-AS54 | 163 | acucuguccauuuccgugg |
| Kylo-11-AS55 | 164 | acaguaaugaaguaugugc |
| Kylo-11-AS56 | 165 | acaggaucuggauuucggc |
| Kylo-11-AS57 | 166 | ucuguuguauaacaccaag |
| Kylo-11-AS58 | 167 | aguccauucugcaucugag |
| Kylo-11-AS59 | 168 | aggcaguccauucugcauc |
| Kylo-11-AS60 | 169 | uaguagcaguccuguaccc |
| Kylo-11-AS61 | 170 | uguccauaaugguaguagc |
| Kylo-11-AS62 | 171 | acucuguccauaaugguag |
| Kylo-11-AS63 | 172 | ucugucaccaggcauugug |
| Kylo-11-AS64 | 173 | uucagaagaagccucugug |
| Kylo-11-AS65 | 174 | ugcuucuucagaagaagcc |
| Kylo-11-AS66 | 175 | uccugugacagugguagag |
| Kylo-11-AS67 | 176 | accaaggacuaaucucagc |
| Kylo-11-AS68 | 177 | auaacaccaaggacuaauc |
| Kylo-11-AS69 | 178 | uccaugguauaacaccaag |
| Kylo-11-AS70 | 179 | uggacauugugucagguug |
| Kylo-11-AS71 | 180 | ucacuggacauugugucag |
| Kylo-11-AS72 | 181 | acacuugauucugucacug |
| Kylo-11-AS73 | 182 | uucagaaacagccguggac |
| Kylo-11-AS74 | 183 | uuguucagaaacagccgug |
| Kylo-11-AS75 | 184 | ugcuuguucagaaacagcc |
| Kylo-11-AS76 | 185 | uuugcuccguuggugcuug |
| Kylo-11-AS77 | 186 | augagccucgauaacucug |
| Kylo-11-AS78 | 187 | uccuguaacagugguggag |
| Kylo-11-AS79 | 188 | aguauucugugguucucug |
| Kylo-11-AS80 | 189 | accauuuggguaguauucu |
| Kylo-11-AS81 | 190 | auccuccgaugccaaugug |
| Kylo-11-AS82 | 191 | auaguauaaugggauccuc |
| Kylo-11-AS83 | 192 | uggauaguauaaugggauc |
| Kylo-11-AS84 | 193 | uggacaucgugucagguug |
| Kylo-11-AS85 | 194 | ucacuggacaucgugucag |
| Kylo-11-AS86 | 195 | ugucacuggacaucguguc |
| Kylo-11-AS87 | 196 | acacucgauucugucacug |
| Kylo-11-AS88 | 197 | augccucgauaacuccguc |
| Kylo-11-AS89 | 198 | ugaccaagauugacagguc |
| Kylo-11-AS90 | 199 | gguaucauagaugaccaag |
| Kylo-11-AS91 | 200 | aucgguuguguaacaccag |
| Kylo-11-AS92 | 201 | ugagcauugugucagauug |
| Kylo-11-AS93 | 202 | augaacgugcugugucuau |
| Kylo-11-AS94 | 203 | ugucccuggaaugaacgug |
| Kylo-11-AS95 | 204 | agggaccauugaugucacc |
| Kylo-11-AS96 | 205 | uggauucauuguguagcac |
| Kylo-11-AS97 | 206 | gaagugcuuuccaaaccuu |
| Kylo-11-AS98 | 207 | ucuucaagcagugagcagc |
| Kylo-11-AS99 | 208 | aggccugcuuagcuuuagc |
| Kylo-11-AS100 | 209 | auucaguccuggcggugac |
| Kylo-11-AS101 | 210 | auucucaauaacaaggagc |
| Kylo-11-AS102 | 211 | augcuucacucugucuccc |
| Kylo-11-AS103 | 212 | agguugaugcuucacucug |
| Kylo-11-AS104 | 213 | acguuucagcuucuaagua |
| Kylo-11-AS105 | 214 | ugcugucuauuauuuccag |
| Kylo-11-AS106 | 215 | ugucuucguuugauugcug |
| Kylo-11-AS107 | 216 | aaaaugccaagguuuggca |

| | | |
|---|---|---|
| wherein g = guanylate, a = adenylate, u = uridylate, c = cytidylate. t = thymidylate. | | |

In some embodiments, the base pairs of the sense strand in Table 1 and the corresponding antisense strand in Table 2 are complementary to form dsRNA, which may be partially complementary or completely complementary. The partial complementarity may be at least 85% base pairing.

In some embodiments, the RNA inhibitor of the present application is selected from Table 3 below:

**Table 3 RNA inhibitor**

| RNA inhibitor | sense strand code | antisense strand code |
|---|---|---|
| Kylo-11-DS01 | Kylo-11-S01 | Kylo-11-AS01 |
| Kylo-11-DS02 | Kylo-11-S01 | Kylo-11-AS02 |
| Kylo-11-DS03 | Kylo-11-S01 | Kylo-11-AS03 |
| Kylo-11-DS04 | Kylo-11-S02 | Kylo-11-AS01 |
| Kylo-11-DS05 | Kylo-11-S01 | Kylo-11-AS04 |
| Kylo-11-DS06 | Kylo-11-S03 | Kylo-11-AS05 |
| Kylo-11-DS07 | Kylo-11-S04 | Kylo-11-AS01 |
| Kylo-11-DS08 | Kylo-11-S05 | Kylo-11-AS01 |
| Kylo-11-DS09 | Kylo-11-S06 | Kylo-11-AS01 |
| Kylo-11-DS10 | Kylo-11-S01 | Kylo-11-AS06 |
| Kylo-11-DS11 | Kylo-11-S05 | Kylo-11-AS04 |
| Kylo-11-DS12 | Kylo-11-S07 | Kylo-11-AS07 |
| Kylo-11-DS13 | Kylo-11-S08 | Kylo-11-AS08 |
| Kylo-11-DS14 | Kylo-11-S09 | Kylo-11-AS07 |
| Kylo-11-DS15 | Kylo-11-S07 | Kylo-11-AS09 |
| Kylo-11-DS16 | Kylo-11-S10 | Kylo-11-AS09 |
| Kylo-11-DS17 | Kylo-11-S11 | Kylo-11-AS09 |
| Kylo-11-DS18 | Kylo-11-S04 | Kylo-11-AS04 |
| Kylo-11-DS19 | Kylo-11-S05 | Kylo-11-AS10 |
| Kylo-11-DS20 | Kylo-11-S12 | Kylo-11-AS11 |
| Kylo-11-DS21 | Kylo-11-S04 | Kylo-11-AS10 |
| Kylo-11-DS22 | Kylo-11-S05 | Kylo-11-AS04 |
| Kylo-11-DS23 | Kylo-11-S04 | Kylo-11-AS12 |
| Kylo-11-DS24 | Kylo-11-S05 | Kylo-11-AS13 |
| Kylo-11-DS25 | Kylo-11-S13 | Kylo-11-AS10 |
| Kylo-11-DS26 | Kylo-11-S02 | Kylo-11-AS04 |
| Kylo-11-DS27 | Kylo-11-S14 | Kylo-11-AS14 |
| Kylo-11-DS28 | Kylo-11-S03 | Kylo-11-AS15 |

In some embodiments, the RNA inhibitor can be added to the cell line for sequence screening by cell transfection or liposome-nucleic acid nanoparticles known to those skilled in the art. The methods for preparing lipid compounds and liposome-nucleic acid nanoparticles in patents US9233971B2, US9080186B2, CN102985548B and CN103189057B are fully incorporated into this specification.

In some embodiments, the amphoteric lipids in the lipid compounds are preferably macrocyclic lipid compounds D1C1, T1C1, T1C6, T4C4, B2C1, B2C6, B2C7 and M10C1.

It is well known to those skilled in the art that dsRNA having a duplex structure of about 20 to 23 base pairs, for example, 21 base pairs, has been found to be particularly effective in inducing RNA inhibition (Elbashir et al., EMBO 2001, 20: 6877-6888). However, others have found that shorter or longer RNA duplex structures are also effective (Chu and Rana (2007) RNA 14: 1714-1719; Kim et al. (2005) Nat Biotech 23: 222-226). It is reasonable to expect that duplexes with a few nucleotides subtracted from or added to one or both ends of a sequence in Tables 1, 2, and 3 will be similarly effective compared to the dsRNA. Therefore, dsRNAs having a sequence with at least 15, 16, 17, 18, 19, 20, 21 or more consecutive nucleotides derived from a sequence in Tables 1, 2 and 3 and having the ability of inhibiting LPA gene expression that differs by no more than about 5, 10, 15, 20, 25, or 30% inhibition compared to a dsRNA containing the entire sequence are included within the scope of the present application.

The dsRNA of the present application may further include one or more single-stranded nucleotide protruding ends, for example, 1, 2, 3 or 4 nucleotides. The nucleotide protruding ends may contain nucleotide/nucleoside analogs or a combination thereof, including deoxynucleotides. The protruding ends may be on the sense strand, the antisense strand or a combination thereof. In addition, the nucleotides on the protruding ends may be at the 5' end, 3' end or both ends of the antisense strand or the sense strand of the dsRNA. The protruding ends may be formed by one strand being longer than the other, or by two strands of the same length being staggered. When the protruding end is on the antisense strand and may form a mismatch or complement with LPA mRNA or may be another sequence. For example, the protruding end is at the 3' end of the sense strand, or alternatively, at the 3' end of the antisense strand.

The dsRNA may also have blunt ends, which means that there is no unpaired nucleotide at that end of the dsRNA, i.e., no nucleotide protruding ends. The blunt end may be at the 5' end of the antisense strand and the 3' end of the sense strand, or vice versa, or double blunt-ended, which is a double-stranded dsRNA over its entire length, i.e., there is no nucleotide protruding end at either end of the molecule.

In some embodiments, the sense strand or antisense strand of the dsRNA has a nucleotide protruding end at the 3' end, the protruding end contains 1, 2, 3 or 4 nucleotides, and the 5' end is blunt.

In some embodiments, the protruding end is present at the 3' ends of both the sense strand and the antisense strand, and the protruding end contains 1, 2, 3, or 4 nucleotides.

In some embodiments, the dsRNA is a double blunt-ended dsRNA of 19, 21, or 23 nucleotides in length, which is double-stranded dsRNA over its entire length, i.e., there is no nucleotide protruding end at either end of the molecule.

In some embodiments, the dsRNA is 21 nucleotides in length, and both the sense and antisense strands have a protruding end of 2 nucleotides at the 3' end.

In order to enhance the in vivo stability of the RNA inhibitor of the present application, the sense strand and antisense strand of the RNA inhibitor may be modified without affecting its activity or even enhancing its activity, the nucleotides therein can have modification groups, and the entire strand or part of the strand can be modified. In some embodiments, one or more nucleotides on the sense strand and/or antisense strand are modified to form modified nucleotides.

In some embodiments, the sense strand and antisense strand of the RNA inhibitor of the present application (e.g., dsRNA) are unmodified. In other embodiments, the sense strand and antisense strand of the RNA inhibitor of the present application are chemically modified or coupled as known in the art and as described herein to enhance stability or other favorable features. In other embodiments of the present application, all nucleotides or substantially all nucleotides of the RNA inhibitor of the present application may be modified, i.e., no more than 5, 4, 3, 2, or 1 unmodified nucleotides are present in the strands of the RNA inhibitor.

The sense strand and antisense strand of the RNA inhibitor of the present application can be synthesized and/or modified by methods known in the art, such as those described in "Current protocols in nucleic acid chemistry", Beaucage, S.L. et al. (eds.), John Wiley & Sons, Inc., New York, NY, USA, which is incorporated by reference into the present application. Modifications include, for example, terminal modifications, such as 5'-end modifications (phosphorylation, coupling, reverse connection) or 3'-end modifications (coupling, DNA nucleotides, reverse connection, etc.); base modifications, such as the use of stabilized bases, destabilized bases or removal of bases (abasic nucleotides) or coupling bases; sugar modifications (e.g., 2'-position or 4'-position) or sugar substitutions; or backbone modifications, including modifications or replacements of phosphodiester linkages. In the RNA inhibitors provided in the present application, both the sense strand and antisense strand of the RNA inhibitor do not need to be uniformly modified, and one or more modifications can be incorporated into their individual nucleotides.

In some embodiments, the modified nucleotides are selected from: deoxyribonucleotides, nucleotide mimetics, abasic nucleotides, 2'-modified nucleotides, 3' to 3' linkage (inverted) nucleotides, nucleotides containing unnatural bases, bridged nucleotides, peptide nucleic acids (PNA), unlocked nucleobase analogues, locked nucleotides, 3'-O-methoxy (2' inter-nucleoside linkage) nucleotides, 2'-F-arabinose nucleotides, 5'-Me / 2'- Fluoro- nucleotides, morpholino nucleotides, vinyl phosphonate deoxyribonucleotides, nucleotides containing vinyl phosphonate and nucleotides containing cyclopropyl phosphonate.

In some embodiments, the 2 ' -modified nucleotides include: 2'-O-methyl nucleotide, 2'-deoxy-2'-fluoronucleotide, 2'-deoxynucleotide, 2'-methoxyethyl nucleotide, 2'-amino nucleotide and/or 2'-alkyl nucleotide.

In some embodiments, the 2' positions of at least two or more nucleotide sugar groups at even positions starting from the 5' end of the antisense strand are fluorine.

In some embodiments, the 2' positions of all nucleotide sugar groups at even positions starting from the 5' end of the antisense strand are fluorine.

In some embodiments, at least one of the 2' positions of nucleotide sugar groups at positions 2, 4, 6, 8, 12 and 14 starting from the 5' end of the antisense strand are fluorine. For example, the 2' positions of the nucleotide sugar groups at positions 2, 4, 6, 8, 12 and 14 starting from the 5' end of the antisense strand are all fluorine.

In some embodiments, except for nucleotides at positions 2, 6, 8, 10, 14 and 16 starting from the 5' end of the antisense strand, at least one of the 2' positions of the remaining nucleotide sugar groups are methoxy group.

In some embodiments, except for nucleotides at positions 2, 4, 6, 8, 14 and 16 starting from the 5' end of the antisense strand, at least one of the 2' positions of the remaining nucleotide sugar groups are methoxy group.

In some embodiments, except for nucleotides at positions 2, 4, 6, 8, 14, 16, 18 and 20 starting from the 5' end of the antisense strand, at least one of the 2' positions of the remaining nucleotide sugar groups are methoxy group.

In some embodiments, the 2' positions of at least two or more nucleotide sugar groups at odd positions starting from the 5' end of the sense strand are fluorine.

In some embodiments, the 2' positions of all nucleotide sugar groups at odd positions starting from the 5' end of the sense strand are fluorine.

In some embodiments, at least one of the 2' positions of nucleotide sugar groups at positions 5, 7, 8 and 9 starting from the 5' end of the sense strand are fluorine. For example, the 2' positions of the nucleotide sugar groups at positions 5, 7, 8 and 9 starting from the 5' end of the sense strand are all fluorine.

In some embodiments, except for nucleotides at positions 5, 7, 8 and 9 starting from the 5' end of the sense strand, at least one of the 2' positions of the remaining nucleotide sugar groups are methoxy group.

In some embodiments, at least one of the 2' positions of nucleotide sugar groups at positions 7, 9, 10 and 11 starting from the 5' end of the sense strand are fluorine. For example, the 2' positions of the nucleotide sugar groups at positions 7, 9, 10 and 11 starting from the 5' end of the sense strand are all fluorine.

In some embodiments, except for nucleotides at positions 7, 9, 10 and 11 starting from the 5' end of the sense strand, at least one of the 2' positions of the remaining nucleotide sugar groups are methoxy group.

In some embodiments, at least one of the 2' positions of nucleotide sugar groups at positions 3, 5, 7, 9, 10, 11, 13 and 15 starting from the 5' end of the sense strand are fluorine. For example, the 2' positions of the nucleotide sugar groups at positions 3, 5, 7, 9, 10, 11, 13 and 15 starting from the 5' end of the sense strand are all fluorine.

In some embodiments, except for nucleotides at positions 3, 5, 7, 9, 10, 11, 13 and 15 starting from the 5' end of the sense strand, at least one of the 2' positions of the remaining nucleotide sugar groups are methoxy group.

In some embodiments, except for nucleotides at positions 7, 8, 9 and 10 starting from the 5' end of the sense strand, at least one of the 2' positions of the remaining nucleotide sugar groups are methoxy group.

For example, the -OH at the 2' position of part or all of the nucleotide sugar groups of the sense strand and/or antisense strand can be substituted, wherein the substituent is fluorine or methoxy, preferably the 2' positions of nucleotide sugar groups at positions 9, 10 and 11 starting from the 5' end of the sense strand are fluorine, and the 2' positions of nucleotide sugar groups at positions 2, 4, 6, 12, 14, 16, 18 and 20 starting from the 5' end of the antisense strand are fluorine, and the 2' positions of the remaining nucleotide sugar groups are all methoxy group; or preferably the 2' positions of nucleotide sugar groups at positions 5, 7, 8 and 9 starting from the 5' end of the sense strand are fluorine, and the 2' positions of nucleotide sugar groups at positions 2, 4, 8, 14 and 16 starting from the 5' end of the antisense strand are fluorine, and the 2' positions of the remaining nucleotide sugar groups are all methoxy group.

In some embodiments, there are at least two consecutive thiophosphate bonds among nucleotides of the sense strand and/or antisense strand.

In some embodiments, there are at least two consecutive thiophosphate bonds among 3 consecutive nucleotides at at least one of the ends of the sense strand and/or antisense strand.

For example, there are at least two consecutive thiophosphate bonds among 3 consecutive nucleotides at the 5' and 3' ends of the sense strand and antisense strand.

For another example, the 2' positions of nucleotide sugar groups at positions 9, 10 and 11 starting from the 5' end of the sense strand are fluorine, and the 2' positions of nucleotide sugar groups at positions 2, 4, 6, 12, 14, 16, 18 and 20 starting from the 5' end of the antisense strand are fluorine, the 2' positions of the remaining nucleotide sugar groups are all methoxy group, and there are at least two consecutive thiophosphate bonds among 3 consecutive nucleotides at the 5' and 3' ends of the sense strand and antisense strand.

In some embodiments, the 2' position of some nucleotides in the sense strand are fluorine or methoxy, and the phosphate bonds among at least 3 adjacent nucleotides at the end of the antisense strand can be thiolated. The 2' positions of nucleotide sugar groups at positions 5, 7, 8 and 9 or positions 3, 5, 7, 8, 9, 11, 13 and 15 starting from the 5' end of the sense strand are fluorine, the 2' positions of the remaining nucleotide sugar groups are methoxy group, and the phosphate bonds among at least 3 adjacent nucleotides at the end of the antisense strand can be thiolated.

In some embodiments, the 2' position of some nucleotides in the sense strand are fluorine or methoxy, and the phosphate bonds among at least 3 adjacent nucleotides at the end of the antisense strand can be thiolated. The 2' positions of nucleotide sugar groups at positions 9, 10 and 11 or positions 3, 5, 7, 8, 9, 11, 13, 15 and/or 17 starting from the 5' end of the sense strand are fluorine, the 2' positions of the remaining nucleotide sugar groups are methoxy group, and the phosphate bonds among at least 3 adjacent nucleotides at the end of the antisense strand can be thiolated.

In some embodiments, the sense strand and antisense strand in the RNA inhibitors of the present application are selected from Table 4 below:

**Table 4 RNA inhibitors with modified sequences**

| Doub le stran ds code | SEQ ID NO. | Sense strand sequence 5'-3' | SEQ ID NO. | Antisense strand sequence 5'-3' |
|---|---|---|---|---|
| Kylo-11-DS29 | 217 | gaCaUUUgUUaaaaaUaaa | 243 | uuUauuuuUaaCaaaUguCau |
| Kylo-11-DS30 | 218 | gaaUgaUgagaaaUaaUUa | 244 | UaauUauuucuCauCauuccc |
| Kylo-11-DS31 | 219 | gggCaggUCUggaaaaaaa | 245 | uuuuuuucCagaccUgccCau |
| Kylo-11-DS32 | 220 | ggUUCUUCUaCUUCUU UUa | 246 | UaaaagaagUagaagaacCac |
| Kylo-11-DS33 | 221 | ggaCaaaUaCaUUUUaCaa | 247 | uUgUaaaaUgUauuUguccuu |
| Kylo-11-DS34 | 222 | ggaggaUCCCaUUaUaCUa | 248 | UagUaUaaUgggauccuccga |
| Kylo-11-DS35 | 223 | CCUUgUUaUUgagaaUgaa | 249 | uuCauucuCaaUaacaaggag |
| Kylo-11-DS36 | 224 | gaaggaCaaaUaCaUUUUa | 250 | UaaaaUgUauuUguccuucuc |
| Kylo-11-DS37 | 225 | gCCCCUUggUgUUaUaCa a | 251 | uUgUaUaaCacCaaggggcUg |
| Kylo-11-DS38 | 226 | CgUUCaUUCCagggaCaaa | 252 | uuUgucccUggaaugaacgUg |
| Kylo-11-DS39 | 227 | gCUCCUUaUUgUUaUaC ga | 253 | ucgUaUaaCaaUaaggagcug |
| Kylo-11-DS40 | 228 | CgUgUUUCaaggUUUgU Ua | 254 | UaaCaaaccuUgaaaCacgag |
| Kylo-11-DS41 | 229 | UsGsGUfAAfUfGfGACA GAGUUAUsUsU | 255 | AsUsAACUfCUGUCfCAf UUACCAsUsU |
| Kylo-11-DS42 | 230 | AsCsAGfAGfUfUfAUCG AGGCUCAsTsT | 256 | UsGsAGCCfUCGAUfAAf CUCUGUsUsU |
| Kylo-11-DS43 | 231 | CsAsUGfGUfAfAfUGGA CAGAGUUsAsU | 257 | AsAsCUCUfGUCCAfUUf ACCAUGsUsU |
| Kylo-11-DS44 | 232 | GsGsACfAGfAfGfUUAU CGAGGCUsCsA | 258 | AsGsCCUCfGAUAAfCUf CUGUCCsUsU |
| Kylo-11-DS45 | 233 | GsUsAAfUGfGfAfCAGA GUUAUCGsAsU | 259 | CsGsAUAAfCUCUGfUCf CAUUACsCsA |
| Kylo-11-DS46 | 234 | AsGsAGfUUfAfUfCGAG GCUCAUUsAsU | 260 | AsAsUGAGfCCUCGfAUf AACUCUsGsU |
| Kylo-11-DS47 | 235 | GsCsCCfCUfUfGfGUGU UAUACsAsA | 261 | UsUsGUAUfAACACfCAf AGGGGCsUsG |
| Kylo-11-DS48 | 236 | GsCsUCfCUfUfAfUUGU UAUACsGsA | 262 | UsCsGUAUfAACAAfUAf AGGAGCsUsG |
| Kylo-11-DS49 | 229 | UsGsGUfAAfUfGfGACA GAGUUAUsUsU | 263 | UsUsAACUfCUGUCfCAf UUACCAsUsU |
| Kylo-11-DS50 | 229 | UsGsGUfAAfUfGfGACA GAGUUAUsUsU | 264 | UsAsUAACfUCUGUfCCf AUUACCAsUsU |
| Kylo-11-DS51 | 237 | UsGsGUfAAfUfGfGACA GAGUUsAsU | 255 | AsUsAACUfCUGUCfCAf UUACCAsUsU |
| Kylo-11-DS52 | 229 | UsGsGUfAAfUfGfGACA GAGUUAUsUsU | 265 | AsUsAACUfCUGUCfCAf UUACCAsUsG |
| Kylo-11-DS53 | 229 | UsGsGUfAAfUfGfGACA GAGUUAUsUsU | 266 | AsfUsAfACfUCfUGUCCA fUUfACCAsUsU |
| Kylo-11-DS54 | 238 | UsGsGUfAAfUfGfGACA GAGUUAUsCsG | 255 | AsUsAACUfCUGUCfCAf UUACCAsUsU |
| Kylo-11-DS55 | 239 | UsGsGUfAAfUfGfGACA GAGUUAUsCsA | 255 | AsUsAACUfCUGUCfCAf UUACCAsUsU |
| Kylo-11-DS56 | 240 | CsGsGUfAAfUfGfGACA GAGUUAUsCsA | 255 | AsUsAACUfCUGUCfCAf UUACCAsUsU |
| Kylo-11-DS57 | 229 | UsGsGUfAAfUfGfGACA GAGUUAUsUsU | 267 | AsUsAACdTfCUGUCfCAf UUACCAsUsU |
| Kylo-11-DS58 | 241 | UsGsfGUfAAfUfGfGAfC AfGAfGUUAUsUsU | 266 | AsfUsAfACfUCfUGUCCA fUUfACCAsUsU |
| Kylo-11-DS59 | 242 | UsGsfGUfAAfUfGfGAfC AfGAfGUUAUsCsA | 268 | AsfUsAfACfUCfUGUCCA fUUfACCAsUsG |

| | | | | |
|---|---|---|---|---|
| wherein, G = 2'-O-methylguanylate, A = 2'-O-methyladenylate, U = 2'-O-methyluridylate, C = 2'-O-methylcytidylate; Gs = 2'-O-methyl-3'-thioguanylate, As = 2'-O-methyl-3'-thioadenylate, Us = 2'-O-methyl-3'-thiouridylate, Cs = 2'-O-methyl-3'-thiocytidylate; fG =2'-fluoroguanylate, fA=2'-fluoroadenylate, fU=2'-fluorouridylate, fC=2'-fluorocytidylate; fGs=2'-fluoro-3'-thioguanylate, fAs=2'-fluoro-3'-thioadenylate, fUs=2'-fluoro-3'-thiouridylate, fCs=2'-fluoro-3'-thiocytidylate, T=2'-deoxy-thymidylate, dT=2'-deoxy-thymidylate. | | | | |

In some embodiments, the sense strand and antisense strand in the RNA inhibitors of the present application are selected from Table 5 below:

**Table 5 RNA inhibitors with modified sequences**

| Doub le stran ds code | SEQ ID NO. | Sense strand sequence 5'-3' | SEQ ID NO. | Antisense strand sequence 5'-3' |
|---|---|---|---|---|
| Kylo-11-DS60 | 229 | UsGsGUfAAfUfGfGAC AGAGUUAUsUsU | 255 | AsUsAACUfCUGUCfC AfUUACCAsUsU |
| Kylo-11-DS61 | 229 | UsGsGUfAAfUfGfGAC AGAGUUAUsUsU | 263 | UsUsAACUfCUGUCfC AfUUACCAsUsU |
| Kylo-11-DS62 | 229 | UsGsGUfAAfUfGfGAC AGAGUUAUsUsU | 264 | UsAsUAACfUCUGUfC CfAUUACCAsUsU |
| Kylo-11-DS63 | 237 | UsGsGUfAAfUfGfGAC AGAGUUsAsU | 255 | AsUsAACUfCUGUCfC AfUUACCAsUsU |
| Kylo-11-DS64 | 229 | UsGsGUfAAfUfGfGAC AGAGUUAUsUsU | 265 | AsUsAACUfCUGUCfC AfUUACCAsUsG |
| Kylo-11-DS65 | 229 | UsGsGUfAAfUfGfGAC AGAGUUAUsUsU | 266 | AsfUsAfACfUCfUGUC CAfUUfACCAsUsU |
| Kylo-11-DS66 | 231 | CsAsUGfGUfAfAfUGG ACAGAGUUsAsU | 276 | AsAsCUCUfGUCCAfU UfACCAUGsGsU |
| Kylo-11-DS67 | 238 | UsGsGUfAAfUfGfGAC AGAGUUAUsCsG | 255 | AsUsAACUfCUGUCfC AfUUACCAsUsU |
| Kylo-11-DS68 | 239 | UsGsGUfAAfUfGfGAC AGAGUUAUsCsA | 255 | AsUsAACUfCUGUCfC AfUUACCAsUsU |
| Kylo-11-DS69 | 240 | CsGsGUfAAfUfGfGACA GAGUUAUsCsA | 255 | AsUsAACUfCUGUCfC AfUUACCAsUsU |
| Kylo-11-DS70 | 229 | UsGsGUfAAfUfGfGAC AGAGUUAUsUsU | 267 | AsUsAACdTfCUGUCf CAfUUACCAsUsU |
| Kylo-11-DS71 | 241 | UsGsfGUfAAfUfGfGAfC AfGAfGUUAUsUsU | 266 | AsfUsAfACfUCfUGUC CAfUUfACCAsUsU |
| Kylo-11-DS72 | 242 | UsGsfGUfAAfUfGfGAfC AfGAfGUUAUsCsA | 268 | AsfUsAfACfUCfUGUC CAfUUfACCAsUsG |
| Kylo-11-DS73 | 269 | CsAsGCCCCUfUfAfUU GUUAUACsGsA | 277 | UsfCsGfUAfUAACAAf UAfAGfGGfGCsfUsG |
| Kylo-11-DS74 | 270 | CsAsGCUCCUfUfAfUU GUUAUACsGsA | 278 | UsfCsGfUAfUAACAAf UAfAGfGAfGCsfUsG |
| Kylo-11-DS75 | 271 | CsAsGCfUCfCfUfUAUU GUUAUACsGsA | 262 | UsCsGUAUfAACAAfU AfAGGAGCsUsG |
| Kylo-11-DS76 | 272 | CsAsfGCfUCfCfUfUAfU UfGUfUAUACsGsA | 279 | UsfCsGfUAfUAfACAA UAfAGfGAGCsUsG |
| Kylo-11-DS77 | 273 | GsCsCCCUUAUUGUUA UACsGsA | 280 | UsfCsGfUAfUAfACAA UAfAGfGGGCsUsG |
| Kylo-11-DS78 | 269 | CsAsGCCCCUfUfAfUU GUUAUACsGsA | 281 | UsfCsGfUAfUAfACAA UAfAGfGGGCUGsGsA |
| Kylo-11-DS79 | 274 | CsAsGCCCCUfUfAfUU GUUAUACGAsGsA | 281 | UsfCsGfUAfUAfACAA UAfAGfGGGCUGsGsA |
| Kylo-11-DS80 | 275 | UsCsCAGCCCCUfUfAf UUGUUAUACsGsA | 281 | UsfCsGfUAfUAfACAA UAfAGfGGGCUGsGsA |

| | | | | |
|---|---|---|---|---|
| wherein G = 2'-O-methylguanylate, A = 2'-O-methyladenylate, U = 2'-O-methyluridylate, C = 2'-O-methylcytidylate; Gs = 2'-O-methyl-3'-thioguanylate, As = 2'-O-methyl-3'-thioadenylate, Us = 2'-O-methyl-3'-thiouridylate, Cs = 2'-O-methyl-3'-thiocytidylate, fG = 2'-fluoroguanylate, fA = 2'-fluoroadenylate, fU = 2'-fluorouridylate, fC = 2'-fluorocytidylate; fGs = 2'-fluoro-3'-thioguanylate, fAs = 2'-fluoro-3'-thioadenylate, fUs = 2'-fluoro-3'-thiouridylate, fCs = 2'-fluoro-3'-thiocytidylate, dT = 2'-deoxy-thymidylate. | | | | |

In some embodiments, the sense strand and antisense strand in the RNA inhibitors of the present application are selected from Table 6 below:

**Table 6 RNA inhibitors with modified sequences**

| Double strands code | SEQ ID NO. | Sense strand sequence 5'-3' | SEQ ID NO. | Antisense strand sequence 5'-3' |
|---|---|---|---|---|
| Kylo-11-DS81 | 282 | UsGsfGUfAAfUfGfGACA GAGUUAUsCsG | 268 | AsfUsAfACfUCfUGUCC AfUUfACCAsUsG |
| Kylo-11-DS82 | 283 | UsGsfGUfAAfUfGfGACA GAGUUAUsCsA | 301 | AsfUsAfACfUCfUGUCC AfUUfACCAsUsA |
| Kylo-11-DS83 | 284 | AsUsfGGfUAfAfUfGGAC AGAGUUAsUsC | 302 | GsfAsUfAAfCUfCUGUC CfAUfUACCsAsU |
| Kylo-11-DS84 | 285 | AsUsfGGfUAAUfGfGfAC AGAGUUAsUsC | 303 | GsfAsUfAAfCUfCUGUC CfAUfUAfCCsfAsU |
| Kylo-11-DS85 | 286 | AsUsGGUAAUfGfGfACA GAGUUAsUsC | 304 | GsAsUfAAfCUfCUGUCC fAUfUACCsAsU |
| Kylo-11-DS86 | 286 | AsUsGGUAAUfGfGfACA GAGUUAsUsC | 305 | GsAsUAACfUCUGUfCCf AUUACCsAsU |
| Kylo-11-DS87 | 287 | UsGsGUAAfUfGfGfACAG AfGUUAUsCsG | 306 | AsfUsAfACfUCfUGUCfC AfUUACCAsUsA |
| Kylo-11-DS88 | 288 | UsGsGUfAAUfGfGfACAG AGUUAUsCsG | 307 | AsfUsAACUCUGUCCAf UUfACCAsUsA |
| Kylo-11-DS89 | 288 | UsGsGUfAAUfGfGfACAG AGUUAUsCsG | 308 | AsfUsAACUCUGUCfCA UUfACCAsUsG |
| Kylo-11-DS90 | 289 | UsGsGUAAfUGfGfAfCAG AGUUAUsCsA | 309 | AsUsAfACUCUGUCfCAf UUACCAsUsG |
| Kylo-11-DS91 | 290 | UsGsGUAAUfGfGfACAG AGUUAUsCsA | 310 | AsfUsAfACUCUGUCfCA fUUACCAsUsG |
| Kylo-11-DS92 | 291 | UsGsGUAAfUfGfGfACAG AGUUAUsCsG | 311 | AsfUsAAfCUCUGUCCAf UUfACCAsUsA |
| Kylo-11-DS93 | 292 | UsGsGUAAUGfGfAfCAG AGUUAUsCsG | 312 | AsfUsAfACTfCUGUCCA fUUfACCAsUsA |
| Kylo-11-DS94 | 239 | UsGsGUfAAfUfGfGACAG AGUUAUsCsA | 313 | AsfUsAfACfUCfUGfUCf CAfUUfACCAsUsA |
| Kylo-11-DS95 | 293 | UsfGsGfUAAfUGGfACAG fAGUUAfUsCsA | 314 | AsUsAACUCUGUCCAU UACCAsUsT |
| Kylo-11-DS96 | 294 | UsGsGUAAfUGfGfAfCAG AGUUAUsUsU | 315 | AsfUsAACUCUGUCCAf UUACCAsUsU |
| Kylo-11-DS97 | 295 | UsGsGUfAAfUfGfGACAG AGUUAsCsG | 316 | AsUsAACUfCUGUCfCAf UUACCAsUsA |
| Kylo-11-DS98 | 296 | UsGsGUAAfUGfGfAfCAG AGUUsAsU | 315 | AsfUsAACUCUGUCCAf UUACCAsUsU |
| Kylo-11-DS99 | 296 | UsGsGUAAfUGfGfAfCAG AGUUsAsU | 317 | AsfUsAACUCUGUCCAf UUACCAsUsG |
| Kylo-11-DS10 0 | 297 | UsGsGUAAfUGfGfAfCAG AGUUAUsUsG | 318 | CsfAsAUAACUCUGUCf CAUUACCAsUsU |
| Kylo-11-DS10 1 | 298 | CsAsUGGUfAAfUfGfGAC AGAGUUsAsU | 319 | AsfUsAACUCUGUCCAf UUACCAUGsGsU |
| Kylo-11-DS10 2 | 229 | UsGsGUfAAfUfGfGACAG AGUUAUsUsU | 320 | AsfUsAACfUCfUGfUCC AfUUfACCAsUsU |
| Kylo-11-DS10 3 | 299 | UsGsfGUfAAfUfGfGACAf GAfGUUAUsUsU | 266 | AsfUsAfACfUCfUGUCC AfUUfACCAsUsU |
| Kylo-11-DS10 4 | 300 | UsGsGUAAUGfGfAfCAG AGUUAUsUsU | 321 | AsUsAACUCUGUCCAfU UfACCAsUsU |
| Kylo-11-DS10 5 | 229 | UsGsGUfAAfUfGfGACAG AGUUAUsUsU | 322 | AsfUsAACUCUGUCfCA UUfACCAsUsU |

| | | | | |
|---|---|---|---|---|
| wherein, G = 2'-O-methylguanylate, A = 2'-O-methyladenylate, U = 2'-O-methyluridylate, C = 2'-O-methylcytidylate; Gs = 2'-O-methyl-3'-thioguanylate, As = 2'-O-methyl-3'-thioadenylate, Us = 2'-O-methyl-3'-thiouridylate, Cs = 2'-O-methyl-3'-thiocytidylate; fG =2'-fluoroguanylate, fA=2'-fluoroadenylate, fU=2'-fluorouridylate, fC=2'-fluorocytidylate; fGs=2'-fluoro-3'-thioguanylate, fAs=2'-fluoro-3'-thioadenylate, fUs=2'-fluoro-3'-thiouridylate, fCs=2'-fluoro-3'-thiocytidylate, T=2'-deoxy-thymidylate, dT=2'-deoxy-thymidylate. | | | | |

In some embodiments, the sense strand and antisense strand in the RNA inhibitors of the present application are selected from Table 7 below:

**Table 7 RNA inhibitors with modified sequences**

| Double strands code | SEQ ID NO. | Sense strand sequence 5'-3' | SEQ ID NO. | Antisense strand sequence 5'-3' |
|---|---|---|---|---|
| Kylo-11-DS10 6 | 241 | UsGsfGUfAAfUfGfGAfC AfGAfGUUAUsUsU | 266 | AsfUsAfACfUCfUGUCCA fUUfACCAsUsU |
| Kylo-11-DS10 7 | 241 | UsGsfGUfAAfUfGfGAfC AfGAfGUUAUsUsU | 330 | UsfUsAfACfUCfUGUCCA fUUfACCAsUsU |
| Kylo-11-DS10 8 | 241 | UsGsfGUfAAfUfGfGAfC AfGAfGUUAUsUsU | 331 | UsfAsUfAAfCUfCUGUCC fAUfUACCAsUsU |
| Kylo-11-DS10 9 | 323 | UsGsfGUfAAfUfGfGAfC AfGAfGUUsAsU | 266 | AsfUsAfACfUCfUGUCCA fUUfACCAsUsU |
| Kylo-11-DS11 0 | 324 | UsGsfGUfAAfUGfGfAfC AfGAfGUUAUsUsU | 268 | AsfUsAfACfUCfUGUCCA fUUfACCAsUsG |
| Kylo-11-DS11 1 | 325 | CsAsfUGfGUfAfAfUGfG AfCAfGAGUUsAsU | 332 | AsfAsCfUCfUGfUCCAUU fACfCAUGsGsU |
| Kylo-11-DS11 2 | 326 | UsGsfGUfAAfUfGfGAfC AfGAfGUUAUsCsG | 266 | AsfUsAfACfUCfUGUCCA fUUfACCAsUsU |
| Kylo-11-DS11 3 | 242 | UsGsfGUfAAfUfGfGAfC AfGAfGUUAUsCsA | 266 | AsfUsAfACfUCfUGUCCA fUUfACCAsUsU |
| Kylo-11-DS11 4 | 327 | CsGsfGUfAAfUfGfGAfC AfGAfGUUAUsCsA | 266 | AsfUsAfACfUCfUGUCCA fUUfACCAsUsU |
| Kylo-11-DS11 5 | 241 | UsGsfGUfAAfUfGfGAfC AfGAfGUUAUsUsU | 333 | AsfUsAfACdTCfUGUCCA fUUfACCAsUsU |
| Kylo-11-DS11 6 | 242 | UsGsfGUfAAfUfGfGAfC AfGAfGUUAUsCsA | 268 | AsfUsAfACfUCfUGUCCA fUUfACCAsUsG |
| Kylo-11-DS11 7 | 239 | UsGsGUfAAfUfGfGACA GAGUUAUsCsA | 268 | AsfUsAfACfUCfUGUCCA fUUfACCAsUsG |
| Kylo-11-DS11 8 | 269 | CsAsGCCCCUfUfAfUUG UUAUACsGsA | 277 | UsfCsGfUAfUAACAAfUA fAGfGGfGCsfUsG |
| Kylo-11-DS11 9 | 270 | CsAsGCUCCUfUfAfUUG UUAUACsGsA | 278 | UsfCsGfUAfUAACAAfUA fAGfGAfGCsfUsG |
| Kylo-11-DS12 0 | 328 | GsCsCCCUUAfUfUfGUU AUACsGsA | 277 | UsfCsGfUAfUAACAAfUA fAGfGGfGCsfUsG |
| Kylo-11-DS12 1 | 269 | CsAsGCCCCUfUfAfUUG UUAUACsGsA | 334 | UsfCsGfUAfUAACAAfUA fAGfGGfGCfUGsGsA |
| Kylo-11-DS12 2 | 274 | CsAsGCCCCUfUfAfUUG UUAUACGAsGsA | 334 | UsfCsGfUAfUAACAAfUA fAGfGGfGCfUGsGsA |
| Kylo-11-DS12 3 | 329 | UsCsCAGCCCfCfUfUAU UGUUAUACsGsA | 334 | UsfCsGfUAfUAACAAfUA fAGfGGfGCfUGsGsA |

| | | | | |
|---|---|---|---|---|
| wherein G = 2'-O-methylguanylate, A = 2'-O-methyladenylate, U = 2'-O-methyluridylate, C = 2'-O-methylcytidylate; Gs = 2'-O-methyl-3'-thioguanylate, As = 2'-O-methyl-3'-thioadenylate, Us = 2'-O-methyl-3'-thiouridylate, Cs = 2'-O-methyl-3'-thiocytidylate, fG = 2'-fluoroguanylate, fA = 2'-fluoroadenylate, fU = 2'-fluorouridylate, fC = 2'-fluorocytidylate; fGs = 2'-fluoro-3'-thioguanylate, fAs = 2'-fluoro-3'-thioadenylate, fUs = 2'-fluoro-3'-thiouridylate, fCs = 2'-fluoro-3'-thiocytidylate, dT = 2'-deoxy-thymidylate. | | | | |

In some embodiments, the sense strand and antisense strand in the RNA inhibitors of the present application are selected from Table 8 below:

**Table 8 RNA inhibitors with modified sequences**

| Doub le stran ds code | SEQ ID NO. | Sense strand sequence 5'-3' | SEQ ID NO. | Antisense strand sequence 5'-3' |
|---|---|---|---|---|
| Kylo-11-DS-124 | 335 | GsCsUCCUUAfUfUfGUU AUACsGsA | 277 | UsfCsGfUAfUAACAAfUA fAGfGGfGCsfUsG |
| Kylo-11-DS-125 | 270 | CsAsGCUCCUfUfAfUUG UUAUACsGsA | 334 | UsfCsGfUAfUAACAAfUA fAGfGGfGCfUGsGsA |
| Kylo-11-DS-126 | 336 | CsAsGCUCCUfUfAfUUG UUAUACGAsGsA | 334 | UsfCsGfUAfUAACAAfUA fAGfGGfGCfUGsGsA |
| Kylo-11-DS-127 | 337 | UsCsCAGCUCfCfUfUAU UGUUAUACsGsA | 334 | UsfCsGfUAfUAACAAfUA fAGfGGfGCfUGsGsA |
| Kylo-11-DS-128 | 270 | CsAsGCUCCUfUfAfUUG UUAUACsGsA | 341 | UsfCsGfUAdTAACAAfUA fAGfGAfGCsfUsG |
| Kylo-11-DS-129 | 335 | GsCsUCCUUAfUfUfGUU AUACsGsA | 342 | UsfCsGfUAdTAACAAfUA fAGfGGfGCsfUsG |
| Kylo-11-DS-130 | 270 | CsAsGCUCCUfUfAfUUG UUAUACsGsA | 343 | UsfCsGfUAdTAACAAfUA fAGfGGfGCfUGsGsA |
| Kylo-11-DS-131 | 336 | CsAsGCUCCUfUfAfUUG UUAUACGAsGsA | 343 | UsfCsGfUAdTAACAAfUA fAGfGGfGCfUGsGsA |
| Kylo-11-DS-132 | 337 | UsCsCAGCUCfCfUfUAU UGUUAUACsGsA | 343 | UsfCsGfUAdTAACAAfUA fAGfGGfGCfUGsGsA |
| Kylo-11-DS-133 | 272 | CsAsfGCfUCfCfUfUAfU UfGUfUAUACsGsA | 278 | UsfCsGfUAfUAACAAfUA fAGfGAfGCsfUsG |
| Kylo-11-DS-134 | 338 | GsCsfUCfCUfUfAfUUfG UfUAfUACsGsA | 277 | UsfCsGfUAfUAACAAfUA fAGfGGfGCsfUsG |
| Kylo-11-DS-135 | 272 | CsAsfGCfUCfCfUfUAfU UfGUfUAUACsGsA | 334 | UsfCsGfUAfUAACAAfUA fAGfGGfGCfUGsGsA |
| Kylo-11-DS-136 | 339 | CsAsfGCfUCfCfUfUAfU UfGUfUAUACGAsGsA | 334 | UsfCsGfUAfUAACAAfUA fAGfGGfGCfUGsGsA |
| Kylo-11-DS-137 | 340 | UsCsfCAfGCfUCfCfUfUA fUUfGUUAUACsGsA | 334 | UsfCsGfUAfUAACAAfUA fAGfGGfGCfUGsGsA |
| Kylo-11-DS-138 | 272 | CsAsfGCfUCfCfUfUAfU UfGUfUAUACsGsA | 341 | UsfCsGfUAdTAACAAfUA fAGfGAfGCsfUsG |
| Kylo-11-DS-139 | 338 | GsCsfUCfCUfUfAfUUfG UfUAfUACsGsA | 342 | UsfCsGfUAdTAACAAfUA fAGfGGfGCsfUsG |
| Kylo-11-DS-140 | 272 | CsAsfGCfUCfCfUfUAfU UfGUfUAUACsGsA | 343 | UsfCsGfUAdTAACAAfUA fAGfGGfGCfUGsGsA |
| Kylo-11-DS-141 | 339 | CsAsfGCfUCfCfUfUAfU UfGUfUAUACGAsGsA | 343 | UsfCsGfUAdTAACAAfUA fAGfGGfGCfUGsGsA |
| Kylo-11-DS-142 | 340 | UsCsfCAfGCfUCfCfUfUA fUUfGUUAUACsGsA | 343 | UsfCsGfUAdTAACAAfUA fAGfGGfGCfUGsGsA |

| | | | | |
|---|---|---|---|---|
| wherein G = 2'-O-methylguanylate, A = 2'-O-methyladenylate, U = 2'-O-methyluridylate, C = 2'-O-methylcytidylate; Gs = 2'-O-methyl-3'-thioguanylate, As = 2'-O-methyl-3'-thioadenylate, Us = 2'-O-methyl-3'-thiouridylate, Cs = 2'-O-methyl-3'-thiocytidylate, fG = 2'-fluoroguanylate, fA = 2'-fluoroadenylate, fU = 2'-fluorouridylate, fC = 2'-fluorocytidylate; fGs = 2'-fluoro-3'-thioguanylate, fAs = 2'-fluoro-3'-thioadenylate, fUs = 2'-fluoro-3'-thiouridylate, fCs = 2'-fluoro-3'-thiocytidylate, dT = 2'-deoxy-thymidylate. | | | | |

In some embodiments, the sense strand or antisense strand of the RNA inhibitor of the present application is a sequence having at least 15 consecutive nucleotides identical to those in the sense or antisense strands in Tables 4 to 8, or a sequence that differs from the sense or antisense strands in Tables 4 to 8 by 1, 2 or 3 nucleotides.

In some embodiments, the distribution, targeting or stability of RNA inhibitors is altered by introducing ligands of target tissue receptors into the vector. For example, a specific ligand can provide an enhanced affinity for a selected target (e.g., molecule, cell, or cell type, compartment (e.g., cell or organ compartment, body tissue, organ, or region) compared to species where no ligand is present.

Ligands can include naturally occurring substances, such as proteins (such as human serum albumin (HSA), low-density lipoprotein (LDL), or globulin); carbohydrates (such as glucan, pullulan, chitin, chitosan, inulin, cyclodextrin, N-acetylglucosamine, N-acetylgalactosamine or hyaluronic acid); or lipids. Ligands can also be recombinant or synthetic molecules, such as synthetic polymers, such as synthetic polyamino acids.

Ligands may also include targeting groups, such as cell or tissue targeting agents, such as lectins, glycoproteins, lipids, or proteins, such as antibodies, that bind to a specified cell type such as kidney cells. The targeting groups can be thyrotropin, melanotropin, lectin, glycoprotein, surfactant protein A, mucin carbohydrate, multivalent lactose, multivalent galactose, N-acetyl-galactosamine, N-acetylglucosamine, multivalent mannose, multivalent fucose, sugar groupated polyamino acids, multivalent galactose, transferrin, bisphosphonate, polyglutamic acid, polyaspartic acid, lipid, cholesterol, steroid, cholic acid, folic acid, vitamin B12, vitamin A, biotin, or RGD peptide or RGD peptide mimetics. In some embodiments, the ligand is a multivalent galactose, for example, N-acetyl-galactosamine.

The sense strand and antisense strand contained in the RNA inhibitors of the present application can be conveniently and routinely prepared by the well-known technique of solid-phase synthesis. Additionally or alternatively, any other method known in the art for such synthesis, such as liquid-phase synthesis or fermentation, may be used.

In some embodiments, in addition to commercially available and commonly used standard nucleoside phosphoramidite monomers and non-standard nucleoside phosphoramidite monomers, the sense strand and antisense strand contained in the RNA inhibitor of the present application can be synthesized by an automated synthesizer using phosphoramidite method derived from the support-nucleoside phosphoramidite monomer.

In some embodiments, the ligand of the present application is coupled to the 5' end and/or 3' end of the antisense strand, and/or the 5' end and/or 3' end of the sense strand via a ligand structure.

For example, the ligand structure can be coupled to the 5' end and/or 3' end of the sense strand; or the ligand structure can be coupled to the 5' end of the antisense strand and the ligand structure is coupled to the 3' end of the sense strand; or the ligand structure can be coupled to the 3' end of the antisense strand, and the ligand is coupled to the 5' end of the sense strand.

In some embodiments, the ligand structure includes 5'MVIP and 3'MVIP, wherein the 5'MVIP is conjugated at the 5' end of the sense strand and/or antisense strand, the 3'MVIP is conjugated at the 3' end of the antisense strand and/or sense strand, the structure of the 5'MVIP is shown in formula I, the structure of the 3'MVIP is shown in formula II,

(X-L)ₙ-B-D-R₁-. I

(X-L)ₘ-B-D-R₂-, II

wherein,
X is a liver targeting specific ligand;
L is a branched chain;
B is a linker;
D is a linking chain;
R₁ and R₂ are transition points;

The 5'MVIP is connected with the 5' end of the sense strand or the 5' end of the antisense strand through the transition point R₁, and the 3'MVIP is connected with the 3' end of the sense strand or the 3' end of the antisense strand through the transition point R₂, n and m are each independently any integer from 0 to 4, and n+m is an integer of 2-6, preferably n+m=2, 3 or 4, more preferably 4.

In some embodiments, the R₁ or R₂ is connected to the sense or antisense strand through a phosphate or modified phosphate, preferably the R₁ or R₂ is connected to the sense or antisense strand through a phosphate or thiophosphate.

In some embodiments, m or n can be 0, that is, there is no 3'MVIP or 5'MVIP.

In some embodiments, when n=0 (that is, there is no 5'MVIP), the structure of the 3'MVIP can be:

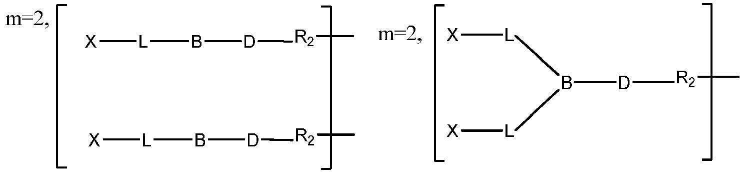

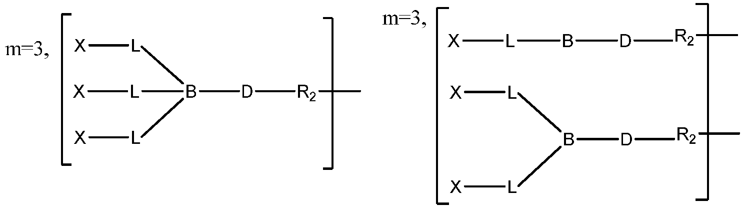

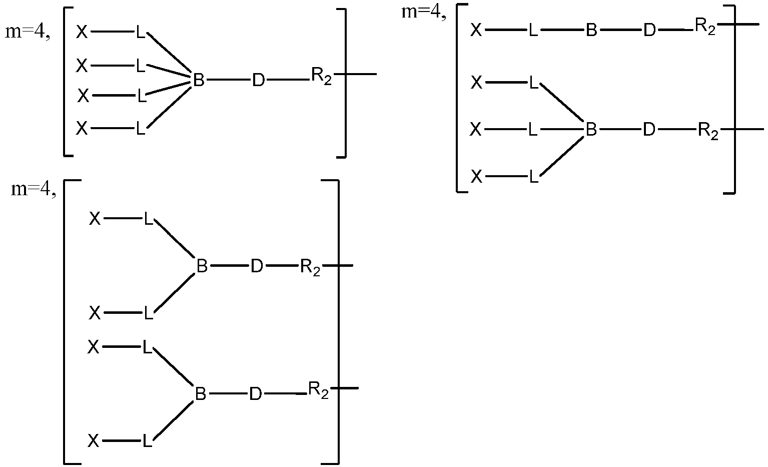

In some embodiments, when n=1, the structure of the 3' MVIP may be:

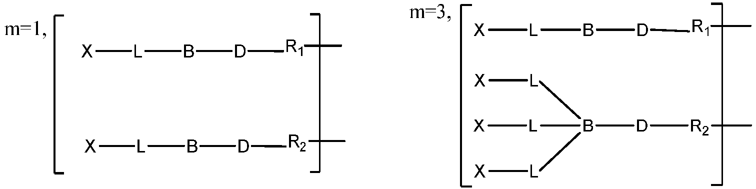

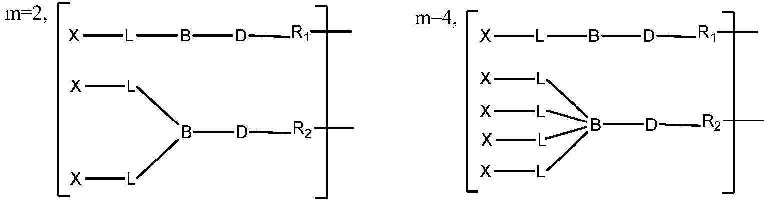

In some embodiments, when n=2, the structure of the 3 ' MVIP may be:

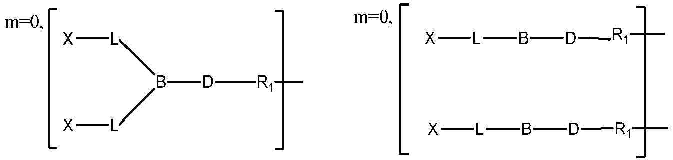

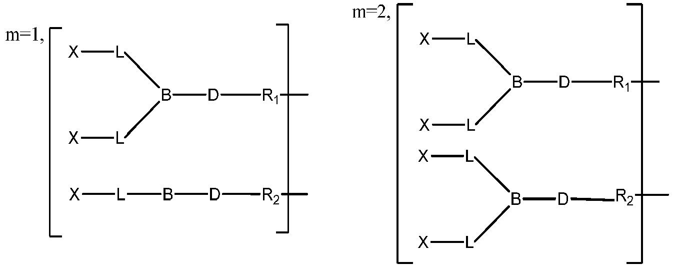

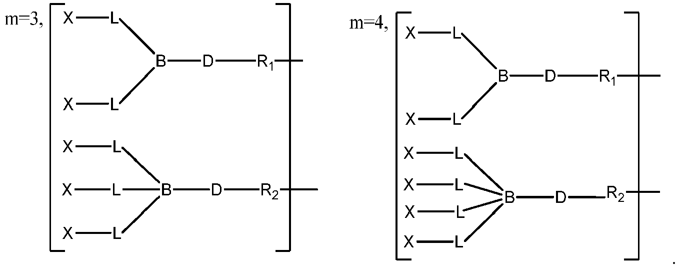

In some embodiments, when n=3, the structure of the 3' MVIP may be:

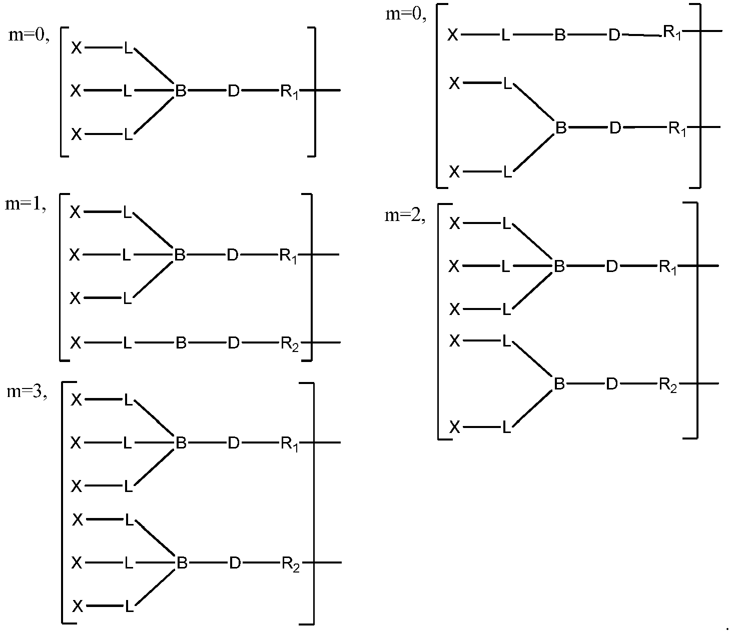

In some embodiments, when n=4, the structure of the 3' MVIP may be:

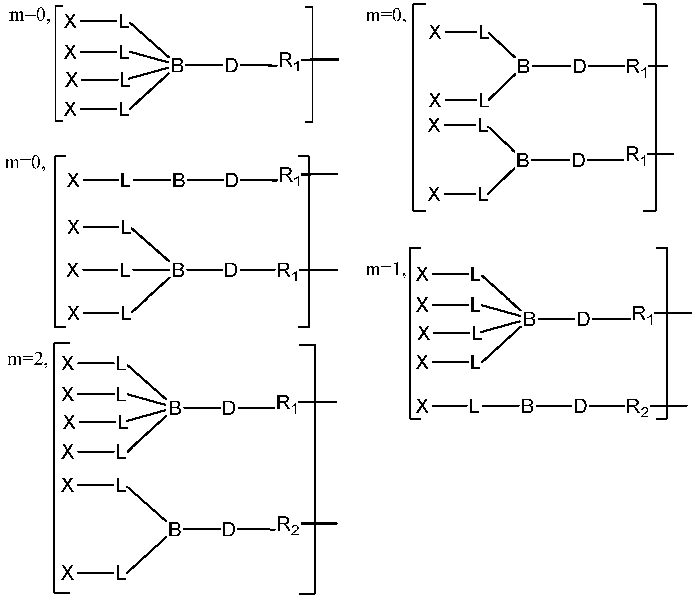

In some embodiments, the n refers to the sum of n in the 5'MVIP at 5' end of the sense strand and antisense strand of the RNA inhibitor at the same time, and the m refers to the sum of m in the 3'MVIP at 3' end of the sense strand and antisense strand of the RNA inhibitor at the same time.

In some embodiments, the R₁ and R₂ contain -NH-, -S-, and/or -O- in their structures, the R₁ and R₂ are respectively connected with the linking chain D and the 5' and 3' ends of the sense strand and/or antisense strand through -NH-, -S-, or -O- in their structures, and the R₁ and R₂ are the same or different.

In some embodiments, the R₁ and R₂ are optionally straight carbon chain, or straight carbon chain or cyclic structure with amido, carboxyl or alkyl branched chain, and the cyclic structure includes saturated or unsaturated aliphatic carbocyclyl, or five or six membered heterocyclyl or aromatic hydrocarbyl containing sulfur, oxygen, or nitrogen atom.

In some embodiments, the R₁ and/or R2 are -E₁(CH₂)ₓCH₂E₂-, wherein x is any integer of 3-12, and the groups E₁ and E₂ can be -NH-, -S- or -O-, respectively.

In some embodiments, the R₁ and/or R₂ are -E₁(CH₂)ₓ₁CH(OH)(CH₂)ₓ₂E₂-, wherein x₁ or x₂ are each independently any integer of 3-10, and E₁ and E₂ can be - NH-, -S- or -O-, respectively.

In some embodiments, the R₁ is a heterocyclic or carbocyclic structure containing N, S or O as shown below:

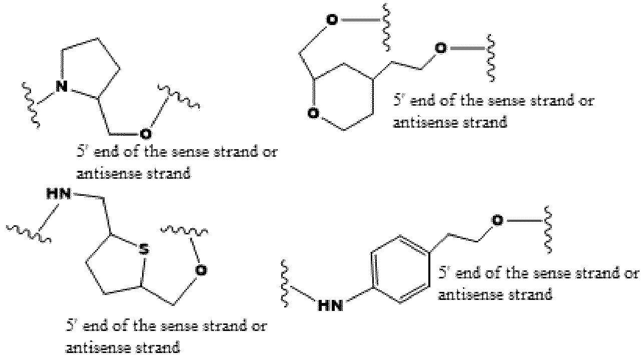

In some embodiments, the transition point R₁ is -NH(CH₂)ₓCH₂O-, wherein x is any integer of 3-12, preferably any integer of 4-6, which can be introduced by the following two phosphoramidite monomers:
i. One -O- or -S- in the R₁ structure is used for the synthesis of R₁ phosphoramidite monomer, and is connected to the 5' end of the sense strand or antisense strand of RNA inhibitor by solid-phase synthesis. In the structure, -NH-, -S-, or -O- is used to connect with the linking chain D in the 5'MVIP, thereby introducing the liver targeting specific ligand X at the 5' end of the sense strand or antisense strand of the RNA inhibitor. An exemplary structure of a monomer introduced into the 5' end of the sense or antisense strand of the RNA inhibitor is as follows: 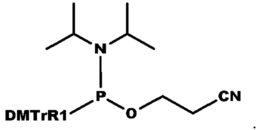 In some embodiments, the following structure is preferred: 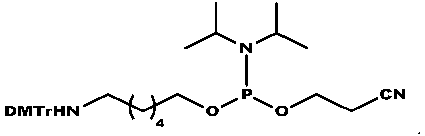
ii. One -NH-, -S- or -O- in R₁ structure is first connected with the linking chain D, and another -NH-, -S-, or -O- is used to form ester with phosphoramidite in the synthesis of 5'MVIP phosphoramidite monomer. The exemplary structure of sense strand or antisense strand 5'MVIP phosphoramidite monomer is as follows:

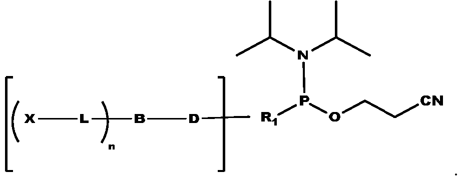

In some embodiments, the sense strand or antisense strand 5'MVIP phosphoramidite monomer preferably has the following structure:

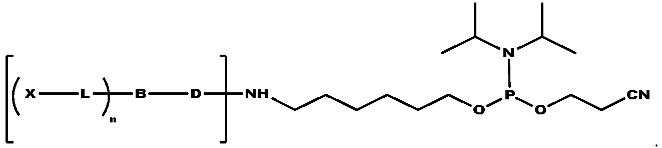

When n in the general formula is 1-4, the linker B part of the above monomer is branched for 1 to 4 times respectively to obtain the corresponding monomer compounds. With the help of the above monomer compounds, the liver targeting specific ligand X is introduced to the 5' end of the sense or antisense strand through solid-phase synthesis.

In some embodiments, the transition point R₁ is -NH(CH₂)ₓCH₂O-, wherein x can be any integer of 3-12, preferably any integer of 4-6.

In some embodiments, the 5'MVIP phosphoramidite monomer has the structure selected from the following structures:

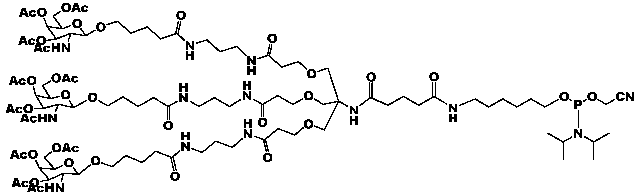

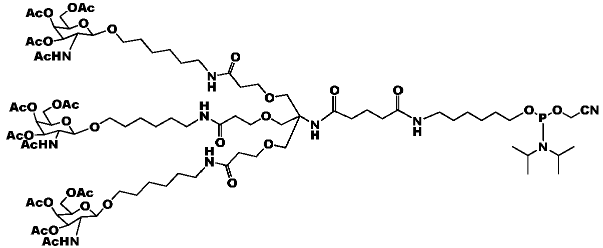

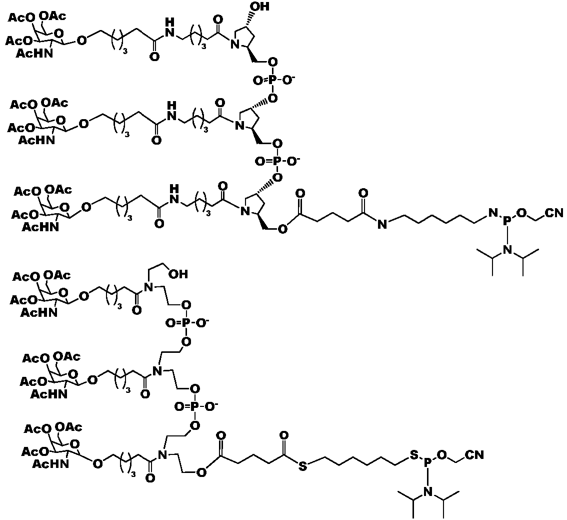

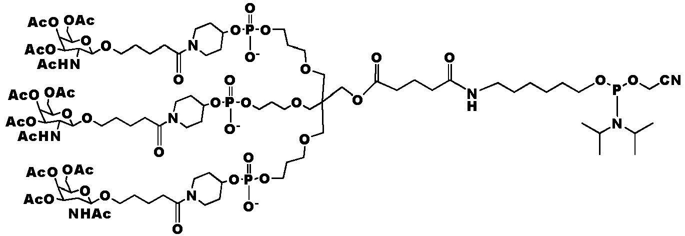

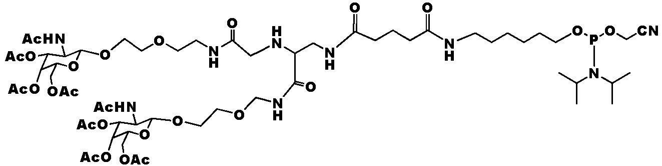

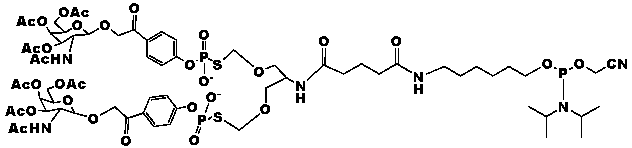

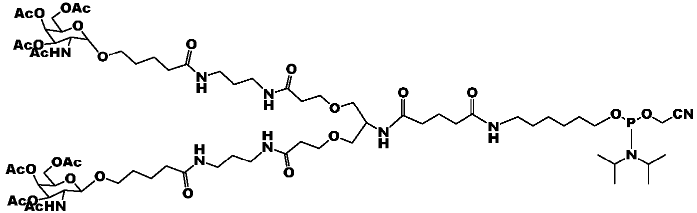

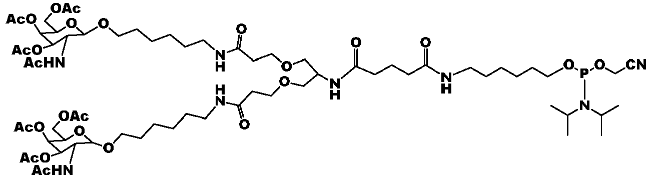

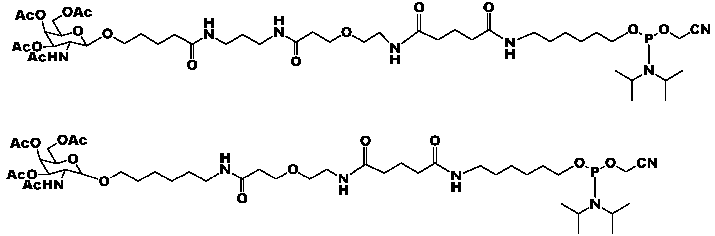

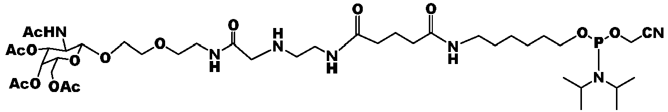

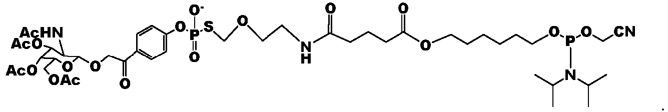

In some embodiments, the transition point R₂ is a heterocyclic or carbocyclic structure containing N, S or O as shown below:

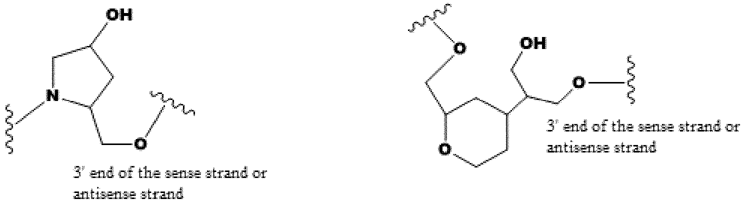

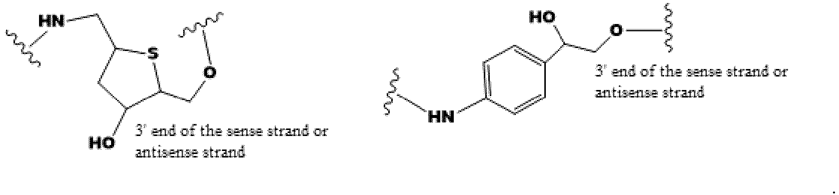

In some embodiments, the transition point R₂ is - NH(CH₂)ₓ₁CH(OH)(CH₂)ₓ₂CH₂O-, wherein x1 is any integer of 1-4, and x2 is any integer of 0-4.

The transition point R₂ described in the present application forms ester or amide with -NH-, -S- or -O- in the R₂ structure through succinic anhydride, and at the same time conjugates with -NH- in blank solid support to form 3'MVIP solid support, and then 3'MVIP is introduced into the 3' end of the sense strand or antisense strand by phosphoramidite solid-phase synthesis.

In some embodiments, the heterocycle in the transition point R₂ structure is a pyrrole ring or a piperidine ring, which is connected with the linking chain D of 3'MVIP through the nitrogen heteroatom in the ring. The exemplary structure of 3'MVIP solid support is as follows:

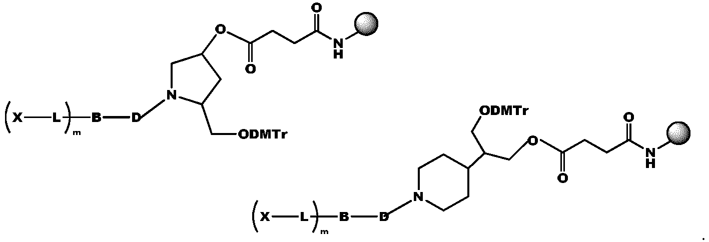

When m in the general formula is 1-4, the linker B part in the monomer above is branched for 1 to 4 times respectively to obtain the corresponding solid supports.

In some embodiments, the transition point R₂ is - B₄(CH₂)ₓ₁CH(OH)(CH₂)ₓ₂CH₂B₅-, wherein x1 is any integer of 1-4, x2 is any integer of 0-4, and B₄ and B₅ are -NH-, -S- or -O-, respectively, the exemplary structure of the introduced 3'MVIP solid support is as follows:

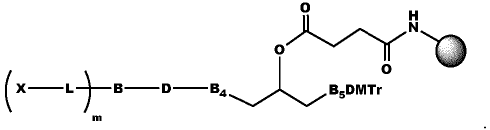

When m in the general formula is 1-4, the linker B part in the monomer above is branched for 1 to 4 times respectively to obtain the corresponding Solid Supports.

In some embodiments, the R₂ is -NHCH₂CH(OH)CH₂O-. The exemplary structure of the introduced 3' MVIP solid support is as follows:

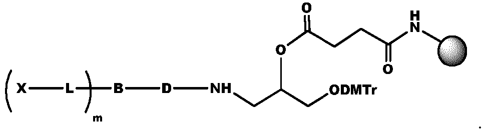

When m in the general formula is 1-4, the linker B part in the monomer above is branched for 1 to 4 times respectively to obtain the corresponding Solid Supports.

In some embodiments, the 3'MVIP solid support has a structure as follows:

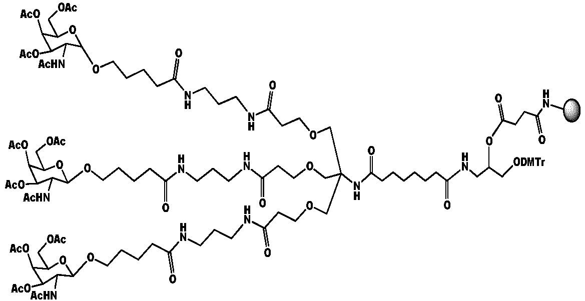

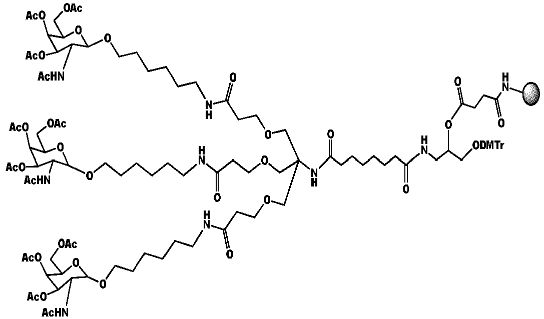

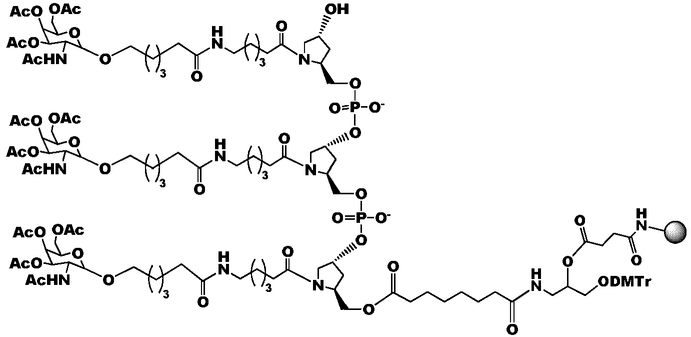

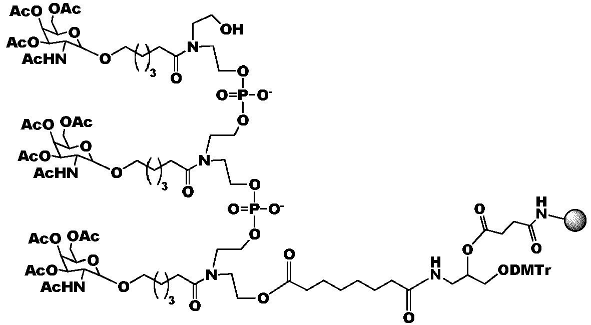

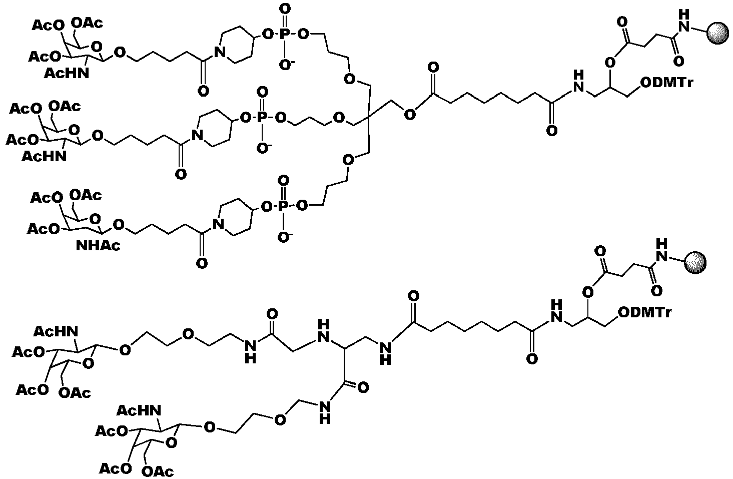

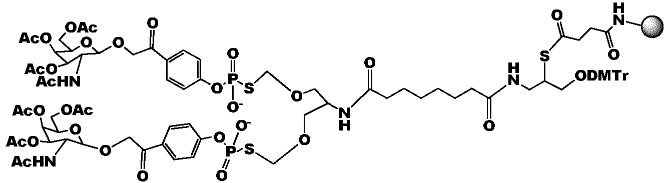

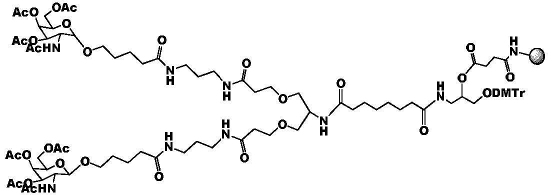

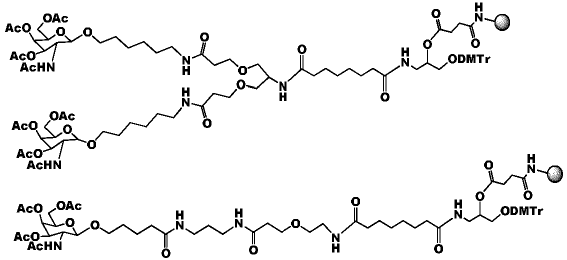

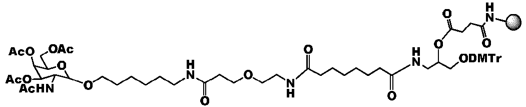

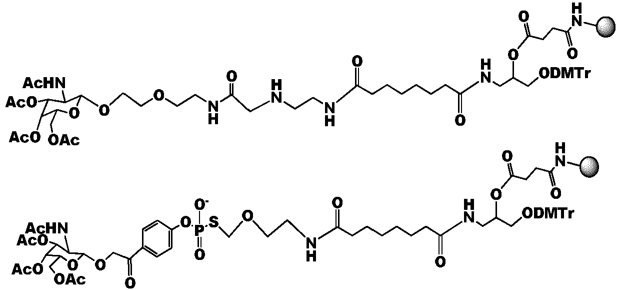

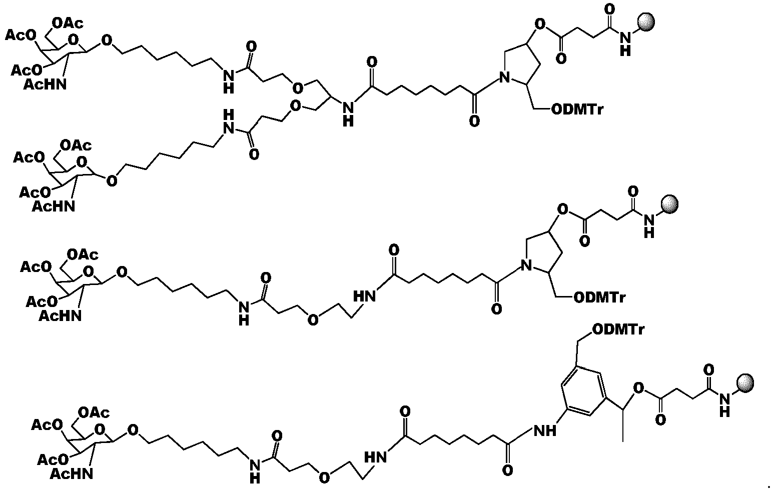

In some embodiments, the liver targeting specific ligand X is selected from structures used to enhance the uptake of RNA inhibitors by hepatocytes, which can be lipids, steroids, vitamins, sugars, proteins, peptides, polyamines and peptide mimetic structures. In the RNA inhibitor provided by the present application, the liver targeting specific ligand X introduced into the end of the sense strand or antisense strand of the RNA inhibitor can be the same or different. For example, in terms of characteristics, some can enhance liver targeting, some can be the structure that regulates the pharmacokinetics of the RNA inhibitor in vivo, and some can be the structure with dissolution activity in vivo. In some embodiments, the liver targeting specific ligand X is selected from one or more monosaccharides and derivatives thereof in the following structures.

In some embodiments, the monosaccharide is selected from one or more of the following structures: mannose, galactose, D-arabinose, glucose, fructose, xylose, glucosamine, ribose. The monosaccharide derivative is selected from mannose derivatives, galactose derivatives, glucose derivatives, ribose derivatives, and other derivatives.

In some embodiments, the liver targeting specific ligand X is selected from galactose, galactosamine, N-acetylgalactosamine and their derivatives, and its general structural formula is as follows: 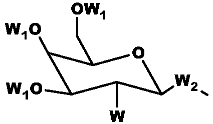 wherein, W₁ is a hydrogen or hydroxyl protecting group, which can be the same or different; W is -OH, -NHCOOH or -NHCO(CH₂)qCH₃, wherein q is an integer of 0-4; W₂ is -NH-, O, S or C.

In some embodiments, the liver targeting specific ligand X is N-acetylgalactosamine and its derivatives.

In some embodiments, the liver targeting specific ligand X is selected from the following structures: 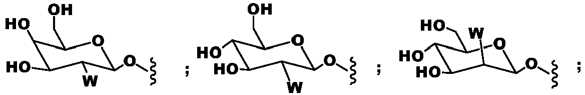 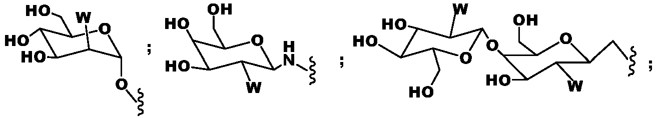 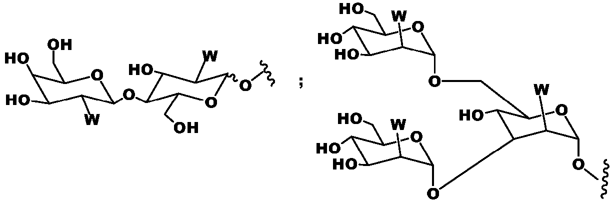 wherein W is selected from one or two of -OH, -NHCOOH or -NHCO(CH₂)_{q}CH₃, wherein q is an integer of 0-4.

In some embodiments, the liver targeting specific ligand X may be the same or different in the same 5'MVIP or 3'MVIP structure.

In some embodiments, the X between 5'MVIP and 3'MVIP can be the same or different.

In some embodiments, the branched chain L is a C₄-C₁₈ carbon chain containing -NH-, -C(=O)-, -O-, -S-, amido, phosphoryl, thiophosphoryl, C₄-C₁₀ aliphatic carbocyclyl, phenyl or a combination of these groups.

In some embodiments, the branched chain L also has a side chain of hydroxyethyl or carboxylic acids group.

In some embodiments, the branched chain L is a C₇-C₁₈ carbon chain containing amido group or six-membered aliphatic carbocyclyl group.

In some embodiments, the branched chain L is selected from one or more of the following structures: 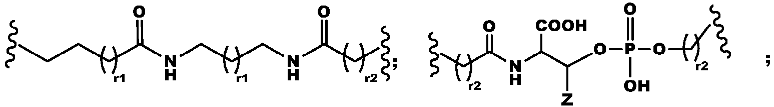 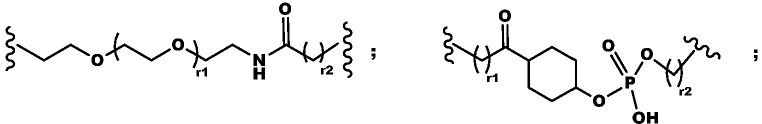 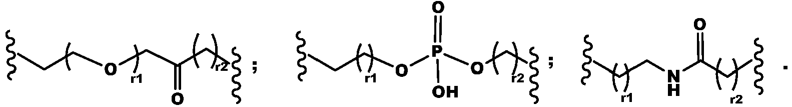 wherein r1 is any integer from 1 to 12, r2 is any integer from 0 to 20, and Z is H, alkyl or amido group, the alkyl group is such as C₁-C₅ alkyl.

In some embodiments, the structure of the linker B is related to the number of X that can be introduced. The linker B contains -NH-, C, O, S, amido, phosphoryl, thiophosphoryl. When n or m is 1, it is a straight carbon chain, and when n or m is 2, 3 or 4, the number of branches is 2, 3 or 4, respectively.

In some embodiments, the linker B is selected from the following structures: 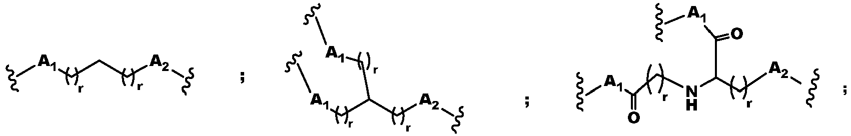 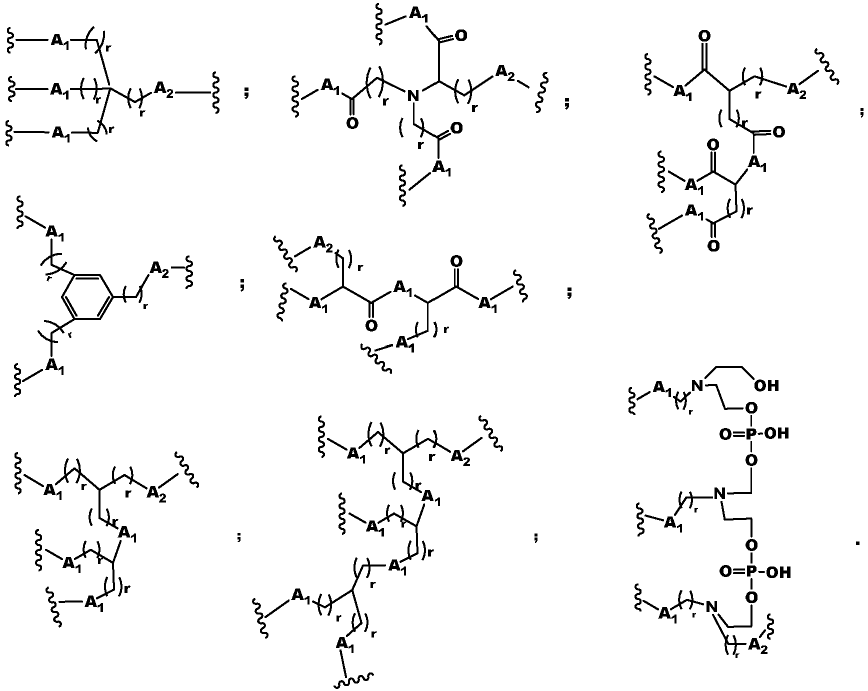 wherein A₁ and A₂ are each independently C, O, S, -NH-, carbonyl, amido, phosphoryl or thiophosphoryl, and r is an integer of 0-4.

In some embodiments, the linker B is selected from the following structures: 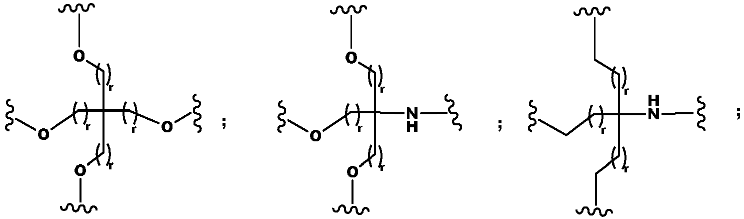 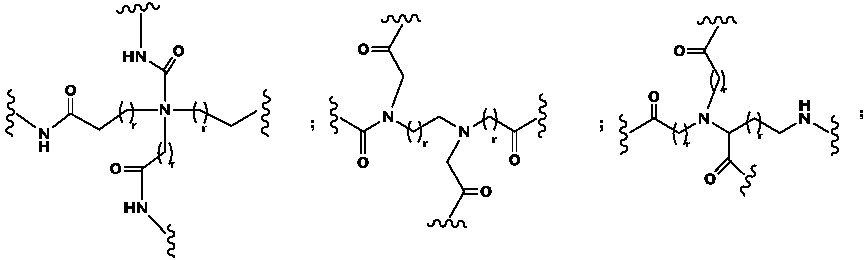 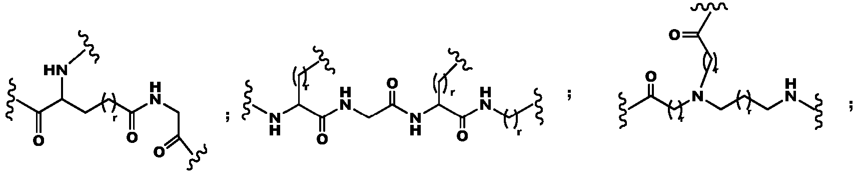 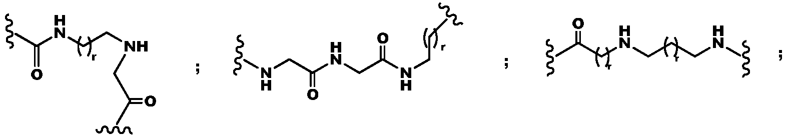 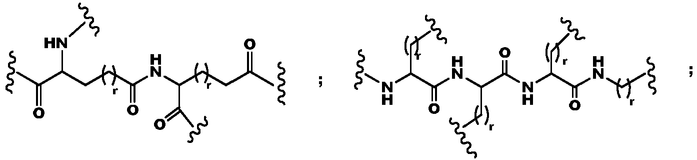 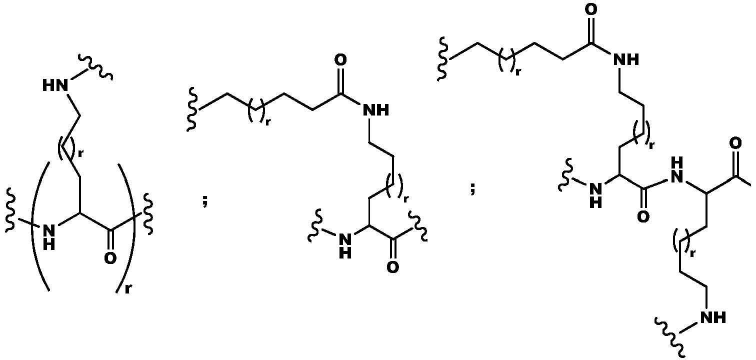 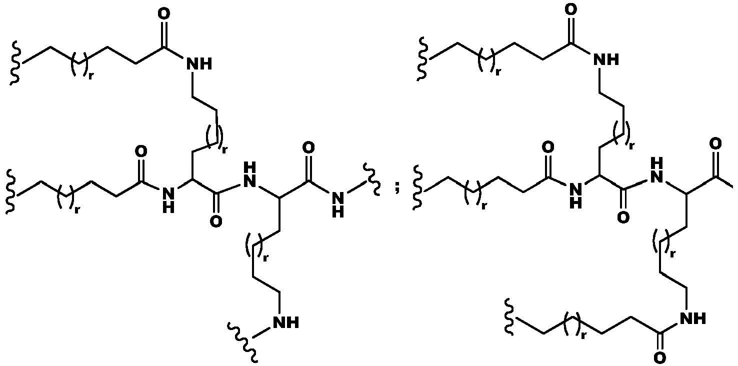 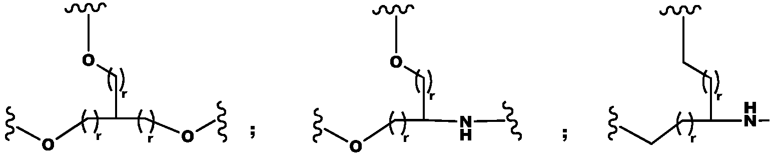 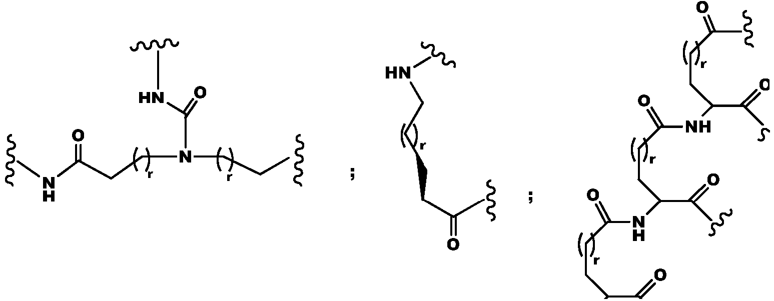 wherein r is any integer from 0 to 4.

In some embodiments, the linker B is selected from the following structures:

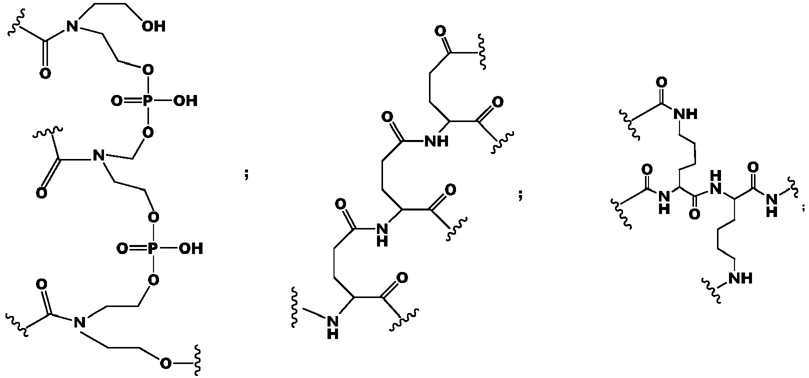

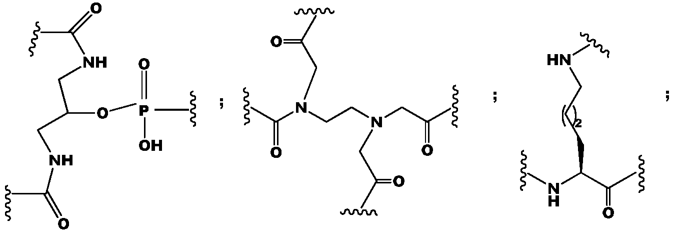

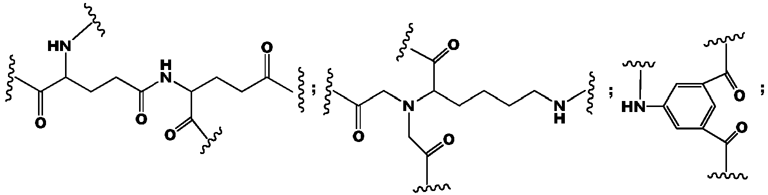

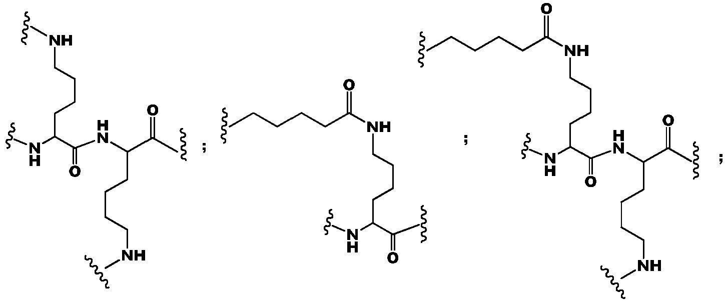

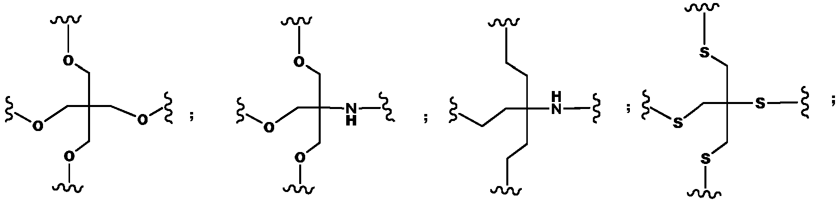

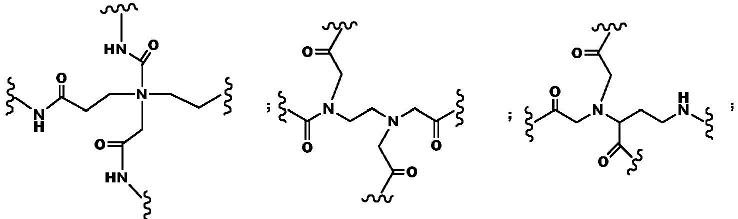

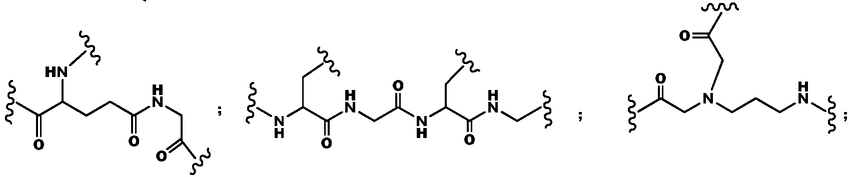

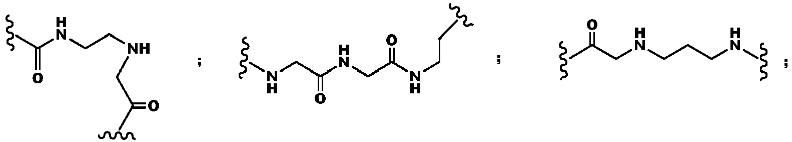

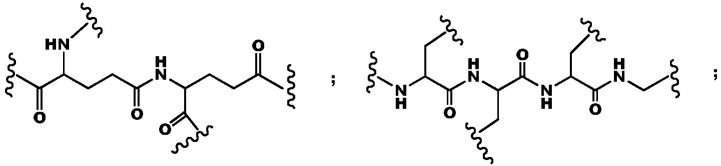

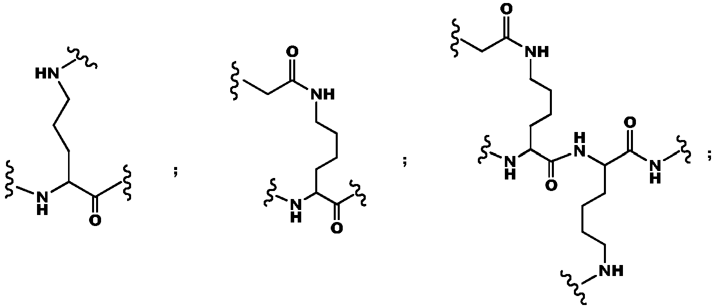

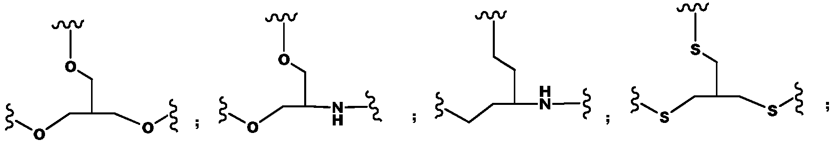

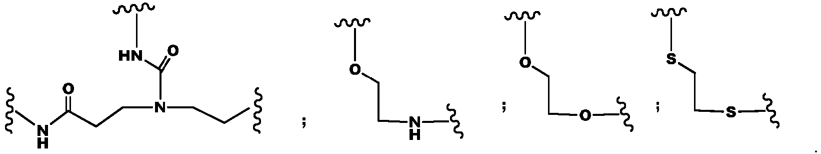

In some embodiments, the linker B is selected from the following structures:

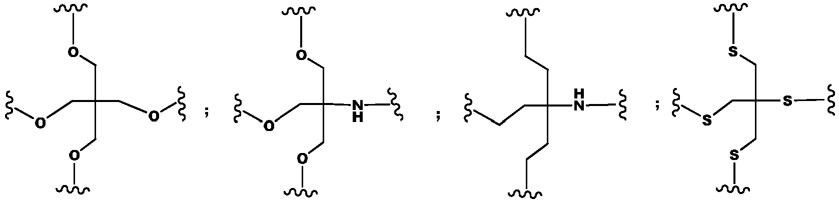

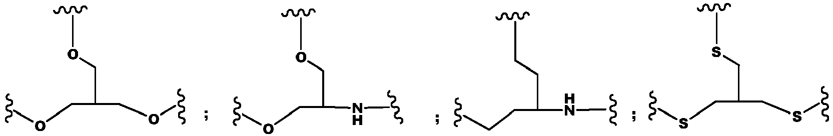

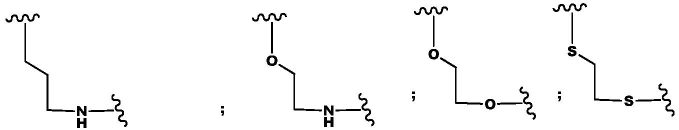

In some embodiments, the linking chain D is a C₃-C₁₈ carbon chain containing - NH-, C=O, O, S, amido, phosphoryl, thiophosphoryl, aromatic hydrocarbyl, C₄-C₁₀ aliphatic carbocyclyl, five- or six-membered heterocyclyl containing 1-3 nitrogen, or a combination of these groups.

In some embodiments, the linking chain D also has side chains of hydroxymethyl, methyl tert-butyl, methylphenoyl, and C₅-C₆ aliphatic cyclic groups.

In some embodiments, the linking chain D is a C₃-C₁₀ carbon chain containing two C=O, six-membered aliphatic carbocyclyl or phenyl groups.

In some embodiments, the linking chain D is a C₃-C₁₀ carbon chain containing two C=O.

In some embodiments, the linking chain D is selected from the following structures: 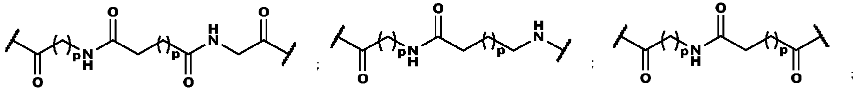 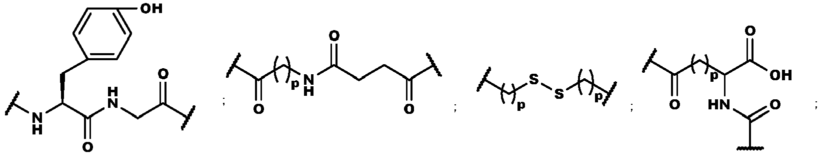 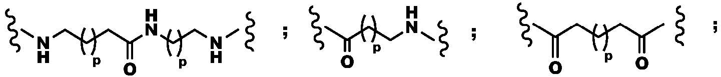 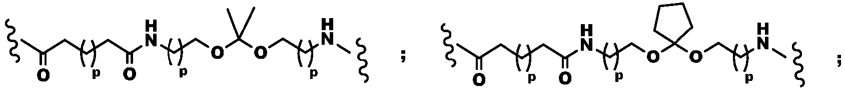 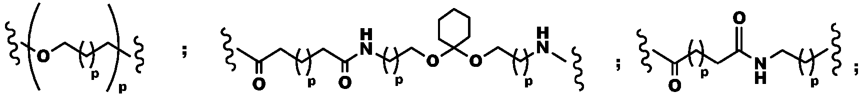 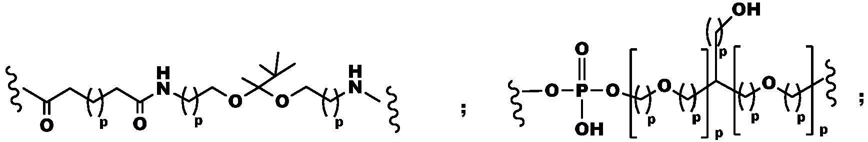 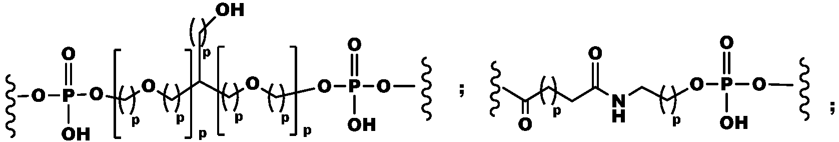 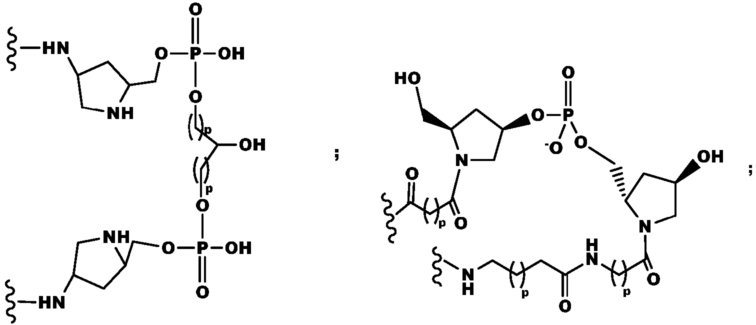 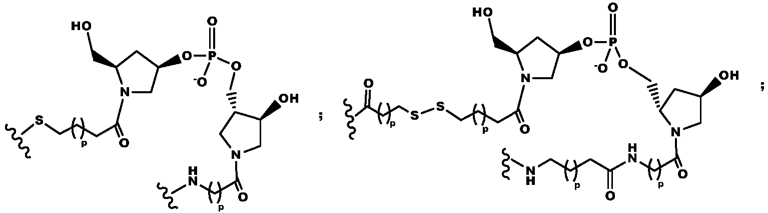 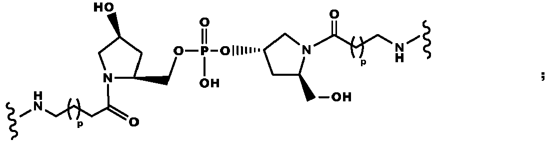 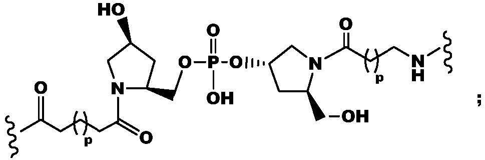 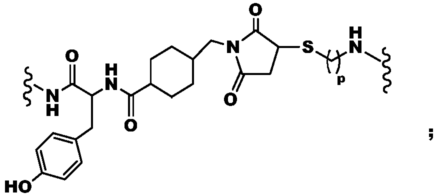 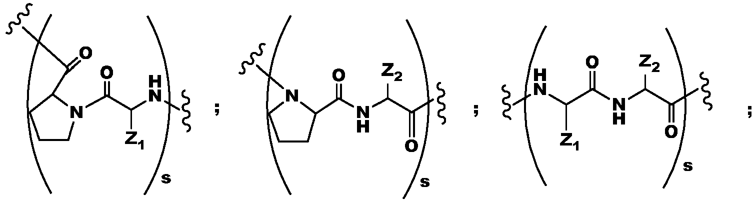 wherein, each p is independently any integer from 1 to 20; s is an integer of 2-13; Z₁ and Z₂ are the same or different substituents, such as C₃-C₁₀ alkyl.

In some embodiments, the linking chain D is selected from the following structures:

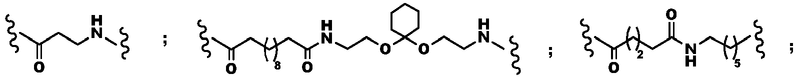

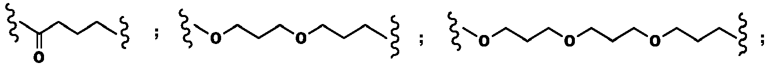

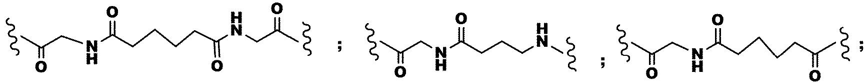

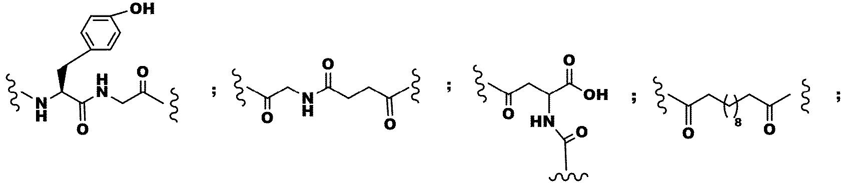

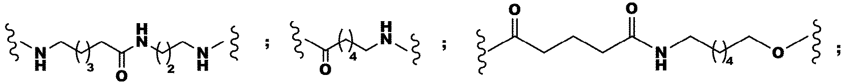

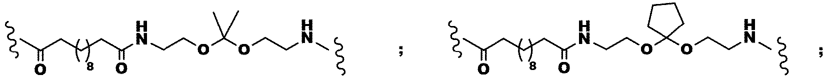

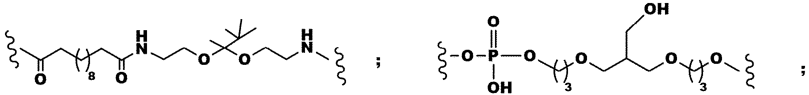

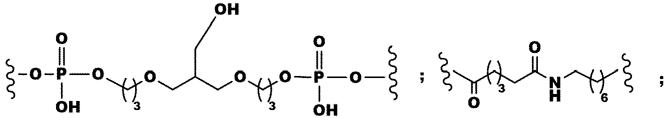

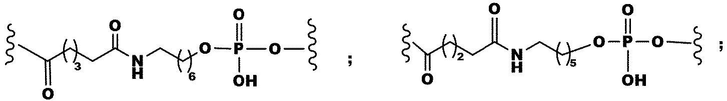

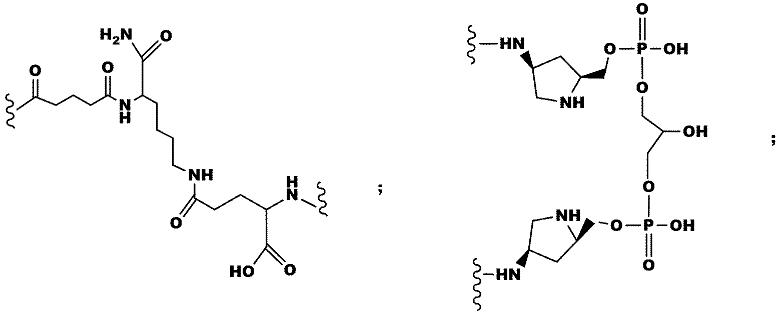

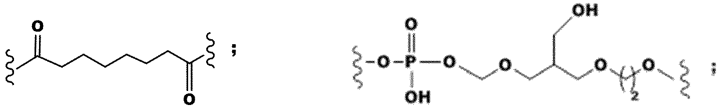

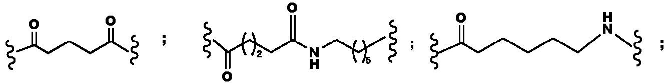

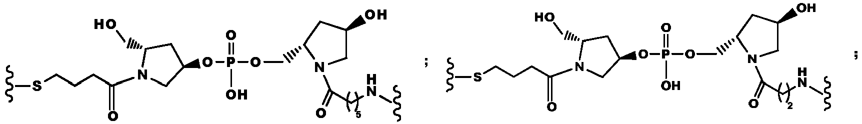

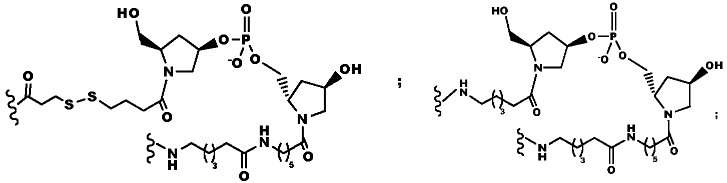

In some embodiments, the linking chain D is selected from the following structures:

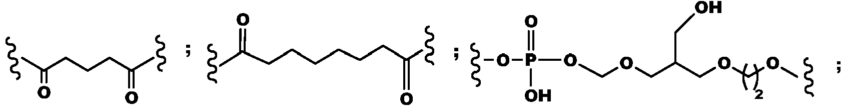

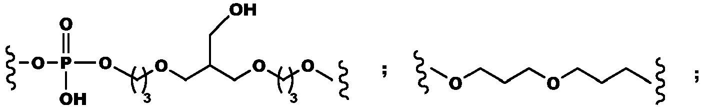

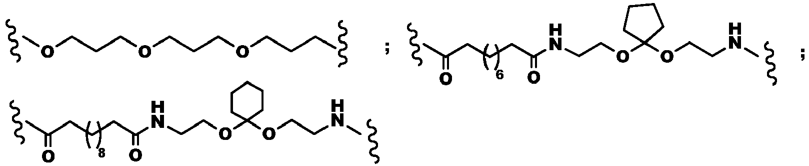

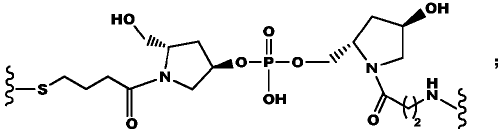

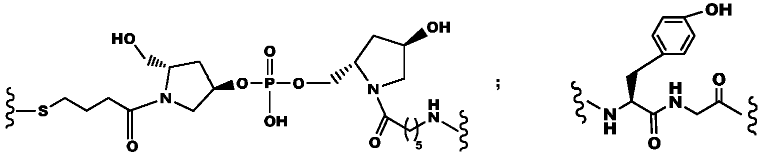

In some embodiments, (X-L)ₙ-B-D- in the 5'MVIP structure and (X-L)ₘ--B-D- in the 3'MVIP structure are selected from one or more of the following structures:

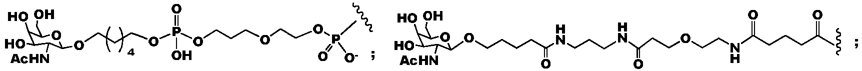

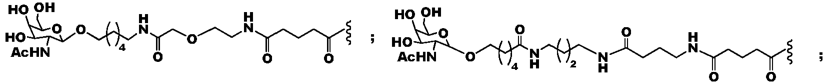

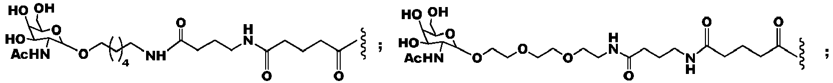

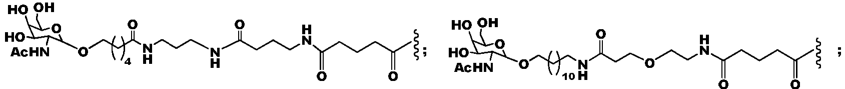

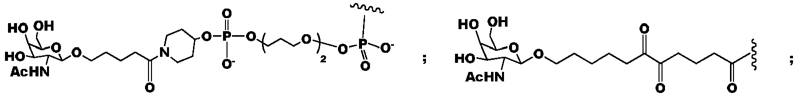

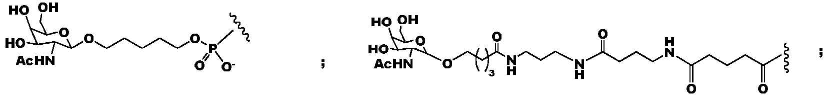

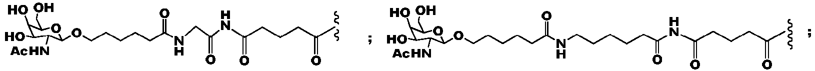

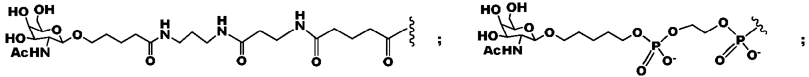

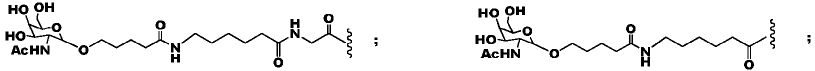

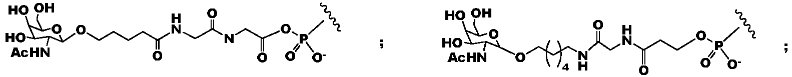

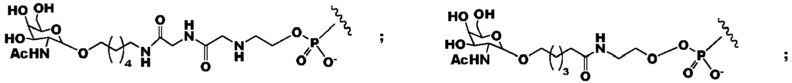

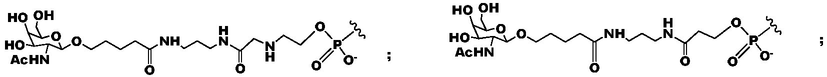

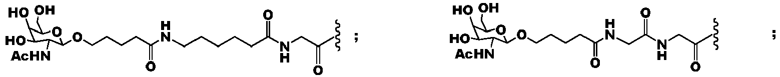

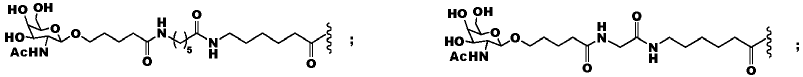

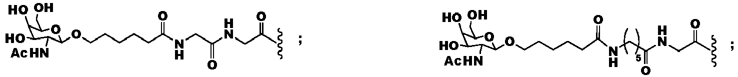

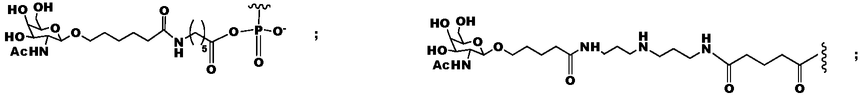

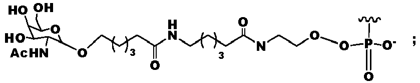

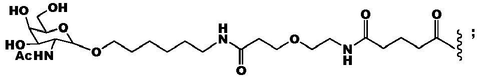

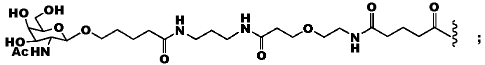

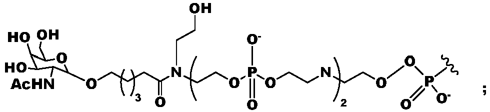

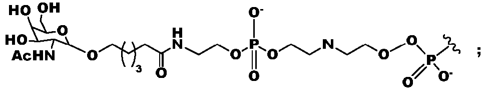

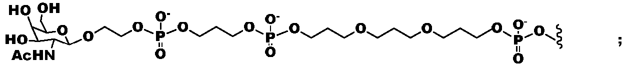

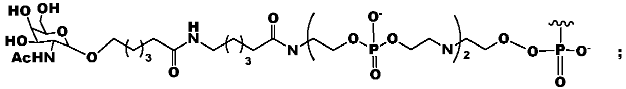

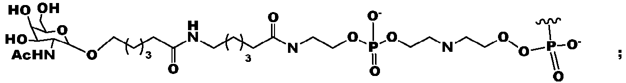

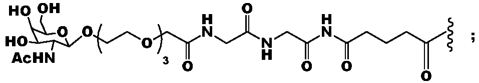

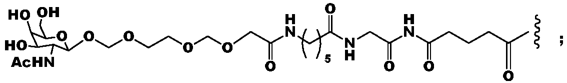

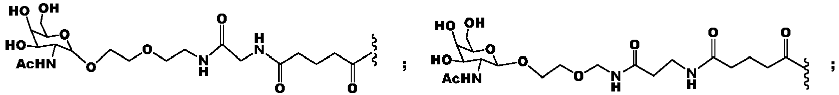

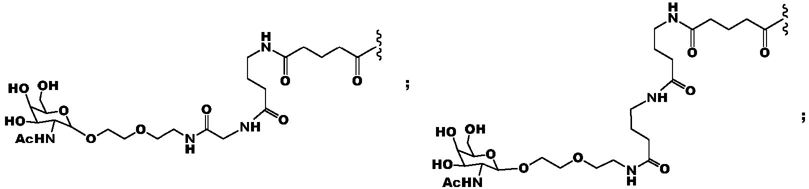

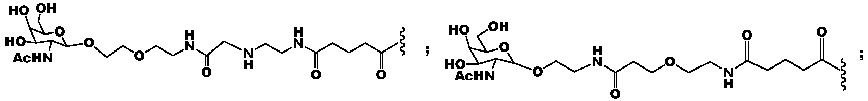

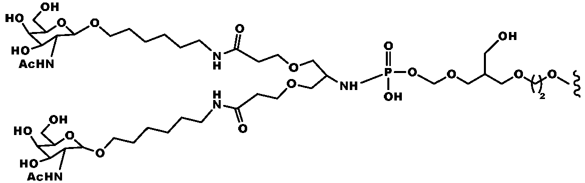

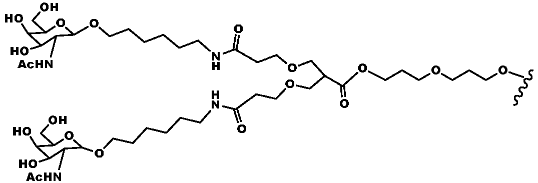

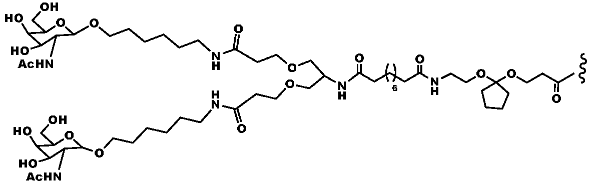

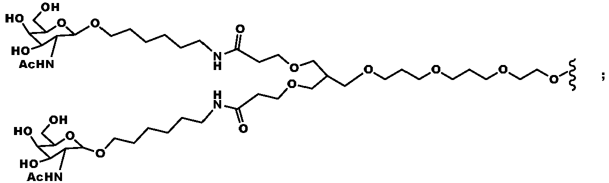

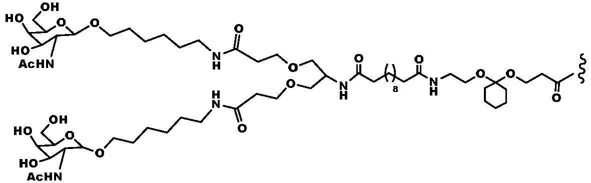

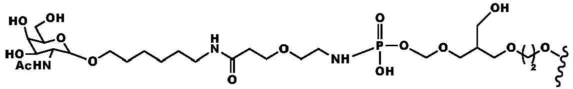

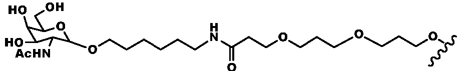

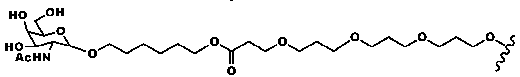

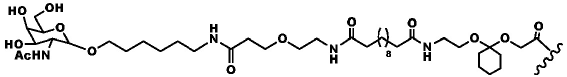

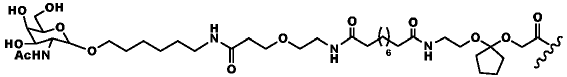

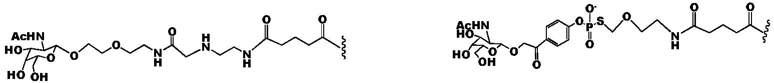

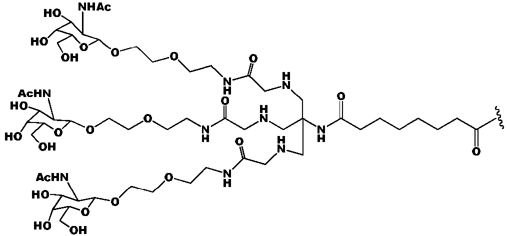

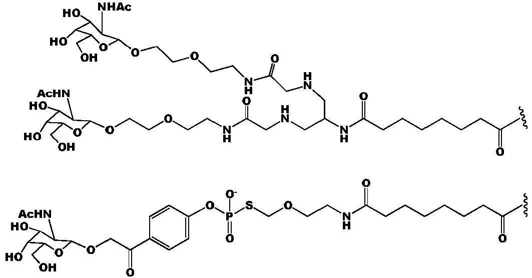

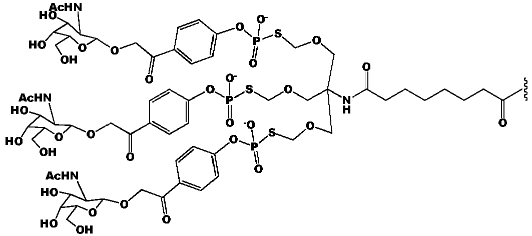

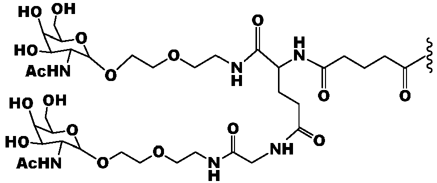

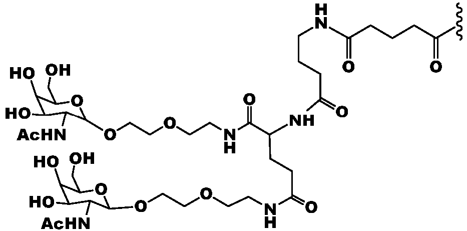

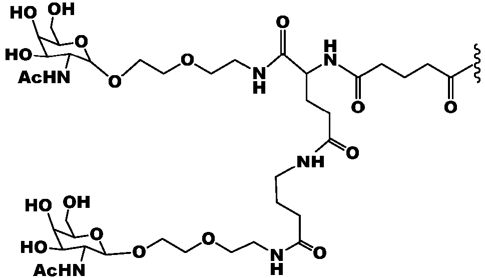

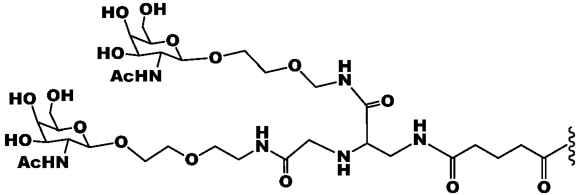

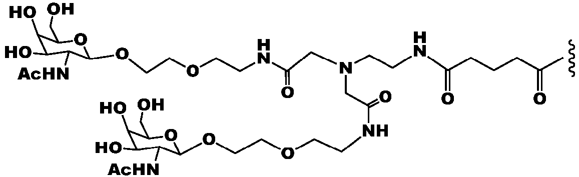

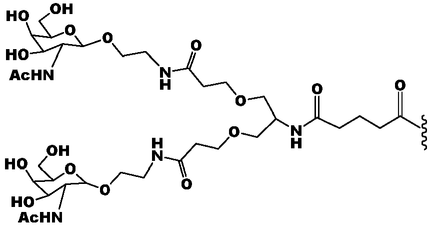

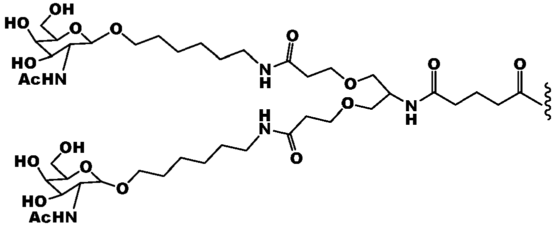

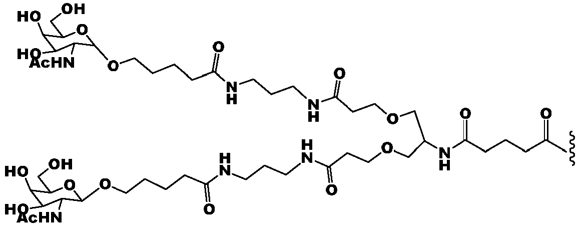

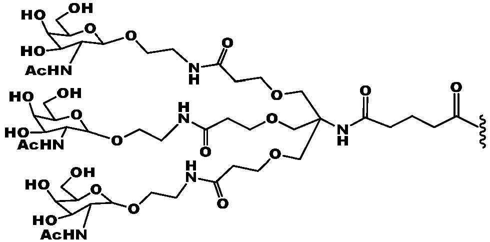

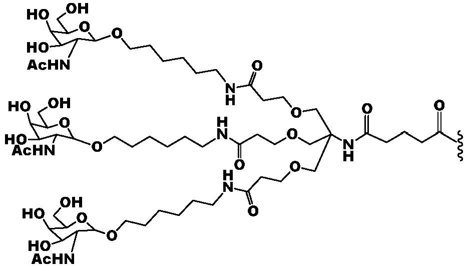

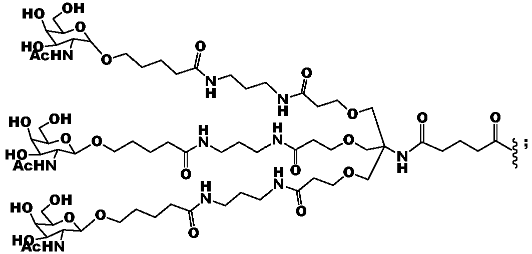

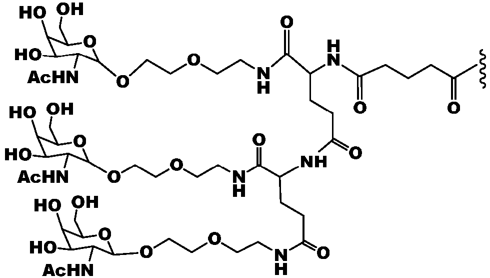

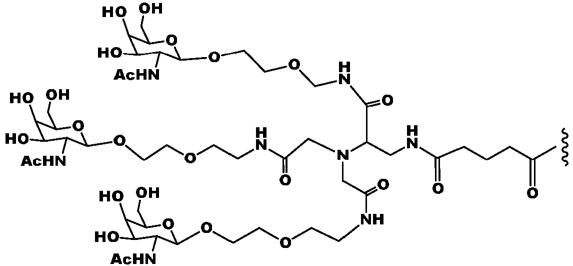

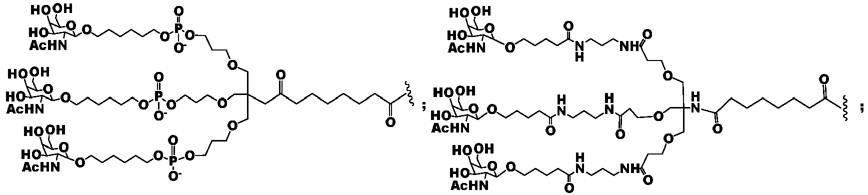

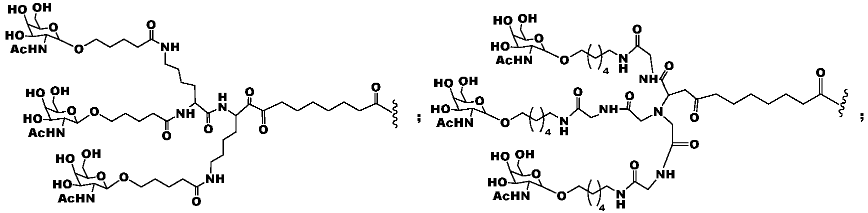

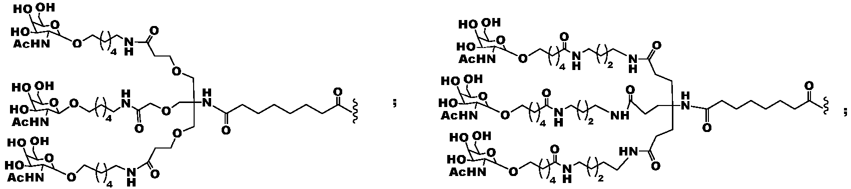

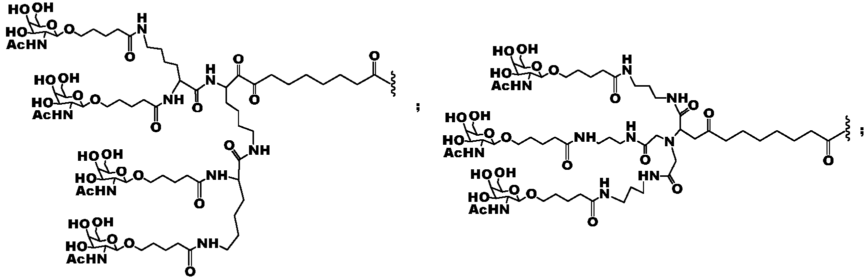

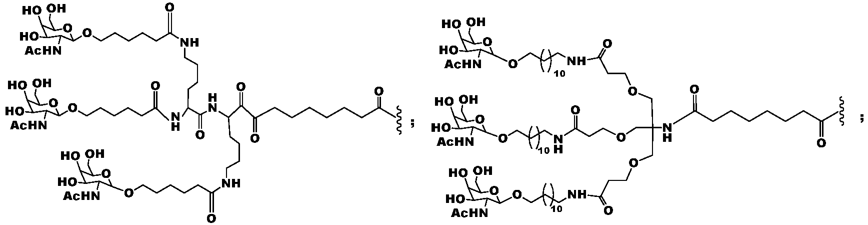

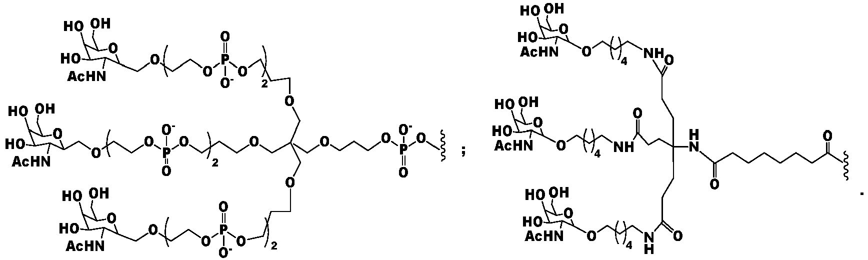

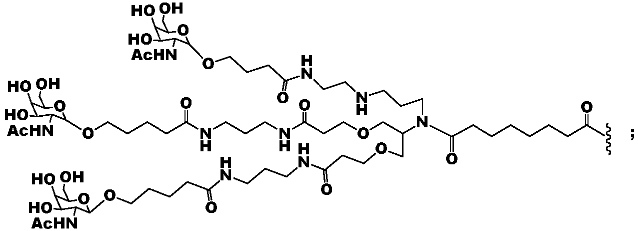

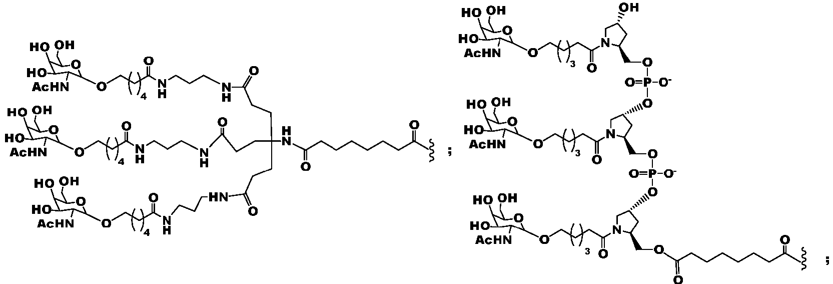

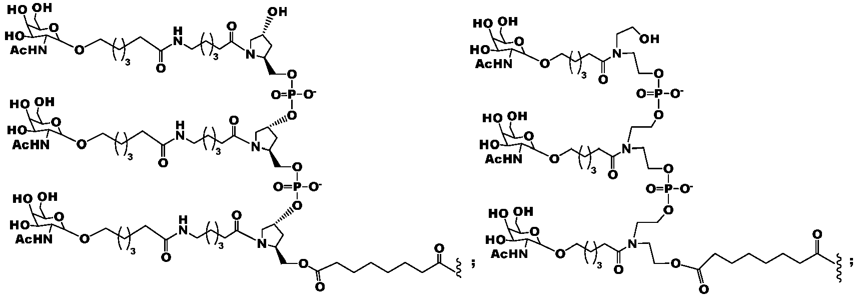

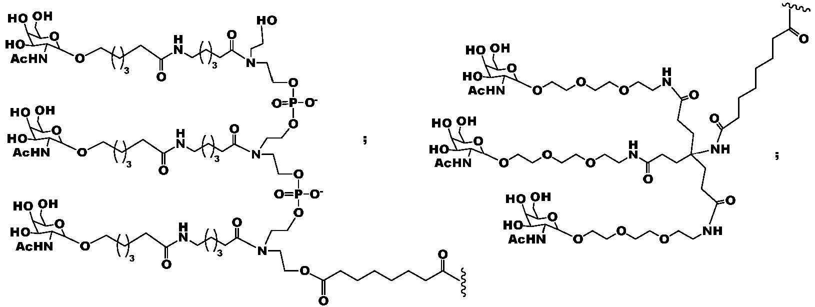

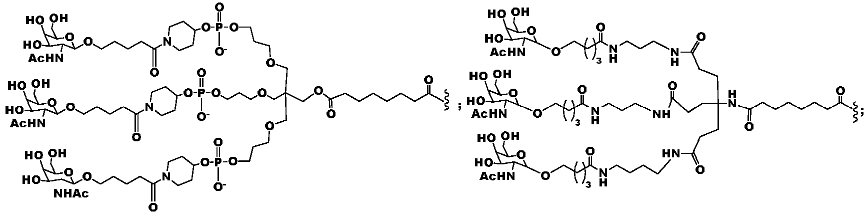

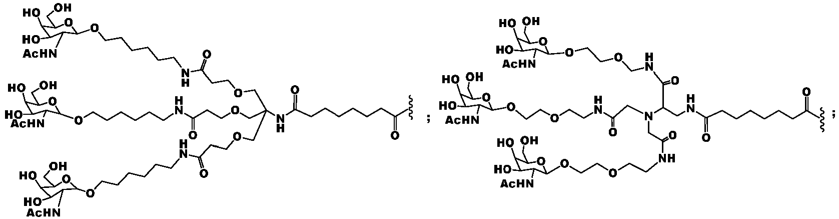

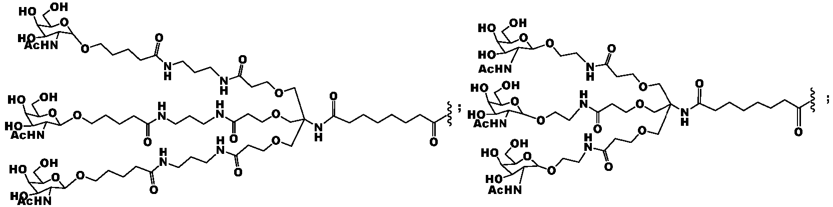

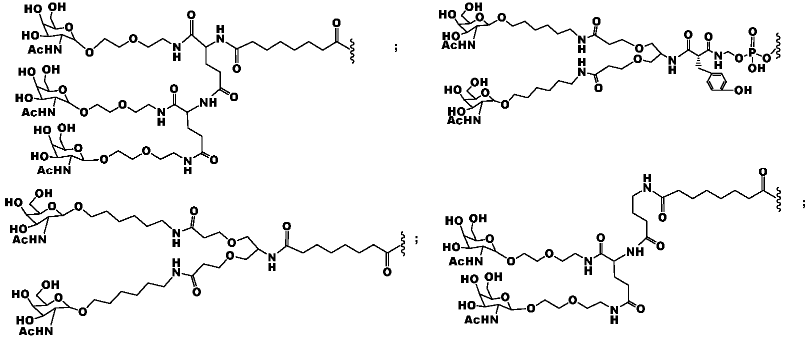

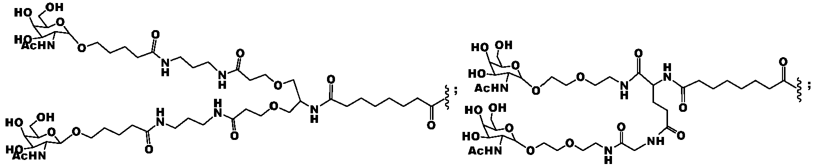

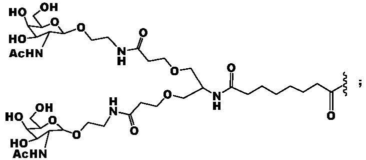

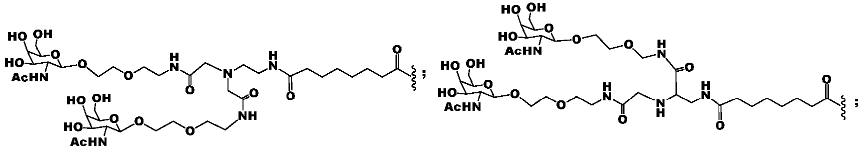

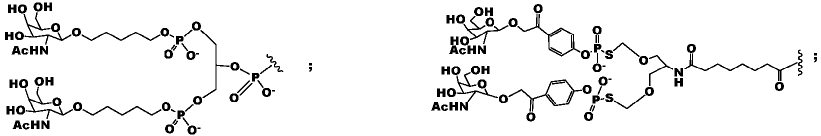

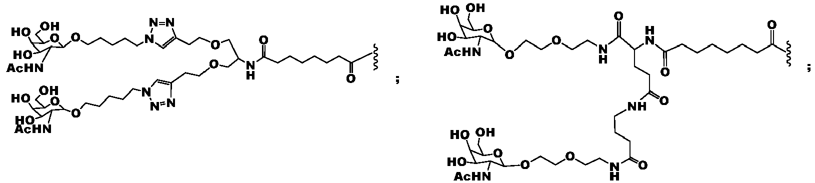

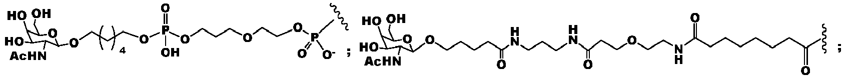

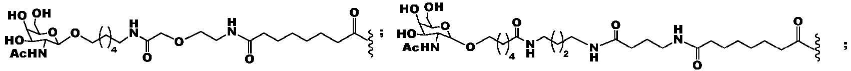

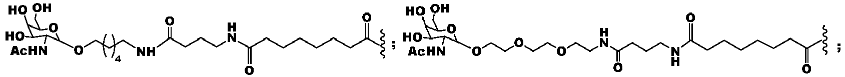

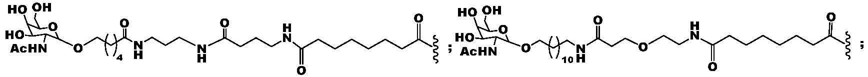

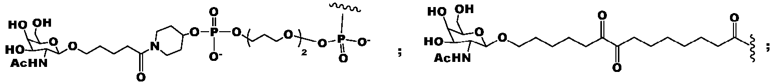

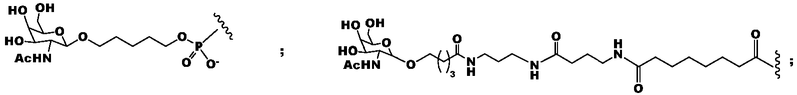

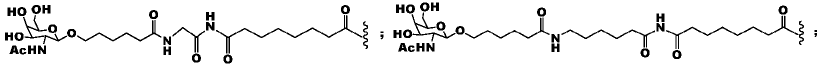

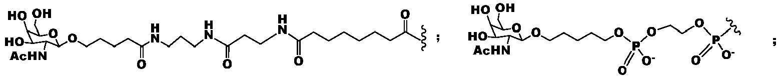

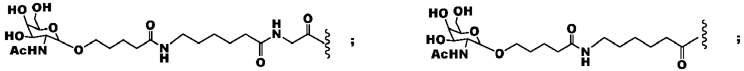

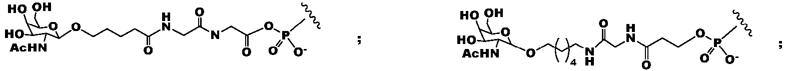

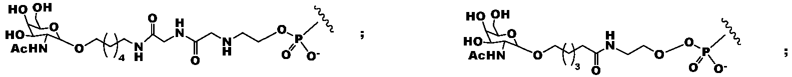

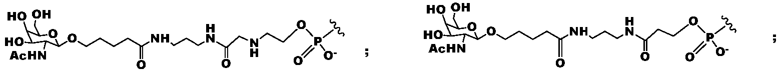

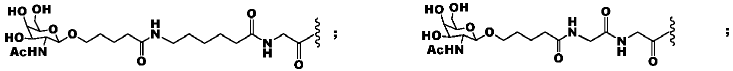

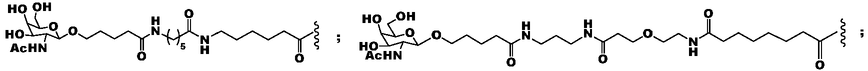

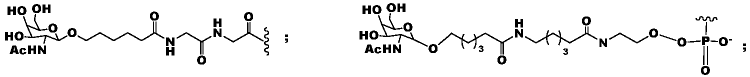

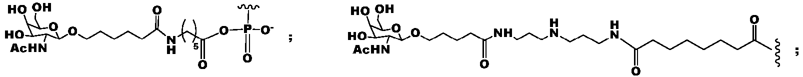

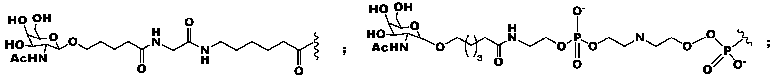

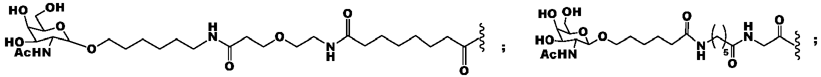

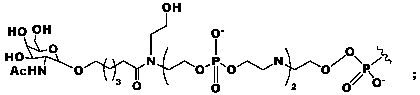

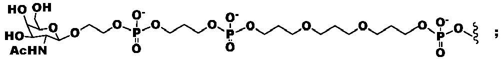

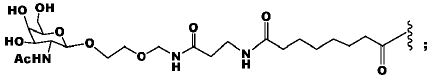

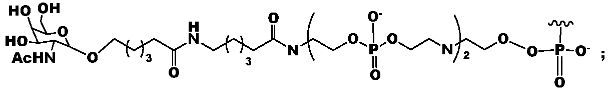

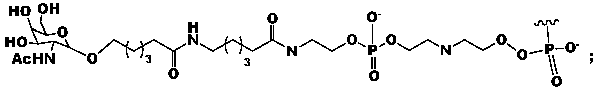

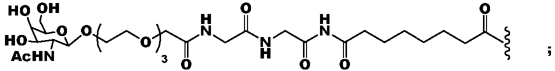

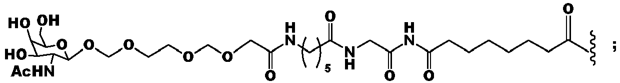

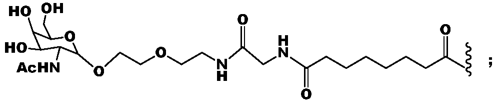

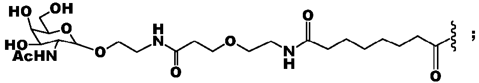

In some embodiments, the X, L, B and D are the same or different within the respective 5 'MVIP and 3' MVIP or between the 5 'MVIP and 3' MVIP.

In some embodiments, (X-L)ₙ-B-D- in the 5'MVIP structure is selected from the structures shown in Table 9:

**Table 9 Structures of (X-L)ₙ-B-D- in 5'MVIP**

| Serial number | code | Structural formula |
|---|---|---|
| 1 | 5'YICdd-01 | 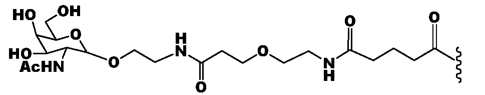 |
| 2 | 5'YICd-01 | 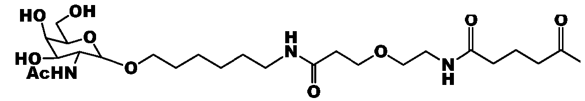 |
| 3 | 5'YICc-01 | 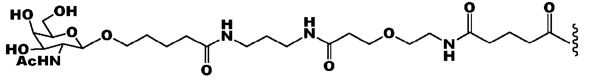 |
| 4 | 5'YICa-01 | 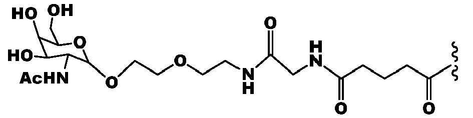 |
| 5 | 5'YICa-02 | 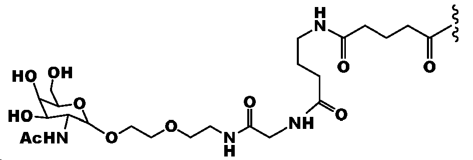 |
| 6 | 5'YICa-03 | 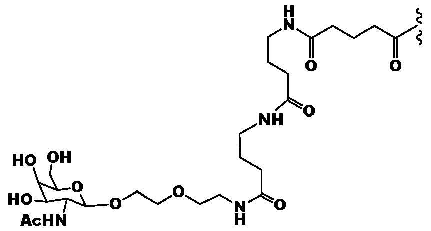 |
| 7 | 5'YICa-04 | 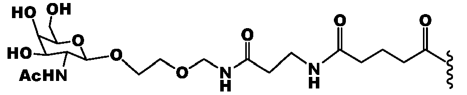 |
| 8 | 5'YICa-05 | 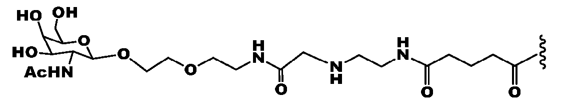 |
| 9 | 5'ERCa-01 | 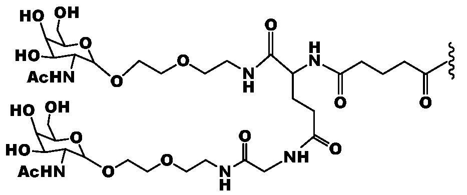 |
| 10 | 5'ERCa-02 | 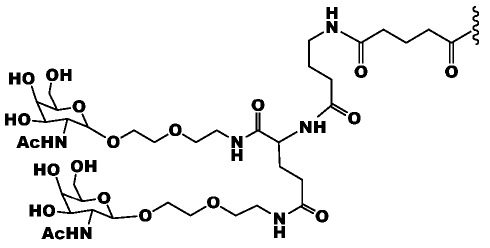 |
| 11 | 5'ERCa-03 | 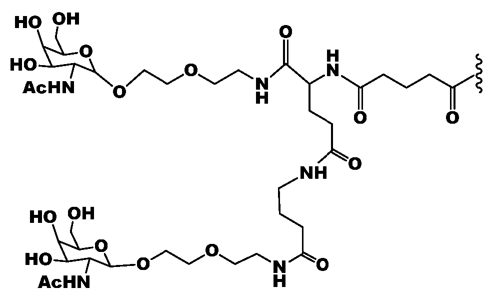 |
| 12 | 5'ERCa-04 | 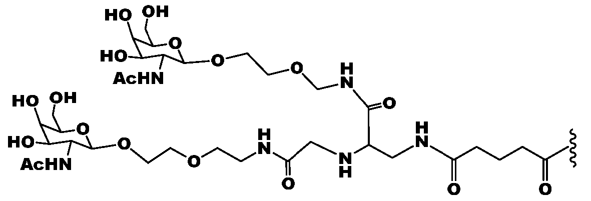 |
| 13 | 5'ERCa-05 | 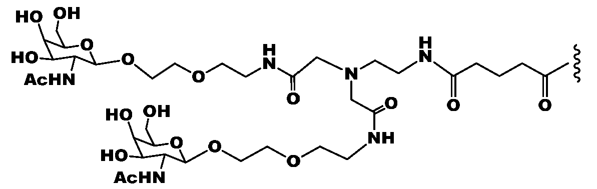 |
| 14 | 5'ERCdd-01 | 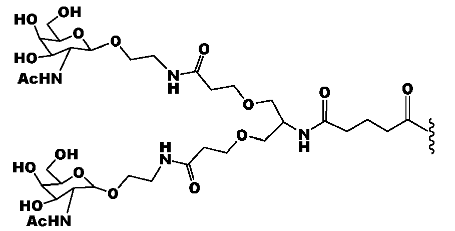 |
| 15 | 5'ERCd-01 | 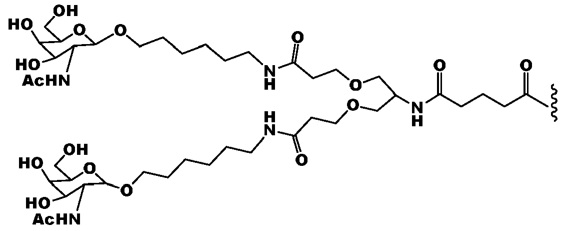 |
| 16 | 5'ERCc-01 | 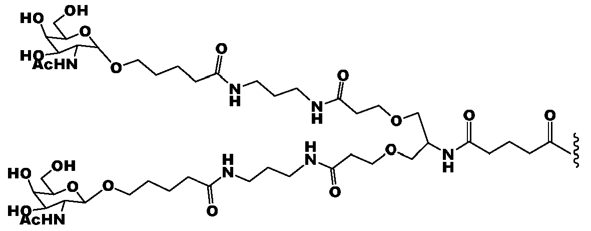 |
| 17 | 5'SANCdd -01 | 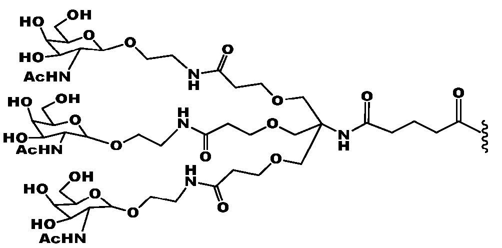 |
| 18 | 5'SANCd-01 | 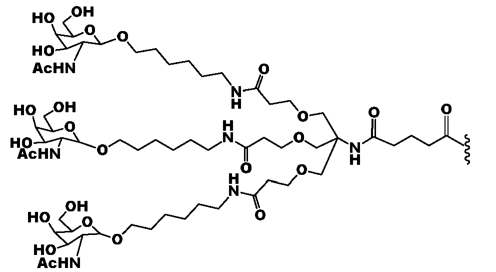 |
| 19 | 5'SANCc-01 | 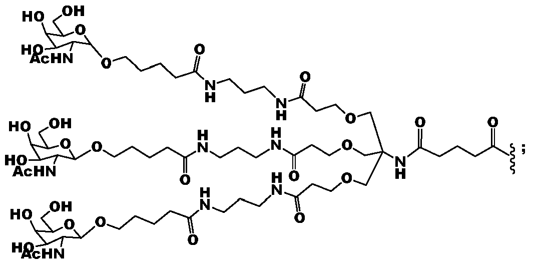 |
| 20 | 5'SANCa-01 | 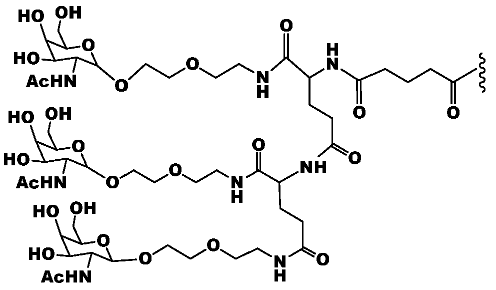 |
| 21 | 5'SANCa-02 | 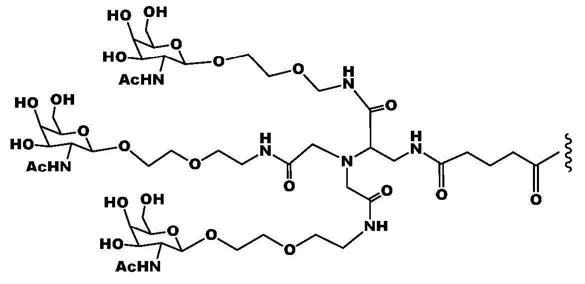 |
| 22 | 5'SANCa-03 | 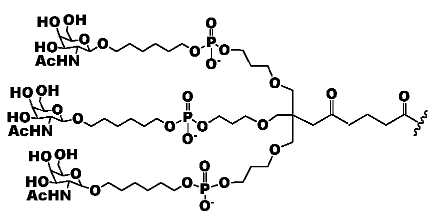 |

In some embodiments, 5'MVIP may not exist, in which case m may be any integer of 2-4.

In some embodiments, (X-L)ₘ-B-D- in the 3'MVIP structure is selected from the structures shown in Table 10:

**Table 10 Structures of (X-L)ₘ-B-D- in 3'MVIP**

| Ser ies nu mb er | code | structure |
|---|---|---|
| 1 | 3'SANCdd-01 | 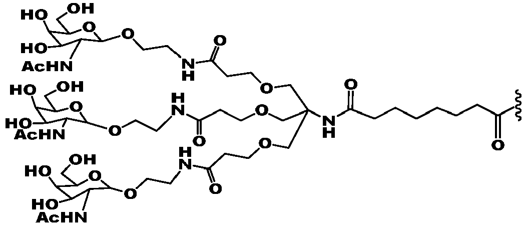 |
| 2 | 3'SANCd-01 | 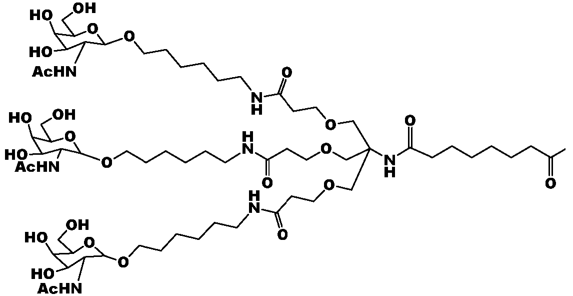 |
| 3 | 3'SANCc-01 | 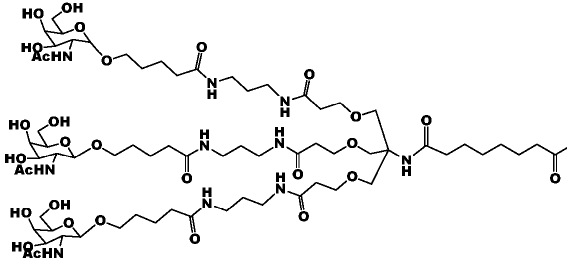 |
| 4 | 3'SANCa-01 | 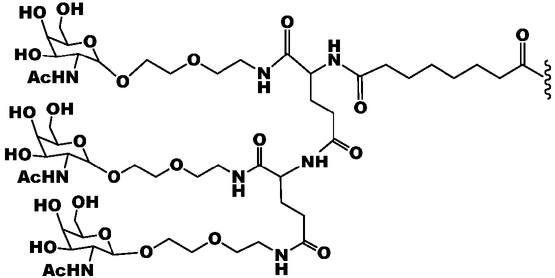 |
| 5 | 3'SANCa-02 | 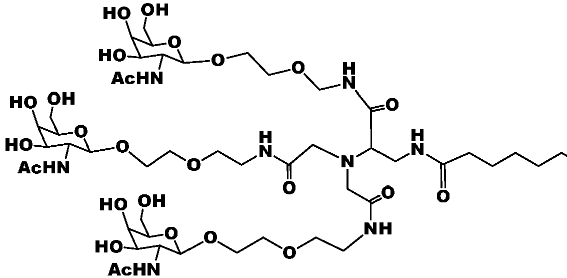 |
| 6 | 3'SANCa-03 | 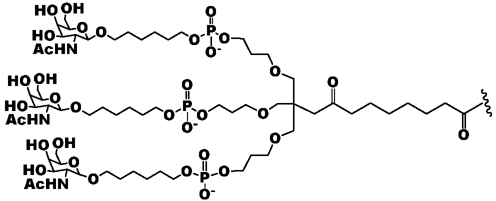 |
| 7 | 3'ERCdd-01 | 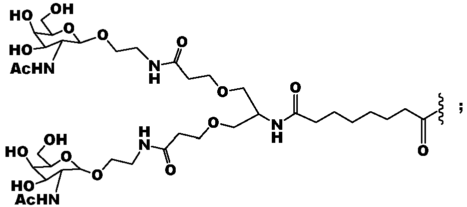 |
| 8 | 3'ERCd-01 | 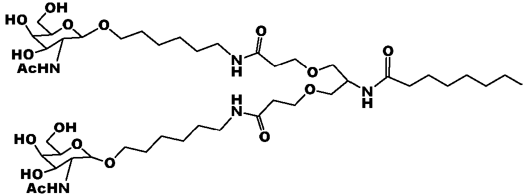 |
| 9 | 3'ERCc-01 | 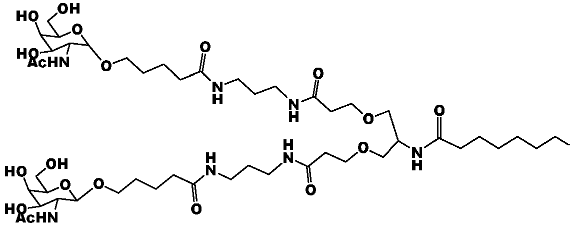 |
| 10 | 3'ERCa-01 | 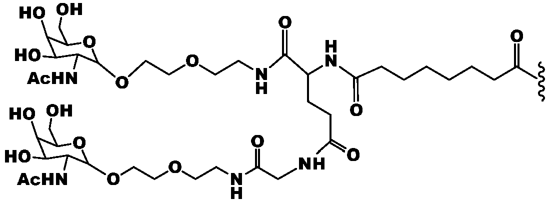 |
| 11 | 3'ERCa-02 | 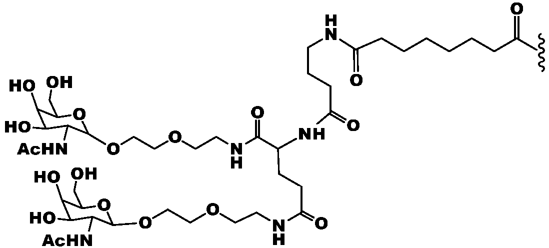 |
| 12 | 3'ERCa-03 | 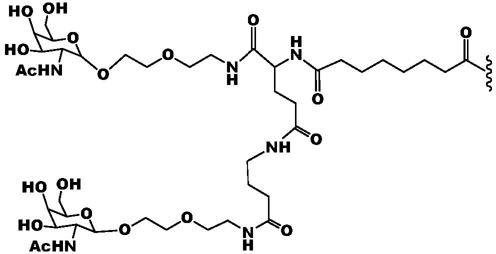 |
| 13 | 3'ERCa-04 | 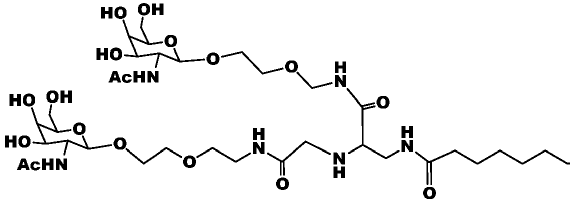 |
| 14 | 3'ERCa-05 | 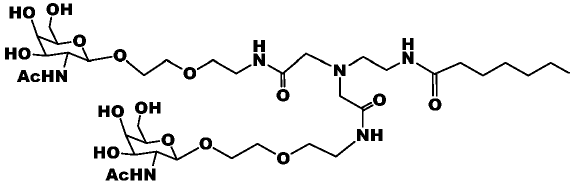 |
| 15 | 3'YICa-01 | 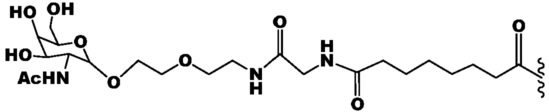 |
| 16 | 3'YICa-02 | 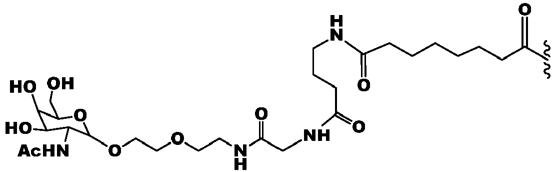 |
| 17 | 3'YICa-03 | 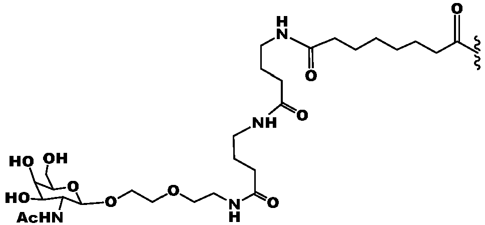 |
| 18 | 3'YICa-04 | 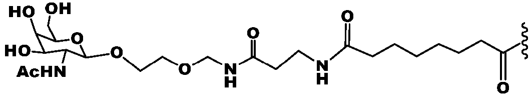 |
| 19 | 3'YICa-05 | 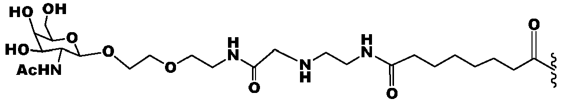 |
| 20 | 3'YICdd-01 | 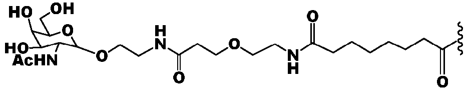 |
| 21 | 3'YICd-01 | 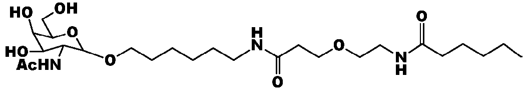 |
| 22 | 3'YICc-01 | 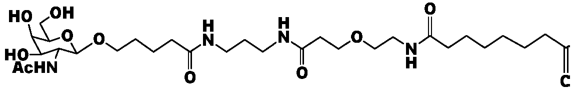 |

In some embodiments, the combinations of (X-L)ₙ-B-D- in the ligand structure 5'MVIP and R₁ are shown in Table 11.

**Table 11 Combinations of (X-L)ₙ-B-D- in 5'MVIP and R₁**

| Serial number | **(X-L)ₙ-B-D-**code | R₁ | 5'MVIP code |
|---|---|---|---|
| 1 | 5'YICd-01 | -NH(CH₂)₆O- | 5'MVIP01 |
| 2 | 5'YICc-01 | -NH(CH₂)₆O- | 5'MVIP02 |
| 3 | 5'YICa-01 | -O(CH₂)₆O- | 5'MVIP03 |
| 4 | 5'YICa-02 | -O(CH₂)₆O- | 5'MVIP04 |
| 5 | 5'YICa-03 | -S(CH₂)₆O- | 5'MVIP05 |
| 6 | 5'YICa-04 | -NH(CH₂)₆S- | 5'MVIP06 |
| 7 | 5'YICa-05 | -NH(CH₂)₈ O- | 5'MVIP07 |
| 8 | 5'YICr-06 | -NH(CH₂)₈O- | 5'MVIP08 |
| 9 | 5'ERCd-01 | -NH(CH₂)₆O- | 5'MVIP09 |
| 10 | 5'ERCc-01 | -NH(CH₂)₆O- | 5'MVIP10 |
| 11 | 5'ERCa-01 | -NH(CH₂)₅CH(CH₂CH₃)O- | 5'MVIP11 |
| 12 | 5'ERCa-02 | -O(CH₂)₆O- | 5'MVIP12 |
| 13 | 5'ERCa-03 | -S(CH₂)₆O- | 5'MVIP13 |
| 14 | 5'ERCa-04 | -O(CH₂)₆O- | 5'MVIP 14 |
| 15 | 5'ERCa-05 | -O(CH₂)₆O- | 5'MVIP15 |
| 16 | 5'ERCr-06 | -S(CH₂)₄CH(CH₃)O- | 5'MVIP16 |
| 17 | 5'SANCd-01 | -NH(CH₂)₆O- | 5'MVIP17 |
| 18 | 5'SANCc-01 | -NH(CH₂)₆O- | 5'MVIP18 |
| 19 | 5'ERCd-01 | 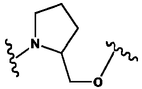 | 5'MVIP19 |
| 20 | 5'ERCd-01 | 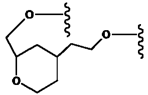 | 5'MVIP20 |
| 21 | 5'YICd-01 | 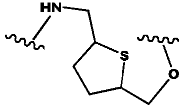 | 5'MVIP21 |
| 22 | 5'SANCd-01 | 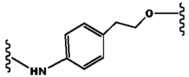 | 5'MVIP22 |

In some embodiments, 3'MVIP may not exist, in which case n may be any integer of 2-4.

In some embodiments, the combinations of (X-L)ₘ-B-D- in the ligand structure 3'MVIP and R₂ are shown in Table 12.

**Table 12 Combinations of (X-L)ₘ-B-D- in 3'MVIP and R₂**

| Serial number | **(X-L)ₘ-B-D**- code | R₂ | 3'MVIP code |
|---|---|---|---|
| 1 | 3'YICd-01 | 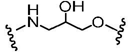 | 3'MVIP01 |
| 2 | 3'YICc-01 | 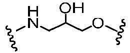 | 3'MVIP02 |
| 3 | 3'YICa-01 | 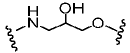 | 3'MVIP03 |
| 4 | 3'YICa-02 | 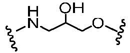 | 3'MVIP04 |
| 5 | 3'YICa-03 | 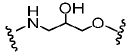 | 3'MVIP05 |
| 6 | 3'YICa-04 | 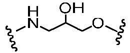 | 3'MVIP06 |
| 7 | 3'YICa-05 | 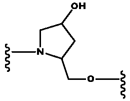 | 3'MVIP07 |
| 8 | 3'YICr-06 | 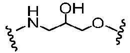 | 3'MVIP08 |
| 9 | 3'ERCd-01 | 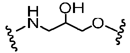 | 3'MVIP09 |
| 10 | 3'ERCc-01 | 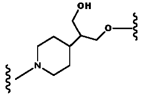 | 3'MVIP10 |
| 11 | 3'ERCa-01 | 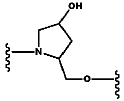 | 3'MVIP11 |
| 12 | 3'ERCa-02 | 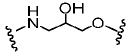 | 3'MVIP12 |
| 13 | 3'ERCa-03 | 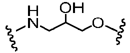 | 3'MVIP13 |
| 14 | 3'ERCa-04 | 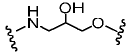 | 3'MVIP14 |
| 15 | 3'ERCa-05 | 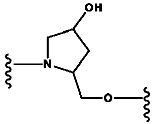 | 3'MVIP15 |
| 16 | 3'ERCr-06 | 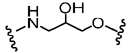 | 3'MVIP16 |
| 17 | 3'SANCd-01 | 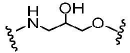 | 3'MVIP17 |
| 18 | 3'SANCc-01 | 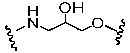 | 3'MVIP18 |
| 19 | 3'SANCa-01 | 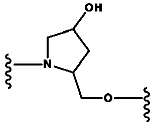 | 3'MVIP19 |
| 20 | 3'ERCd-01 | 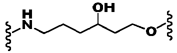 | 3'MVIP20 |
| 21 | 3'ERCd-01 | 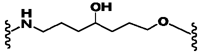 | 3'MVIP21 |
| 22 | 3'ERCd-01 | 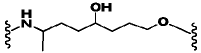 | 3'MVIP22 |
| 23 | 3'ERCd-01 | 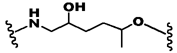 | 3'MVIP23 |
| 24 | 3'ERCd-01 | 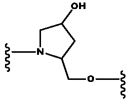 | 3'MVIP24 |
| 25 | 3'ERCd-01 | 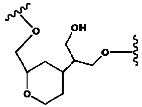 | 3'MVIP25 |
| 26 | 3'ERCd-01 | 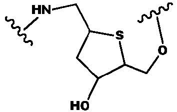 | 3'MVIP26 |
| 27 | 3'ERCd-01 | 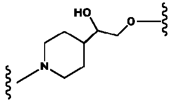 | 3'MVIP27 |

In some embodiments, the 5'MVIP is selected from any one or more of 5'MVIP01 to 5'MVIP22 in Table 11.

In some embodiments, the 3'MVIP is selected from any one or more of 3'MVIP01 to 3'MVIP27 in Table 12.

In some embodiments, there is a possibility of combination of any of the 5'MVIP in Table 11 and any of the 3'MVIP in Table 12, where n+m =2, 3, 4, 5 or 6.

In some embodiments, the sense strand in the RNA inhibitor can be a sequence selected from the Table 13 below:

**Table 13 Sense strands coupled to 5'MVIP09**

| Sense strand code | Sense strand sequence 5'-3' |
|---|---|
| S196 | 5'MVIP09-CsAsGCUCCUfUfAfUUGUUAUACsGsA |
| S197 | 5'MVIP09-GsCsCCCUUAfUfUfGUUAUACsGsA |
| S198 | 5'MVIP09-CsAsGCCCCUfUfAfUUGUUAUACsGsA |
| S199 | 5'MVIP09-CsAsGCCCCUfUfAfUUGUUAUACGAsGsA |
| S200 | 5'MVIP09-UsCsCAGCCCfCfUfUAUUGUUAUACsGsA |
| S201 | 5'MVIP09-UsGsfGUfAAfUfGfGAfCAfGAfGUUAUsUsU |
| S201 | 5'MVIP09-UsGsfGUfAAfUfGfGAfCAfGAfGUUAUsUsU |
| S201 | 5'MVIP09-UsGsfGUfAAfUfGfGAfCAfGAfGUUAUsUsU |
| S202 | 5'MVIP09-UsGsfGUfAAfUfGfGAfCAfGAfGUUsAsU |
| S203 | 5'MVIP09-UsGsfGUfAAfUGfGfAfCAfGAfGUUAUsUsU |
| S204 | 5'MVIP09-CsAsfUGFGUfAfATUGIGAfCAfGAGUUsAsU |
| S205 | 5'MVIP09-UsGsfGUfAAfUfGfGAfCAfGAfGUUAUsCsG |
| S206 | 5'MVIP09-UsGsfGUfAAfUfGIGAfCAfGAfGUUAUsCsA |
| S265 | 5'MVIP09-CsGsfGUfAAfUfGfGAfCAfGAfGUUAUsCsA |
| S201 | 5'MVIP09-UsGsfGUfAAfUfGfGAfCAfGAfGUUAUsUsU |
| S206 | 5'MVIP09-UsGsfGUfAAfUfGfGAfCAfGAfGUUAUsCsA |
| S266 | 5'MVIP09-UsGsGUfAAfUfGfGACAGAGUUAUsCsA |
| S266 | 5'MVIP09-UsGsGUfAAfUfGfGACAGAGUUAUsCsA |

In some embodiments, the sense strand of the RNA inhibitor of the present application is a sequence that has at least 15 consecutive nucleotides identical to the sense strand in Table 13, or a sequence that differs from the sense strand in Table 13 by 1, 2 or 3 nucleotides.

In some embodiments, the antisense strand in the RNA inhibitor can be a sequence selected from the Table 14 below:

**Table 14 Antisense strands coupled to 3'MVIP09**

| Antisense Strand code | Antisense strand sequence 5'-3' |
|---|---|
| AS196 | UsfCsGfUAfUAACAAfUAfAGfGAfGCsfUsG-3'MVIP09 |
| AS197 | UsfCsGfUAfUAACAAfUAfAGfGGfGCsfUsG-3'MVIP09 |
| AS198 | UsfCsGfUAfUAACAAfUAfAGfGGfGCfUGsGsA-3'MVIP09 |
| AS198 | UsfCsGfUAfUAACAAfUAfAGfGGfGCfUGsGsA-3'MVIP09 |
| AS198 | UsfCsGfUAfUAACAAfUAfAGfGGfGCfUGsGsA-3'MVIP09 |
| AS199 | AsfUsAfACfUCfUGUCCAfUUfACCAsUsU-3'MVIP09 |
| AS200 | UsfUsAfACTUCTUGUCCAfUUfACCAsUsU-3'MVIP09 |
| AS201 | UsfAsUfAAfCUfCUGUCCfAUfUACCAsUsU-3'MVIP09 |
| AS199 | AsfUsAfACfUCfUGUCCAfUUfACCAsUsU-3'MVIP09 |
| AS202 | AsfUsAfACfUCfUGUCCAfUUfACCAsUsG-3'MVIP09 |
| AS203 | AsfAsCfUCfUGfUCCAUUfACfCAUGsGsU-3'MVIP09 |
| AS199 | AsfUsAfACfUCfUGUCCAfUUfACCAsUsU-3'MVIP09 |
| AS199 | AsfUsAfACfUCfUGUCCAfUUfACCAsUsU-3'MVIP09 |
| AS199 | AsfUsAfACfUCfUGUCCAfUUfACCAsUsU-3'MVIP09 |
| AS204 | AsfUsAfACdTCfUGUCCAfUUfACCAsUsU-3'MVIP09 |
| AS205 | AsfUsAfACfUCfUGUCCAfUUfACCAsUsG-3'MVIP09 |
| AS205 | AsfUsAfACfUCfUGUCCAfUUfACCAsUsG-3'MVIP09 |
| AS206 | AsfUsAfACdTCfUGUCCAfUUfACCAsUsG-3'MVIP09 |

In some embodiments, the antisense strand of the RNA inhibitor of the present application is a sequence that has at least 15 consecutive nucleotides identical to the antisense strand in Table 14, or a sequence that differs from the antisense strand in Table 14 by 1, 2 or 3 nucleotides.

In some in vivo experimental embodiments, the RNA inhibitor of the present application is selected from the sequences in Table 15.

**Table 15 RNA inhibitors containing 5'MVIP09/3'MVIP09 combination**

| Double strands code | Sense strand sequence 5'-3' | Antisense strand sequence 5'-3' |
|---|---|---|
| Kylo-11-DS124 | 5'MVIP09- CsAsGCUCCUfUfAfUUGUU AUACsGsA | UsfCsGfUAfUAACAAfUAfAGf GAfGCsfUsG-3'MVIP09 |
| Kylo-11-DS125 | 5'MVIP09- GsCsCCCUUAfUfUfGUUAU ACsGsA | UsfCsGfUAfUAACAAfUAfAGf GGfGCsfUsG-3'MVIP09 |
| Kylo-11-DS126 | 5'MVIP09- CsAsGCCCCUfUfAfUUGUU AUACsGsA | UsfCsGfUAfUAACAAfUAfAGf GGfGCfUGsGsA-3'MVIP09 |
| Kylo-11-DS127 | 5'MVIP09- CsAsGCCCCUfUfAfUUGUU AUACGAsGsA | UsfCsGfUAfUAACAAfUAfAGf GGfGCfUGsGsA-3'MVIP09 |
| Kylo-11-DS128 | 5'MVIP09- UsCsCAGCCCfCfUfUAUUG UUAUACsGsA | UsfCsGfUAfUAACAAfUAfAGf GGfGCfUGsGsA-3'MVIP09 |
| Kylo-11-DS129 | 5'MVIP09- UsGsfGUfAAfUfGfGAfCAfG AfGUUAUsUsU | AsfUsAfACfUCfUGUCCAfUUf ACCAsUsU-3'MVIP09 |
| Kylo-11-DS130 | 5'MVIP09- UsGsfGUfAAfUfGfGAfCAfG AfGUUAUsUsU | UsfUsAfACfUCfUGUCCAfUUf ACCAsUsU-3'MVIP09 |
| Kylo-11-DS131 | 5'MVIP09- UsGsfGUfAAfUfGfGAfCAfG AfGUUAUsUsU | UsfAsUfAAfCUfCUGUCCfAUf UACCAsUsU-3'MVIP09 |
| Kylo-11-DS132 | 5'MVIP09- UsGsfGUfAAfUfGfGAfCAfG AfGUUsAsU | AsfUsAfACfUCfUGUCCAfUUf ACCAsUsU-3'MVIP09 |
| Kylo-11-DS133 | 5'MVIP09- UsGsfGUfAAfUGfGfAfCAfG AfGUUAUsUsU | AsfUsAfACfUCfUGUCCAfUUf ACCAsUsG-3'MVIP09 |
| Kylo-11-DS134 | 5'MVIP09- CsAsfUGfGUfAfAfUGfGAfC AfGAGUUsAsU | AsfAsCfUCfUGfUCCAUUfACfC AUGsGsU-3'MVIP09 |
| Kylo-11-DS135 | 5'MVIP09- UsGsfGUfAAfUfGfGAfCAfG AfGUUAUsCsG | AsfUsAfACfUCfUGUCCAfUUf ACCAsUsU-3'MVIP09 |
| Kylo-11-DS136 | 5'MVIP09- UsGsfGUfAAfUfGfGAfCAfG AfGUUAUsCsA | AsfUsAfACfUCfUGUCCAfUUf ACCAsUsU-3'MVIP09 |
| Kylo-11-DS137 | 5'MVIP09- CsGsfGUfAAfUfGfGAfCAfG AfGUUAUsCsA | AsfUsAfACfUCfUGUCCAfUUf ACCAsUsU-3'MVIP09 |
| Kylo-11-DS138 | 5'MVIP09- UsGsfGUfAAfUfGfGAfCAfG AfGUUAUsUsU | AsfUsAfACdTCfUGUCCAfUUf ACCAsUsU-3'MVIP09 |
| Kylo-11-DS139 | 5'MVIP09- UsGsfGUfAAfUfGfGAfCAfG AfGUUAUsCsA | AsfUsAfACfUCfUGUCCAfUUf ACCAsUsG-3'MVIP09 |
| Kylo-11-DS140 | 5'MVIP09- UsGsGUfAAfUfGfGACAGA GUUAUsCsA | AsfUsAfACfUCfUGUCCAfUUf ACCAsUsG-3'MVIP09 |
| Kylo-11-DS141 | 5'MVIP09- UsGsGUfAAfUfGfGACAGA GUUAUsCsA | AsfUsAfACdTCfUGUCCAfUUf ACCAsUsG-3'MVIP09 |

In some embodiments, the sense strand and antisense strand of the RNA inhibitor of the present application are sequences that have at least 15 consecutive nucleotides identical to the sense strand and antisense strand in Table 15, or sequences that differ from the sequences in Table 15 by 1, 2 or 3 nucleotides.

In some embodiments, the antisense strand of the RNA inhibitor of the present application is: UsfCsGfUAfUAACAAfUAfAGfGAfGCsfUsG (SEQ ID NO: 278), the 5' end and/or 3' end of which is connected to 5'MVIP and/or 3'MVIP of different structures, and the antisense strand connecting the ligand structure is selected from Table 16 below:

**Table 16 Antisense strands coupled with 5'MVIP and/or 3'MVIP**

| Antisense strand code | Antisense strand sequence 5'→3' |
|---|---|
| AS196 | UsfCsGfUAfUAACAAfUAfAGfGAfGCsfUsG-3'MVIP09 |
| AS207 | 5' MVIP17-UsfCsGfUAfUAACAAfUAfAGfGAfGCsfUsG |
| AS208 | UsfCsGfUAfUAACAAfUAfAGfGAfGCsfUsG -3' MVIP01 |
| AS209 | UsfCsGfUAfUAACAAfUAfAGfGAfGCsfUsG -3' MVIP17 |
| AS210 | 5' MVIP01-UsfCsGfUAfUAACAAfUAfAGfGAfGCsfUsG -3' MVIP01 |
| AS211 | 5' MVIP09-UsfCsGfUAfUAACAAfUAfAGfGAfGCsfUsG -3' MVIP09 |
| AS212 | 5' MVIP17-UsfCsGfUAfUAACAAfUAfAGfGAfGCsfUsG -3' MVIP17 |
| AS213 | 5' MVIP01-UsfCsGfUAfUAACAAfUAfAGfGAfGCsfUsG -3' MVIP17 |
| AS214 | 5' MVIP17-UsfCsGfUAfUAACAAfUAfAGfGAfGCsfUsG -3' MVIP01 |
| AS215 | 5' MVIP01-UsfCsGfUAfUAACAAfUAfAGfGAfGCsfUsG -3' MVIP09 |
| AS216 | 5' MVIP09-UsfCsGfUAfUAACAAfUAfAGfGAfGCsfUsG -3' MVIP01 |
| AS217 | 5' MVIP09-UsfCsGfUAfUAACAAfUAfAGfGAfGCsfUsG -3' MVIP17 |
| AS218 | 5' MVIP17-UsfCsGfUAfUAACAAfUAfAGfGAfGCsfUsG -3' MVIP09 |
| AS219 | 5' MVIP12-UsfCsGfUAfUAACAAfUAfAGfGAfGCsfUsG |
| AS220 | UsfCsGfUAfUAACAAfUAfAGfGAfGCsfUsG-3' MVIP19 |
| AS221 | 5'MVIP16- UsfCsGfUAfUAACAAfUAfAGfGAfGCsfUsG - 3'MVIP16 |
| AS222 | UsfCsGfUAfUAACAAfUAfAGfGAfGCsfUsG-3' MVIP17 |
| AS223 | UsfCsGfUAfUAACAAfUAfAGfGAfGCsfUsG-3' MVIP18 |
| AS224 | 5' MVIP03-UsfCsGfUAfUAACAAfUAfAGfGAfGCsfUsG |
| AS225 | 5' MVIP08-UsfCsGfUAfUAACAAfUAfAGfGAfGCsfUsG |
| AS226 | 5'MVIP16-UsfCsGfUAfUAACAAfUAfAGfGAfGCsfUsG |
| AS227 | 5'MVIP13-UsfCsGfUAfUAACAAfUAfAGfGAfGCsfUsG- 3'MVIP06 |
| AS228 | 5'MVIP04-UsfCsGfUAfUAACAAfUAfAGfGAfGCsfUsG- 3'MVIP06 |
| AS229 | 5' MVIP11-UsfCsGfUAfUAACAAfUAfAGfGAfGCsfUsG |
| AS230 | 5' MVIP11-UsfCsGfUAfUAACAAfUAfAGfGAfGCsfUsG-3' MVIP14 |
| AS231 | 5' MVIP15-UsfCsGfUAfUAACAAfUAfAGfGAfGCsfUsG |
| AS232 | 5' MVIP02-UsfCsGfUAfUAACAAfUAfAGfGAfGCsfUsG |
| AS233 | 5' MVIP05-UsfCsGfUAfUAACAAfUAfAGfGAfGCsfUsG |
| AS234 | 5' MVIP06-UsfCsGfUAfUAACAAfUAfAGfGAfGCsfUsG |
| AS235 | 5' MVIP07-UsfCsGfUAfUAACAAfUAfAGfGAfGCsfUsG |
| AS236 | 5' MVIP10- UsfCsGfUAfUAACAAfUAfAGfGAfGCsfUsG |
| AS237 | 5' MVIP14- UsfCsGfUAfUAACAAfUAfAGfGAfGCsfUsG |

| Antisense strand code | Antisense strand sequence 5'-3' |
|---|---|
| AS238 | 5' MVIP18- UsfCsGfUAfUAACAAfUAfAGfGAfGCsfUsG |
| AS239 | UsfCsGfUAfUAACAAfUAfAGfGAfGCsfUsG -3' MVIP02 |
| AS240 | UsfCsGfUAfUAACAAfUAfAGfGAfGCsfUsG-3' MVIP03 |
| AS241 | UsfCsGfUAfUAACAAfUAfAGfGAfGCsfUsG -3' MVIP04 |
| AS242 | 5' MVIP04-UsfCsGfUAfUAACAAfUAfAGfGAfGCsfUsG-3' MVIP04 |
| AS243 | 5' MVIP03-UsfCsGfUAfUAACAAfUAfAGfGAfGCsfUsG -3' MVIP19 |
| AS244 | 5' MVIP18- UsfCsGfUAfUAACAAfUAfAGfGAfGCsfUsG-3' MVIP18 |
| AS245 | 5' MVIP08- UsfCsGfUAfUAACAAfUAfAGfGAfGCsfUsG-3' MVIP18 |
| AS246 | UsfCsGfUAfUAACAAfUAfAGfGAfGCsfUsG -3' MVIP05 |
| AS247 | UsfCsGfUAfUAACAAfUAfAGfGAfGCsfUsG -3' MVIP07 |
| AS248 | UsfCsGfUAfUAACAAfUAfAGfGAfGCsfUsG -3' MVIP10 |
| AS249 | UsfCsGfUAfUAACAAfUAfAGfGAfGCsfUsG -3' MVIP11 |
| AS250 | 5' MVIP11- UsfCsGfUAfUAACAAfUAfAGfGAfGCsfUsG-3' MVIP11 |
| AS251 | 5' MVIP15- UsfCsGfUAfUAACAAfUAfAGfGAfGCsfUsG-3' MVIP15 |
| AS252 | UsfCsGfUAfUAACAAfUAfAGfGAfGCsfUsG -3' MVIP06 |
| AS253 | UsfCsGfUAfUAACAAfUAfAGfGAfGCsfUsG -3' MVIP08 |
| AS254 | UsfCsGfUAfUAACAAfUAfAGfGAfGCsfUsG -3' MVIP12 |
| AS255 | UsfCsGfUAfUAACAAfUAfAGfGAfGCsfUsG -3' MVIP13 |
| AS256 | UsfCsGfUAfUAACAAfUAfAGfGAfGCsfUsG -3' MVIP14 |
| AS257 | UsfCsGfUAfUAACAAfUAfAGfGAfGCsfUsG -3' MVIP15 |
| AS258 | UsfCsGfUAfUAACAAfUAfAGfGAfGCsfUsG -3' MVIP16 |
| AS259 | UsfCsGfUAfUAACAAfUAfAGfGAfGCsfUsG -3' MVIP24 |
| AS260 | UsfCsGfUAfUAACAAfUAfAGfGAfGCsfUsG -3' MVIP27 |
| AS261 | 5' MVIP19-UsfCsGfUAfUAACAAfUAfAGfGAfGCsfUsG |
| AS262 | 5' MVIP20-UsfCsGfUAfUAACAAfUAfAGfGAfGCsfUsG |
| AS263 | 5' MVIP21-UsfCsGfUAfUAACAAfUAfAGfGAfGCsfUsG |
| AS264 | 5' MVIP22-UsfCsGfUAfUAACAAfUAfAGfGAfGCsfUsG |
| AS265 | 5' MVIP01-UsfCsGfUAfUAACAAfUAfAGfGAfGCsfUsG |
| AS266 | 5' MVIP09-UsfCsGfUAfUAACAAfUAfAGfGAfGCsfUsG |

In some embodiments, the antisense strand of the RNA inhibitor of the present application is a sequence that has at least 15 consecutive nucleotides identical to the antisense strand in Table 16, or a sequence that differs from the antisense strand in Table 16 by 1, 2 or 3 nucleotides.

In some embodiments, the antisense strand of the RNA inhibitor of the present application could be obtained by coupling the antisense strand in Table 5-Table 8 with 5'MVIP and/or 3'MVIP.

In some embodiments, the antisense strand of the RNA inhibitor of the present application is obtained by coupling a sequence having at least 15 consecutive nucleotides identical to the antisense strand in Table 5-Table 8, or a sequence differing from the antisense strand in Table 5-Table 8 by 1, 2 or 3 nucleotides with 5'MVIP and/or 3'MVIP.

In some embodiments, the sense strand of the RNA inhibitor of the present application is: CsAsGCUCCUfUfAfUUGUUAUACsGsA (SEQ ID NO: 270), the 5' end and/or 3' end of which is connected to a 5'MVIP and/or 3'MVIP of different structures, and the sense strand connecting the ligand structure is selected from Table 17 below:

**Table 17 Sense strands coupled with 5'MVIP and/or 3'MVIP**

| Sense strand code | Sense strand sequence 5'-3' |
|---|---|
| S196 | 5'MVIP09-CsAsGCUCCUfUfAfUUGUUAUACsGsA |
| S207 | CsAsGCUCCUfUfAfUUGUUAUACsGsA - 3'MVIP17 |
| S208 | CsAsGCUCCUfUfAfUUGUUAUACsGsA -3'MVIP09 |
| S209 | CsAsGCUCCUfUfAfUUGUUAUACsGsA-3'MVIP01 |
| S210 | 5'MVIP17- CsAsGCUCCUfUfAfUUGUUAUACsGsA |
| S211 | 5'MVIP01- CsAsGCUCCUfUfAfUUGUUAUACsGsA |
| S212 | 5'MVIP01-CsAsGCUCCUfUfAfUUGUUAUACsGsA -3'MVIP01 |
| S213 | 5'MVIP09-CsAsGCUCCUfUfAfUUGUUAUACsGsA -3'MVIP09 |
| S214 | 5'MVIP17-CsAsGCUCCUfUfAfUUGUUAUACsGsA -3'MVIP17 |
| S215 | 5'MVIP01-CsAsGCUCCUfUfAfUUGUUAUACsGsA -3'MVIP17 |
| S216 | 5'MVIP17-CsAsGCUCCUfUfAfUUGUUAUACsGsA -3'MVIP01 |
| S217 | 5'MVIP01-CsAsGCUCCUfUfAfUUGUUAUACsGsA -3'MVIP09 |
| S218 | 5'MVIP09-CsAsGCUCCUfUfAfUUGUUAUACsGsA -3'MVIP01 |
| S219 | 5'MVIP09-CsAsGCUCCUfUfAfUUGUUAUACsGsA -3'MVIP17 |
| S220 | 5'MVIP17-CsAsGCUCCUfUfAfUUGUUAUACsGsA -3'MVIP09 |
| S221 | 5'MVIP12-CsAsGCUCCUfUfAfUUGUUAUACsGsA |
| S222 | CsAsGCUCCUfUfAfUUGUUAUACsGsA -3'MVIP19 |
| S223 | 5'MVIP16-CsAsGCUCCUfUfAfUUGUUAUACsGsA -3'MVIP16 |
| S224 | CsAsGCUCCUfUfAfUUGUUAUACsGsA -3'MVIP17 |
| S225 | CsAsGCUCCUfUfAfUUGUUAUACsGsA -3'MVIP18 |
| S226 | 5' MVIP03-CsAsGCUCCUfUfAfUUGUUAUACsGsA |
| S227 | 5' MVIP08-CsAsGCUCCUfUfAfUUGUUAUACsGsA |
| S228 | 5' MVIP16-CsAsGCUCCUfUfAfUUGUUAUACsGsA |
| S229 | 5'MVIP13-CsAsGCUCCUfUfAfUUGUUAUACsGsA -3'MVIP06 |
| S230 | 5'MVIP04-CsAsGCUCCUfUfAfUUGUUAUACsGsA -3'MVIP06 |
| S231 | 5'MVIP11-CsAsGCUCCUfUfAfUUGUUAUACsGsA |
| S232 | 5'MVIP11-CsAsGCUCCUfUfAfUUGUUAUACsGsA -3'MVIP14 |
| S233 | 5'MVIP15-CsAsGCUCCUfUfAfUUGUUAUACsGsA |
| S234 | 5'MVIP02-CsAsGCUCCUfUfAfUUGUUAUACsGsA |
| S235 | 5'MVIP05-CsAsGCUCCUfUfAfUUGUUAUACsGsA |
| S236 | 5'MVIP06-CsAsGCUCCUfUfAfUUGUUAUACsGsA |
| S237 | 5'MVIP07-CsAsGCUCCUfUfAfUUGUUAUACsGsA |
| S238 | 5'MVIP10-CsAsGCUCCUfUfAfUUGUUAUACsGsA |
| S239 | 5'MVIP14-CsAsGCUCCUfUfAfUUGUUAUACsGsA |
| S240 | 5'MVIP18-CsAsGCUCCUfUfAfUUGUUAUACsGsA |
| S241 | CsAsGCUCCUfUfAfUUGUUAUACsGsA -3' MVIP02 |
| S242 | CsAsGCUCCUfUfAfUUGUUAUACsGsA -3' MVIP03 |
| S243 | CsAsGCUCCUfUfAfUUGUUAUACsGsA -3' MVIP04 |
| S244 | 5' MVIP04-CsAsGCUCCUfUfAfUUGUUAUACsGsA -3' MVIP04 |
| S245 | 5' MVIP03-CsAsGCUCCUfUfAfUUGUUAUACsGsA -3' MVIP19 |
| S246 | 5' MVIP18-CsAsGCUCCUfUfAfUUGUUAUACsGsA -3' MVIP18 |
| S247 | 5' MVIP08-CsAsGCUCCUfUfAfUUGUUAUACsGsA -3' MVIP18 |
| S248 | CsAsGCUCCUfUfAfUUGUUAUACsGsA -3' MVIP05 |
| S249 | CsAsGCUCCUfUfAfUUGUUAUACsGsA -3' MVIP07 |
| S250 | CsAsGCUCCUfUfAfUUGUUAUACsGsA -3' MVIP10 |
| S251 | CsAsGCUCCUfUfAfUUGUUAUACsGsA -3' MVIP11 |
| S252 | 5' MVIP11-CsAsGCUCCUfUfAfUUGUUAUACsGsA -3' MVIP11 |
| S253 | 5' MVIP15-CsAsGCUCCUfUfAfUUGUUAUACsGsA -3' MVIP15 |
| S254 | CsAsGCUCCUfUfAfUUGUUAUACsGsA -3' MVIP06 |
| S255 | CsAsGCUCCUfUfAfUUGUUAUACsGsA -3' MVIP08 |
| S256 | CsAsGCUCCUfUfAfUUGUUAUACsGsA -3' MVIP12 |
| S257 | CsAsGCUCCUfUfAfUUGUUAUACsGsA -3' MVIP13 |
| S258 | CsAsGCUCCUfUfAfUUGUUAUACsGsA -3' MVIP14 |
| S259 | CsAsGCUCCUfUfAfUUGUUAUACsGsA -3' MVIP15 |
| S260 | CsAsGCUCCUfUfAfUUGUUAUACsGsA -3' MVIP16 |
| S261 | 5' MVIP19-CsAsGCUCCUfUfAfUUGUUAUACsGsA |
| S262 | 5' MVIP20-CsAsGCUCCUfUfAfUUGUUAUACsGsA |
| S263 | 5' MVIP21-CsAsGCUCCUfUfAfUUGUUAUACsGsA |
| S264 | 5' MVIP22-CsAsGCUCCUfUfAfUUGUUAUACsGsA |

In some embodiments, the sense strand of the RNA inhibitor of the present application is a sequence that has at least 15 consecutive nucleotides identical to the sense strand in Table 17, or a sequence that differs from the sense strand in Table 17 by 1, 2 or 3 nucleotides.

In some embodiments, the sense strand of the RNA inhibitor of the present application could be obtained by coupling the sense strand in Table 5-Table 8 with 5'MVIP and/or 3'MVIP.

In some embodiments, the sense strand of the RNA inhibitor of the present application is obtained by coupling a sequence having at least 15 consecutive nucleotides identical to the sense strand in Table 5-Table 8, or a sequence differing from the sense strand in Table 5-Table8 by 1, 2 or 3 nucleotides with 5'MVIP and/or 3'MVIP.

In some embodiments, the RNA inhibitor of the present application is formed by random pairing of the antisense strand in Table 16 or a sequence that differs from these antisense strands by 1, 2 or 3 nucleotides and the sense strand in Table 17 or a sequence that differs from these sense strands by 1, 2 or 3 nucleotides.

In some embodiments, the sense strand and/or antisense strand of the RNA inhibitor has at least 15 consecutive nucleotides identical to the sense strand and/or antisense strand in Table 19, or differs from them by 1, 2, or 3 nucleotides.

The patent CN113171371B provides a detailed examination of the effects of different X, L, B, D, R₁ and R₂ in the structures of 5'MVIP and/or 3'MVIP on the activity of RNA inhibitors. The full text of this patent is incorporated into this specification.

When X is galactose, galactosamine, N-acetylgalactosamine and their derivatives respectively, among the RNA inhibitors provided by the present application, N-acetylgalactosamine and its derivatives are preferably used as liver-targeting specific ligands.

| X code | X structure |
|---|---|
| X1 | 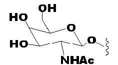 |
| X2 | 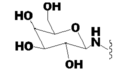 |
| X3 | 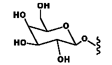 |
| X4 | 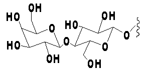 |
| X5 | 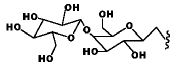 |
| X6 | 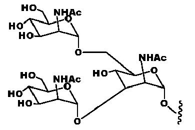 |

The length of L has a greater impact on the effect of RNA inhibitors, L chain cannot be too short or too long. When L contains -NH-, C=O, O, S, amido, phosphoryl, thiophosphoryl, aliphatic carbocyclyl such as cyclohexane, or a combination of these groups, or has different structures in the same 5'MVIP, 3'MVIP structures or between 5'MVIP and 3'MVIP, the activities of the resulting RNA inhibitors do not differ significantly in the range of chain length of C7-C18.

| L code | L structure |
|---|---|
| L1 | 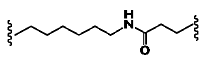 |
| L2 | 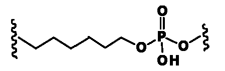 |
| L3 | 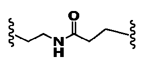 |
| L4 | 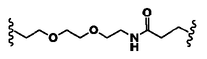 |
| L5 | 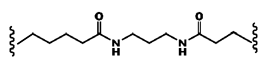 |
| L6 | 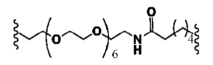 |
| L7 | 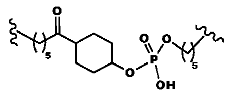 |
| L8 | 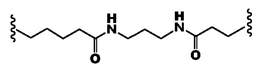 |
| L9 | 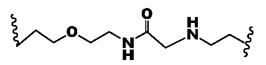 |
| L10 | 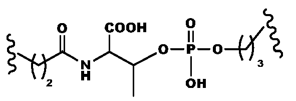 |
| L11 | L in the S of 5' MVIP: |
| | 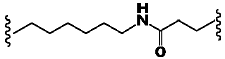 |
| | L in the AS of 3'MVIP : |
| | 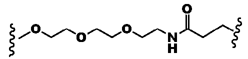 |
| L12 | L in the S of 5' MVIP: |
| | 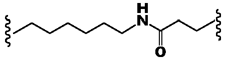 |
| | L in the AS of 3'MVIP : |
| | 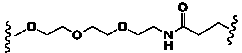 |
| L13 | L in the S of 5' MVIP: |
| | 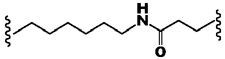 |
| | L in the AS of 3'MVIP: |
| | 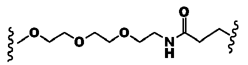 |
| L14 | 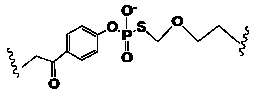 |

Except for the change in the structure of linker B, when X, L, D and R₁/R₂ are consistent with those in the combination 5'MVIP09/3'MVIP09, A₁ and A₂ in the general formula of linker B are each independently C, O, S, -NH-, carbonyl, amido, phosphoryl or thiophosphoryl, and r is any integer of 0-4, and when linker B is the same or different between 5'MVIP and 3'MVIP, the activities of resulting RNA inhibitors do not differ significantly.

| B code | B structure | |
|---|---|---|
| B1 | 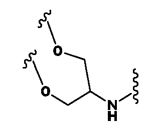 | |
| B2 | 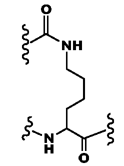 | |
| B3 | 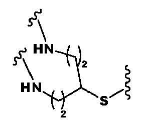 | |
| B4 | 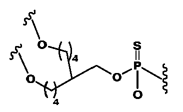 | |
| B5 | 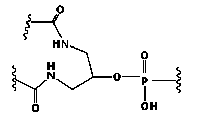 | |
| B6 | 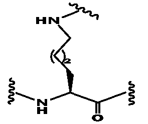 | |
| B7 | 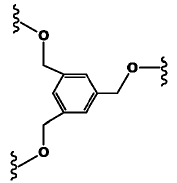 | |
| B8 | B in the S of 5'MVIP: | |
| | 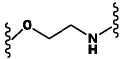 | |
| | B in the AS of 3'MVIP: | |
| | 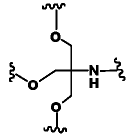 | |
| B9 | B in the S of 5'MVIP: | |
| | 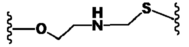 | |
| | B in the AS of 3'MVIP: | |
| | 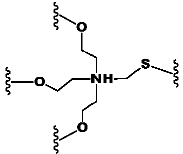 | |
| B10 | B in the S of 5'MVIP: | |
| | 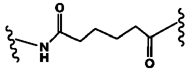 | |
| | B in the AS of 3'MVIP: | |
| | 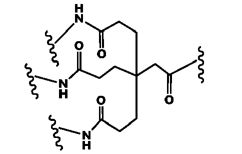 | |
| B11 | B in the S of 5'MVIP: | |
| | 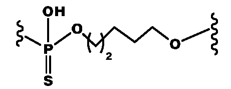 | |
| | B in the AS of 3'MVIP: | |
| | 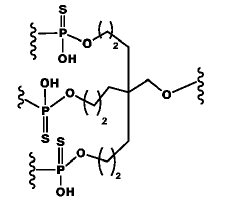 | |
| B12 | B in the S of 5'MVIP: | |
| | 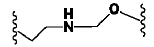 | |
| | B in the AS of 3'MVIP: | |
| | 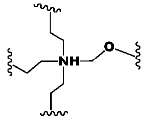 | |
| B13 | B in the S of 5'MVIP: | |
| | 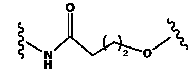 | |
| | B in the AS of 3'MVIP: | |
| | 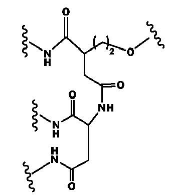 | |
| B14 | B in the S of 5'MVIP: | |
| | 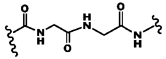 | |
| | B in the AS of 3'MVIP: | |
| | 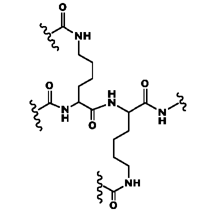 | |
| B15 | B in the S of 5'MVIP: | |
| | 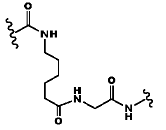 | |
| | B in the AS of 3'MVIP: | |
| | 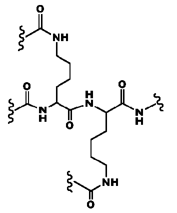 | |
| B16 | B in the S of 5'MVIP: | |
| | 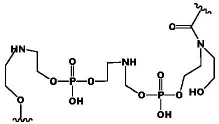 | |
| | B in the AS of 3'MVIP: | |
| | 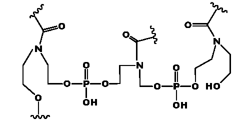 | |
| B17 | B in the S of 5'MVIP: | |
| | 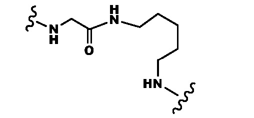 | |
| | B in the AS of 3'MVIP: | |
| | 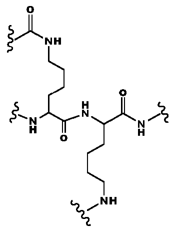 | |
| B18 | B in the S of 5'MVIP: | |
| | 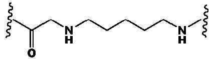 | |
| | B in the AS of 3'MVIP: | |
| | 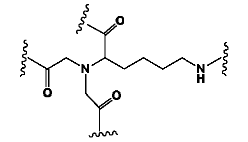 | |
| B19 | B in the S of 5'MVIP: | |
| | 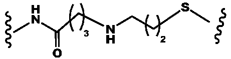 | |
| | B in the AS of 3'MVIP: | |
| | 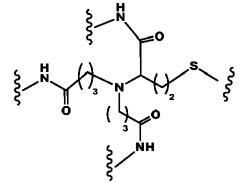 | |
| B20 | 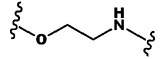 | |
| B21 | 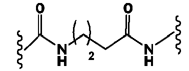 | |
| B22 | 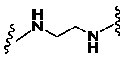 | |
| B23 | 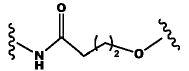 | |
| B24 | 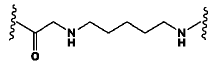 | |
| B25 | 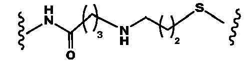 | |
| B26 | 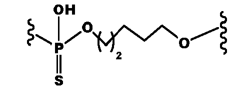 | |
| B27 | B in the S of 5'MVIP: | |
| | 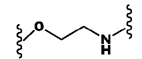 | |
| | B in the AS of 3'MVIP: | |
| | 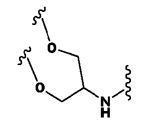 | |
| B28 | B in the S of 5'MVIP: | |
| | 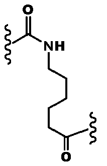 | |
| | B in the AS of 3'MVIP: | |
| | 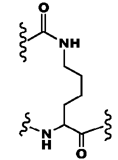 | |
| B29 | B in the S of 5'MVIP: | |
| | 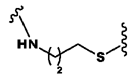 | |
| | B in the AS of 3'MVIP: | |
| | 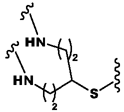 | |
| B30 | B in the S of 5'MVIP: | 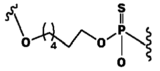 |
| | B in the AS of 3'MVIP: | 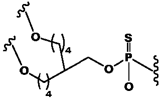 |
| B31 | B in the S of 5'MVIP: | 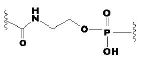 |
| | B in the AS of 3'MVIP: | 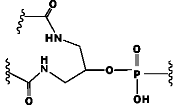 |
| B32 | B in the S of 5'MVIP: | |
| | 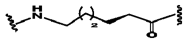 | |
| | B in the AS of 3'MVIP: | |
| | 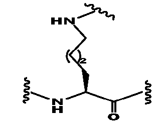 | |
| B33 | B in the S of 5'MVIP: | |
| | 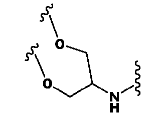 | |
| | B in the AS of 3'MVIP: | |
| | 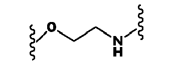 | |
| B34 | B in the S of 5'MVIP: | |
| | 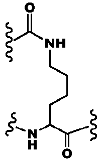 | |
| | B in the AS of 3'MVIP: | |
| | 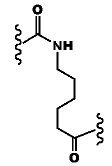 | |
| B35 | B in the S of 5'MVIP: | |
| | 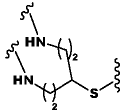 | |
| | B in the AS of 3'MVIP: | |
| | 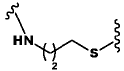 | |
| B36 | B in the S of 5'MVIP: | 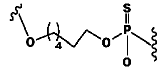 |
| | B in the AS of 3'MVIP: | 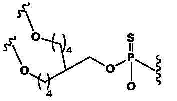 |
| B37 | B in the S of 5'MVIP: | 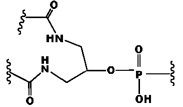 |
| | B in the AS of 3'MVIP: | 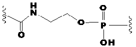 |
| B38 | B in the S of 5'MVIP: | |
| | 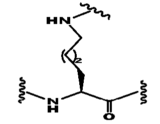 | |
| | B in the AS of 3'MVIP: | |
| | 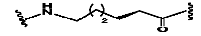 | |

When MVIP structure and RNA inhibitor are the same, different connecting chains D will affect the activities of the RNA inhibitors, among which D1, D2 and D4 have similar effects and are better than D3.

| D code | D structure |
|---|---|
| D1 | D in S of 5'MVIP: |
| | 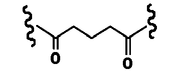 |
| | D in AS of 3'MVIP: |
| | 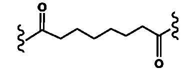 |
| D2 | D in S of 5'MVIP: |
| | 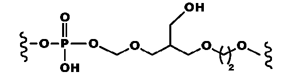 |
| | D in AS of 3'MVIP: |
| | 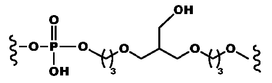 |
| D3 | D in S of 5'MVIP: |
| | 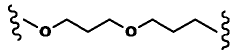 |
| | D in AS of 3'MVIP: |
| | 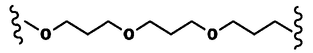 |
| D4 | D in S of 5'MVIP: |
| | 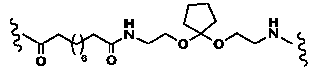 |
| | D in AS of 3'MVIP: |
| | 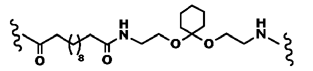 |
| D5 | D in S of 5'MVIP: |
| | 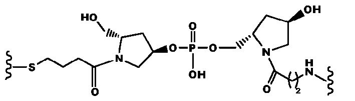 |
| | D in AS of 3'MVIP: |
| | 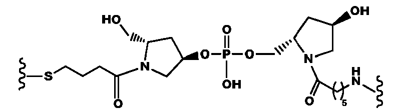 |

Different transition points R₁ will affect the activities of RNA inhibitors, among which the activity of obtained RNA inhibitor using R1-1 as the transition point is the best.

| R₁ code | R₁ structure |
|---|---|
| R1-1 | -NH(CH₂)₆O- |
| R1-2 | -O(CH₂)₆O- |
| R1-3 | -S(CH₂)₆O- |
| R1-4 | -NH(CH₂)₈ O- |
| R1-5 | -NH(CH₂)₅CH(CH₂CH₃)O- |
| R1-6 | -S(CH₂)₄CH(CH₃)O- |

Different transition points R₂ will affect the activities of RNA inhibitors, among which the resulting RNA inhibitor using R2-1 as the transition point is the best.

| R₂ code | R₂ structure |
|---|---|
| R2-1 | 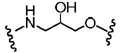 |
| R2-2 | 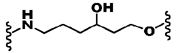 |
| R2-3 | 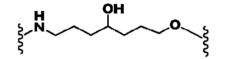 |
| R2-4 | 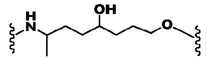 |
| R2-5 | 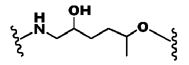 |
| R2-6 | 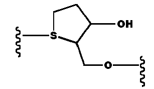 |
| R2-7 | 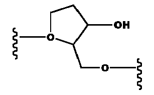 |
| R2-8 | 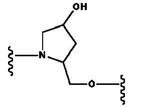 |
| R2-9 | 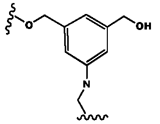 |
| R2-10 | 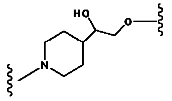 |
| R2-11 | 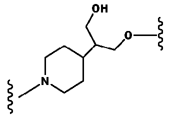 |
| R2-12 | 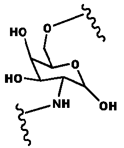 |

In some embodiments, the n+m of the RNA inhibitors of the present application are 2, 3, 4, 5 and 6, respectively. The positions for 5'MVIP and/or 3'MVIP coupling include the 5' end and/or 3' end of antisense strand, the 5' end and/or 3' end of sense strand, the 5' end of antisense strand and 3' end of sense strand, and the 5' end of sense strand and 3' end of antisense strand.

In some embodiments, the n+m of the RNA inhibitors of the present application are 2, 3, 4, 5 and 6, respectively. The positions for 5'MVIP and/or 3'MVIP coupling include the 5' end and/or 3' end of antisense strand in Table 5-Table 8, the 5' end and/or 3' end of sense strand in Table 5-Table 8, the 5' end of antisense strand and 3' end of sense strand in Table 5-Table 8, and the 5' end of sense strand and 3' end of antisense strand in Table 5-Table 8, the obtained 5'MVIP and 3'MVIP combinations are shown in Table 18:

**Table 18 5'MVIP and 3'MVIP combination list**

| Ligand structure | | Ligand structure | | n+ m | Position of the ligand structure coupled to the sense strand and/or antisense strand (S:AS) |
|---|---|---|---|---|---|
| 5'MVIP09 | n=2 | 3'MVIP09 | m=2 | 4 | n:m |
| 5'MVIP01 | n=1 | 3' MVIP01 | m=1 | 2 | n:m |
| 5'MVIP21 | n=1 | 3' MVIP01 | m=1 | 2 | n:m |
| 5'MVIP01 | n=1 | 3' MVIP07 | m=1 | 2 | n:m |
| 5'MVIP01/3'MVIP 01 | n=1/m= 1 | / | / | 2 | nm:/ |
| 3'MVIP01 | m=1 | 5'MVIP01 | n=1 | 2 | m:n |
| 3' MVIP07 | m=1 | 5'MVIP21 | n=1 | 2 | m:n |
| 5'MVIP01 | n=1 | 3'MVIP09 | m=2 | 3 | n:m |
| 5' MVIP08 | n=1 | 3'MVIP 15 | m=2 | 3 | n:m |
| 5'MVIP09 | n=2 | 3'MVIP01 | m=1 | 3 | n:m |
| 5' MVIP19 | n=2 | 3' MVIP07 | m=1 | 3 | n:m |
| 5'MVIP01/3'MVIP 09 | n=1/m= 2 | / | / | 3 | nm:/ |
| 5'MVIP09/3'MVIP 01 | n=2/m= 1 | / | / | 3 | nm:/ |
| 3'MVIP09 | m=2 | 5'MVIP01 | n=1 | 3 | m:n |
| 3'MVIP11 | m=2 | 5'MVIP06 | n=1 | 3 | m:n |
| 3'MVIP01 | m=1 | 5' MVIP20 | n=2 | 3 | m:n |
| 3' MVIP07 | m=1 | 5' MVIP19 | n=2 | 3 | m:n |
| 5'MVIP01 | n=1 | 3' MVIP17 | m=3 | 4 | n:m |
| 5' MVIP21 | n=1 | 3' MVIP17 | m=3 | 4 | n:m |
| 5'MVIP17 | n=3 | 3' MVIP01 | m=1 | 4 | n:m |
| 5' MVIP22 | n=3 | 3' MVIP07 | m=1 | 4 | n:m |
| 5' MVIP20 | n=2 | 3' MVIP10 | m=2 | 4 | n:m |
| 5' MVIP19 | n=2 | 3'MVIP 15 | m=2 | 4 | n:m |
| 5' MVIP10 | n=2 | 3'MVIP12 | m=2 | 4 | n:m |
| 5'MVIP09/3'MVIP 09 | n=2/m= 2 | / | / | 4 | nm:/ |
| 3'MVIP09 | m=2 | 5' MVIP09 | n=2 | 4 | m:n |
| 3'MVIP10 | m=2 | 5' MVIP20 | n=2 | 4 | m:n |
| 3'MVIP01 | m=1 | 5' MVIP17 | n=3 | 4 | m:n |
| 3'MVIP02 | m=1 | 5' MVIP18 | n=3 | 4 | m:n |
| 3'MVIP17 | m=3 | 5' MVIP01 | n=1 | 4 | m:n |
| 3'MVIP18 | m=3 | 5' MVIP21 | n=1 | 4 | m:n |
| 5'MVIP09 | n=2 | 3' MVIP17 | m=3 | 5 | n:m |
| 5'MVIP20 | n=2 | 3' MVIP19 | m=3 | 5 | n:m |
| 5'MVIP17 | n=3 | 3'MVIP09 | m=2 | 5 | n:m |
| 5'MVIP22 | n=3 | 3'MVIP 11 | m=2 | 5 | n:m |
| 5'MVIP09/3'MVIP 17 | n=2/m= 3 | / | / | 5 | nm:/ |
| 5'MVIP17/3'MVIP 09 | n=3/m= 2 | / | / | 5 | nm:/ |
| 3'MVIP17 | m=3 | 5' MVIP20 | n=2 | 5 | m:n |
| 3'MVIP19 | m=3 | 5' MVIP16 | n=2 | 5 | m:n |
| 3'MVIP09 | m=2 | 5' MVIP17 | n=3 | 5 | m:n |
| 3' MVIP15 | m=2 | 5' MVIP17 | n=3 | 5 | m:n |
| 5'MVIP17 | n=3 | 3' MVIP17 | m=3 | 6 | n:m |
| 5' MVIP22 | n=3 | 3' MVIP19 | m=3 | 6 | n:m |
| 5'MVIP17/3'MVIP 17 | n=3/m= 3 | / | | 6 | nm:/ |
| 3'MVIP17 | m=3 | 5' MVIP17 | n=3 | 6 | m:n |
| 3'MVIP18 | m=3 | 5' MVIP22 | n=3 | 6 | m:n |

In some embodiments, n and m are each independently any integer of 0-4, each independently preferably an integer of 1-3, and n+m= an integer of 2-6, preferably n+m= 2, 3 or 4, more preferably 4.

In some embodiments, the RNA inhibitor is selected from Table 19:

**Table 19 RNA inhibitor**

| Double strands code | Sense strand sequence 5'-3' | Antisense strand sequence 5'-3' |
|---|---|---|
| Kylo-11-DS146 | 5'MVIP09- CsAsGCUCCUfUfAfUUGUU AUACsGsA | UsfCsGfUAfUAACAAfUAfA GfGAfGCsfUsG-3'MVIP09 |
| Kylo-11-DS147 | 5'MVIP09- GsCsCCCUUAfUfUfGUUAU ACsGsA | UsfCsGfUAfUAACAAfUAfA GfGGfGCsfUsG-3'MVIP09 |
| Kylo-11-DS148 | 5'MVIP09- UsGsGUfAAfUfGfGACAGA GUUAUsCsA | AsfUsAfACdTCfUGUCCAfUU fACCAsUsG-3'MVIP09 |
| Kylo-11-DS149 | 5'MVIP09- CsAsGCCCCUfUfAfUUGUU AUACGAsGsA | UsfCsGfUAfUAACAAfUAfA GfGGfGCfUGsGsA-3'MVIP09 |
| Kylo-11-DS150 | 5'MVIP09- UsCsCAGCCCfCfUfUAUUG UUAUACsGsA | UsfCsGfUAfUAACAAfUAfA GfGGfGCfUGsGsA-3'MVIP09 |
| Kylo-11-DS151 | 5'MVIP09- UsGsfGUfAAfUfGfGAfCAfG AfGUUAUsUsU | AsfUsAfACfUCfUGUCCAfUU fACCAsUsU-3'MVIP09 |
| Kylo-11-DS152 | 5'MVIP09- UsGsfGUfAAfUfGfGAfCAfG AfGUUAUsUsU | UsfUsAfACfUCfUGUCCAfUU fACCAsUsU-3'MVIP09 |
| Kylo-11-DS153 | 5'MVIP09- UsGsfGUfAAfUfGfGAfCAfG AfGUUAUsUsU | UsfAsUfAAfCUfCUGUCCfAU fUACCAsUsU-3'MVIP09 |
| Kylo-11-DS154 | 5'MVIP09- UsGsfGUfAAfUfGfGAfCAfG AfGUUsAsU | AsfUsAfACfUCfUGUCCAfUU fACCAsUsU-3'MVIP09 |
| Kylo-11-DS155 | 5'MVIP09- UsGsfGUfAAfUGfGfAfCAfG AfGUUAUsUsU | AsfUsAfACfUCfUGUCCAfUU fACCAsUsG-3'MVIP09 |
| Kylo-11-DS156 | 5'MVIP09- CsAsfUGfGUfAfAfUGfGAfC AfGAGUUsAsU | AsfAsCfUCfUGfUCCAUUfAC fCAUGsGsU-3'MVIP09 |
| Kylo-11-DS157 | 5'MVIP09- UsGsfGUfAAfUfGfGAfCAfG AfGUUAUsCsG | AsfUsAfACfUCfUGUCCAfUU fACCAsUsU-3'MVIP09 |
| Kylo-11-DS158 | 5'MVIP09- UsGsfGUfAAfUfGfGAfCAfG AfGUUAUsCsA | AsfUsAfACfUCfUGUCCAfUU fACCAsUsU-3'MVIP09 |
| Kylo-11-DS159 | 5'MVIP09- CsGsfGUfAAfUfGfGAfCAfG AfGUUAUsCsA | AsfUsAfACfUCfUGUCCAfUU fACCAsUsU-3'MVIP09 |
| Kylo-11-DS160 | 5'MVIP09- UsGsfGUfAAfUfGfGAfCAfG AfGUUAUsUsU | AsfUsAfACdTCfUGUCCAfUU fACCAsUsU-3'MVIP09 |
| Kylo-11-DS161 | 5'MVIP09- UsGsfGUfAAfUfGfGAfCAfG AfGUUAUsCsA | AsfUsAfACfUCfUGUCCAfUU fACCAsUsG-3'MVIP09 |
| Kylo-11-DS162 | 5'MVIP09- UsGsGUfAAfUfGfGACAGA GUUAUsCsA | AsfUsAfACfUCfUGUCCAfUU fACCAsUsG-3'MVIP09 |
| Kylo-11-DS163 | 5'MVIP09- CsAsGCCCCUfUfAfUUGUU AUACsGsA | UsfCsGfUAfUAACAAfUAfA GfGGfGCfUGs-3'MVIP09 |

In some embodiments, the sense strand and/or antisense strand of the RNA inhibitor is a sequence having at least 15 consecutive nucleotides identical to the sense strand and/or antisense strand in Table 19, or a sequence differing from the sense strand and/or antisense strand in Table 19 by 1, 2 or 3 nucleotides.

In some embodiments, the RNA inhibitor or pharmaceutically acceptable salt thereof of the present application is preferably prepared or synthesized in the form of sodium salt and triethylamine salt or other pharmaceutically acceptable salt.

In some embodiments, the RNA inhibitor or pharmaceutically acceptable salt thereof of the present application is more preferably sodium salt or triethylamine salt thereof.

In another aspect, the present application also provides a pharmaceutical composition comprising the above-mentioned RNA inhibitor or pharmaceutically acceptable salt thereof.

In one embodiment, the present application provides a pharmaceutical composition comprising the above-mentioned RNA inhibitor or pharmaceutically acceptable salt thereof and optional pharmaceutically acceptable adjuvants. The pharmaceutical composition containing the above-mentioned RNA inhibitor provided by the present application can be used to prevent and/or treat some disorders, such as hypertension. Such pharmaceutical compositions are formulated according to the mode of delivery. An embodiment is to formulate as a composition for systemic administration by parenteral delivery, such as, subcutaneous (SC), intramuscular (IM), or intravenous (IV) delivery. The pharmaceutical composition of the present application can be administered at a dose sufficient to inhibit LPA gene expression.

The pharmaceutically acceptable "adjuvants" or "excipients" are pharmaceutically acceptable solvents, suspensions or any other pharmaceutically inert vectors used to deliver one or more nucleic acids to animals. The excipients may be liquid or solid and are selected taking account of the intended mode of administration to provide the required volume, consistency, etc. when combined with nucleic acids and other components in a given pharmaceutical composition. The RNA inhibitor of the present application can be delivered in a manner that targets specific tissues (e.g., hepatocytes).

In some embodiments, the pharmaceutical composition of the present application further comprises a delivery vector (such as nanoparticles, dendrimers, polymers, liposomes, or cation delivery systems).

In some embodiments, the delivery vector of the present application includes liposomes.

In some embodiments, the delivery vector of the present application includes nanolipids, which can form liposome-nucleic acid nanoparticles with nucleic acid molecules.

In some embodiments, the delivery vector of the present application includes the amphoteric lipid compounds M10C1.

The pharmaceutical compositions provided by the present application include, but are not limited to, solutions, emulsions and formulations containing liposomes. These compositions can be produced from a variety of components, including, but not limited to, preformed liquids, self-emulsifying solids and self-emulsifying semisolids. The formulations include those targeted to the liver. The pharmaceutical formulations of the present application, which can be conveniently presented in unit dosage form, can be prepared according to conventional techniques known in the pharmaceutical industry. Such techniques include the steps of combining the active ingredients with pharmaceutically acceptable excipients or vectors.

### Purpose

In another aspect, the present application provides a method for reducing LPA mRNA or protein expression in cells or tissues, comprising contacting the cells or tissues with an effective amount of the aforementioned RNA inhibitor or pharmaceutically acceptable salt thereof for inhibiting LPA gene expression, and/or the aforementioned pharmaceutical composition.

Cells suitable for treatment using the method of the present application can be any cells expressing LPA gene, such as liver cells, brain cells, gallbladder cells, heart cells or kidney cells, but preferably liver cells. Cells suitable for use in the method of the present application can be mammalian cells. When in contact with cells expressing LPA gene, RNA inhibitor inhibits the expression of LPA gene (for example, human, primate, non-primate or rat LPA gene) by at least about 50%, for example, as determined by PCR or methods based on branched DNA (bDNA), or methods based on protein, such as immunofluorescence analysis, Western blotting or flow cytometry.

In some embodiments, the tissue is liver tissue.

In some embodiments, the cells and tissues are ex vivo.

In some embodiments, the cells and tissues are in vivo in a subject.

The term "inhibit" as used in the present application may be used interchangeably with "reduce," "lower," "silence," "downregulate," "suppress" and other similar terms, and includes any level of inhibition. The expression of the LPA gene may be evaluated based on the level or change in level of any variables related to the expression of the LPA gene, for example, the level of LPA mRNA. This level may be analyzed in a single cell or in a population of cells (including, for example, a sample derived from a subject). The control level may be any type of control level used in the art, for example, a baseline level before administration or a level measured from a similar subject, cell, or sample that has not been treated or treated with a control (such as, for example, a buffer-only control or a no-active-agent control).

The inhibition of LPA gene expression can be manifested by a decrease in the amount of mRNA expressed by the first cell or cell population that inhibit LPA gene expression (such cells may be, for example, present in a sample derived from a subject) in which the LPA gene is transcribed and treated (for example, by contacting one or more cells with the RNA inhibitor of the present application, or by administering the RNA inhibitor of the present application to a subject in which the cell is present) compared to a second cell or cell population (control cells not treated with RNA inhibitor or not treated with RNA inhibitor targeting the gene of interest) that is essentially the same as that first cell or cell population but not so treated.

In a preferred embodiment, evaluation was performed in cell lines that highly express LPA by using appropriate concentrations of siRNA, and the mRNA level in the interfered cells is expressed as a percentage of the mRNA level in the uninterfered control cells.

In other embodiments, the inhibition of LPA gene expression can be evaluated by the reduction of parameters functionally related to LPA gene expression, such as LP(a) levels in the blood or serum of subjects. LPA gene inhibition can be measured in any cell expressing LPA (either endogenous or exogenous from an expression construct) and by any assay known in the art.

The inhibition of LPA expression can be manifested by the reduction of LP(a) levels expressed by cells or cell populations or subject samples (e.g., protein levels in blood samples derived from subjects).

Control cells, cell populations, or subject samples that can be used to evaluate LPA gene inhibition include cells, cell populations, or subject samples that have not been contacted with the RNA inhibitor of the present application. For example, control cells, cell populations, or subject samples can be derived from a single subject (e.g., a human or animal subject) or an appropriately matched group control before treatment with the RNA inhibitor.

LPA mRNA levels expressed by cells or cell populations can be determined using any method known in the art for evaluating mRNA expression. For example, qRT-PCR is used to evaluate the reduction of gene expression. Any method known in the art, for example, ELISA could be used to evaluate the reduction in protein production. In some embodiments, the liver biopsy sample is used as tissue material for monitoring the reduction of LPA gene expression. In other embodiments, blood samples are used as subject samples for monitoring the reduction of LP(a) expression.

In yet another aspect, the present application provides use of the above-mentioned RNA inhibitor or pharmaceutically acceptable salt thereof for inhibiting LPA gene expression, or the above-mentioned pharmaceutical composition in the preparation of a drug, wherein the drug is used to prevent and/or treat a disease or condition or to reduce the risk of a disease or condition.

In some embodiments, the disease or condition described in the present application includes a disease or condition associated with the LPA gene, such as cardiovascular disease.

In some embodiments, the disease or condition described in the present application is selected from: hyperlipoprotein(a)emia, hyperlipidemia, stroke, atherosclerosis, thrombosis, coronary heart disease, aortic valve stenosis, vascular disease, myocardial infarction, angina pectoris, renal disease, renal failure, obesity, glucose intolerance, type 2 diabetes (non-insulin-dependent diabetes) and metabolic syndrome.

In yet another aspect, the present application provides a method for preventing and/or treating diseases or conditions, which includes administering to a subject in need an effective amount of the above-mentioned RNA inhibitor or pharmaceutically acceptable salt thereof that inhibits LPA gene expression, and/or the above-mentioned pharmaceutical composition.

The in vivo method of the present application may comprise administering to a subject a pharmaceutical composition comprising an RNA inhibitor, wherein the RNA inhibitor comprises a nucleotide sequence complementary to at least a portion of LPA mRNA of a mammal receiving the administration of the RNA inhibitor. The pharmaceutical composition of the present application may be administered in any manner known in the art, including (but not limited to): oral, intraperitoneal or parenteral routes, including intracranial (e.g., intraventricular, intraparenchymal and intrathecal), intravenous, intramuscular, subcutaneous, transdermal, airway (aerosol), nasal, rectal and local (including buccal and sublingual) administration. In some embodiments, the pharmaceutical composition is administered by intravenous infusion or injection. In some embodiments, the pharmaceutical composition is administered by subcutaneous injection. In some embodiments, the composition is administered by intramuscular injection.

The RNA inhibitor of the present application can also be administered as a "free RNA inhibitor". The free RNA inhibitors are administered in the absence of a pharmaceutical composition. Naked RNA inhibitors can be in a suitable buffer. The buffer may contain acetate, citrate, prolamin, carbonate or phosphate, or any combination thereof. In one embodiment, the buffer is phosphate buffered saline (PBS). The pH and osmotic pressure of the buffer containing RNA inhibitor can be adjusted to be suitable for administration to subjects.

Alternatively, the RNA inhibitor of the present application may be administered as a pharmaceutical composition, such as a liposome preparation.

The pharmaceutical composition of the present application can be administered at a dosage sufficient to inhibit LPA gene expression. Generally, the appropriate dosage of the RNA inhibitor of the present application is in the range of about 0.001 to about 200.0 mg per kilogram of recipient body weight per day, typically in the range of about 1 to 50 mg per kilogram body weight per day. Generally, the appropriate dosage of the RNA inhibitor of the present application is in the range of about 0.1 mg/kg to about 5.0 mg/kg, for example, in the range of about 0.3mg/kg to about 3.0mg/kg.

In one embodiment, the method includes administering the pharmaceutical composition of the present application such that the target LPA gene expression is reduced for, such as approximately 1, 2, 3, 4, 5, 6, 1-6, 1-3 or 3-6 months per dose. In some embodiments, the composition is administered every 3-6 months.

In some embodiments, after the initial treatment regimen, treatment is administered at less frequency. The repeated dosage regimen may include regular administration of therapeutic amounts of the RNA inhibitor, such as once a month to once a year. In some embodiments, the RNA inhibitor is administered approximately once a month to approximately once every three months, or approximately once every three months to approximately once every six months.

After the initial treatment regimen, the RNA inhibitor could be administered less frequently for treatment. The duration of treatment could be determined according to the severity of the disease.

In other embodiments, a single dose of the pharmaceutical composition may be long-acting, such that the dose is administered at intervals of no more than 1, 2, 3, or 4 months. In some embodiments of the present application, a single dose of the pharmaceutical composition of the present application is administered approximately once a month. In other embodiments of the present application, a single dose of the pharmaceutical composition of the present application is administered quarterly (i.e. about every 3 months). In other embodiments of the present application, a single dose of the pharmaceutical composition of the present application is administered twice a year (i.e., about once every 6 months).

The person skilled in the art should understand that certain factors can affect the dosage and duration of administration required to effectively treat a subject, including (but not limited to): the mutation existing in the subject, previous treatment, the general health/age and presence of other diseases of the subject. In addition, preventing and/or treating the subject as needed may include a single treatment or a series of treatments.

In some embodiments, the method further includes determining LP(a) levels in samples from the subject.

For example, the method further includes determining LP(a) level in blood sample, serum sample or urine sample from the subject.

In some embodiments, the method further includes administering to the subject an additional therapeutic agent for the treatment of hyperlipoprotein(a)emia, hyperlipidemia, stroke, atherosclerosis, thrombosis, coronary heart disease and aortic valve stenosis.

For example, the additional therapeutic agent could be selected from: statins, such as atorvastatin, rosuvastatin etc.; cholesterol absorption inhibitors such as ezetimibe; PCSK9 inhibitors; ANGPTL3 inhibitors; APOC3 inhibitors and AGT inhibitors.

In yet another aspect, the present application provides a cell comprising the above-mentioned RNA inhibitor or pharmaceutically acceptable salt thereof for inhibiting LPA gene expression.

In yet another aspect, the present application provides a drug kit comprising the above-mentioned RNA inhibitor or pharmaceutically acceptable salt thereof for inhibiting LPA gene expression, or the above-mentioned pharmaceutical composition.

Without being bound by any theory, the examples in the following are only for the purpose of explaining the RNA inhibitor, preparation method and use of the present application, and are not used to limit the scope of the invention of the application.

### Examples

### Description:

The name of DMSO is dimethyl sulfoxide;
The name of DMF is N, N-dimethylformamide;
The name of HOBt is 1-hydroxybenzotriazole;
The name of HBTU is O-benzotriazole-tetramethylurea hexafluorophosphate;
The name of DIPEA (DIEA) is N, N-diisopropylethylamine;
The name of DCM is dichloromethane;
The name of DMAP is 4-dimethylaminopyridine;
The name of DMT-CL is 4,4'- dimethoxytriphenylchloromethane;
The name of MEOH is methanol;
The name of TBTU is O-benzotriazole-N, N, N', N'-tetramethylurea tetrafluoroboric acid;
The name of 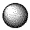 is solid-phase support, such as macroporous aminomethyl resin (Resin).

### Example 1 Synthesis of RNA inhibitors

The sense strand and antisense strand not coupled to the ligand structure were synthesized by a standard solid-phase phosphoramidite method using a multi-channel solid-phase synthesizer, and then the sense strand and the corresponding antisense strand were complementary annealed to yield the corresponding RNA inhibitor.

The basic steps of the solid-phase phosphoramidite method include:
1) Deprotection: removing the hydroxyl protecting group (DMTr) of starting monomer Solid Support;
2) Coupling: adding the first phosphoramidite monomer to occur coupling reaction in the 3' to 5' direction;
3) Oxidation: oxidizing the resulting nucleoside phosphite to a more stable nucleoside phosphate (that is, trivalent phosphorus is oxidized to pentavalent phosphorus);
4) Blocking: adding cap to 5'-OH of the nucleotide sequence that failed in the previous step to prevent further reaction; repeating the above steps until the last phosphoramidite monomer is introduced; then cleaving the ester bond between Solid Support and the starting monomer with methylamine aqueous solution and ammonia water, and removing the protective groups on each base and phosphate on the resulting nucleotide sequence; after separation and purification by HPLC, filtering to sterilize and lyophilizing to obtain the corresponding sense strand or antisense strand.

### Description of the synthesis process of RNA inhibitors:

Redissolved the sense strand and antisense strand freeze-dried powders separately, mixed them in an equal molar ratio, added into an appropriate amount of water for injection, and added into an appropriate amount of TRIS buffer solution. Shaked gently for about 1-2 min to mix the solution evenly. Heated a water bath to 92°C~95 °C and placed the above reaction solution in the water bath to heat for 3~ 5min, shaked gently to heat the solution evenly. Let it to cool naturally to room temperature to obtain colorless or yellowish transparent liquid. Took samples for testing to measure concentrations.

### Example 2 Experiment 1 of RNA inhibitors to inhibit LPA gene expression in vitro

The RNA inhibitors of this Example were selected from Table 3 and prepared by the method described in Example 1. Transferred plasmid DNA (LPA_PSICHECK(TM)-2 plasmid) into Huh7 cells using Fugene HD. Inoculated the transfected cells into a 96-well plate at a density of 10,000 cells per well, and the culture medium per well was 100 µL. Cultured the cells overnight in a 5% CO₂, 37°C incubator. Then, formulated the RNA inhibitors into a nanolipid-coated RNA inhibitor sample solutions of corresponding concentrations using PBS. Added RNAiMAX/Opti-MEM to each well at the corresponding positions, and took the RNA inhibitor sample solutions diluted to the corresponding concentrations to add to the wells, mixed and incubated, and took the incubated mixture to mix evenly with DMEM containing 10% FBS. Removed the culture medium from each well, then added new culture medium containing the sample, and then placed it in a 5% CO₂, 37 °C incubator for culture. The final concentrations of the test samples were 5nM, 0.5nM, and 0.05nM.

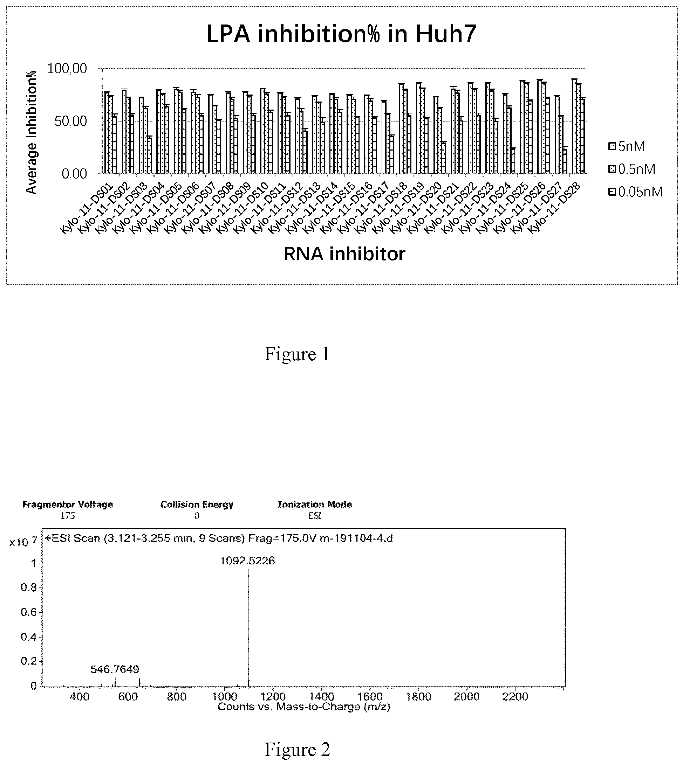

Took the cells out of the incubator, aspirated and discarded the supernatant, added fresh culture medium and detection reagent, shaked in the dark. After the cells are fully lysed, transferred the sample to an Opaque white board to detect the Firefly luciferase signal. Added Dual-Glo^{®} Stop & Glo^{®} detection reagent to each well, shaked in the dark, and detected the Renilla luciferase signal. Calculated the ratio of the signal of the main reporter gene to that of the internal reference reporter gene in each well. The test results were shown in Table 20 and Figure 1 below.

**Table 20 Inhibitory effect of RNA inhibitors on LPA mRNA in Huh7 cells**

| Double strands code | Average Inhibition% | | |
|---|---|---|---|
| | 5nM | 0.5nM | 0.05nM |
| Kylo-11-DS01 | 76.96 | 73.12 | 53.87 |
| Kylo-11-DS02 | 79.03 | 72.11 | 55.49 |
| Kylo-11-DS03 | 72.20 | 61.81 | 33.56 |
| Kylo-11-DS04 | 79.04 | 75.43 | 63.35 |
| Kylo-11-DS05 | 79.76 | 77.29 | 61.00 |
| Kylo-11-DS06 | 77.13 | 72.18 | 54.95 |
| Kylo-11-DS07 | 75.04 | 64.38 | 50.45 |
| Kylo-11-DS08 | 76.73 | 70.54 | 52.49 |
| Kylo-11-DS09 | 77.35 | 73.64 | 55.14 |
| Kylo-11-DS10 | 80.76 | 75.54 | 58.02 |
| Kylo-11-DS11 | 76.57 | 71.96 | 54.93 |
| Kylo-11-DS12 | 70.94 | 58.92 | 40.23 |
| Kylo-11-DS13 | 73.14 | 67.61 | 48.68 |
| Kylo-11-DS14 | 75.34 | 70.48 | 58.43 |
| Kylo-11-DS15 | 74.60 | 70.55 | 53.79 |
| Kylo-11-DS16 | 74.20 | 68.89 | 52.86 |
| Kylo-11-DS17 | 68.25 | 56.40 | 35.49 |
| Kylo-11-DS18 | 85.29 | 79.33 | 55.07 |
| Kylo-11-DS19 | 85.70 | 80.90 | 52.49 |
| Kylo-11-DS20 | 73.02 | 61.90 | 28.94 |
| Kylo-11-DS21 | 80.46 | 76.47 | 50.10 |
| Kylo-11-DS22 | 85.69 | 80.19 | 55.25 |
| Kylo-11-DS23 | 85.72 | 78.46 | 49.50 |
| Kylo-11-DS24 | 74.87 | 62.29 | 23.45 |
| Kylo-11-DS25 | 88.20 | 85.95 | 69.11 |
| Kylo-11-DS26 | 88.35 | 85.47 | 71.72 |
| Kylo-11-DS27 | 73.05 | 54.81 | 22.80 |
| Kylo-11-DS28 | 89.99 | 85.19 | 70.65 |

The test results show that the RNA inhibitors in Table 3 exhibited different degrees of inhibitory effects on the LPA mRNA level in Huh7 cells at different concentrations, and were significantly dose-dependent. At a low concentration of 0.05nM, except for Kylo-11-DS-03, Kylo-11-DS-17, Kylo-11-DS-20, Kylo-11-DS-24 and Kylo-11-DS-27, which had the inhibition rates lower than 40%, the remaining inhibitors all had inhibition rates higher than 40%.

### Example 3 Synthesis of ligand structures

When the 3' end of the sense strand or antisense strand of the RNA inhibitor of the present application is coupled with a ligand structure 3'MVIP, the Solid Support of 3'MVIP is used as the starting monomer for solid-phase synthesis. When the 5' end of the sense strand or antisense strand of the RNA inhibitor of the present application is coupled with a ligand structure 5'MVIP, the 5'MVIP phosphoramidite monomer is used as the last monomer for solid-phase synthesis.

The general formula of solid support of 3'MVIP is as follows:

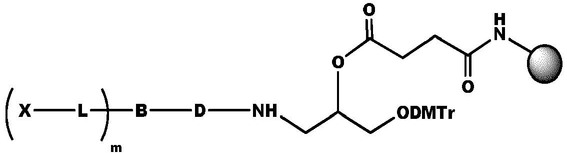

When m is 1-4, the linker B part in the general formula is branched for 1 to 4 times respectively to obtain the corresponding Solid Support of 3'MVIP.

The general formula of 5'MVIP phosphoramidite monomer is as follows:

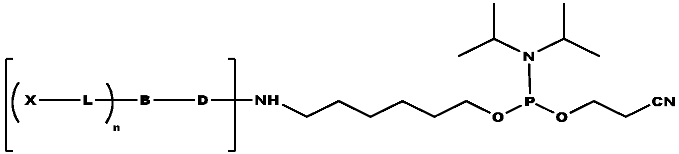

When n is 1-4, the linker B part in the general formula is branched for 1 to 4 times respectively to obtain the corresponding 5'MVIP phosphoramidite monomer.

The following are only exemplary examples of the chemical synthesis processes of several 3'MVIP Solid Supports and 5'MVIP phosphoramidite monomers. With reference to the methods described in the Examples, those skilled in the art can easily synthesize the remaining 3'MVIP Solid Supports and 5'MVIP phosphoramidite monomers involved in the present application. The synthesis process is described as follows:

### 3.1 Synthesis of Solid Support of 3'MVIP

### 3.1.1 Synthesis of Solid Support of 3'MVIP09

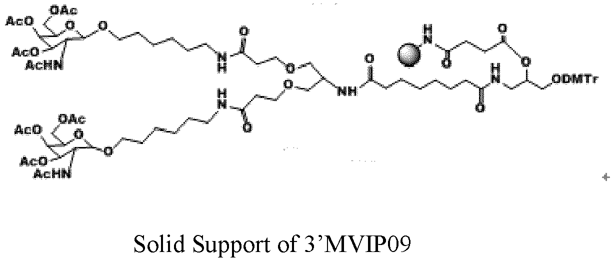

### Description of synthesis process:

### 3.1.1.1 Synthesis of ERC-01-c1

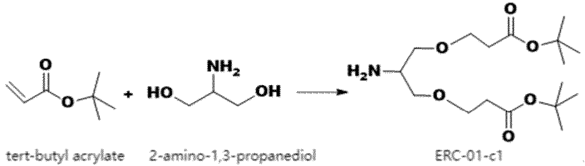

Weighed 2-amino-1,3-propanediol (5.0g, 54.9mmol) and added 50ml of DMSO, 5ml of sodium hydroxide solution (1g/ml), cooled it down to 0°C, added tert-butyl acrylate (20ml, 137.8mol) dropwise for 2 hours, let it react at room temperature for 48h, added petroleum ether (100ml), washed twice with saturated salt water, and dried the organic layer. Passed it through a chromatography column (eluent: ethyl acetate: petroleum ether = 25%-75%), added 0.05% triethylamine to the column, and obtained 6.2g of colorless oil.

### 3.1.1.2 Synthesis of ERC-01-c2

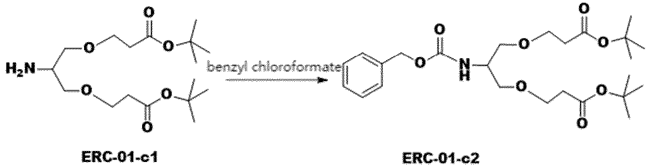

Weighed ERC-01-c1 (6.2g, 17.9mmol), added 50ml of dichloromethane, 23ml of sodium carbonate solution (25%), added benzyl chloroformate (8.2ml, 57.4mmol) dropwise at room temperature for 2 hours, let it react overnight at room temperature, washed with saturated salt water three times, dried with anhydrous sodium sulfate, evaporated the solvent, passed it through a chromatography column (ethyl acetate: petroleum ether =5%-30%), and obtained 4.0g of oil.

### 3.1.1.3 Synthesis of ERC-01-c3

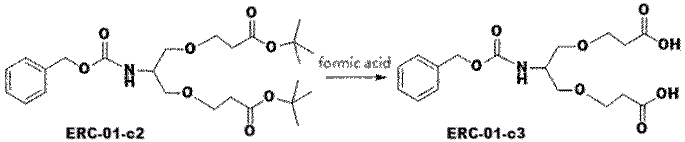

Weighed ERC-01-c2 (4.0g, 8.3mmol), added 12ml of formic acid, let it react overnight at room temperature, evaporated the solvent under reduced pressure to obtain 2.8g of product.

### 3.1.1.4 Synthesis of ERCd-01-c1

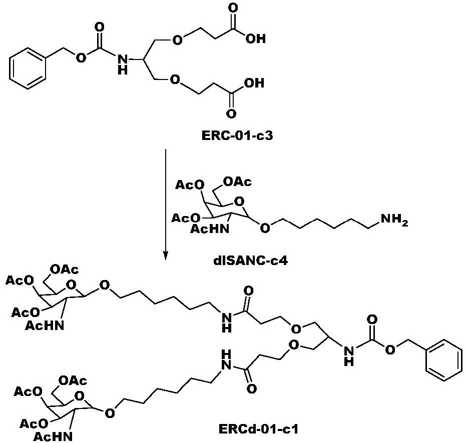

Added compounds ERC-01-c3 (1.11g, 3.0mmol) and dlSANC-c4 (3.6g, 8.04mmol) to DMF (60 ml), then added HOBt (2.24g) and HBTU (3.36g), and then slowly added DIEA (4.16ml). Stirred the reaction solution for 3 hours at room temperature. Then added water and extracted the aqueous layer with dichloromethane (2x10ml). Combined the organic layers, and then washed with saturated sodium bicarbonate (80 ml), water (2x60 ml), and saturated salt water (60ml) successively. Dried with anhydrous sodium sulfate, evaporated to dryness under reduced pressure, and purified by silica gel column chromatography (eluent: 3-15% MeOH in DCM). Obtained light yellow solid 3.24g.

### 3.1.1.5 Synthesis of ERCd-01-c2

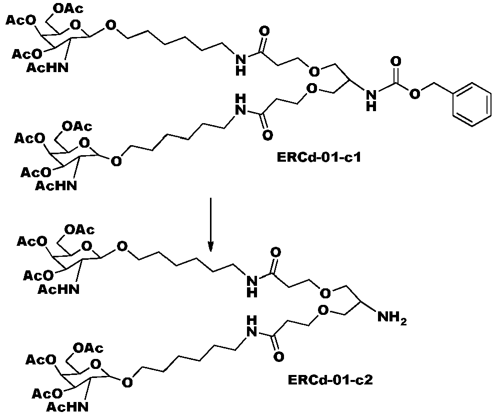

Dissolved ERCd-01-c1 (3.24g, 2.6mmol) in methanol (60ml), and added 10% palladium carbon (0.3g) and acetic acid (2.0ml). Then introduced hydrogen at atmospheric pressure to react overnight. The reaction solution was filtered with diatomite, and the filtrate was evaporated to dryness under reduced pressure to obtain 2.9g of oil ERCd-01-c2, its high-resolution mass spectrum was shown in Figure 2.

### 3.1.1.6 Synthesis of 3'MVIP09-c1

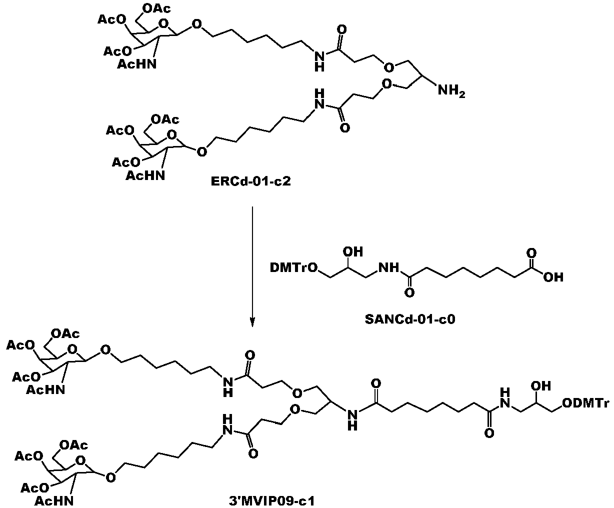

Successively added SANCd-01-c0 (0.824g, 1.5mmol) and ERCd-01-c2 (1.09g, 1.0mmol) into a vial, then added 10ml of DCM, stirred until dissolved, then successively added TBTU (0.963g) and DIPEA (0.517g), let it react overnight, added water, extracted with DCM, washed the organic phase with saturated salt water, dried, filtered and concentrated, and finally purified by a silica gel column to obtain 1.3g of product.

### 3.1.1.7 Synthesis of 3'MVIP09-c2

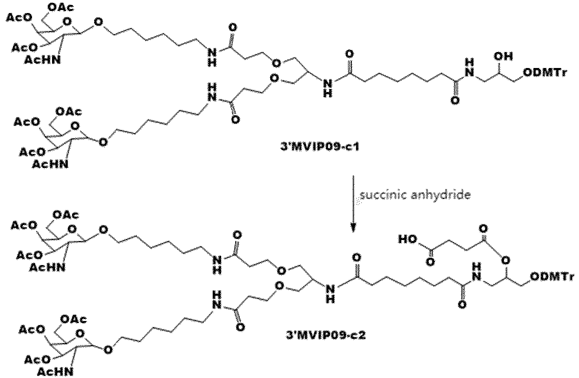

Added 3'MVIP09-c1 (1.62g, 1 µmol) and 10ml of DCM into a vial in turn, stirred at room temperature until dissolved, then added DMAP (0.366g) and succinic anhydride (0.2g, 3 µmol) in turn, stirred at room temperature to react. Concentrated DCM after the reaction is validated by TLC analysis, added water, extracted with DCM, washed the organic phase with saturated salt water, dried the organic phase by anhydrous sodium sulfate, filtered and concentrated, and finally purified by a silica gel column to obtain 1.55g product.

### 3.1.1.8 Synthesis of Solid Support of 3'MVIP09

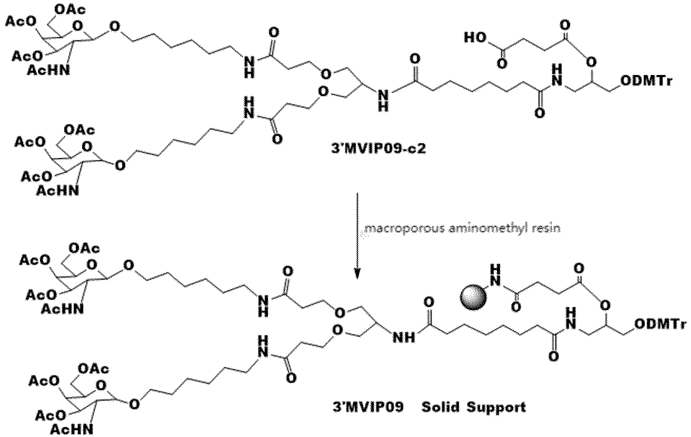

Added 3'MVIP09-c2 (0.86g, 0.5 µmol) and 10ml DMF into a vial in turn, after dissolution, added HBTU (0.19g), DIPEA (0.194g) and macroporous aminomethyl resin (2.0g) in turn, placed on the shaker for 24h, filtered, washed the resin with 10% methanol/DCM, and then used 25% acetic acid/pyridine for end capping, with a degree of substitution of 150 µmol/g.

### 3.1.2 Synthesis of Solid Support of 3'MVIP17

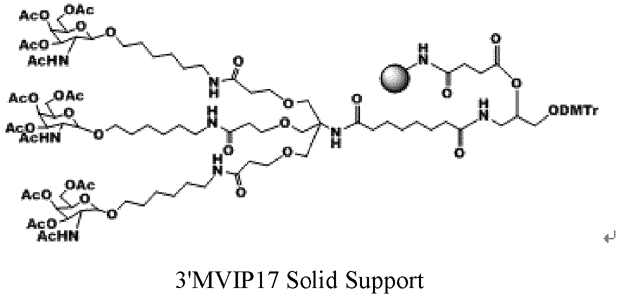

### 3.1.2.1 Synthesis of SANC-01-c1

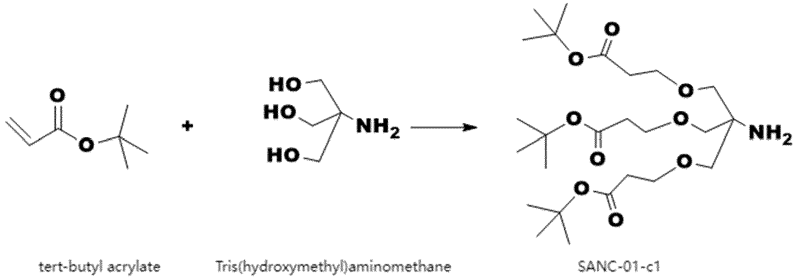

The synthesis steps are referred to 3.1.1.1 synthesis of ERC-01-c1.

### 3.1.2.2 Synthesis of SANC-01-c2

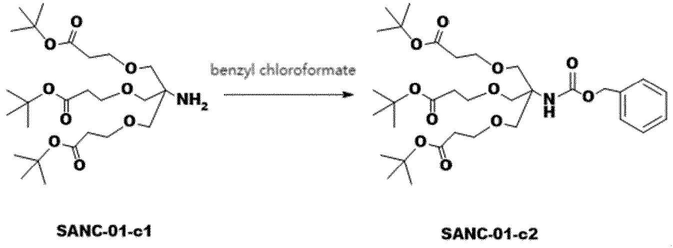

The synthesis steps are referred to 3.1.1.2. synthesis of ERC-01-c2.

### 3.1.2.3 Synthesis of SANC-01-c3

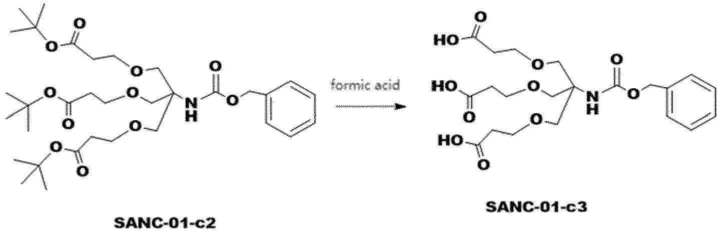

The synthesis steps are referred to 3.1.1.3. Synthesis of ERC-01-c3.

### 3.1.2.4 Synthesis of SANCd-01-c1

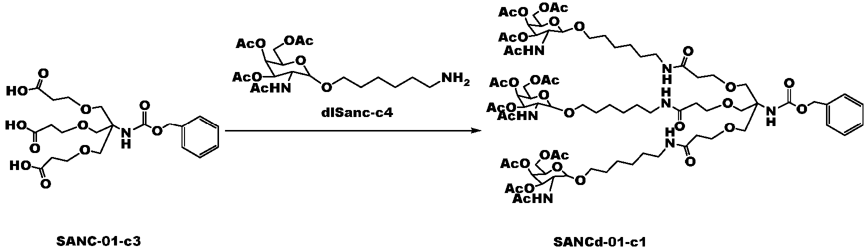

The synthesis steps are referred to 3.1.1.4. Synthesis of ERCd-01-c1.

### 3.1.2.5 Synthesis of SANCd-01-c2

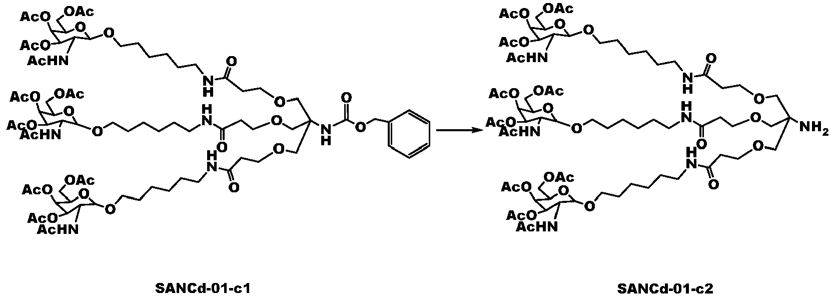

The synthesis steps are referred to 3.1.1.5. Synthesis of ERCd-01-c2.

### 3.1.2.6 Synthesis of 3'MVIP17-c1

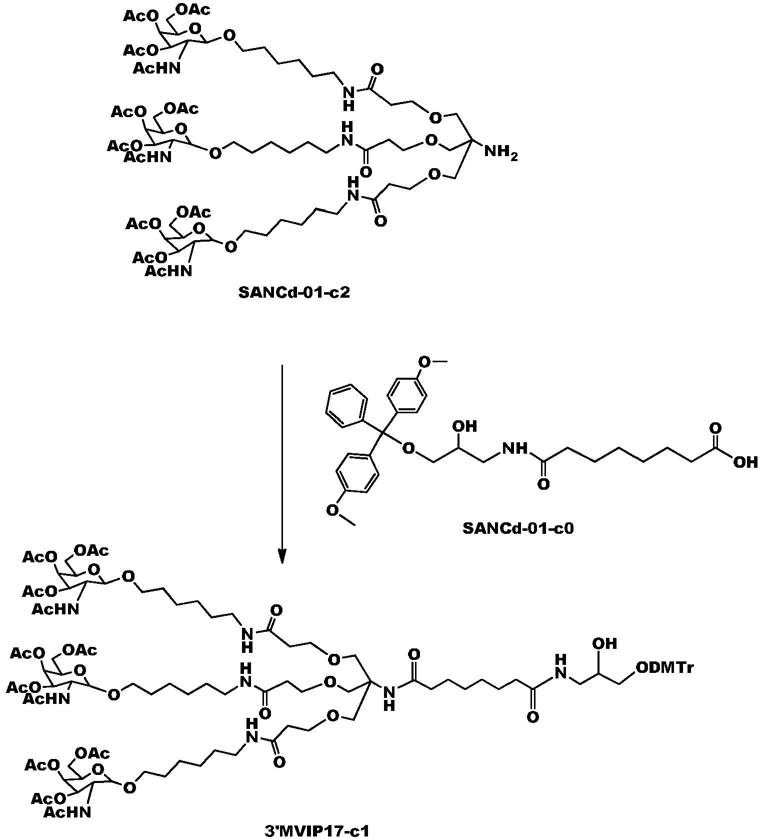

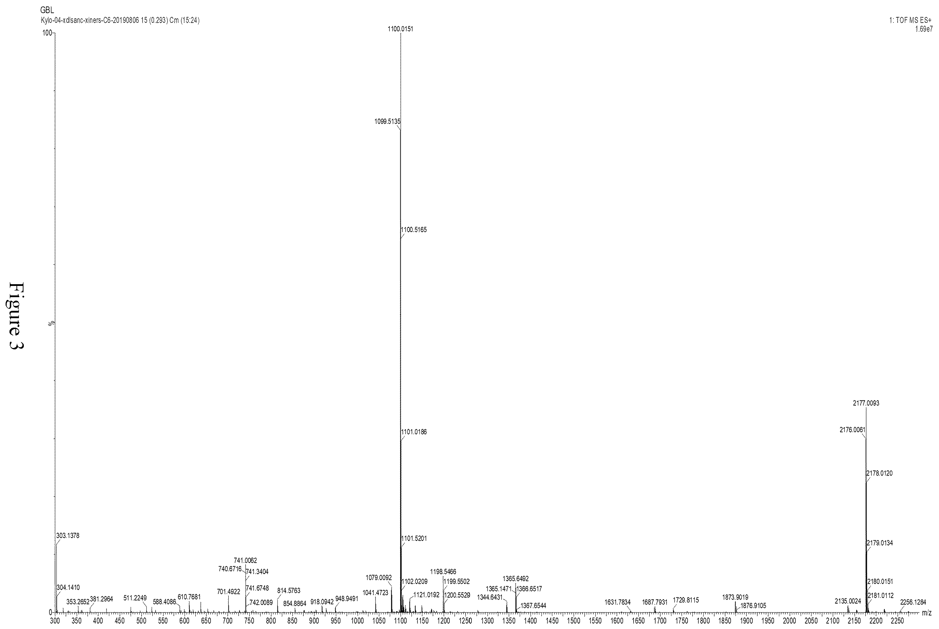

The synthesis steps are referred to 3.1.1.6. Synthesis of 3'MVIP09-c1, the synthesized 3'MVIP17-c1 has high-resolution mass spectrum as shown in Figure 3.

### 3.1.2.7 Synthesis of 3'MVIP17-c2

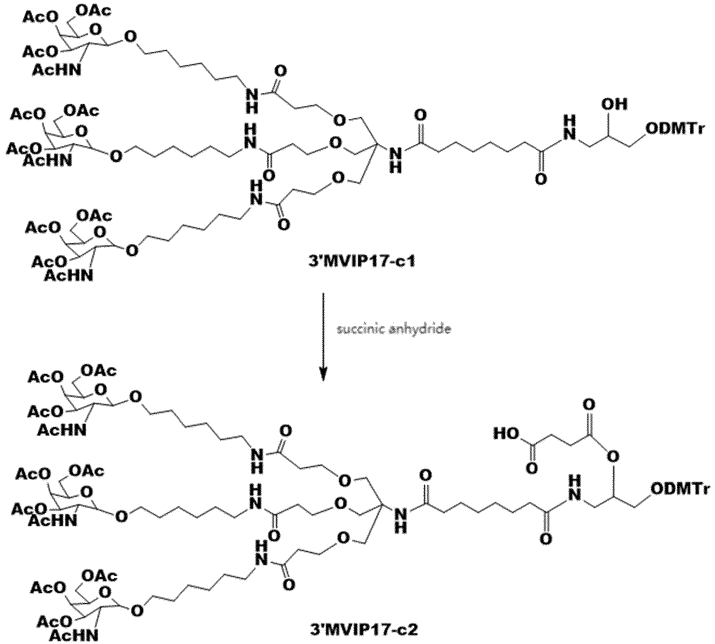

The synthesis steps are referred to 3.1.1.7. Synthesis of 3'MVIP09-c2.

### 3.1.2.8 Synthesis of Solid Support of 3'MVIP17

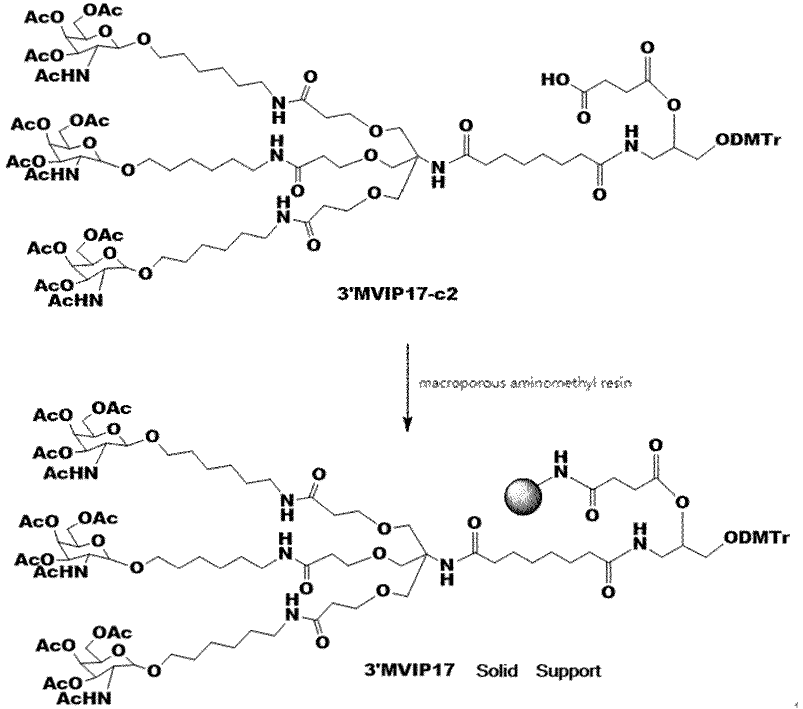

The synthesis steps are referred to 3.1.1.8 Synthesis of Solid Support of 3'MVIP09.

### 3.1.3 Synthesis of Solid Support of 3'MVIP01

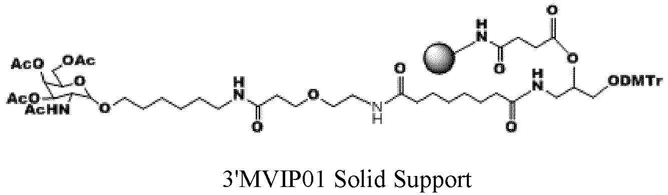

### Description of synthesis process:

### 3.1.3.1 Synthesis of 3'MVIP01-c1

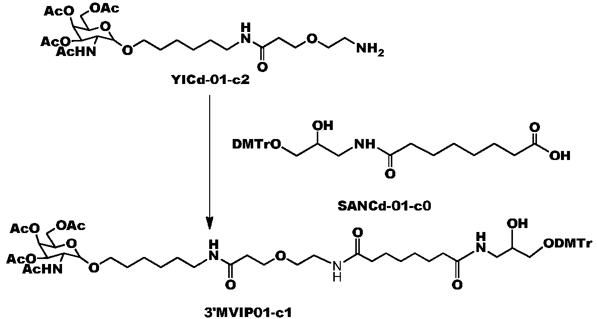

The synthesis steps are referred to 3.1.1.6 synthesis of 3'MVIP09-c1.

### 3.1.3.2 Synthesis of 3'MVIP01-c2

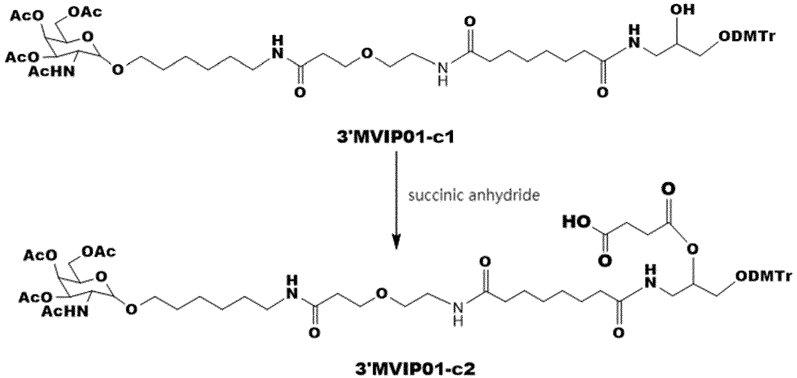

The synthesis steps are referred to3.1.1.7 synthesis of 3'MVIP09-c2.

### 3.1.3.3 Synthesis of Solid Support of 3'MVIP01

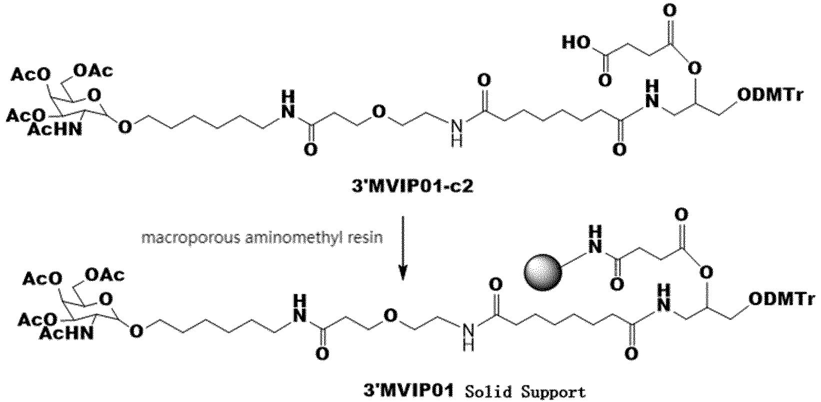

The synthesis steps are referred to 3.1.1.8 synthesis of Solid Support of 3'MVIP09.

### 3.2 Synthesis of 5'MVIP phosphoramidite monomer

### 3.2.1 Synthesis of 5'MVIP09 phosphoramidite monomer:

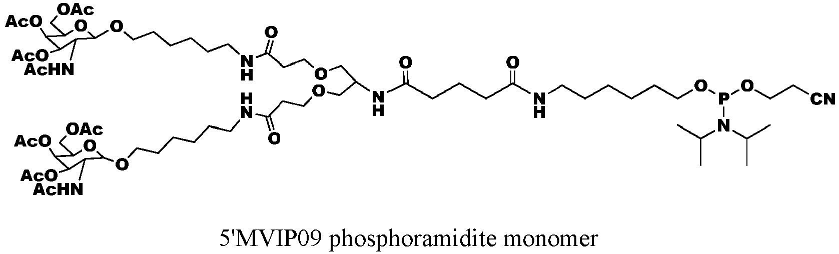

### 3.2.1.1 Synthesis of 5'MVIP09-ERCd-PFP-c1

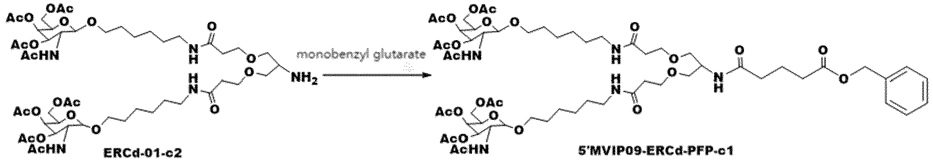

Weighed ERCd-01-c2 (2.18g, 2.0mmol) to dissolve in DMF (50ml), added monobenzyl glutarate (0.53g, 2.4mmol), DIPEA (0.78g) and TBTU (0.84g), stirred at room temperature overnight, added water to quench (50ml), extracted with DCM (30ml*3), washed with 10% citric acid (50ml*3), 50ml saturated sodium bicarbonate and 100ml pyridine, dried with anhydrous sodium sulfate, filtered, conducted rotary evaporation, and purified by a column to obtain the product 5'MVIP09-ERCd-PFP-c1 (2.15g).

### 3.2.1.2 Synthesis of 5'MVIP09-ERCd-PFP-c2

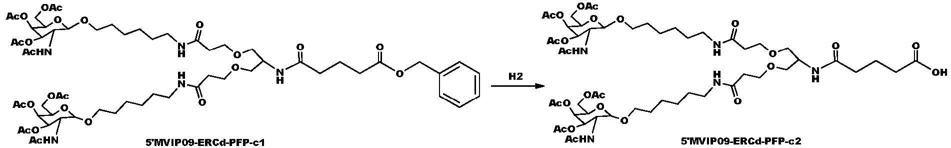

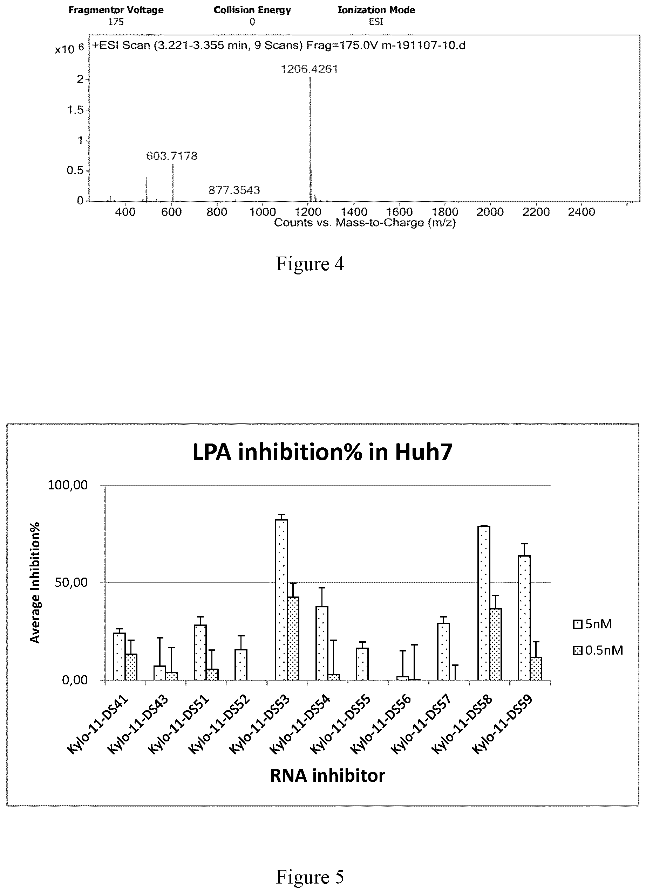

Weighed 5'MVIP09-ERCd-PFP-c1 (2.15g, 1.66mmol) and 10% palladium carbon (0.21g), added methanol (50ml), and hydrogenated overnight with stirring at room temperature, filtered palladium carbon with diatomite after the reaction, conducted rotary evaporation to obtain 5'MVIP09-ERCd-PFP-c2 crude product (1.9g), its high-resolution mass spectrum is shown in Figure 4.

### 3.2.1.3 Synthesis of 5'MVIP09-ERCd-PFP

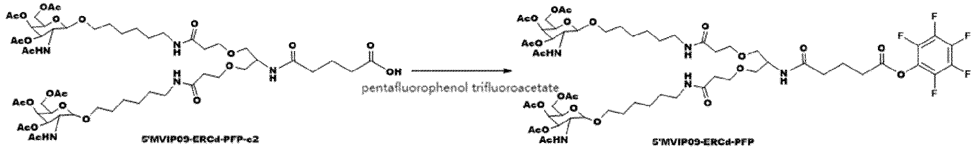

Weighed 5'MVIP09-ERCd-PFP-c2 crude product (1.9g, 1.58mmol) to dissolve in DCM (60ml), added DIPEA (1.33g), cooled, added pentafluorophenol trifluoroacetate (2.21g, 7.9mmol), let it react for 2h with stirring at room temperature, then conducted rotary evaporation, redissolved in DCM (60ml), washed with saturated sodium bicarbonate (30ml*3), 10% citric acid (30ml*1), saturated salt water (50ml*1), dried with anhydrous sodium sulfate, filtered, conducted rotary evaporation to obtain 5'MVIP09-ERCd-PFP crude product (2.35g). After being drained, it was directly used for the next reaction without purification.

### 3.2.1.4 Synthesis of 5'MVIP09 phosphoramidite monomer-c1

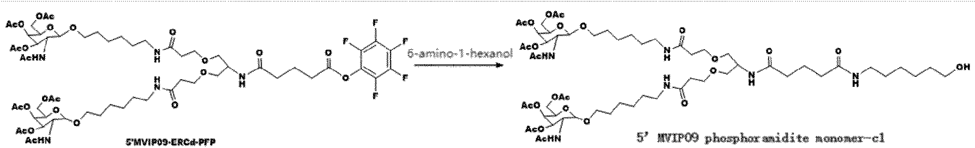

Dissolved 5'MVIP09-ERCd-PFP crude product (2.35g, 1.58mmol) in DCM (60ml), added DIPEA (0.82g, 6.32mmol), 6-amino-1-hexanol (0.37g, 3.16mmol), and let it react overnight with stirring at room temperature. Added 10% citric acid (30ml), extracted with DCM (30ml*3), washed with saturated salt water (50ml), dried with anhydrous sodium sulfate, filtered, conducted rotary evaporation, and purified by a column to obtain the product 5'MVIP09 monomer-c1 (1.73g).

### 3.2.1.5 5'MVIP09 phosphoramidite monomer

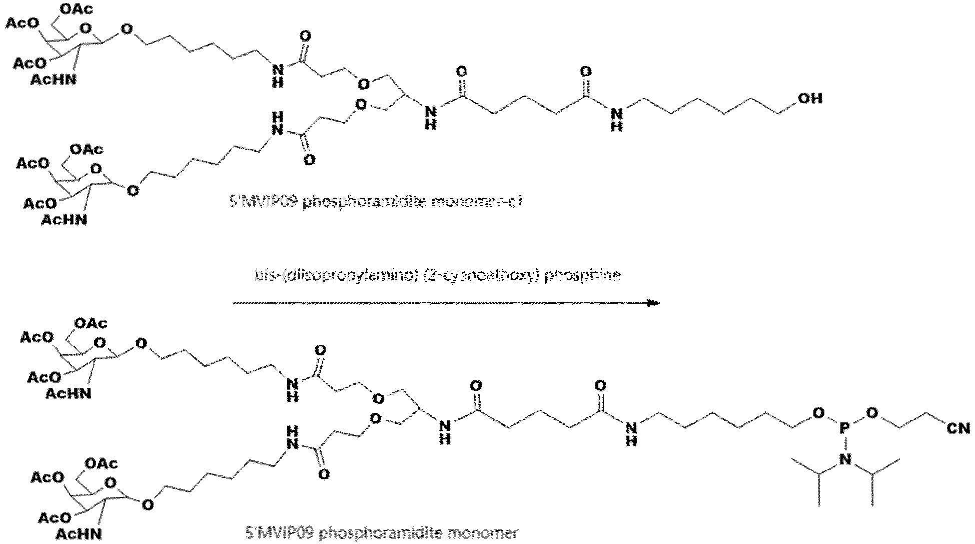

Weighed 5'MVIP09 phosphoramidite monomer-c1 (1.3g, 1.0mmol) to dissolve in acetonitrile (30ml), added diisopropylamine triazole (0.22g), added bis-(diisopropylamino) (2-cyanoethoxy) phosphine (0.36g, 1.2mmol) dropwise in an ice bath, let it react for 4h at room temperature, after the reaction is validated by HPLC in-process control , concentrated and purified by a column to obtain the product 5'MVIP09 monomer (1.2g).

### 3.2.2 Synthesis of 5'MVIP01 phosphoramidite monomer:

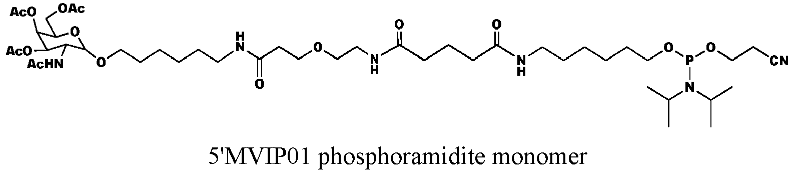

Except for weighing YICd-01-c2 (1.12g, 2.0mmol) as the phosphoramidite monomer of 5'MVIP01, refer to 3.2.1.1~ 3.2.1.5 for the remaining operations.

### Example 4 Synthesis of RNA inhibitors coupled to ligands

Description of synthesis of the antisense strand coupled to the ligand (Coupled to 3'MVIP 09): purged a reagent bottle with argon for at least 2 min. Added phosphoramidite monomer and acetonitrile into the reagent bottle in turn, tightened the bottle cap and shook until the solid is completely dissolved by visual inspection. Then added 3A molecular sieve and let it stand for more than 8 h for later use. Purged a reagent bottle with argon for at least 2 min. Added xanthane hydride and dried pyridine into the reagent bottle in turn, tightened the bottle cap, shook until the solid is completely dissolved by visual inspection, and stored temporarily for later use. Ensured to carry out the following operations at room temperature of 20~30 °C : weighed 3'MVIP Solid Support, added it to a reagent bottle, then added acetonitrile, shook until mixed evenly, transferred into a synthesis column, and eluted the residual part in the reagent bottle with acetonitrile and transferred into the synthesis column. After elution, added acetonitrile to fill the synthetic column, and recorded the amount of acetonitrile used. Installed and fixed the synthetic column according to the instrument instructions.

Connected the monomer solution formulated above, CAP A, CAP B, oxidant, thioreagent, activator, decapping agent and acetonitrile to the pipeline corresponding to AKTA PILOT100, and ensured that the pipeline is inserted into the bottom of the reagent bottle.

After the synthesis method was set, the instrument was ready for all work, clicked Run to start the synthesis. Observed and recorded each detritylation peak area online. During the synthesis process, added additional amount according to the actual amount of deprotection reagent used.

After the synthesis, purged the synthetic column with argon for at least 2 h, and unloaded the synthetic column according to the operating procedures. Transferred the solid phase support in the synthesis column to a reaction bottle, added methylamine aqueous solution and ammonia water, and put the reaction bottle into a shaker at 35°C for 2-3 hours. Filtered the solution into a round bottom flask, washed the residual solid phase with 50% ethanol aqueous solution, filtered again and mixed with the previous filtrate, connected the round bottom flask with a rotary evaporator, set the water temperature at 50°C and evaporated until there was no distillate, added ethanol into the round bottom flask, mixed well and evaporated again until there was no distillate. Repeated the operation until white powder appeared at the bottom of the flask. Formulated the obtained white powder into a solution, purified by a reverse chromatography column, and sampled to detect OD260 and purity. Divided the purified antisense strand solution into vials and lyophilized for later use, and stored the product sealed in a -20°C refrigerator.

The synthesis process of sense strand coupled to the ligand (coupled to 5'MVIP 09) is the same as that of antisense strand, in which the Solid Support loaded in the column is Universal support. Added DIPEA to the obtained intermediate to formulate a solution, added 5'MVIP phosphoramidite monomer, mixed well, and put the reaction bottle into a shaker at 35 °C for 2-3 hours.

### Description of synthetic annealing process of RNA inhibitor:

Took the obtained sense strand and antisense strand and mixed them in a reaction bottle in an equimolar ratio of 1:1. Turned off the power of the water bath after 5 minutes of 95°C water bath, let it naturally cool down to below 40°C. Added 3M sodium acetate aqueous solution to the solution containing double strands, mixed well, then added an appropriate volume of absolute ethanol, mixed well, and put the reaction solution into a -20 °C refrigerator for 45min. Set the high-speed refrigerated centrifuge to 4°C for pre-cooling, put in the solution containing double strands after the temperature is reached, and started the centrifuge. Took out the centrifuged solution containing double strands, removed the supernatant, added ultrapure water to completely dissolve the solid, and took samples to detect OD260 and purity, obtained the RNA inhibitors in Table 14. Divided the purified finished solution into vials and lyophilized for later use, and stored the product sealed in a -20 °C refrigerator.

The above only exemplifies the synthesis of RNA inhibitors coupled to 5'MVIP09/3'MVIP09. The RNA inhibitors described in the present application but not listed here are all applicable to this rule, that is, when the 3' end of the sense strand or antisense strand of the RNA inhibitor is coupled to the ligand structure 3'MVIP, the solid support of 3'MVIP is used as the starting monomer for solid phase synthesis; when the 5' end of the sense strand or antisense strand of the RNA inhibitor is coupled to the ligand structure 5'MVIP, the 5'MVIP phosphoramidite monomer is used as the last monomer for solid phase synthesis. Referring to the method described in this example, those skilled in the art can easily synthesize the remaining RNA inhibitors involved in the present application.

### Example 5 Experiment 2 of RNA inhibitor to inhibit LPA gene expression in vitro

The RNA inhibitors in this Example were selected from Table 4, the 2' positions of different nucleotide sugar groups in the sense and antisense strands have a methoxy or fluorine modification.

The RNA inhibitors were prepared by the method described in Example 4. Referring to the experimental method of Example 2, the inhibitory effects of the RNA inhibitors on LPA mRNA in Huh7 cells at concentrations of 5 nM and 0.5 nM were investigated. The obtained results were shown in Table 21 and Figure 5.

**Table 21 Inhibitory effects of RNA inhibitors on LPA mRNA in Huh7 cells**

| Double Stands Code | Average Inhibition% | |
|---|---|---|
| | 5nM | 0.5nM |
| Kylo-11-DS41 | 24.20 | 13.30 |
| Kylo-11-DS43 | 7.27 | 4.04 |
| Kylo-11-DS51 | 28.27 | 5.59 |
| Kylo-11-DS52 | 15.72 | 0.00 |
| Kylo-11-DS53 | 82.30 | 42.51 |
| Kylo-11-DS54 | 37.68 | 2.99 |
| Kylo-11-DS55 | 16.48 | 0.00 |
| Kylo-11-DS56 | 1.98 | 0.41 |
| Kylo-11-DS57 | 29.10 | 0.00 |
| Kylo-11-DS58 | 78.88 | 36.68 |
| Kylo-11-DS59 | 63.65 | 11.75 |

The test results showed that the RNA inhibitor Kylo-11-DS53 had a significant inhibitory effect on the LPA mRNA level of Huh7 cells at different concentrations. It is characterized by the fact that the 2' positions of nucleotide sugar groups at positions 5, 7, 8 and 9 starting from the 5' end of the sense strand are all fluorine, the rest are methoxy group, and the phosphate bonds among the 3 consecutive nucleotides at the 5' and 3' ends are thiolated, the 2' positions of nucleotide sugar groups at positions 2, 4, 8, 14 and 16 starting from the 5' end of the antisense strand are all fluorine, the rest are methoxy, and the phosphate bonds among the 3 consecutive nucleotides at the 5' and 3' ends are thiolated.

### Example 6 Experiment 3 of RNA inhibitor to inhibit LPA gene expression in vitro

The RNA inhibitors in this Example were selected from Table 5, the 2' positions of different nucleotide sugar groups in the sense and antisense strands have a methoxy or fluorine modification.

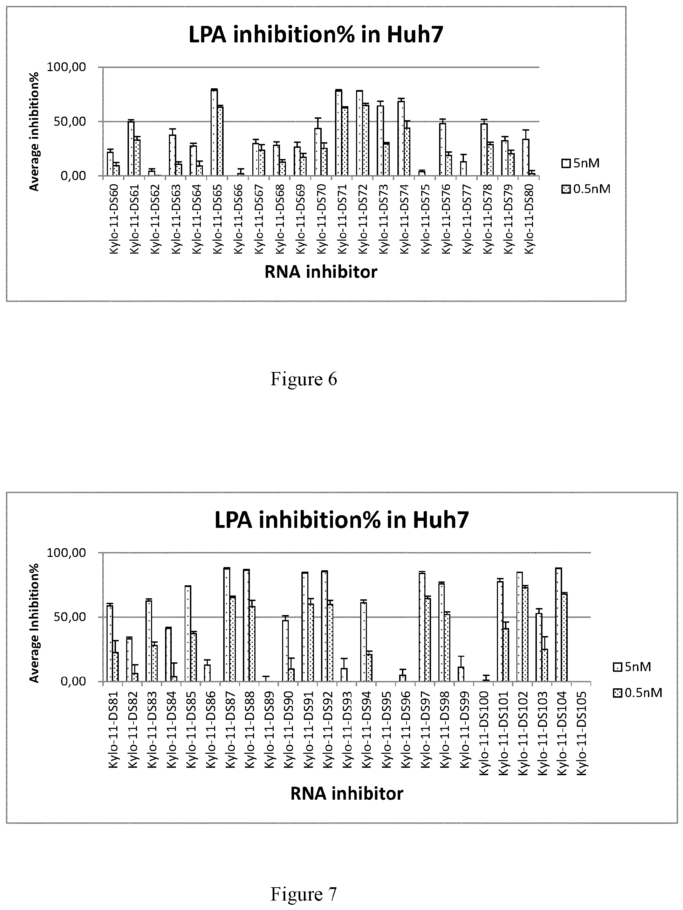

The RNA inhibitors were prepared by the method described in Example 4. Referring to the experimental method of Example 2, the inhibitory effects of the RNA inhibitors on LPA mRNA in Huh7 cells at concentrations of 5 nM and 0.5 nM were investigated. The obtained results were shown in Table 22 and Figure 6.

**Table 22 Inhibitory effects of RNA inhibitors on LPA mRNA in Huh7 cells**

| Double Stands Code | Average Inhibition % | |
|---|---|---|
| | 5nM | 0.5nM |
| Kylo-11-DS60 | 21.35 | 9.44 |
| Kylo-11-DS61 | 49.64 | 32.85 |
| Kylo-11-DS62 | 3.98 | 0.00 |
| Kylo-11-DS63 | 37.35 | 10.68 |
| Kylo-11-DS64 | 27.66 | 9.05 |
| Kylo-11-DS65 | 78.68 | 62.97 |
| Kylo-11-DS66 | 0.00 | 1.87 |
| Kylo-11-DS67 | 29.69 | 23.25 |
| Kylo-11-DS68 | 28.24 | 12.74 |
| Kylo-11-DS69 | 26.42 | 16.98 |
| Kylo-11-DS70 | 43.31 | 25.39 |
| Kylo-11-DS71 | 77.97 | 62.39 |
| Kylo-11-DS72 | 77.80 | 65.07 |
| Kylo-11-DS73 | 64.23 | 29.45 |
| Kylo-11-DS74 | 68.32 | 43.74 |
| Kylo-11-DS75 | 3.62 | 0.00 |
| Kylo-11-DS76 | 47.98 | 18.69 |
| Kylo-11-DS77 | 12.90 | 0.00 |
| Kylo-11-DS78 | 47.82 | 28.89 |
| Kylo-11-DS79 | 32.36 | 20.36 |
| Kylo-11-DS80 | 33.70 | 1.74 |

The test results showed that the RNA inhibitors Kylo-11-DS65, Kylo-11-DS71, Kylo-11-DS72 and Kylo-11-DS74 had significant inhibitory effects on the LPA mRNA levels of Huh7 cells at different concentrations. The Kylo-11-DS65 is characterized by the fact that the 2' positions of nucleotide sugar groups at positions 5, 7, 8 and 9 starting from the 5' end of the sense strand are all fluorine, the rest are methoxy, and the phosphate bonds among the 3 consecutive nucleotides at the 5' and 3' ends are thiolated, the 2' positions of nucleotide sugar groups at positions 2, 4, 8, 14 and 16 starting from the 5' end of the antisense strand are all fluorine, the rest are methoxy, and the phosphate bonds among the 3 consecutive nucleotides at the 5' and 3' ends are thiolated.

The Kylo-11-DS71 and Kylo-11-DS72 are characterized by the fact that the 2' positions of nucleotide sugar groups at positions 3, 5, 7, 8, 9, 10, 11, 13 and 15 starting from the 5' end of the sense strand are all fluorine, the rest are methoxy, and the phosphate bonds among the 3 consecutive nucleotides at the 5' and 3' ends are thiolated, the 2' positions of nucleotide sugar groups at positions 2, 4, 6, 8, 14 and 16 starting from the 5' end of the antisense strand are all fluorine, the rest are methoxy, and the phosphate bonds among the 3 consecutive nucleotides at the 5' and 3' ends are thiolated. The Kylo-11-DS74 is characterized by the fact that the 2' positions of nucleotide sugar groups at positions 9, 10 and 11 starting from the 5' end of the sense strand are all fluorine, the rest are methoxy, and the phosphate bonds among the 3 consecutive nucleotides at the 5' and 3' ends are thiolated, the 2' positions of nucleotide sugar groups at positions 2, 4, 6, 8, 14, 16, 18 and 20 starting from the 5' end of the antisense strand are all fluorine, the rest are methoxy, and the phosphate bonds among the 3 consecutive nucleotides at the 5' and 3' ends are thiolated.

### Example 7 Experiment 4 of RNA inhibitor to inhibit LPA gene expression in vitro

The RNA inhibitors in this Example were selected from Table 6. The candidate RNA inhibitors Kylo-11-DS81~Kylo-11-DS105 have methoxy or fluorinated modifications at the 2' positions of different nucleotide sugar groups in the sense and antisense strands.

The RNA inhibitors were prepared by the method described in Example 4. Referring to the experimental method of Example 2, the inhibitory effects of the RNA inhibitors on LPA mRNA in Huh7 cells at concentrations of 5 nM and 0.5 nM were investigated. The obtained results were shown in Table 23 and Figure 7.

**Table 23 Inhibitory effects of RNA inhibitors on LPA mRNA in Huh7 cells**

| Double Stands Code | Average Inhibition% | |
|---|---|---|
| | 5nM | 0.5nM |
| Kylo-11-DS81 | 58.83 | 22.54 |
| Kylo-11-DS82 | 32.95 | 6.17 |
| Kylo-11-DS83 | 62.48 | 28.05 |
| Kylo-11-DS84 | 41.30 | 3.77 |
| Kylo-11-DS85 | 73.95 | 37.30 |
| Kylo-11-DS86 | 12.75 | 0.00 |
| Kylo-11-DS87 | 87.75 | 65.04 |
| Kylo-11-DS88 | 86.67 | 58.07 |
| Kylo-11-DS89 | 0.00 | 0.00 |
| Kylo-11-DS90 | 47.35 | 9.72 |
| Kylo-11-DS91 | 84.16 | 60.20 |
| Kylo-11-DS92 | 85.33 | 59.79 |
| Kylo-11-DS93 | 10.05 | 0.00 |
| Kylo-11-DS94 | 61.18 | 21.11 |
| Kylo-11-DS95 | 0.00 | 0.00 |
| Kylo-11-DS96 | 5.00 | 0.00 |
| Kylo-11-DS97 | 84.02 | 64.28 |
| Kylo-11-DS98 | 75.80 | 52.05 |
| Kylo-11-DS99 | 11.18 | 0.00 |
| Kylo-11-DS100 | 0.00 | 0.94 |
| Kylo-11-DS101 | 77.50 | 41.00 |
| Kylo-11-DS102 | 84.70 | 72.99 |
| Kylo-11-DS103 | 52.80 | 25.00 |
| Kylo-11-DS104 | 87.78 | 67.75 |
| Kylo-11-DS105 | 0.00 | 0.00 |

The test results showed that the RNA inhibitors Kylo-11-DS87, Kylo-11-DS88, Kylo-11-DS91, Kylo-11-DS92, Kylo-11-DS97, Kylo-11-DS98, Kylo-11-DS101, Kylo-11-DS102 and Kylo-11-DS104 had significant inhibitory effects on the LPA mRNAlevels of Huh7 cells at different concentrations. The Kylo-11-DS87 is characterized by the fact that the 2' positions of nucleotide sugar groups at positions 7, 8, 9 and 10 starting from the 5' end of the sense strand are all fluorine, the 2' positions of nucleotide sugar groups at positions 2, 4, 6, 8 and 14 starting from the 5' end of the antisense strand are all fluorine. The Kylo-11-DS88 is characterized by the fact that the 2' positions of nucleotide sugar groups at positions 5, 8, 9 and 10 starting from the 5' end of the sense strand are all fluorine, the 2' positions of nucleotide sugar groups at positions 2, 15 and 17 starting from the 5' end of the antisense strand are all fluorine. The Kylo-11-DS91 is characterized by the fact that the 2' positions of nucleotide sugar groups at positions 8, 9 and 10 starting from the 5' end of the sense strand are all fluorine, the 2' positions of nucleotide sugar groups at positions 2, 4, 12 and 14 starting from the 5' end of the antisense strand are all fluorine. The Kylo-11-DS92 is characterized by the fact that the 2' positions of nucleotide sugar groups at positions 7, 8, 9 and 10 starting from the 5' end of the sense strand are all fluorine, the 2' positions of nucleotide sugar groups at positions 2, 5, 14 and 16 starting from the 5' end of the antisense strand are all fluorine. The Kylo-11-DS97 is characterized by the fact that the 2' positions of nucleotide sugar groups at positions 5, 7, 8 and 9 starting from the 5' end of the sense strand are all fluorine, the 2' positions of nucleotide sugar groups at positions 7, 12 and 14 starting from the 5' end of the antisense strand are all fluorine. The Kylo-11-DS98 is characterized by the fact that the 2' positions of nucleotide sugar groups at positions 7, 9, 10 and 11 starting from the 5' end of the sense strand are all fluorine, the 2' positions of nucleotide sugar groups at positions 2 and 14 starting from the 5' end of the antisense strand are all fluorine. The Kylo-11-DS101 is characterized by the fact that the 2' positions of nucleotide sugar groups at positions 7, 9, 10 and 11 starting from the 5' end of the sense strand are all fluorine, the 2' positions of nucleotide sugar groups at positions 2 and 14 starting from the 5' end of the antisense strand are all fluorine. The Kylo-11-DS102 is characterized by the fact that the 2' positions of nucleotide sugar groups at positions 5, 7, 8 and 9 starting from the 5' end of the sense strand are all fluorine, the 2' positions of nucleotide sugar groups at positions 2, 6, 8, 10, 14 and 16 starting from the 5' end of the antisense strand are all fluorine. The Kylo-11-DS104 is characterized by the fact that the 2' positions of nucleotide sugar groups at positions 9, 10 and 11 starting from the 5' end of the sense strand are all fluorine, the 2' positions of nucleotide sugar groups at positions 14 and 16 starting from the 5' end of the antisense strand are all fluorine. The common characteristics of these RNA inhibitors are that, except for the fluorinated 2' positions of the nucleotide sugar groups at the above positions, the 2' positions of the remaining nucleotide sugar groups are methoxy, and the phosphate bonds among the 3 consecutive nucleotides at the 5' and 3' ends are thiolated. Among them, Kylo-11-DS102 had the best inhibitory effect on the LPA mRNA level of Huh7 cells, with an inhibition rate of 72.99% at a concentration of 0.5nM.

### Example 8 Experiment 5 of RNA inhibitor to inhibit LPA gene expression in vitro

The RNA inhibitors in this Example were selected from Table 7. The 2' positions of different nucleotide sugar groups in the sense and antisense strands have a methoxy or fluorine modification.

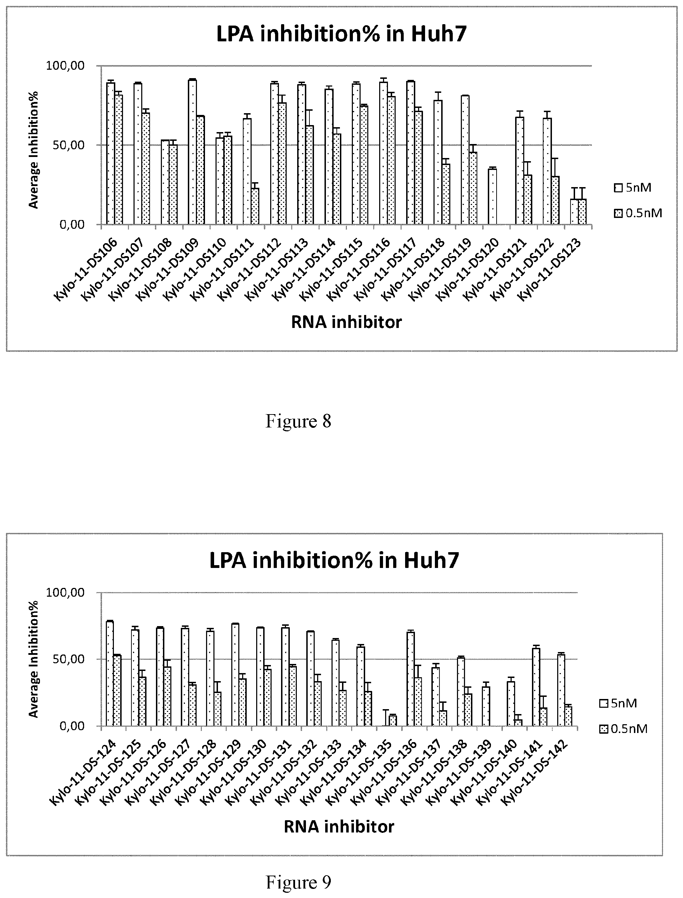

The RNA inhibitors were prepared by the method described in Example 4. Referring to the experimental method of Example 2, the inhibitory effects of the RNA inhibitors on LPA mRNA in Huh7 cells at concentrations of 5 nM and 0.5 nM were investigated. The obtained results were shown in Table 24 and Figure 8.

**Table 24 Inhibitory effects of RNA inhibitors on LPA mRNA in Huh7 cells**

| Double Strands Code | Average Inhibition% | |
|---|---|---|
| | 5nM | 0.5nM |
| Kylo-11-DS106 | 89.27 | 81.44 |
| Kylo-11-DS107 | 88.79 | 70.30 |
| Kylo-11-DS108 | 53.10 | 50.04 |
| Kylo-11-DS109 | 90.81 | 68.15 |
| Kylo-11-DS110 | 54.41 | 55.60 |
| Kylo-11-DS111 | 66.56 | 22.69 |
| Kylo-11-DS112 | 88.78 | 76.72 |
| Kylo-11-DS113 | 88.11 | 62.14 |
| Kylo-11-DS114 | 85.23 | 57.14 |
| Kylo-11-DS115 | 88.44 | 74.48 |
| Kylo-11-DS116 | 89.66 | 80.35 |
| Kylo-11-DS117 | 90.05 | 71.21 |
| Kylo-11-DS118 | 78.16 | 37.89 |
| Kylo-11-DS119 | 81.15 | 45.59 |
| Kylo-11-DS120 | 34.89 | 0.00 |
| Kylo-11-DS121 | 67.41 | 31.06 |
| Kylo-11-DS122 | 66.92 | 30.29 |
| Kylo-11-DS123 | 15.87 | 15.87 |

The test results showed that the RNA inhibitors Kylo-11-DS106~Kylo-11-DS110, Kylo-11-DS112~ Kylo-11-DS117 and Kylo-11-DS119 had significant inhibitory effects on the LPA mRNA levels of Huh7 cells at different concentrations. Among them, Kylo-11-DS106~Kylo-11-DS110, Kylo-11-DS112~ Kylo-11-DS114 and Kylo-11-DS116 have at least 15 consecutive nucleotides in common with each other, and the 2' positions of the nucleotide sugar groups at positions 3, 5, 7, 8, 9, 10, 11, 13 and 15 starting from the 5' end of the sense strand are all fluorine, and the rest of the 2' positions are methoxy, the phosphate bonds among the 3 consecutive nucleotides at the 5' and 3' ends are thiolated, and the 2' positions of the nucleotide sugar groups at positions 2, 4, 6, 8, 14 and 16 starting from the 5' end of the antisense chain are all fluorine, the rest are methoxy, the phosphate bonds among the 3 consecutive nucleotides at the 5' and 3' ends are thiolated, and both the sense and antisense strands contain 21 nucleotides (except Kylo-11-DS109), and there are 2 protruding ends of nucleotide at the 3' ends of both sense and antisense strands. Kylo-11-DS106 and Kylo-11-DS115 differ by only 1 nucleotide. The 6th nucleotide starting from the 5' end of the antisense strand is fU in the former and dT in the latter. The two have similar activities and both have high inhibitory activity. The unmodified sequences of the sense and antisense strands of Kylo-11-DS116 and Kylo-11-DS117 are the same, SEQ ID NO. 8 and SEQ ID NO. 21, respectively. The modification characteristics of the two are different. For the latter, the 2' positions of the nucleotide sugar groups at positions 5, 7, 8 and 9 starting from the 5' end of the sense strand are all fluorine, the 2' positions of the nucleotide sugar groups at positions 2, 6, 8, 10, 14 and 16 starting from the 5' end of the antisense strand are all fluorine. The unmodified sequences of the sense and antisense strands of the RNA inhibitor Kylo-11-DS119 are SEQ ID NO. 11 and SEQ ID NO. 25, respectively. The modification characteristics are that the 2' positions of the nucleotide sugar groups at positions 9, 10 and 11 starting from the 5' end of the sense strand are all fluorine, the 2' positions of the nucleotide sugar groups at positions 2, 4, 6, 12, 14, 16, 18 and 20 starting from the 5' end of the antisense strand are all fluorine. Both the sense and antisense strands contain 21 nucleotides and are blunt-ended at both ends.

### Example 9 Experiment 6 of RNA inhibitor to inhibit LPA gene expression in vitro

The RNA inhibitors in this Example were selected from Table 8. The 2' positions of different nucleotide sugar groups in the sense and antisense strands have a methoxy or fluorine modification. The RNA inhibitors were prepared by the method described in Example 4. Referring to the experimental method of Example 2, the inhibitory effects of the RNA inhibitors on LPA mRNA in Huh7 cells at concentrations of 5 nM and 0.5 nM were investigated. The obtained results were shown in Table 25 and Figure 9.

**Table 25 Inhibitory effects of RNA inhibitors on LPA mRNA in Huh7 cells**

| Double strands code | Average Inhibition% | |
|---|---|---|
| | 5nM | 0.5nM |
| Kylo-11-DS-124 | 78.29 | 52.85 |
| Kylo-11-DS-125 | 72.12 | 36.57 |
| Kylo-11-DS-126 | 73.43 | 44.36 |
| Kylo-11-DS-127 | 73.16 | 30.95 |
| Kylo-11-DS-128 | 70.76 | 25.24 |
| Kylo-11-DS-129 | 76.41 | 35.20 |
| Kylo-11-DS-130 | 73.75 | 42.31 |
| Kylo-11-DS-131 | 73.56 | 44.50 |
| Kylo-11-DS-132 | 70.62 | 33.00 |
| Kylo-11-DS-133 | 64.15 | 26.57 |
| Kylo-11-DS-134 | 59.31 | 25.74 |
| Kylo-11-DS-135 | 0.13 | 7.50 |
| Kylo-11-DS-136 | 70.19 | 36.20 |
| Kylo-11-DS-137 | 43.67 | 11.41 |
| Kylo-11-DS-138 | 51.16 | 24.10 |
| Kylo-11-DS-139 | 29.33 | 0.00 |
| Kylo-11-DS-140 | 33.08 | 4.61 |
| Kylo-11-DS-141 | 58.19 | 13.26 |
| Kylo-11-DS-142 | 53.29 | 14.30 |

The test results showed that the RNA inhibitors Kylo-11-DS-124, Kylo-11-DS-126, Kylo-11-DS-130 and Kylo-11-DS-131 had significant inhibitory effects on the LPA mRNA of Huh7 cells at different concentrations. The common characteristics of these RNA inhibitors are that the 2' positions of the nucleotide sugar groups at positions 9, 10 and 11 starting from the 5' end of the sense strand are all fluorine, the rest are methoxy, and the phosphate bonds among the 3 consecutive nucleotides at the 5' and 3' ends are thiolated, and the 2' positions of the nucleotide sugar groups at positions 2, 4, 6, 12, 14, 16, 18 and 20 starting from the 5' end of the antisense strand are all fluorine. Among them, Kylo-11-DS-126 and Kylo-11-DS-131 both have 21 nucleotides in the sense and antisense strands, and both have 2 protruding ends of nucleotide at the 3' ends. The difference is that the 6th nucleotide starting from the 5' end of the antisense strand of the former is fA, while the same position of the latter is dT. The two exhibit similar activities. Kylo-11-DS-124 and Kylo-11-DS-130 have a sense strand containing 19 nucleotides and an antisense strand containing 21 nucleotides. The 3' end of the antisense strand has 2 protruding ends of nucleotide, and the 3' end of the sense strand and the 5' end of the antisense strand are blunt ends. The two exhibit similar activities.

### Example 10 Evaluation of RNA inhibitor in vivo activity using cynomolgus monkeys

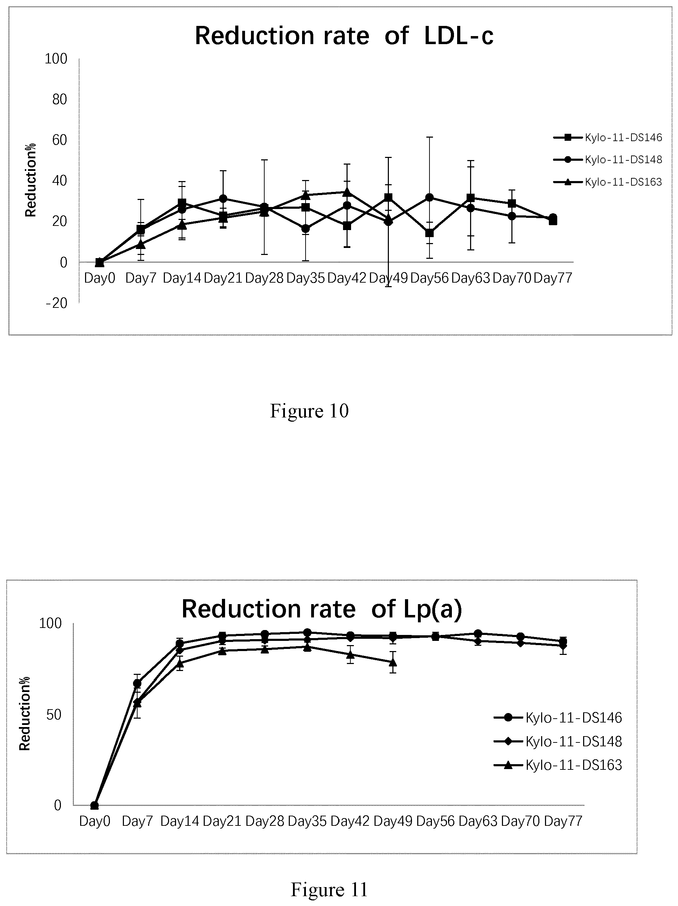

Cynomolgus monkeys of appropriate age were used for experiments to evaluate the RNA inhibitors Kylo-11-DS148, Kylo-11-DS146 and Kylo-11-DS163. Each dose was administered at 6 mg/kg via subcutaneous injection on Day 0. After administration, collected blood every week to measure LDL-c and Lp(a) levels. The results of LDL-c and Lp(a) levels after RNA inhibitor intervention were shown in Tables 26-27 and Figures 10-11.

**Table 26 Effect of RNA inhibitors on reducing LDL-c levels in cynomolgus monkey plasma**

| Times | Reduction rate of LDL-c | | |
|---|---|---|---|
| | Kylo-11-DS146 | Kylo-11-DS148 | Kylo-11-DS163 |
| Day0 | 0.00 | 0.00 | 0.00 |
| Day7 | 16.17 | 15.82 | 8.83 |
| Day14 | 29.01 | 25.76 | 18.45 |
| Day21 | 22.76 | 31.06 | 21.59 |
| Day28 | 26.30 | 27.04 | 24.73 |
| Day35 | 26.79 | 16.48 | 32.81 |
| Day42 | 17.84 | 27.72 | 34.37 |
| Day49 | 31.65 | 19.72 | 21.36 |
| Day56 | 14.37 | 31.65 | / |
| Day63 | 31.45 | 26.39 | / |
| Day70 | 28.66 | 22.46 | / |
| Day77 | 20.13 | 21.78 | / |

**Table 27 Effect of RNA inhibitors on reducing Lp(a) levels in cynomolgus monkey plasma**

| Times | Reduction rate of Lp(a) | | |
|---|---|---|---|
| | Kylo-11-DS146 | Kylo-11-DS148 | Kylo-11-DS163 |
| Day0 | 0.00 | 0.00 | 0.00 |
| Day7 | 67.05 | 56.96 | 56.25 |
| Day14 | 88.87 | 85.19 | 77.96 |
| Day21 | 93.04 | 90.14 | 84.76 |
| Day28 | 93.99 | 90.64 | 85.65 |
| Day35 | 94.94 | 91.01 | 87.04 |
| Day42 | 93.26 | 91.90 | 82.80 |
| Day49 | 92.96 | 91.80 | 78.50 |
| Day56 | 92.55 | 92.80 | / |
| Day63 | 94.29 | 90.15 | / |
| Day70 | 92.66 | 89.12 | / |
| Day77 | 89.98 | 87.58 | / |

The test results showed that the RNA inhibitors Kylo-11-DS146, Kylo-11-DS 148 and Kylo-11-DS163 could reduce the LDL-c level in cynomolgus monkey plasma and significantly and continuously reduce the Lp(a) level in the plasma. After the intervention of Kylo-11-DS146, the individual plasma Lp(a) level could be reduced by up to 95.25% compared with before administration. By day 77, the average reduction rate could still be maintained at 89.98%.

### Example 11 Comparison of in vitro inhibitory effects of different duplexes

CN202210241706.1 (hereinafter referred to as D1) is a Chinese patent application submitted by the applicant "KYLONOVA (XIAMEN) BIOPHARMA CO., LTD." on March 11, 2022. The title of the application is LPA inhibitors and use thereof and the publication number is CN114703184A. The applicant performed the following experiments on different duplexes:

| | Double strands code | SEQ ID NO. of sense strand | Sense strand sequence 5'-3' | SEQ ID NO. of antisense strand | Antisense strand sequence 5'-3' |
|---|---|---|---|---|---|
| the present application | Kylo-11-DS13 | SEQ ID NO. 11 | cagcuccuuauuguuaua cga | SEQ ID NO. 25 | ucguauaacaauaaggag cug |
| D1 | Ky-11-DS12 | SEQ ID NO. 345 | gcuccuuauuguuauacg a | SEQ ID NO. 346 | ucguauaacaauaaggag cug |
| the present application | Kylo-11-DS11 | SEQ ID NO. 8 | ugguaauggacagaguua uca | SEQ ID NO. 21 | auaacucuguccauuacc aug |
| D1 | Ky-11-DS08 | SEQ ID NO. 347 | ugguaauggacagaguua uuu | SEQ ID NO. 348 | auaacucuguccauuacc auu |

Took Huh7 cells, washed them with PBS first, digested with 0.05% trypsin, pipeted the cells gently with DMEM containing 10% FBS to single cells and then counted them. Transferred Plasmid DNA into Huh7 cells using Fugene HD. Inoculated the transfected cells into a 96-well plate at a density of 10,000 cells per well, and the culture medium per well was 100 µL. Cultured the cells overnight in a 5% CO₂, 37°C incubator.

Prepared RNAiMAX transfection reagent, used the ratio of RNAiMAX:Opti-MEM =1.5:48.5 to add an appropriate volume as needed in a 15mL centrifuge tube, vortexed for 15 seconds, mixed well, and incubate at room temperature for 15 minutes. Added 60 µL RNAiMAX/Opti-MEM to each well at the corresponding positions, and took 60 µL of the compound diluted to the corresponding concentrations to add into the wells, mixed well and incubated for 15 minutes. Aspirated the medium in each well, and then added new medium containing the compound, each well now contained 120 µL of medium. After addition, placed in a 5% CO₂, 37°C incubator for incubation. The final concentrations of the compound tested were 5 nM, 0.5 nM, and 0.05 nM.

The experimental steps refer to the DUAL-GLO^{®} Luciferase Assay System manuals. The method is briefly described as follows:
Took the cells out of the incubator, aspirated and discarded the supernatant, added 75 µL fresh culture medium and 75 µL detection reagent, shaked in the dark for 10 mins. After the cells are fully lysed, transfered 100 µL sample to an Opaque white board to detect the Firefly luciferase signal. Added 50 µL Dual-Glo^{®} Stop & Glo^{®} detection reagent to each well, shaked in the dark for 10 mins, and detected the Renilla luciferase signal. Calculated the ratio of the signal of the main reporter gene to that of the internal reference reporter gene in each well, and calculated the inhibition rate % based on the ratio.

The test results were as follows:

| | Double strands code | Inhibition% | | |
|---|---|---|---|---|
| | | 5nM | 0.5nM | 0.05nM |
| | | MEAN±SD | | |
| the present application | Kylo-11-DS13 | 89.99±0.23 | 85.19±0.65 | 70.65±1.24 |
| D1 | Ky-11-DS12 | 80.46±2.66 | 76.47±2.88 | 50.10±4.36 |
| the present application | Kylo-11-DS11 | 85.69±1.16 | 80.19±0.85 | 55.25±2.39 |
| D1 | Ky-11-DS08 | 76.96±0.72 | 73.12±1.61 | 53.86±2.75 |

As shown in the above test results, the region of LPA mRNA NM_005577.4 (SEQ ID NO. 1) contained in the target sequence acted upon by the 2 pairs of original sequences for which protection is claimed in this application is different from that in D1, and is expanded by 2 positions forward or 1 position backward compared to the region in D1. The inhibition rates of Kylo-11-DS13 and Kylo-11-DS11 in the present application at concentrations of 5nM, 0.5nM and 0.05nM are respectively higher than those of Ky-11-DS12 and Ky-11-DS08 in D1.

## Claims

1. An RNA inhibitor or pharmaceutically acceptable salt thereof for inhibiting LPA gene expression, wherein:
the RNA inhibitor is formed by base pairing of a sense strand and an antisense strand with a chain length of 15-30, preferably 19-23, and at least 85% of the bases between the sense strand and the antisense strand are complementary;
the -OH at the 2' positions of part or all nucleotide sugar groups of the sense strand and/or the antisense strand can be substituted, the substituent group is fluorine or methoxy; and
the phosphate bonds among 3 adjacent nucleotides at at least one of the ends of the sense and/or antisense strand can be thiolated.

2. The RNA inhibitor or pharmaceutically acceptable salt thereof according to claim 1, wherein the antisense strand forms complementary regions with a target sequence, the target sequence is multiple regions at different positions of LPA mRNA, the multiple regions have at least 15 consecutive nucleotides that are identical, and the target sequence is selected from any one of the nucleotide regions among 312-332, 654-674, 996-1016, 1338-1358, 1680-1700, 2022-2042 and 2364-2384 in LPA mRNA (NM_005577.4).

3. The RNA inhibitor or pharmaceutically acceptable salt thereof according to claim 1, wherein the antisense strand forms complementary regions with a target sequence, the target sequence is multiple regions at different positions of LPA mRNA, the multiple regions have at least 15 consecutive nucleotides that are identical, and the target sequence is selected from any one of the nucleotide regions among 493-512, 1861-1880 and 2203-2222 in LPA mRNA (NM_005577.4).

4. The RNA inhibitor or pharmaceutically acceptable salt thereof according to any one of claims 1 to 3, wherein the antisense strand is selected from the following sequences:
5' ucguauaacaauaaggagcug 3' SEQ ID NO. 25
5' auaacucuguccauuaccaug 3' SEQ ID NO. 21
or is a sequence having at least 15 consecutive nucleotides identical to the above antisense strand, or is a sequence differing from the above antisense strand by 1, 2 or 3 nucleotides,
wherein, g = guanylate, a = adenylate, u = uridylate, c = cytidylate.

5. The RNA inhibitor or pharmaceutically acceptable salt thereof according to any one of claims 1 to 3, wherein the sense strand is selected from the following sequences:
| | |
|---|---|
| 5' cagcuccuuauuguuauacga 3' | SEQ ID NO. 11 |
| 5' ugguaauggacagaguuauca 3' | SEQ ID NO. 8 |
or is a sequence having at least 15 consecutive nucleotides identical to the above sense strand, or is a sequence differing from the above sense strand by 1, 2 or 3 nucleotides,
wherein, g = guanylate, a = adenylate, u = uridylate, c = cytidylate.

6. The RNA inhibitor or pharmaceutically acceptable salt thereof according to any one of claims 1 to 5, wherein the sense strand is SEQ ID NO. 11, or a sequence having at least 15 consecutive nucleotides identical to it, or a sequence differing from it by 1, 2 or 3 nucleotides; and the antisense strand is SEQ ID NO. 25, or a sequence having at least 15 consecutive nucleotides identical to it, or a sequence differing from it by 1, 2 or 3 nucleotides,
Sense strand: 5' cagcuccuuauuguuauacga 3' SEQ ID NO. 11
Antisense strand: 5' ucguauaacaauaaggagcug 3' SEQ ID NO. 25;
alternatively, the sense strand is SEQ ID NO. 8, or a sequence having at least 15 consecutive nucleotides identical to it, or a sequence differing from it by 1, 2 or 3 nucleotides; and the antisense strand is SEQ ID NO. 21, or a sequence having at least 15 consecutive nucleotides identical to it, or a sequence differing from it by 1, 2 or 3 nucleotides,
Sense strand: 5' ugguaauggacagaguuauca 3' SEQ ID NO. 8
Antisense strand: 5' auaacucuguccauuaccaug 3' SEQ ID NO. 21;
wherein, g = guanylate, a = adenylate, u = uridylate, c = cytidylate.

7. The RNA inhibitor or pharmaceutically acceptable salt thereof according to claim 6, wherein the sense strand is SEQ ID NO. 270, or a sequence differing from it by 1, 2 or 3 nucleotides; and the antisense strand is SEQ ID NO. 278, or a sequence differing from it by 1, 2 or 3 nucleotides,
Sense strand: 5' CsAsGCUCCUfUfAfUUGUUAUACsGsA 3' SEQ ID NO. 270
Antisense strand: 5' UsfCsGfUAfUAACAAfUAfAGfGAfGCsfUsG 3' SEQ ID NO. 278;
alternatively, the sense strand is SEQ ID NO. 239, or a sequence differing from it by 1, 2 or 3 nucleotides; and the antisense strand is SEQ ID NO. 344, or a sequence differing from it by 1, 2 or 3 nucleotides,
Sense strand: 5' UsGsGUfAAfUfGfGACAGAGUUAUsCsA 3' SEQ ID NO. 239
Antisense strand: 5' AsfUsAfACdTCfUGUCCAfUUfACCAsUsG 3' SEQ ID NO. 344;
wherein, G = 2'-O-methylguanylate, A = 2'-O-methyladenylate, U = 2'-O-methyluridylate, C = 2'-O-methyl cytidylate; Gs = 2'-O-methyl-3'-thioguanylate, As = 2'-O-methyl-3'-thioadenylate, Us = 2'-O-methyl-3'-thiouridylate, Cs = 2'-O-methyl-3'-thiocytidylate, fG = 2'-fluoroguanylate, fA = 2'-fluoroadenylate, fU = 2'-fluorouridylate, fC = 2'-fluorocytidylate; fGs = 2'-fluoro-3'-thioguanylate, fAs = 2'-fluoro-3'-thioadenylate, fUs = 2'-fluoro-3'-thiouridylate, fCs = 2'-fluoro-3'-thio cytidylate, dT = 2'-deoxythymidylate.

8. The RNA inhibitor or pharmaceutically acceptable salt thereof according to any one of claims 1 to 7, the RNA inhibitor further contains ligand structures 5'MVIP and 3'MVIP, and the structure of the RNA inhibitor is shown in Formula Ia, Ib or Ic: 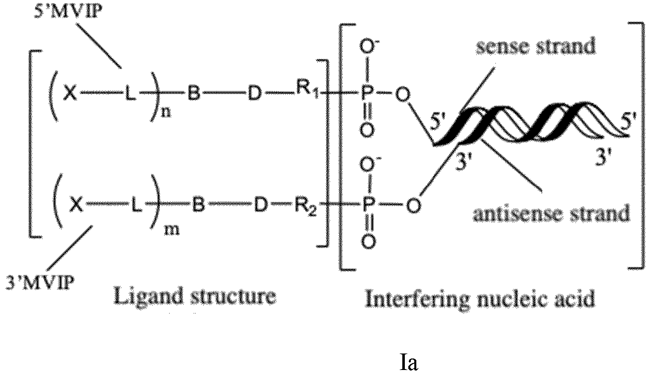 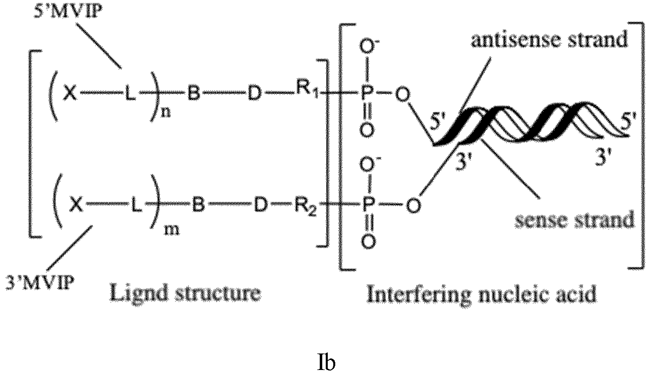 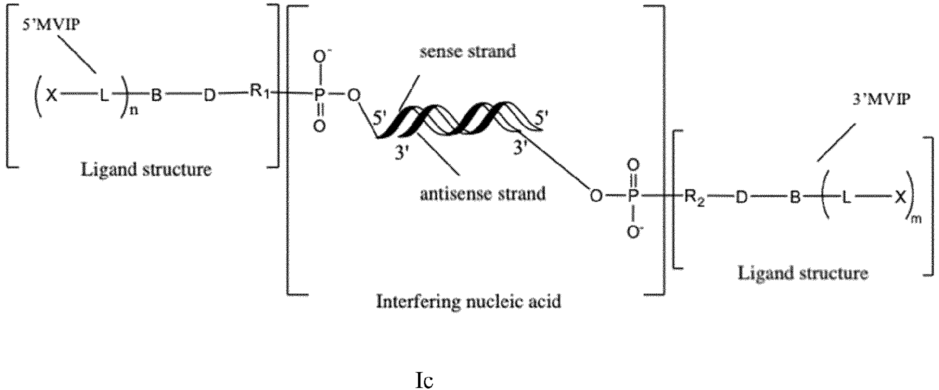 wherein,
the 5'MVIP is composed of a transition point R₁, a linking chain D, a linker B, a branched chain L and a liver targeting specific ligand X, which is connected to the 5' end of the sense strand or the 5' end of the antisense strand through the transition point R₁ and has the structure as shown in the general formula I:
(X-L)ₙ-B-D-R₁-, I
the 3'MVIP is composed of a transition point R₂, a linking chain D, a linker B, a branched chain L and a liver targeting specific ligand X, which is connected to the 3' end of the sense strand or the 3' end of the antisense strand through the transition point R₂ and has the structure as shown in the general formula II:
(X-L)ₘ-B-D-R₂-, II
wherein,
n and m are each independently any integer from 0 to 4, preferably each independently an integer of 1-3, and n+m is an integer of 2-6, preferably n+m=2, 3 or 4, more preferably 4;
the transition point R₁ is a heterocyclic or carbocyclic structure containing N, S or O as shown below: 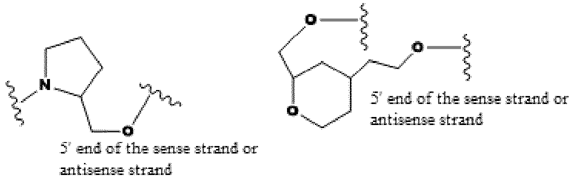 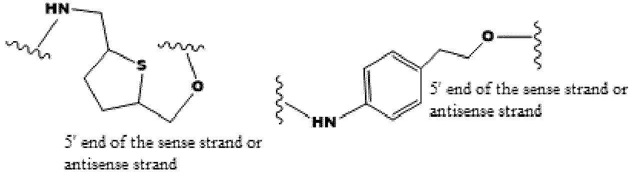
alternatively, the R₁ is -NH(CH₂)ₓCH₂O-, wherein x is any integer of 3-12, preferably any integer of 4-6,
the transition point R₂ is a heterocyclic or carbocyclic structure containing N, S or O as shown below: 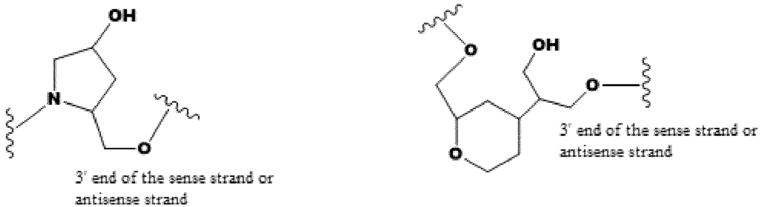 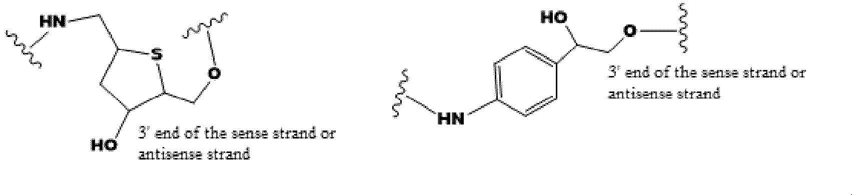
alternatively, the transition point R₂ is -NH(CH₂)ₓ₁CH(OH)(CH₂)ₓ₂CH₂O-, wherein x1 is any integer of 1-4, and x2 is any integer of 0-4,
the liver targeting specific ligand X is the same or different within each of 5'MVIP and 3'MVIP or between 5'MVIP and 3'MVIP, and is selected from monosaccharides and their derivatives, preferably N-acetylgalactosamine and its derivatives, and more preferably selected from the following structures: 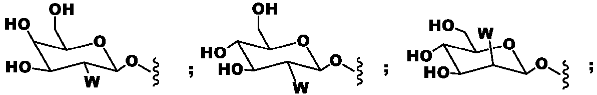 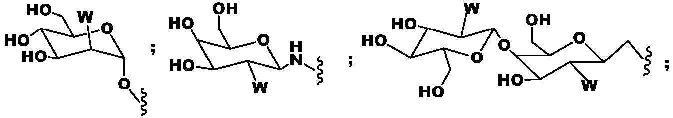 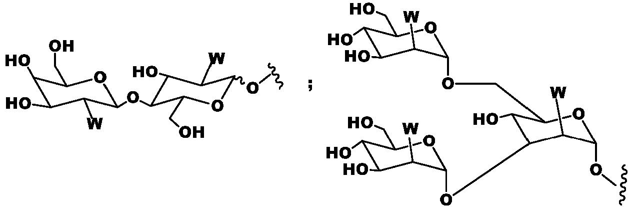 wherein W is selected from one or two of -OH, -NHCOOH or -NHCO(CH₂)_{q}CH₃, wherein q is an integer of 0-4,
the branched chain L is the same or different within each of 5'MVIP and 3'MVIP or between 5'MVIP and 3'MVIP, and is selected from one or more of the following structures: 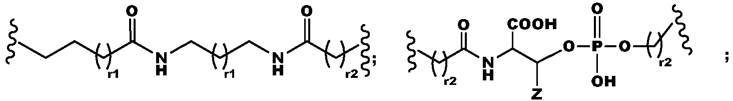 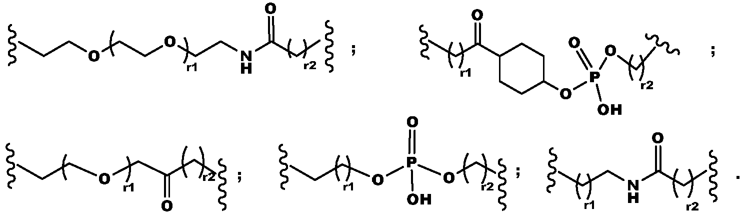 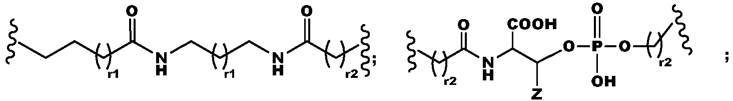 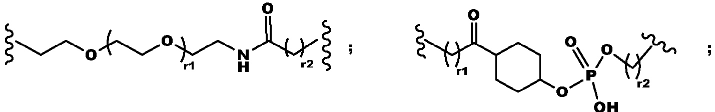 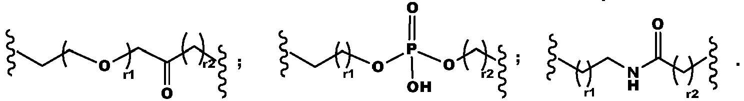 wherein r1 is any integer from 1 to 12, r2 is any integer from 0 to 20, and Z is H, alkyl or amido group, the alkyl group is such as C₁-C₅ alkyl,
the linker B is the same or different within each of 5'MVIP and 3'MVIP or between 5'MVIP and 3'MVIP, and is selected from the following structures: 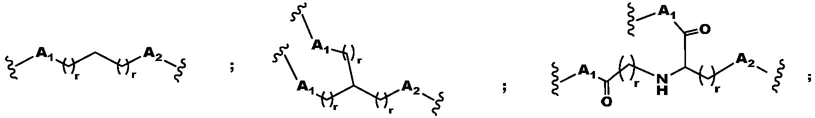 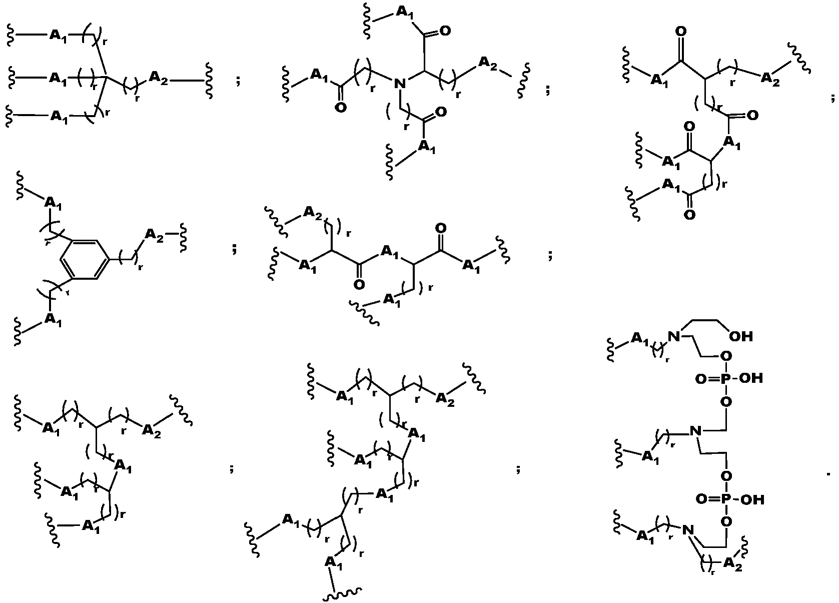 wherein A₁ and A₂ are each independently C, O, S, -NH-, carbonyl, amido, phosphoryl or thiophosphoryl, and r is any integer of 0-4.
the linking chain D is the same or different within each of 5'MVIP and 3'MVIP or between 5'MVIP and 3'MVIP, and is selected from the following structures: 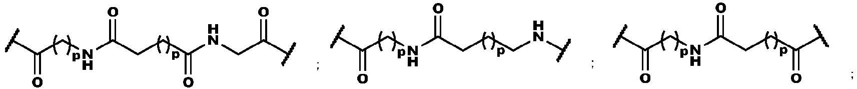 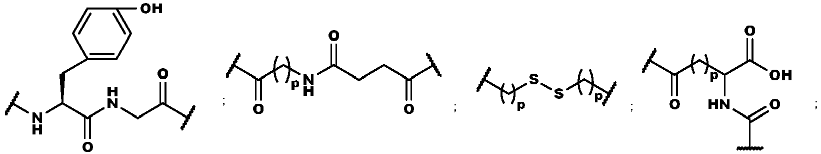 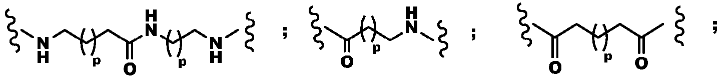 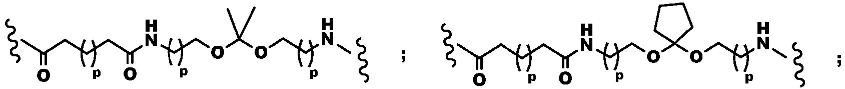 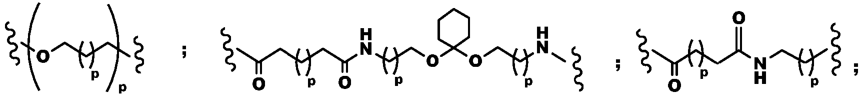 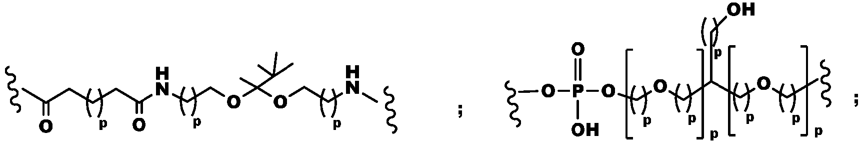 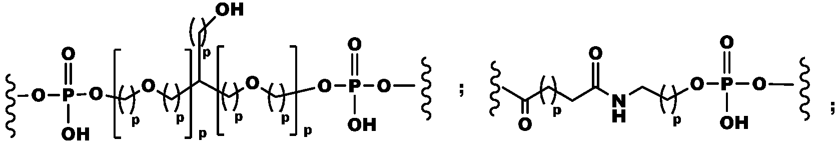 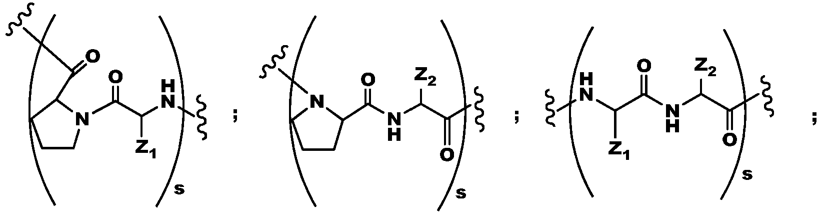 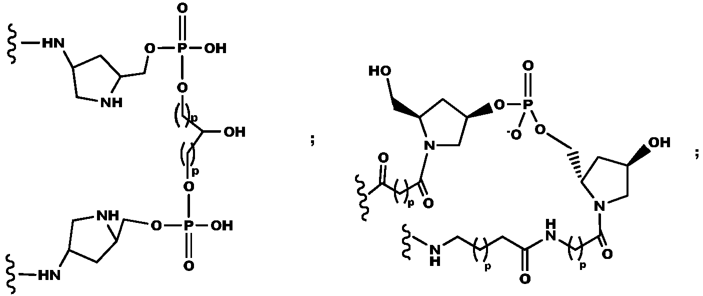 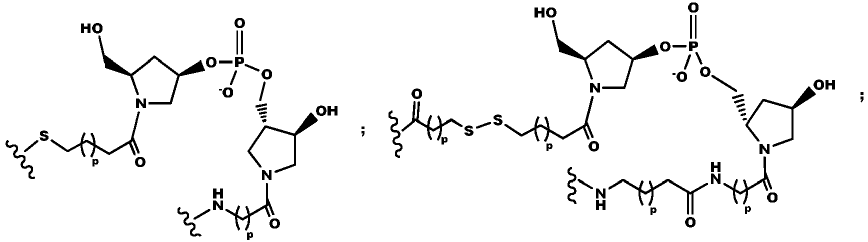 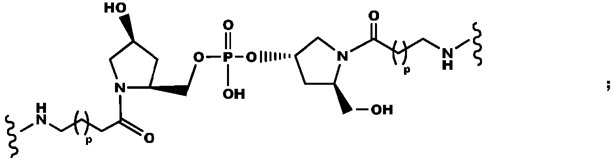 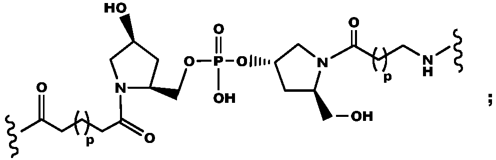 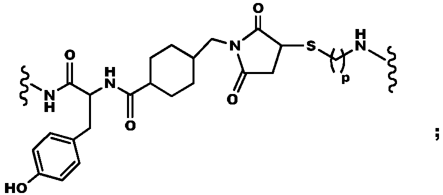 wherein, each p is independently any integer from 1 to 20; s is any integer of 2-13; Z₁ and Z₂ are the same or different substituents.

9. The RNA inhibitor or pharmaceutically acceptable salt thereof according to claim 8, wherein the 5'MVIP is 5'MVIP01 or 5'MVIP09 as shown below, and the 3'MVIP is 3'MVIP01, 3'MVIP09 or 3'MVIP17 as shown below:

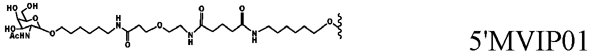

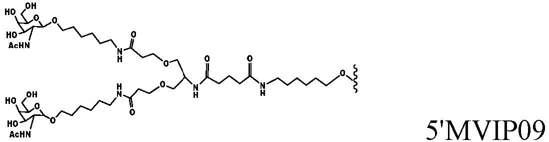

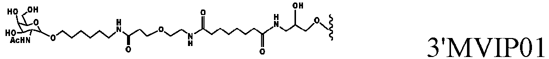

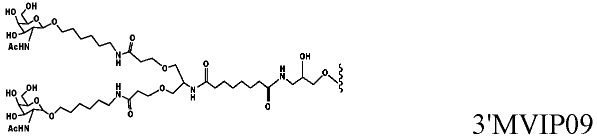

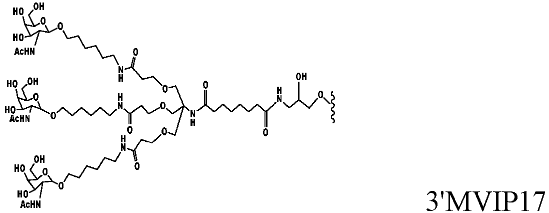

10. The RNA inhibitor or pharmaceutically acceptable salt thereof according to claim 9, wherein the combination of the sense strand 5'MVIP and the antisense strand 3'MVIP is 5'MVIP01/3'MVIP01, 5'MVIP01/3'MVIP17 or 5'MVIP09/3'MVIP09, or the combination of the sense strand 5'MVIP and the antisense strand 3'MVIP is 5'MVIP01/3'MVIP09 or 5'MVIP09/3'MVIP01.

11. The RNA inhibitor or pharmaceutically acceptable salt thereof according to claim 10, wherein the RNA inhibitor is selected from Kylo-11-DS146, Kylo-11-DS148 and Kylo-11-DS163.

12. Use of the RNA inhibitor or pharmaceutically acceptable salt thereof according to any one of claims 1-11 in the preparation of a medicament for treating and/or preventing diseases associated with elevated LP(a) level, wherein the diseases include but are not limited to inflammatory diseases, cardio-cerebrovascular diseases and metabolic diseases, wherein the cardio-cerebrovascular diseases include hyperlipoprotein(a)emia, hyperlipidemia, stroke, atherosclerosis, thrombosis, coronary heart disease and aortic valve stenosis

13. A pharmaceutical composition comprising the RNA inhibitor or pharmaceutically acceptable salt thereof according to any one of claims 1-11, and pharmaceutically acceptable adjuvants, wherein the dosage form thereof is oral administration, intravenous injection, or subcutaneous or intramuscular injection, preferably subcutaneous injection.
